# EUROPÄISCHE PATENTSCHRIFT

(11) **EP 4 200 282 B1**
(45) Veröffentlichungstag und Bekanntmachung des Hinweises auf die Patenterteilung: **11.09.2024**
(21) Anmeldenummer: 21766152.9
(22) Anmeldetag: 20.08.2021
(51) Int. Cl.: C07D 239/54, A01N 43/54

(54) **SUBSTITUIERTE N-PHENYLURACILE SOWIE DEREN SALZE UND IHRE VERWENDUNG ALS HERBIZIDE WIRKSTOFFE**
SUBSTITUTED N-PHENYLURACILS AS WELL AS THEIR SALTS AND THEIR USE AS HERBICIDES.
N-PHÉNYLURACILS SUBSTITUÉS, LEUR SELS ET LEUR USAGE AUTANT QU'HERBICIDES.

(30) Priorität: 24.08.2020 EP 20192356
(43) Veröffentlichungstag der Anmeldung: 28.06.2023
(73) Patentinhaber: Bayer Aktiengesellschaft, 51373 Leverkusen (DE)
(72) Erfinder: HEINEMANN, Ines, 65719 Hofheim (DE); JAKOBI, Harald, 60598 Frankfurt (DE); HELMKE, Hendrik, 65835 Liederbach (DE); FRACKENPOHL, Jens, 60439 Frankfurt (DE); ROSINGER, Christopher, Hugh, 65719 Hofheim (DE); GATZWEILER, Elmar, 61231 Bad Nauheim (DE); ASMUS, Elisabeth, 63768 Hösbach (DE); BOLLENBACH-WAHL, Birgit, 55413 Weiler/Bingen (DE)
(74) Vertreter: BIP Patents
(86) Internationale Anmeldenummer: PCT/EP2021/073129
(87) Internationale Veröffentlichungsnummer: WO 2022/043205

(56) Entgegenhaltungen:
- EP-A1- 1 397 958
- EP-A1- 1 470 753
- EP-A2- 1 106 607
- WO-A1-2011/137088
- JP-A- 2001 348 376
- US-A1- 2004 152 597

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen.

Speziell betrifft diese Erfindung substituierte N-Phenyluracile sowie deren Salze, Verfahren zu ihrer Herstellung und ihre Verwendung als Herbizide, insbesondere zur Bekämpfung von Unkräutern und/oder Ungräsern in Nutzpflanzenkulturen und/oder als Pflanzenwachstumsregulatoren zur Beeinflussung des Wachstums von Nutzpflanzenkulturen.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Die herbizide Wirkung dieser bekannten Verbindungen, insbesondere bei niedrigen Aufwandmengen, bzw. deren Verträglichkeit gegenüber Kulturpflanzen bleiben verbesserungswürdig.

Es ist aus verschiedenen Schriften bekannt, dass bestimmte substituierte N-verknüpfte Aryluracile als herbizide Wirkstoffe verwendet werden können (vgl. EP1106607, EP408382, EP473551, EP648749, US4943309, US5084084, US5127935, US6537948, JP2001/348376, JP2001/354661, JP2002/003480, JP2002/363010, JP2002/363170, WO91/00278, WO95/29168, WO95/30661, WO96/35679, WO97/01541, WO98/25909, WO 2001/034575, WO2001/39597, WO2001/85907, WO2002/098227, WO2002/098228, WO2003/028462, WO2003/028463, WO2003/0284647, WO2016/095768). Die bekannten Aryluracile weisen jedoch eine Reihe von Wirkungslücken, insbesondere gegenüber monokotylen Unkräutern auf. Eine Reihe von herbiziden Wirkstoffkombinationen auf Basis von N-verknüpften Aryluracilen sind ebenfalls bekannt geworden (vgl. DE4437197, EP714602, WO96/07323, WO96/08151, JP11189506, JP2003/104808, JP2003/104809, JP2003/104810, JP2003/160415 und JP2003/160416). Die Eigenschaften dieser Wirkstoffkombinationen waren jedoch auch nicht in allen Belangen zufriedenstellend.

Ebenfalls bekannt sind substituierte Uracile, die eine N-verknüpfte und weiter substituierte Diarylethergruppe oder einen entsprechenden Heteroarylaryletherrest enthalten (vgl. US6333296, US6121201, WO2001/85907, EP1122244, EP1397958, EP1422227, WO 2002/098227). Weiterhin sind hochsubstituierte N-Phenyluracile mit spezifisch substituierter Carbonylalkyloxygruppe beschrieben (vgl. WO2011/137088). In der WO2018/019842 wird die Verwendung von spezifisch substituierten N-Phenyluracilen zur Bekämpfung bestimmter dikotyler Unkräuter, die spezifische Resistenzen gegen etablierte Herbizide aufweisen, beschrieben. Substituierte 3-Phenyl-5-alkyl-6-(trifluormethyl)pyrimidin-2,4(1H,3H)-dione (vgl. WO2019/101551) sind ebenfalls bekannt.

Überraschenderweise wurde nun gefunden, dass ausgewählte N-Phenyluracile mit substituierter Alkylesterseitenkette oder deren Salze als Herbizide gut geeignet sind und besonders vorteilhaft als Wirkstoffe zur Bekämpfung von monokotylen und dikotylen Unkräutern in Nutzpflanzenkulturen eingesetzt werden können.

Ein Gegenstand der vorliegenden Erfindung sind damit substituierte N-Phenyluracile der allgemeinen Formel (I) oder deren Salze 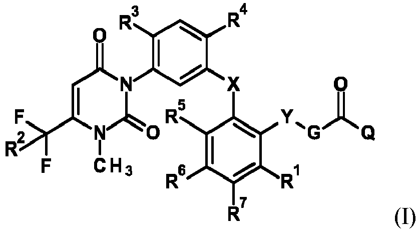 worin
- R¹: für Wasserstoff, Halogen, Cyano, (C₁-C₈)-Alkyl, (C₁-C₈)-Haloalkyl, (C₁-C₈)-Alkoxy, (C₁-C₈)-Haloalkoxy steht,
- R²: für Wasserstoff, Fluor, Chlor, Brom, Trifluormethyl, (C₁-C₈)-Alkoxy steht,
- R³: für Wasserstoff, Halogen, (C₁-C₈)-Alkoxy steht,
- R⁴: für Halogen, Cyano, NOz, C(O)NH₂, C(S)NH₂, (C₁-C₈)-Haloalkyl, (C₂-C₈)-Alkinyl steht,
- R⁵, R⁶ und R⁷: unabhängig voneinander für Wasserstoff, Halogen, Cyano, (C₁-C₈)-Alkyl, (C₁-C₈)-Haloalkyl, (C₁-C₈)-Alkoxy, (C₁-C₈)-Haloalkoxy stehen,
- G: für unverzweigtes oder verzweigtes (C₁-C₈)-Alkylen steht,
- Q: für einen Rest der Formel 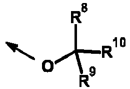 steht,
- R⁸: für Wasserstoff, (C₁-C₈)-Alkyl, (C₁-C₈)-Haloalkyl, Aryl, Aryl-(C₁-C₈)-alkyl, Heteroaryl, (C₂-C₈)-Alkinyl, (C₂-C₈)-Alkenyl, C(O)R¹³, C(O)OR¹³, (C₁-C₈)-Alkoxy-(C₁-C₈)-alkyl steht,
- R⁹: für Wasserstoff oder (C₁-C₈)-Alkyl steht,
- R¹⁰: für Cyano, NOz, Heteroaryl, Heteroaryl-(C₁-C₈)-alkyl, Heterocyclyl, Heterocyclyl-(C₁-C₈)-alkyl, R¹¹R¹²N-(C₁-C₈)-alkyl, R¹³O-(C₁-C₈)-alkyl, Cyano-(C₁-C₈)-alkyl, (C₁-C₈)-Alkylcarbonyloxy-(C₁-C₈)-alkyl, (C₃-C₈)-Cycloalkylcarbonyloxy-(C₁-C₈)-alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, OR¹³, NR¹¹R¹², SR¹⁴, S(O)R¹⁴, SO₂R¹⁴, R¹⁴S-(C₁-C₈)-alkyl, R¹⁴(O)S-(C₁-C₈)-alkyl, R¹⁴O₂S-(C₁-C₈)-alkyl, Tris-[(C₁-C₈)-Alkyl]silyl-(C₁-C₈)-alkyl, Bis-[(C₁-C₈)-Alkyl](aryl)silyl(C₁-C₈)-alkyl, [(C₁-C₈)-Alkyl]-bis-(aryl)silyl-(C₁-C₈)-alkyl, Tris-[(C₁-C₈)-Alkyl]silyl, Bis-hydroxyboryl-(C₁-C₈)-alkyl, Bis-[(C₁-C₈)-alkoxy]boryl-(C₁-C₈)-alkyl, Tetramethyl-1,3,2-Dioxaborolan-2-yl, Tetramethyl-1,3,2-Dioxaborolan-2-yl-(C₁-C₈)-alkyl, Nitro-(C₁-C₈)-alkyl, C(O)R¹⁴, Bis-(C₁-C₈)-alkoxymethyl, Bis-(C₁-C₈)-alkoxymethyl-(C₁-C₈)-alkyl steht, oder
- R⁸ und R¹⁰: mit dem Kohlenstoffatom, an das sie gebunden sind, ein vollständig gesättigtes oder teilgesättigtes, und gegebenenfalls weiter substituiertes 3 bis 10-gliedriges monocyclisches oder bicyclisches Heterocyclyl bilden,
- R¹¹ und R¹²: gleich oder verschieden sind und unabhängig voneinander für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, COR¹³, SO₂R¹⁴, Heterocyclyl, (C₁-C₈)-Alkoxycarbonyl, Bis-[(C₁-C₈)-alkyl]aminocarbonyl-(C₁-C₈)-alkyl, (C₁-C₈)-Alkyl-aminocarbonyl-(C₁-C₈)-alkyl, Aryl-(C₁-C₈)-alkyl-aminocarbonyl-(C₁-C₈)-alkyl, Aryl-(C₁-C₈)-alkoxycarbonyl, Heteroaryl-(C₁-C₈)-alkoxycarbonyl, (C₂-C₈)-Alkenyloxycarbonyl, (C₂-C₈)-Alkinyloxycarbonyl, Heterocyclyl-(C₁-C₈)-alkyl stehen, oder
- R¹¹ und R¹²: mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
- R¹³: für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkoxy-(C₁-C₈)-haloalkyl, (C₁-C₈)-Alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkoxy-(C₁-C₈)-alkyl, Aryl, Aryl-(C₁-C₈)-alkyl, Aryl-(C₁-C₈)-alkoxy-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, Bis-[(C₁-C₈)-alkyl]aminocarbonyl-(C₁-C₈)-alkyl, (C₁-C₈)-Alkyl-aminocarbonyl-(C₁-C₈)-alkyl, Aryl-(C₁-C₈)-alkyl-aminocarbonyl-(C₁-C₈)-alkyl, Bis-[(C₁-C₈)-alkyl]amino-(C₂-C₆)-alkyl, (C₁-C₈)-Alkyl-amino-(C₂-C₆)-alkyl, Aryl-(C₁-C₈)-alkyl-amino-(C₂-C₆)-alkyl, R¹⁴S-(C₁-C₈)-alkyl, R¹⁴(O)S-(C₁-C₈)-alkyl, R¹⁴O₂S-(C₁-C₈)-alkyl, Hydroxycarbonyl-(C₁-C₈)-alkyl, Heterocyclyl, Heterocyclyl-(C₁-C₈)-alkyl, Tris-[(C₁-C₈)-Alkyl]silyl-(C₁-C₈)-alkyl, Bis-[(C₁-C₈)-Alkyl](aryl)silyl(C₁-C₈)-alkyl, [(C₁-C₈)-Alkyl]-bis-(aryl)silyl-(C₁-C₈)-alkyl, (C₁-C₈)-Alkylcarbonyloxy-(C₁-C₈)-alkyl, (C₃-C₈)-Cycloalkylcarbonyloxy-(C₁-C₈)-alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, Aryloxy-(C₁-C₈)-alkyl, Heteroaryloxy-(C₁-C₈)-alkyl, (C₁-C₈)-Alkoxycarbonyl steht,
- R¹⁴: für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, Heterocyclyl-(C₁-C₈)-alkyl, (C₃-C₈)-Cycloalkyl-(C₁-C₈)-alkyl, (C₄-C₁₀)-Cycloalkenyl-(C₁-C₈)-alkyl, Bis-[(C₁-C₈)-alkyl]amino, (C₁-C₈)-Alkyl-amino, Aryl-(C₁-C₈)-amino, Aryl-(C₁-C₆)-alkyl-amino, Aryl-[(C₁-C₈)-alkyl]amino; (C₃-C₈)-Cycloalkyl-amino, (C₃-C₈)-Cycloalkyl-[(C₁-C₈)-alkyl]amino; N-Azetidinyl, N-Pyrrolidinyl, N-Piperidinyl. N-Morpholinyl, steht
und
X und Y unabhängig voneinander für O (Sauerstoff) oder S (Schwefel) stehen.

Besonders bevorzugter Erfindungsgegenstand sind Verbindungen der allgemeinen Formel (I), worin
- R¹: für Wasserstoff, Halogen, Cyano, (C₁-C₆)-Alkyl, (C₁-C₆)-Haloalkyl, (C₁-C₆)-Alkoxy, (C₁-C₆)-Haloalkoxy steht,
- R²: für Wasserstoff, Fluor, Chlor, Brom, Trifluormethyl, (C₁-C₆)-Alkoxy steht,
- R³: für Wasserstoff, Halogen, (C₁-C₆)-Alkoxy steht,
- R⁴: für Halogen, Cyano, NOz, C(O)NH₂, C(S)NH₂, (C₁-C₆)-Haloalkyl, (C₂-C₆)-Alkinyl steht,
- R⁵, R⁶ und R⁷: unabhängig voneinander für Wasserstoff, Halogen, Cyano, (C₁-C₆)-Alkyl, (C₁-C₆)-Haloalkyl, (C₁-C₆)-Alkoxy, (C₁-C₆)-Haloalkoxy stehen,
- G: für unverzweigtes oder verzweigtes (C₁-C₆)-Alkylen steht,
- Q: für einen Rest der Formel 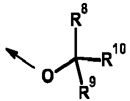 steht,
- R⁸: für Wasserstoff, (C₁-C₆)-Alkyl, (C₁-C₆)-Haloalkyl, Aryl, Aryl-(C₁-C₆)-alkyl, Heteroaryl, (C₂-C₆)-Alkinyl, (C₂-C₆)-Alkenyl, C(O)R¹³, C(O)OR¹³, (C₁-C₆)-Alkoxy-(C₁-C₆)-alkyl steht,
- R⁹: für Wasserstoff oder (C₁-C₄)-Alkyl steht,
- R¹⁰: für Cyano, NOz, Heteroaryl, Heteroaryl-(C₁-C₆)-alkyl, Heterocyclyl, Heterocyclyl-(C₁-C₆)-alkyl, R¹¹R¹²N-(C₁-C₆)-alkyl, R¹³O-(C₁-C₆)-alkyl, Cyano-(C₁-C₆)-alkyl, (C₁-C₆)-Alkylcarbonyloxy-(C₁-C₆)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₆)-alkyl, Heterocyclylcarbonyloxy-(C₁-C₆)-alkyl, OR¹³, NR¹¹R¹², SR¹⁴, S(O)R¹⁴, SO₂R¹⁴, R¹⁴S-(C₁-C₆)-alkyl, R¹⁴(O)S-(C₁-C₆)-alkyl, R¹⁴O₂S-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, Tris-[(C₁-C₆)-Alkyl]silyl, Bis-hydroxyboryl-(C₁-C₆)-alkyl, Bis-[(C₁-C₆)-alkoxy]boryl-(C₁-C₆)-alkyl, Tetramethyl-1,3,2-Dioxaborolan-2-yl, Tetramethyl-1,3,2-Dioxaborolan-2-yl-(C₁-C₆)-alkyl, Nitro-(C₁-C₆)-alkyl, , C(O)R¹³, , Bis-(C₁-C₆)-alkoxymethyl, Bis-(C₁-C₆)-alkoxymethyl-(C₁-C₆)-alkyl steht,
- R⁸ und R¹⁰: mit dem Kohlenstoffatom, an das sie gebunden sind, ein vollständig gesättigtes oder teilgesättigtes, und gegebenenfalls weiter substituiertes 3 bis 10-gliedriges monocyclisches oder bicyclisches Heterocyclyl bilden,
- R¹¹ und R¹²: gleich oder verschieden sind und unabhängig voneinander für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, COR¹³, SO₂R¹⁴, Heterocyclyl, (C₁-C₆)-Alkoxycarbonyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Aryl-(C₁-C₆)-alkoxycarbonyl, Heteroaryl-(C₁-C₆)-alkoxycarbonyl, (C₂-C₆)-Alkenyloxycarbonyl, (C₂-C₆)-Alkinyloxycarbonyl, Heterocyclyl-(C₁-C₆)-alkyl stehen, oder
- R¹¹ und R¹²: mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
- R¹³: für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkoxy-(C₁-C₆)-haloalkyl, (C₁-C₆)-Alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkoxy-(C₁-C₆)-alkyl, Aryl, Aryl-(C₁-C₆)-alkyl, Aryl-(C₁-C₆)-alkoxy-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Bis-[(C₁-C₆)-alkyl]amino-(C₂-C₄)-alkyl, (C₁-C₆)-Alkyl-amino-(C₂-C₄)-alkyl, Aryl-(C₁-C₆)-alkyl-amino-(C₂-C₄)-alkyl, R¹⁴S-(C₁-C₆)-alkyl, R¹⁴(O)S-(C₁-C₆)-alkyl, R¹⁴O₂S-(C₁-C₆)-alkyl, Hydroxycarbonyl-(C₁-C₆)-alkyl, Heterocyclyl, Heterocyclyl-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, (C₁-C₆)-Alkylcarbonyloxy-(C₁-C₆)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₆)-alkyl, Heterocyclylcarbonyloxy-(C₁-C₆)-alkyl, Aryloxy-(C₁-C₆)-alkyl, Heteroaryloxy-(C₁-C₆)-alkyl, (C₁-C₆)-Alkoxycarbonyl steht,
- R¹⁴: für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, Heterocyclyl-(C₁-C₆)-alkyl, (C₃-C₆)-Cycloalkyl-(C₁-C₆)-alkyl, (C₄-C₁₀)-Cycloalkenyl-(C₁-C₆)-alkyl, Bis-[(C₁-C₆)-alkyl]amino, (C₁-C₆)-Alkyl-amino, Aryl-(C₁-C₆)-amino, Aryl-(C₁-C₂)-alkyl-amino, Aryl-[(C₁-C₆)-alkyl]amino; (C₃-C₆)-Cycloalkyl-amino, (C₃-C₆)-Cycloalkyl-[(C₁-C₆)-alkyl]amino; N-Azetidinyl, N-Pyrrolidinyl, N-Piperidinyl. N-Morpholinyl, steht
und
X und Y unabhängig voneinander für O (Sauerstoff) oder S (Schwefel) stehen.

Ganz besonders bevorzugter Erfindungsgegenstand sind Verbindungen der allgemeinen Formel (I), worin
- R¹: für Wasserstoff, Fluor, Chlor, Brom, Iod, Cyano, Methyl, Ethyl, Prop-1-yl, 1-Methylethyl, But-1-yl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Trifluormethyl, Difluormethyl, Pentafluorethyl, 2,2-Difluorethyl, 2,2,2-Trifluorethyl, Methoxy, Ethoxy, Prop-1-yloxy, Prop-2-yloxy, But-1-yloxy, But-2-yloxy, 2-Methylprop-1-yloxy, 1,1-Dimethyleth-1-yloxy, Difluormethoxy, Trifluormethoxy, Pentafluorethoxy, 2,2-Difluorethoxy, 2,2,2-Trifluorethoxy steht,
- R²: für Wasserstoff, Fluor, Chlor, Brom, Trifluormethyl, Methoxy, Ethoxy, Prop-1-yloxy, But-1-yloxy steht,
- R³: für Wasserstoff, Fluor, Chlor, Brom, Methoxy, Ethoxy, Prop-1-yloxy, Prop-2-yloxy, But-1-yloxy, But-2-yloxy, 2-Methylprop-1-yloxy, 1,1-Dimethyleth-1-yloxy steht,
- R⁴: für Fluor, Chlor, Brom, Cyano, NOz, C(O)NH₂, C(S)NH₂, Trifluormethyl, Difluormethyl, Pentafluorethyl, Ethinyl, Propin-1-yl, 1-Butin-1-yl, Pentin-1-yl, Hexin-1-yl steht,
- R⁵, R⁶ und R⁷: unabhängig voneinander für Wasserstoff, Fluor, Chlor, Brom, Iod, Cyano, Methyl, Ethyl, Prop-1-yl, 1-Methylethyl, But-1-yl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Trifluormethyl, Difluormethyl, Pentafluorethyl, 2,2-Difluorethyl, 2,2,2-Trifluorethyl, Methoxy, Ethoxy, Prop-1-yloxy, Prop-2-yloxy, But-1-yloxy, But-2-yloxy, 2-Methylprop-1-yloxy, 1,1-Dimethyleth-1-yloxy, Difluormethoxy, Trifluormethoxy, Pentafluorethoxy, 2,2-Difluorethoxy, 2,2,2-Trifluorethoxy stehen,
- G: für Methylen, (Methyl)methylen, (Ethyl)methylen, (Prop-1-yl)methylen, (Prop-2-yl)methylen, (But-1-yl)methylen, (But-2-yl)methylen, (Pent-1-yl)methylen, (Pent-2-yl)methylen, (Pent-3-yl)methylen, (Dimethyl)methylen, (Diethyl)methylen, Ethylen, n-Propylen, (1-Methyl)ethyl-1-en, (2-Methyl)ethyl-1-en, n-Butylen, 1-Methylpropyl-1-en, 2-Methylpropyl-1-en, 3-Methylpropyl-1-en, 1,1-Dimethylethyl-1-en, 2,2-Dimethylethyl-1-en, 1-Ethylethyl-1-en, 2-Ethylethyl-1-en, 1-(Prop-1-yl)ethyl-1-en, 2-(Prop-1-yl)ethyl-1-en, 1-(Prop-2-yl)ethyl-1-en, 2-(Prop-2-yl)ethyl-1-en, 1,1,2-Trimethylethyl-1-en, 1,2,2-Trimethylethyl-1-en, 1,1,2,2-Tetramethylethyl-1-en, n-Pentylen, 1-Methylbutyl-1-en, 2-Methylbutyl-1-en, 3-Methylbutyl-1-en, 4-Methylbutyl-1-en, 1,1-Dimethylpropyl-1-en, 2,2-Dimethylpropyl-1-en, 3,3-
- X und Y: Dimethylpropyl-1-en, 1,2-Dimethylpropyl-1-en, 1,3-Dimethylpropyl-1-en, 1-Ethylpropyl-1-en, n-Hexylen, 1-Methylpentyl-1-en, 2-Methylpentyl-1-en, 3-Methylpentyl-1-en, 4-Methylpentyl-1-en, 1,1-Dimethylbutyl-1-en, 1,2-Dimethylbutyl-1-en, 1,3-Di-methylbutyl-1-en, 2,2-Dimethylbutyl-1-en, 2,3-Dimethylbutyl-1-en, 3,3-Dimethylbutyl-1-en, 1-Ethylbutyl-1-en, 2-Ethylbutyl-1-en, 1,1,2-Trimethylpropyl-1-en, 1,2,2-Trimethylpropyl-1-en, 1-Ethyl-1-methylpropyl-1-en, 1-Ethyl-2-methylpropyl-1-en steht, unabhängig voneinander für O (Sauerstoff) oder S (Schwefel) stehen
- und Q: für eine der nachfolgend spezifisch genannten Gruppierungen Q-1 bis Q-406 steht, wobei in den Strukturformeln der nachfolgenden Tabelle der Pfeil für eine Bindung der jeweiligen Gruppe Q zur Carbonylgruppe in der allgemeinen Formel (I) steht:

| | | | | |
|---|---|---|---|---|
| 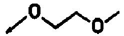 | 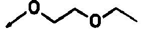 | 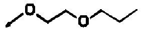 | 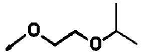 | 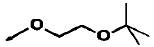 |
| Q-1 | Q-2 | Q-3 | Q-4 | Q-5 |

| | | | | |
|---|---|---|---|---|
| 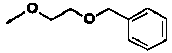 | 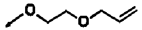 | 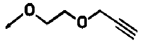 | 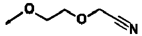 | 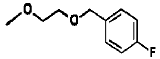 |
| Q-6 | Q-7 | Q-8 | Q-9 | Q-10 |

| | | | | |
|---|---|---|---|---|
| 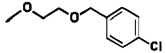 | 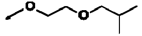 | 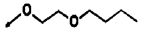 | 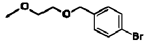 | 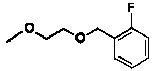 |
| Q-11 | Q-12 | Q-13 | Q-14 | Q-15 |

| | | | | |
|---|---|---|---|---|
| 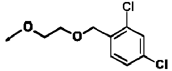 | 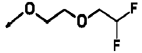 | 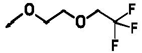 | 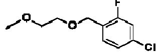 | 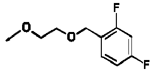 |
| Q-16 | Q-17 | Q-18 | Q-19 | Q-20 |

| | | | | |
|---|---|---|---|---|
| 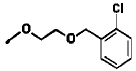 | 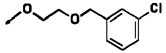 | 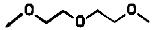 | 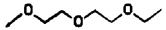 | 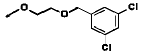 |
| Q-21 | Q-22 | Q-23 | Q-24 | Q-25 |

| | | | | |
|---|---|---|---|---|
| 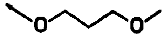 | 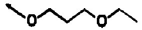 | 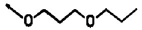 | 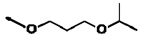 | 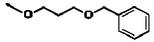 |
| Q-26 | Q-27 | Q-28 | Q-29 | Q-30 |

| | | | | |
|---|---|---|---|---|
| 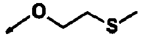 | 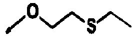 | 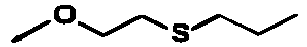 | 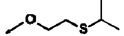 | 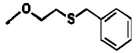 |
| Q-31 | Q-32 | Q-33 | Q-34 | Q-35 |

| | | | | |
|---|---|---|---|---|
| 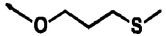 | 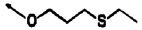 | 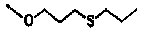 | 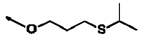 | 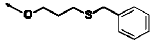 |
| Q-36 | Q-37 | Q-38 | Q-39 | Q-40 |

| | | | | |
|---|---|---|---|---|
| 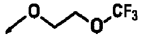 | 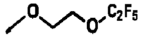 | 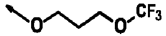 | 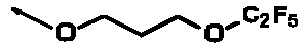 | 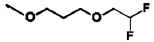 |
| Q-41 | Q-42 | Q-43 | Q-44 | Q-45 |

| | | | | |
|---|---|---|---|---|
| 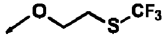 | 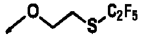 | 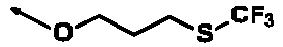 | 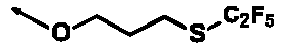 | 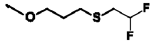 |
| Q-46 | Q-47 | Q-48 | Q-49 | Q-50 |

| | | | | |
|---|---|---|---|---|
| 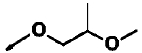 | 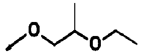 | 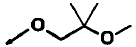 | 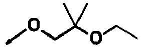 | 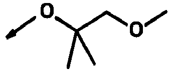 |
| Q-51 | Q-52 | Q-53 | Q-54 | Q-55 |

| | | | | |
|---|---|---|---|---|
| 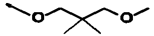 | 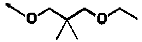 | 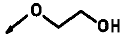 | 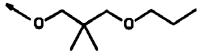 | 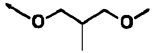 |
| Q-56 | Q-57 | Q-58 | Q-59 | Q-60 |

| | | | | |
|---|---|---|---|---|
| 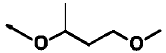 | 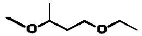 | 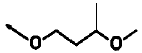 | 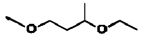 | 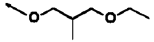 |
| Q-61 | Q-62 | Q-63 | Q-64 | Q-65 |

| | | | | |
|---|---|---|---|---|
| 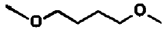 | 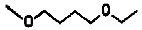 | 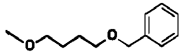 | 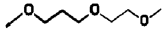 | 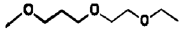 |
| Q-66 | Q-67 | Q-68 | Q-69 | Q-70 |

| | | | | |
|---|---|---|---|---|
| 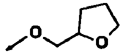 | 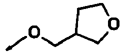 | 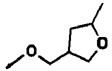 | 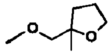 | 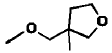 |
| Q-71 | Q-72 | Q-73 | Q-74 | Q-75 |

| | | | | |
|---|---|---|---|---|
| 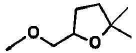 | 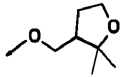 | 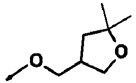 | 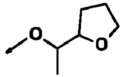 | 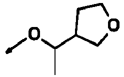 |
| Q-76 | Q-77 | Q-78 | Q-79 | Q-80 |

| | | | | |
|---|---|---|---|---|
| 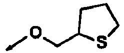 | 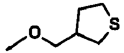 | 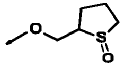 | 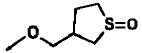 | 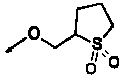 |
| Q-81 | Q-82 | Q-83 | Q-84 | Q-85 |

| | | | | |
|---|---|---|---|---|
| 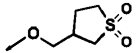 | 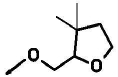 | 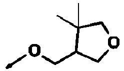 | 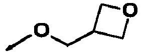 | 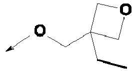 |
| Q-86 | Q-87 | Q-88 | Q-89 | Q-90 |

| | | | | |
|---|---|---|---|---|
| 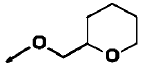 | 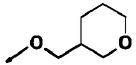 | 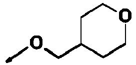 | 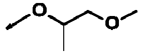 | 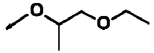 |
| Q-91 | Q-92 | Q-93 | Q-94 | Q-95 |

| | | | | |
|---|---|---|---|---|
| 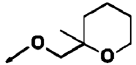 | 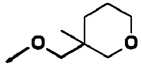 | 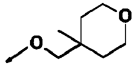 | 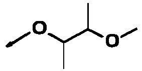 | 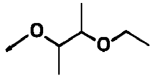 |
| Q-96 | Q-97 | Q-98 | Q-99 | Q-100 |

| | | | | |
|---|---|---|---|---|
| 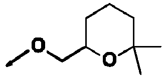 | 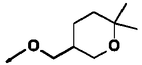 | 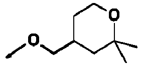 | 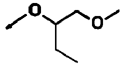 | 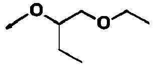 |
| Q-101 | Q-102 | Q-103 | Q-104 | Q-105 |

| | | | | |
|---|---|---|---|---|
| 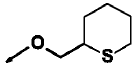 | 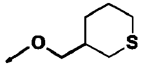 | 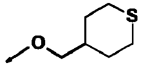 | 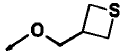 | 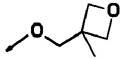 |
| Q-106 | Q-107 | Q-108 | Q-109 | Q-110 |

| | | | | |
|---|---|---|---|---|
| 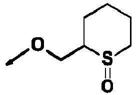 | 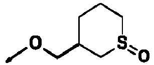 | 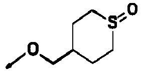 | 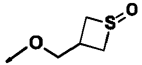 | 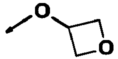 |
| Q-111 | Q-112 | Q-113 | Q-114 | Q-115 |

| | | | | |
|---|---|---|---|---|
| 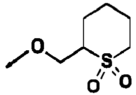 | 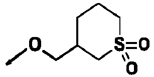 | 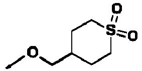 | 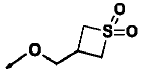 | 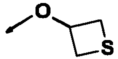 |
| Q-116 | Q-117 | Q-118 | Q-119 | Q-120 |

| | | | | |
|---|---|---|---|---|
| 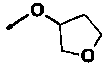 | 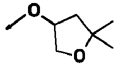 | 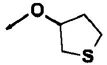 | 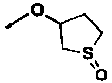 | 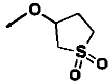 |
| Q-121 | Q-122 | Q-123 | Q-124 | Q-125 |

| | | | | |
|---|---|---|---|---|
| 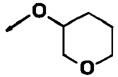 | 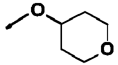 | 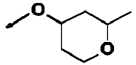 | 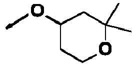 | 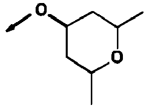 |
| Q-126 | Q-127 | Q-128 | Q-129 | Q-130 |

| | | | | |
|---|---|---|---|---|
| 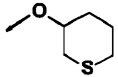 | 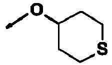 | 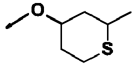 | 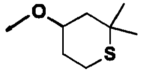 | 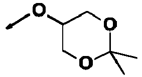 |
| Q-131 | Q-132 | Q-133 | Q-134 | Q-135 |

| | | | | |
|---|---|---|---|---|
| 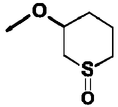 | 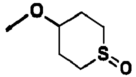 | 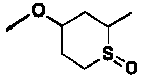 | 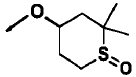 | 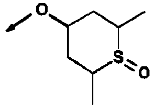 |
| Q-136 | Q-137 | Q-138 | Q-139 | Q-140 |

| | | | | |
|---|---|---|---|---|
| 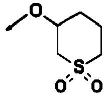 | 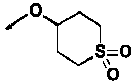 | 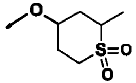 | 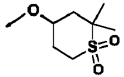 | 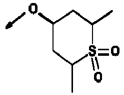 |
| Q-141 | Q-142 | Q-143 | Q-144 | Q-145 |

| | | | | |
|---|---|---|---|---|
| 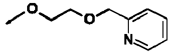 | 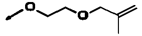 | 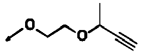 | 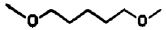 | 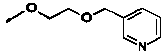 |
| Q-146 | Q-147 | Q-148 | Q-149 | Q-150 |

| | | | | |
|---|---|---|---|---|
| 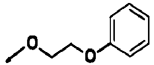 | 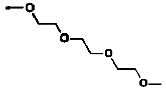 | 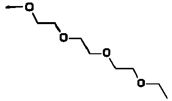 | 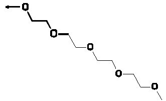 | 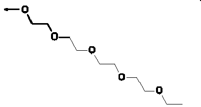 |
| Q-151 | Q-152 | Q-153 | Q-154 | Q-155 |

| | | | | |
|---|---|---|---|---|
| 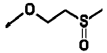 | 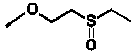 | 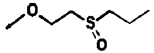 | 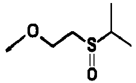 | 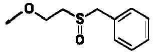 |
| Q-156 | Q-157 | Q-158 | Q-159 | Q-160 |

| | | | | |
|---|---|---|---|---|
| 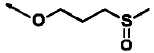 | 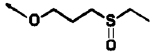 | 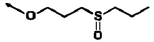 | 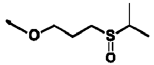 | 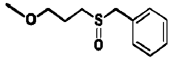 |
| Q-161 | Q-162 | Q-163 | Q-164 | Q-165 |

| | | | | |
|---|---|---|---|---|
| 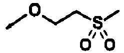 | 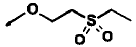 | 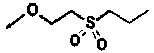 | 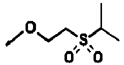 | 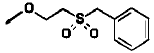 |
| Q-166 | Q-167 | Q-168 | Q-169 | Q-170 |

| | | | | |
|---|---|---|---|---|
| 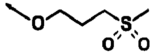 | 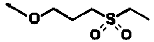 | 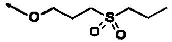 | 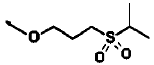 | 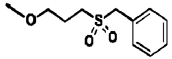 |
| Q-171 | Q-172 | Q-173 | Q-174 | Q-175 |

| | | | | |
|---|---|---|---|---|
| 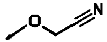 | 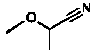 | 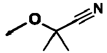 | 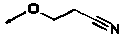 | 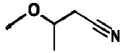 |
| Q-176 | Q-177 | Q-178 | Q-179 | Q-180 |

| | | | | |
|---|---|---|---|---|
| 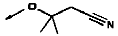 | 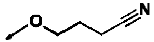 | 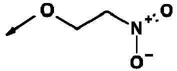 | 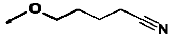 | 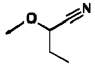 |
| Q-181 | Q-182 | Q-183 | Q-184 | Q-185 |

| | | | | |
|---|---|---|---|---|
| 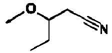 | 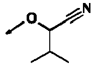 | 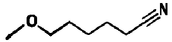 | 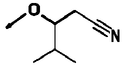 | 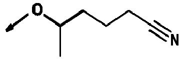 |
| 186 | 187 | 188 | 189 | 190 |

| | | | | |
|---|---|---|---|---|
| 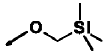 | 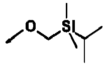 | 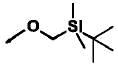 | 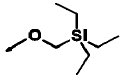 | 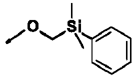 |
| Q-191 | Q-192 | Q-193 | Q-194 | Q-195 |

| | | | | |
|---|---|---|---|---|
| 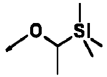 | 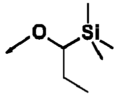 | 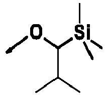 | 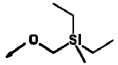 | 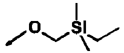 |
| Q-196 | Q-197 | Q-198 | Q-199 | Q-200 |

| | | | | |
|---|---|---|---|---|
| 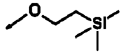 | 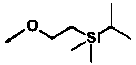 | 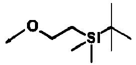 | 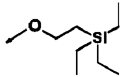 | 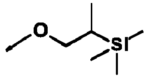 |
| Q-201 | Q-202 | Q-203 | Q-204 | Q-205 |

| | | | | |
|---|---|---|---|---|
| 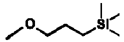 | 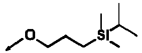 | 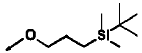 | 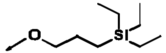 | 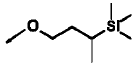 |
| Q-206 | Q-207 | Q-208 | Q-209 | Q-210 |

| | | | | |
|---|---|---|---|---|
| 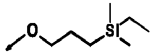 | 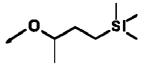 | 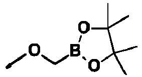 | 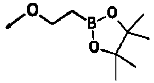 | 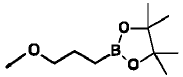 |
| Q-211 | Q-212 | Q-213 | Q-214 | Q-215 |

| | | | | |
|---|---|---|---|---|
| 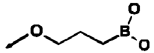 | 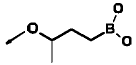 | 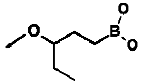 | 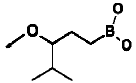 | 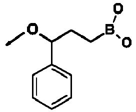 |
| Q-216 | Q-217 | Q-218 | Q-219 | Q-220 |

| | | | | |
|---|---|---|---|---|
| 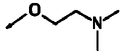 | 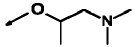 | 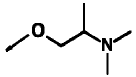 | 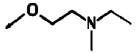 | 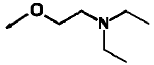 |
| Q-221 | Q-222 | Q-223 | Q-224 | Q-225 |

| | | | | |
|---|---|---|---|---|
| 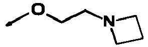 | 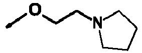 | 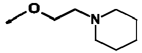 | 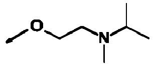 | 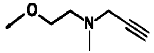 |
| Q-226 | Q-227 | Q-228 | Q-229 | Q-230 |

| | | | | |
|---|---|---|---|---|
| 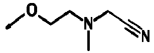 | 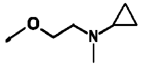 | 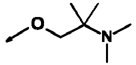 | 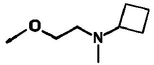 | 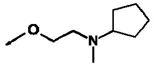 |
| Q-231 | Q-232 | Q-233 | Q-234 | Q-235 |

| | | | | |
|---|---|---|---|---|
| 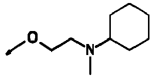 | 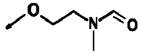 | 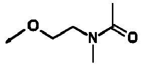 | 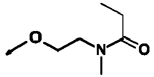 | 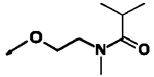 |
| Q-236 | Q-237 | Q-238 | Q-239 | Q-240 |

| | | | | |
|---|---|---|---|---|
| 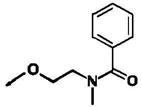 | 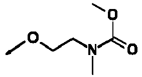 | 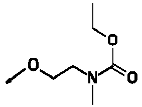 | 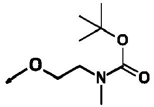 | 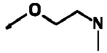 |
| Q-241 | Q-242 | Q-243 | Q-244 | Q-245 |

| | | | | |
|---|---|---|---|---|
| 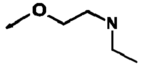 | 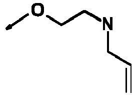 | 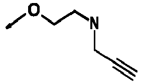 | 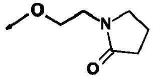 | 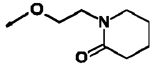 |
| Q-246 | Q-247 | Q-248 | Q-249 | Q-250 |

| | | | | |
|---|---|---|---|---|
| 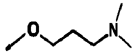 | 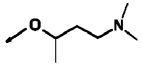 | 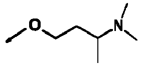 | 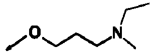 | 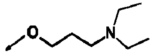 |
| Q-251 | Q-252 | Q-253 | Q-254 | Q-255 |

| | | | | |
|---|---|---|---|---|
| 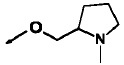 | 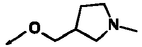 | 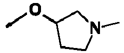 | 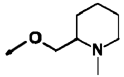 | 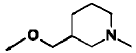 |
| Q-256 | Q-257 | Q-258 | Q-259 | Q-260 |

| | | | | |
|---|---|---|---|---|
| 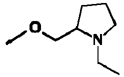 | 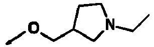 | 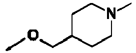 | 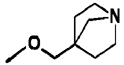 | 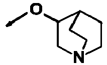 |
| Q-261 | Q-262 | Q-263 | Q-264 | Q-265 |

| | | | | |
|---|---|---|---|---|
| 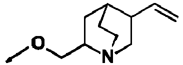 | 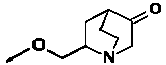 | 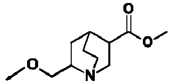 | 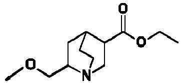 | 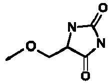 |
| Q-266 | Q-267 | Q-268 | Q-269 | Q-270 |

| | | | | |
|---|---|---|---|---|
| 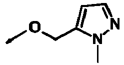 | 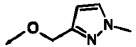 | 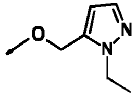 | 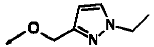 | 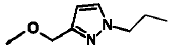 |
| Q-271 | Q-272 | Q-273 | Q-274 | Q-275 |

| | | | | |
|---|---|---|---|---|
| 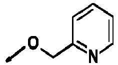 | 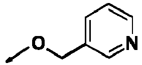 | 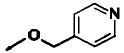 | 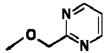 | 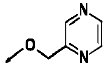 |
| Q-276 | Q-277 | Q-278 | Q-279 | Q-280 |

| | | | | |
|---|---|---|---|---|
| 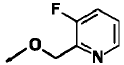 | 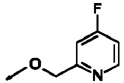 | 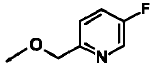 | 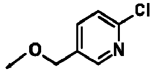 | 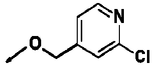 |
| Q-281 | Q-282 | Q-283 | Q-284 | Q-285 |

| | | | | |
|---|---|---|---|---|
| 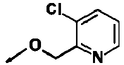 | 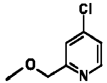 | 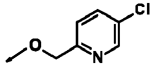 | 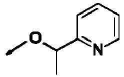 | 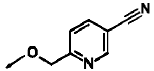 |
| Q-286 | Q-287 | Q-288 | Q-289 | Q-290 |

| | | | | |
|---|---|---|---|---|
| 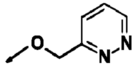 | 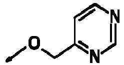 | 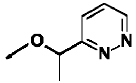 | 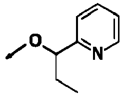 | 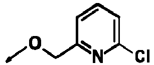 |
| Q-291 | Q-292 | Q-293 | Q-294 | Q-295 |

| | | | | |
|---|---|---|---|---|
| 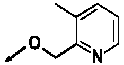 | 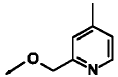 | 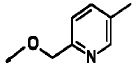 | 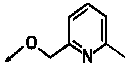 | 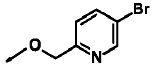 |
| Q-296 | Q-297 | Q-298 | Q-299 | Q-300 |

| | | | | |
|---|---|---|---|---|
| 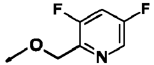 | 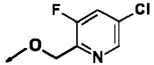 | 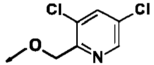 | 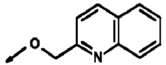 | 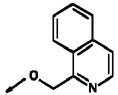 |
| Q-301 | Q-302 | Q-303 | Q-304 | Q-305 |

| | | | | |
|---|---|---|---|---|
| 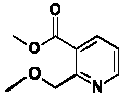 | 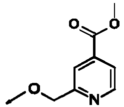 | 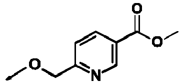 | 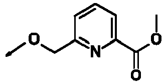 | 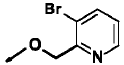 |
| Q-306 | Q-307 | Q-308 | Q-309 | Q-310 |

| | | | | |
|---|---|---|---|---|
| 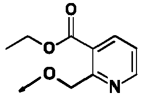 | 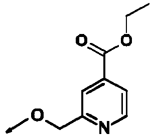 | 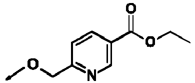 | 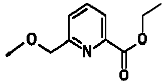 | 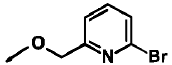 |
| Q-311 | Q-312 | Q-313 | Q-314 | Q-315 |

| | | | | |
|---|---|---|---|---|
| 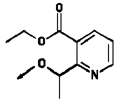 | 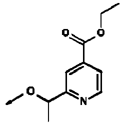 | 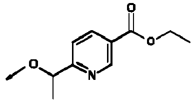 | 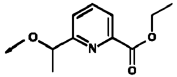 | 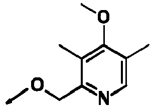 |
| Q-316 | Q-317 | Q-318 | Q-319 | Q-320 |

| | | | | |
|---|---|---|---|---|
| 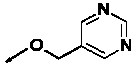 | 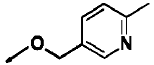 | 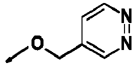 | 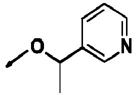 | 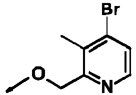 |
| Q-321 | Q-322 | Q-323 | Q-324 | Q-325 |

| | | | | |
|---|---|---|---|---|
| 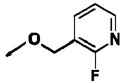 | 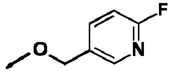 | 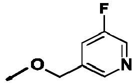 | 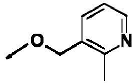 | 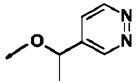 |
| Q-326 | Q-327 | Q-328 | Q-329 | Q-330 |

| | | | | |
|---|---|---|---|---|
| 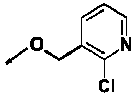 | 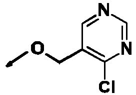 | 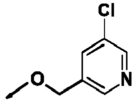 | 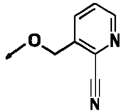 | 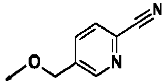 |
| Q-331 | Q-332 | Q-333 | Q-334 | Q-335 |

| | | | | |
|---|---|---|---|---|
| 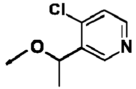 | 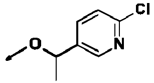 | 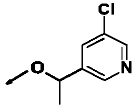 | 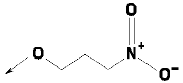 | 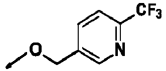 |
| Q-336 | Q-337 | Q-338 | Q-339 | Q-340 |

| | | | | |
|---|---|---|---|---|
| 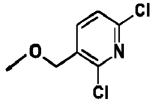 | 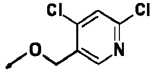 | 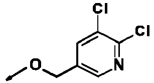 | 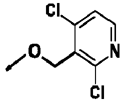 | 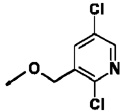 |
| Q-341 | Q-342 | Q-343 | Q-344 | Q-345 |

| | | | | |
|---|---|---|---|---|
| 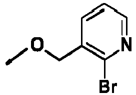 | 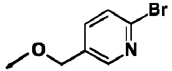 | 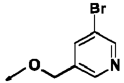 | 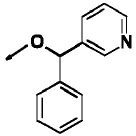 | 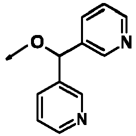 |
| Q-346 | Q-347 | Q-348 | Q-349 | Q-350 |

| | | | | |
|---|---|---|---|---|
| 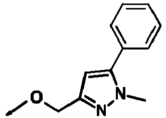 | 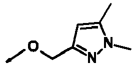 | 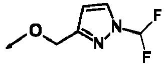 | 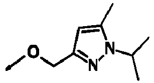 | 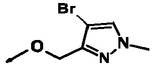 |
| Q-351 | Q-352 | Q-353 | Q-354 | Q-355 |

| | | | | |
|---|---|---|---|---|
| 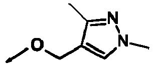 | 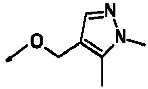 | 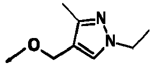 | 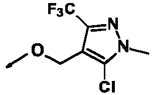 | 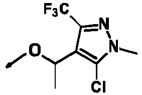 |
| Q-356 | Q-357 | Q-358 | Q-359 | Q-360 |

| | | | | |
|---|---|---|---|---|
| 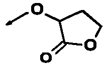 | 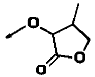 | 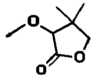 | 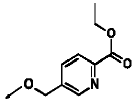 | 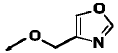 |
| Q-361 | Q-362 | Q-363 | Q-364 | Q-365 |

| | | | | |
|---|---|---|---|---|
| 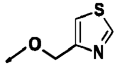 | 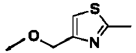 | 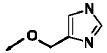 | 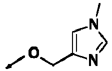 | 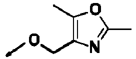 |
| Q-366 | Q-367 | Q-368 | Q-369 | Q-370 |

| | | | | |
|---|---|---|---|---|
| 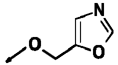 | 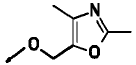 | 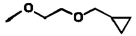 | 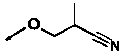 | 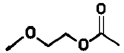 |
| Q-371 | Q-372 | Q-373 | Q-374 | Q-375 |

| | | | | |
|---|---|---|---|---|
| 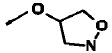 | 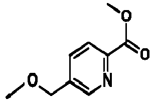 | 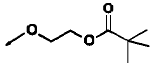 | 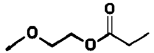 | 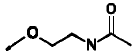 |
| Q-376 | Q-377 | Q-378 | Q-379 | Q-380 |

| | | | | |
|---|---|---|---|---|
| 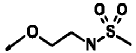 | 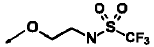 | 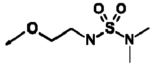 | 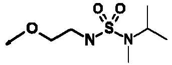 | 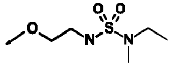 |
| Q-381 | Q-382 | Q-383 | Q-384 | Q-385 |

| | | | | |
|---|---|---|---|---|
| 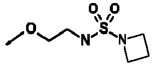 | 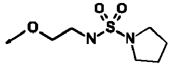 | 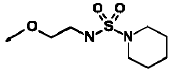 | 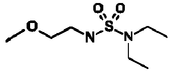 | 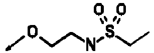 |
| Q-386 | Q-387 | Q-388 | Q-389 | Q-390 |

| | | | | |
|---|---|---|---|---|
| 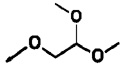 | 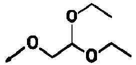 | 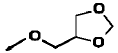 | 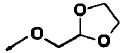 | 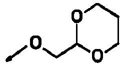 |
| Q-391 | Q-392 | Q-393 | Q-394 | Q-395 |

| | | | | |
|---|---|---|---|---|
| 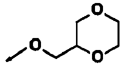 | 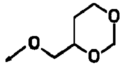 | 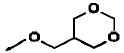 | 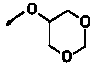 | 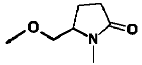 |
| Q-396 | Q-397 | Q-398 | Q-399 | Q-400 |

| | | | | |
|---|---|---|---|---|
| 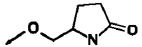 | 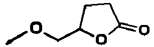 | 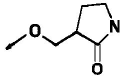 | 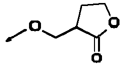 | 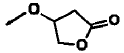 |
| Q-401 | Q-402 | Q-403 | Q-404 | Q-405 |

| |
|---|
| 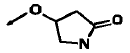 |
| Q-406 |

Im Speziellen bevorzugter Erfindungsgegenstand sind Verbindungen der allgemeinen Formel (I), worin
- R¹: für Wasserstoff, Fluor, Chlor, Brom, Iod, Cyano, Methyl, Ethyl, Trifluormethyl, Difluormethyl, Methoxy, Ethoxy, Difluormethoxy, Trifluormethoxy steht,
- R²: für Fluor steht,
- R³: für Wasserstoff, Fluor, Chlor, Brom, Methoxy steht,
- R⁴: für Fluor, Chlor, Brom, Cyano, NOz, C(O)NH₂, C(S)NH₂, Trifluormethyl, Ethinyl, Propin-1-yl steht,
- R⁵, R⁶ und R⁷: unabhängig voneinander für Wasserstoff, Fluor, Chlor, Brom, Iod, Cyano, Methyl, Ethyl, Trifluormethyl, Difluormethyl, Methoxy, Ethoxy, Difluormethoxy, Trifluormethoxy stehen,
- G: für Methylen, (Methyl)methylen, (Ethyl)methylen, (Dimethyl)methylen, Ethylen, n-Propylen, (1-Methyl)ethyl-1-en, (2-Methyl)ethyl-1-en, n-Butylen, 1-Methylpropyl-1-en, 2-Methylpropyl-1-en, 3-Methylpropyl-1-en, 1,1-Dimethylethyl-1-en, 2,2-Dimethylethyl-1-en, 1-Ethylethyl-1-en, 2-Ethylethyl-1-en, 1-(Prop-1-yl)ethyl-1-en, 2-(Prop-1-yl)ethyl-1-en, 1-(Prop-2-yl)ethyl-1-en, 2-(Prop-2-yl)ethyl-1-en, n-Pentylen, 1-Methylbutyl-1-en, 2-Methylbutyl-1-en, 3-Methylbutyl-1-en, 4-Methylbutyl-1-en, 1,1-Dimethylpropyl-1-en, 2,2-Dimethylpropyl-1-en, 3,3-Dimethylpropyl-1-en, 1,2-Dimethylpropyl-1-en, 1,3-Dimethylpropyl-1-en, 1-Ethylpropyl-1-en, n-Hexylen, steht,
- X und Y: unabhängig voneinander für O (Sauerstoff) oder S (Schwefel) stehen
- und Q: für eine der oben spezifisch genannten Gruppierungen Q-1 bis Q-406 steht.

Im ganz Speziellen bevorzugter Erfindungsgegenstand sind Verbindungen der allgemeinen Formel (I), worin
- R¹: für Wasserstoff, Fluor, Chlor, Brom, Cyano, Methyl, Trifluormethyl, Methoxy, Trifluormethoxy steht,
- R²: für Fluor steht,
- R³: für Fluor steht,
- R⁴: für Chlor, Brom, Cyano, NOz, C(O)NHz, C(S)NH₂ steht,
- R⁵, R⁶ und R⁷: unabhängig voneinander für Wasserstoff, Fluor, Chlor, Brom, Cyano, Methyl, Trifluormethyl, Methoxy, Trifluormethoxy stehen,
- G: für Methylen, (Methyl)methylen, (Ethyl)methylen, (Dimethyl)methylen, Ethylen, n-Propylen, (1-Methyl)ethyl-1-en, (2-Methyl)ethyl-1-en, n-Butylen, 1-Methylpropyl-1-en, 2-Methylpropyl-1-en, 3-Methylpropyl-1-en, n-Pentylen, n-Hexylen steht,
- X und Y: unabhängig voneinander für O (Sauerstoff) oder S (Schwefel) stehen
und
- Q: für eine der oben spezifisch genannten Gruppierungen Q-1 bis Q-406 steht.

Im besonders Speziellen bevorzugter Erfindungsgegenstand sind Verbindungen der allgemeinen Formel (I), worin
- R¹: für Wasserstoff, Fluor, Chlor, Brom steht,
- R²: für Fluor steht,
- R³: für Fluor steht,
- R⁴: für Chlor, Brom, Cyano, NOz steht,
- R⁵: für Wasserstoff, Fluor, Chlor, Brom steht,
- R⁶: für Wasserstoff, Fluor, Chlor, Brom, Cyano steht,
- R⁷: für Wasserstoff, Fluor, Chlor, Brom steht,
- G: für Methylen, (Methyl)methylen, (Ethyl)methylen steht,
- X und Y: unabhängig voneinander für O (Sauerstoff) oder S (Schwefel) stehen
- und Q: für eine der oben spezifisch genannten Gruppierungen Q-1 bis Q-406 steht.

Im ganz besonders Speziellen bevorzugter Erfindungsgegenstand sind Verbindungen der allgemeinen Formel (I), worin
- R¹: für Wasserstoff steht,
- R²: für Fluor steht,
- R³: für Fluor steht,
- R⁴: für Chlor, Brom, Cyano, NOz steht,
- R⁵: für Wasserstoff, Fluor, Chlor, Brom steht,
- R⁶: für Wasserstoff, Fluor, Chlor, Brom, Cyano steht,
- R⁷: für Wasserstoff steht,
- G: für Methylen, (Methyl)methylen steht,
- X: für O (Sauerstoff) oder S (Schwefel) steht,
- Y: für O (Sauerstoff) steht
- und Q: für eine der oben spezifisch genannten Gruppierungen Q-1 bis Q-406 steht.

Im äußerst Speziellen bevorzugter Erfindungsgegenstand sind Verbindungen der allgemeinen Formel (I), worin
- R¹: für Wasserstoff steht,
- R²: für Fluor steht,
- R³: für Fluor steht,
- R⁴: für Chlor, Brom, Cyano, NOz steht,
- R⁵: für Wasserstoff, Fluor steht,
- R⁶: für Wasserstoff, Fluor, Brom, Cyano steht,
- R⁷: für Wasserstoff steht,
- G: für Methylen, (Methyl)methylen steht,
- X: für O (Sauerstoff) oder S (Schwefel) steht,
- Y: für O (Sauerstoff) steht
und
- Q: für eine der oben spezifisch genannten Gruppierungen Q-1 bis Q-35, Q-41 bis Q-45, Q-58, Q-71 bis Q-80, Q-89, Q-94, Q-95, Q-115, Q-120 bis Q-123, Q-152 bis Q-155, Q-166 bis Q-170, Q-176 bis Q-190, Q-261 bis Q-348, Q-352 bis Q-372, Q-377, Q-391-Q-399 steht.

Im alleräußerst Speziellen bevorzugter Erfindungsgegenstand sind Verbindungen der allgemeinen Formel (I), worin
- R¹: für Wasserstoff steht,
- R²: für Fluor steht,
- R³: für Fluor steht,
- R⁴: für Chlor, Brom, Cyano, NOz steht,
- R⁵: für Wasserstoff, Fluor steht,
- R⁶: für Wasserstoff, Fluor, Brom, Cyano steht,
- R⁷: für Wasserstoff steht,
- G: für Methylen, (Methyl)methylen steht,
- X: für O (Sauerstoff) oder S (Schwefel) steht,
- Y: für O (Sauerstoff) steht
- und Q: für eine der oben spezifisch genannten Gruppierungen Q-1, Q-2, Q-3, Q-4, Q-7, Q-8, Q-9, Q-17, Q-18, Q-23, Q-24, Q-26, Q-27, Q-41, Q-42, Q-43, Q-58, Q-71, Q-72, Q-89, Q-94, Q-115, Q-121, Q-176, Q-177, Q-179, Q-183, Q-272, Q-274, Q-275, Q-276, Q-277, Q-278, Q-281, Q-282, Q-283, Q-284, Q-286, Q-288, Q-291, Q-296, Q-301, Q-302, Q-303, Q-308, Q-309, Q-321, Q-327, Q-328, Q-329, Q-331, Q-335, Q-339, Q-356, Q-365, Q-366, Q-367, Q-371, Q-394 steht.

Die oben aufgeführten allgemeinen oder in Vorzugsbereichen aufgeführten Restedefinitionen gelten sowohl für die Endprodukte der allgemeinen Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Vor allem aus den Gründen der höheren herbiziden Wirkung, besseren Selektivität und/oder besseren Herstellbarkeit sind erfindungsgemäße Verbindungen der genannten allgemeinen Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Wenn die Verbindungen durch Wasserstoffverschiebung Tautomere bilden können, welche strukturell formal nicht durch die allgemeine Formel (I) erfasst würden, so sind diese Tautomere gleichwohl von der Definition der erfindungsgemäßen Verbindungen der allgemeinen Formel (I) umfasst, sofern nicht ein bestimmtes Tautomer Gegenstand der Betrachtung ist. So können beispielsweise viele Carbonylverbindungen sowohl in der Ketoform wie auch in der Enolform vorliegen, wobei beide Formen durch die Definition der Verbindung der allgemeinen Formel (I) umfasst werden.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der allgemeinen Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische.

Sofern die Verbindungen als Feststoffe erhalten werden, kann die Reinigung auch durch Umkristallisieren oder Digerieren erfolgen. Sofern einzelne Verbindungen (I) nicht auf den nachstehend beschriebenen Wegen zufriedenstellend zugänglich sind, können sie durch Derivatisierung anderer Verbindungen (I) hergestellt werden.

Als Isolierungs-, Reinigungs- und Stereoisomerenauftrennungsverfahren von Verbindungen der allgemeinen Formel (I) kommen Methoden in Frage, die dem Fachmann aus analogen Fällen allgemein bekannt sind, z.B. durch physikalische Verfahren wie Kristallisation, Chromatographieverfahren, vor allem Säulenchromatographie und HPLC (Hochdruckflüssigchromatographie), Destillation, gegebenenfalls unter reduziertem Druck, Extraktion und andere Verfahren, können gegebenfalls verbleibende Gemische in der Regel durch chromatographische Trennung, z.B. an chiralen Festphasen, getrennt werden. Für präparative Mengen oder im industriellen Maßstab kommen Verfahren in Frage wie Kristallisation, z.B. diastereomerer Salze, die aus den Diastereomerengemischen mit optisch aktiven Säuren und gegebenenfalls bei vorhandenen sauren Gruppen mit optisch aktiven Basen erhalten werden können.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:
Sofern nicht anders definiert, gilt generell für die Bezeichnung von chemischen Gruppen, dass die Anbindung an das Gerüst bzw. den Rest des Moleküls über das zuletzt genannte Strukturelement der betreffenden chemischen Gruppe erfolgt, d.h. beispielsweise im Falle von (C₂-C₈)-Alkenyloxy über das Sauerstoffatom, und im Falle von Heterocyclyl-(C₁-C₈)-alkyl oder (C₁-C₆)-Alkoxy-(C₁-C₆)-alkoxy-(C₁-C₆)-alkyl jeweils über das C-Atom der Alkylgruppe.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 8, oder mit 1 bis 6 Kohlenstoffatomen, wie (C₁-C₁₀)-, (C₁-C₆)- oder (C₁-C₄)-Alkylthio, z.B. (aber nicht beschränkt auf) (C₁-C₆)-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio.

"Alkoxy" bedeutet ein über ein Sauerstoffatom gebundenen Alkylrest, z. B. (aber nicht beschränkt auf) (C₁-C₆)-Alkoxy wie Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Alkenyloxy bedeutet ein über ein Sauerstoffatom gebundenen Alkenylrest, Alkinyloxy bedeutet ein über ein Sauerstoffatom gebundenen Alkinylrest wie (C₂-C₁₀)-, (C₂-C₆)- oder (C₂-C₄)-Alkenoxy bzw. (C₃-C₁₀)-, (C₃-C₆)- oder (C₃-C₄)-Alkinoxy.

"Alkylcarbonyl" (Alkyl-C(=O)-), soweit nicht an anderer Stelle anders definiert, steht erfindungsgemäß für Alkylreste, die über -C(=O)- an das Gerüst gebunden sind, wie (C₁-C₁₀)-, (C₁-C₆)- oder (C₁-C₄)-Alkylcarbonyl. Die Anzahl der C-Atome bezieht sich dabei auf den Alkylrest in der Alkylcarbonylgruppe.

"Alkoxycarbonyl (Alkyl-O-C(=O)-)", soweit nicht an anderer Stelle anders definiert: Alkylreste, die über -O-C(=O)- an das Gerüst gebunden sind, wie (C₁-C₁₀)-, (C₁-C₆)- oder (C₁-C₄)-Alkoxycarbonyl. Die Anzahl der C-Atome bezieht sich dabei auf den Alkylrest in der Alkoxycarbonylgruppe. Analog stehen "Alkenyloxycarbonyl" und "Alkinyloxycarbonyl", soweit nicht an anderer Stelle anders definiert, erfindungsgemäß für Alkenyl- bzw. Alkinylreste, die über -O-C(=O)- an das Gerüst gebunden sind, wie (C₂-C₁₀)-, (C₂-C₆)- oder (C₂-C₄)-Alkenyloxycarbonyl bzw. (C₃-C₁₀)-, (C₃-C₆)- oder (C₃-C₄)-Alkinyloxycarbonyl. Die Anzahl der C-Atome bezieht sich dabei auf den Alkenyl- bzw. Alkinylrest in der Alken- bzw. Alkinyloxycarbonylgruppe.

Der Begriff "Alkylcarbonyloxy" (Alkyl-C(=O)-O-) steht erfindungsgemäß, soweit nicht an anderer Stelle anders definiert, für Alkylreste, die über eine Carbonyloxygruppe (-C(=O)-O-) mit dem Sauerstoff an das Gerüst gebunden sind, wie (C₁-C₁₀)-, (C₁-C₆)- oder (C₁-C₄)-Alkylcarbonyloxy. Die Anzahl der C-Atome bezieht sich dabei auf den Alkylrest in der Alkylcarbonyloxygruppe.

In Kurzformen wie z.B. C(O)R¹³, C(O)OR¹³, OC(O)NR¹¹R¹², oder C(O)NR¹¹R¹² steht die in Klammern aufgeführte Kurzform O für ein über eine Doppelbindung an das benachbarte Kohlenstoffatom gebundenes Sauerstoffatom.

In Kurzformen wie z.B. OC(S)OR¹³, OC(S)SR¹⁴, OC(S)NR¹¹R¹², steht die in Klammern aufgeführte Kurzform S für ein über eine Doppelbindung an das benachbarte Kohlenstoffatom gebundenes Schwefelatom.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst. Bevorzugte Aryl-Substituenten sind hier zum Beispiel Wasserstoff, Halogen, Alkyl, Cycloalkyl, Cycloalkylalkyl, Cycloalkenyl, Halocycloalkyl, Alkenyl, Alkinyl, Aryl, Arylalkyl, Arylalkenyl, Heteroaryl, Heteroarylalkyl, Heterocyclyl, Heterocyclylalkyl, Alkoxyalkyl, Alkylthio, Haloalkylthio, Haloalkyl, Alkoxy, Haloalkoxy, Cycloalkoxy, Cycloalkylalkoxy, Aryloxy, Heteroraryloxy, Alkoxyalkoxy, Alkinylalkoxy, Alkenyloxy, Bis-alkylaminoalkoxy, Tris-[alkyl]silyl, Bis-[alkyl]arylsilyl, Bis-[alkyl]alkylsilyl, Tris-[alkyl]silylalkinyl, Arylalkinyl, Heteroarylalkinyl, Alkylalkinyl, Cycloalkylalkinyl, Haloalkylalkinyl, Heterocyclyl-N-alkoxy, Nitro, Cyano, Amino, Alkylamino, Bis-alkylamino, Alkylcarbonylamino, Cycloalkylcarbonylamino, Arylcarbonylamino, Alkoxycarbonylamino, Alkoxycarbonylalkylamino, Arylalkoxycarbonylalkylamino, Hydroxycarbonyl, Alkoxycarbonyl, Aminocarbonyl, Alkylaminocarbonyl, Cycloalkylaminocarbonyl, Bis-Alkylaminocarbonyl, Heteroarylalkoxy, Arylalkoxy.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfasst, wie beispielsweise 8-Aza-bicyclo[3.2.1]octanyl, 8-Aza-bicyclo[2.2.2]octanyl oder 1-Azabicyclo[2.2. 1]heptyl. Im Falle von gegebenenfalls substituiertem Heterocyclyl werden auch spirocyclische Systeme umfasst, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3- oder 4- oder 5- oder 6-yl; 1,2,3,4-Tetrahydropyridin-1- oder 2- oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4- oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6- oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2- oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3- oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4- oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3- oder 4- oder 5- oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4- oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2- oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3- oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5- oder 6-yl; 1,2,3,4-Tetrahydropyrazin-1- oder 2- oder 3- oder 4- oder 5- oder 6-yl; 1,2-Dihydropyrazin-1- oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2- oder 4-yl; 1,3-Dioxan-2- oder 4- oder 5-yl; 4H-1,3-Dioxin-2- oder 4- oder 5- oder 6-yl; 1,4-Dioxan-2- oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2- oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2- oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3- oder 4- oder 5- oder 6-yl; 6H-1,2-Oxazin-3- oder 4- oder 5- oder 6-yl; 4H-1,2-Oxazin-3- oder 4- oder 5- oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2- oder 4- oder 5- oder 6-yl; Morpholin-2- oder 3- oder 4-yl; 3,4-Dihydro-2H-1,4-oxazin-2- oder 3- oder 4- oder 5- oder 6-yl; 3,6-Dihydro-2H-1,4-oxazin-2- oder 3- oder 5- oder 6-yl; 2H-1,4-oxazin-2- oder 3- oder 5- oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2- oder 3- oder 4- oder 5- oder 6- oder 7-yl; 2,5-Dihydro-1,2-oxazepin-2- oder 3- oder 4- oder 5- oder 6- oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3- oder 4- oder 5- oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro-1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5- oder 6- oder 7-yl; 1,3-Oxazepin-2- oder 4- oder 5- oder 6- oder 7-yl; 1,4-Oxazepan-2- oder 3- oder 5- oder 6- oder 7-yl; 2,3,4,5-Tetrahydro-1,4-oxazepin-2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7-Tetrahydro-1,4-oxazepin-2- oder 3- oder 5- oder 6- oder 7-yl; 4,5,6,7-Tetrahydro-1,4-oxazepin-2- oder 3- oder 4- oder 5- oder 6- oder 7-yl; 2,3-Dihydro-1,4-oxazepin-2- oder 3- oder 5- oder 6- oder 7-yl; 2,5-Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro-1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2- oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5- oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5- oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3-Dihydro-5H-1,4,2-Dioxazepin-2- oder 3- oder 5- oder 6- oder 7-yl; 5H-1,4,2-Dioxazepin-3- oder 5- oder 6- oder 7-yl; 7H-1,4,2-Dioxazepin-3- oder 5- oder 6- oder 7-yl. Strukturbeispiele für gegebenenfalls weiter substituierte Heterocyclen sind auch im Folgenden aufgeführt:

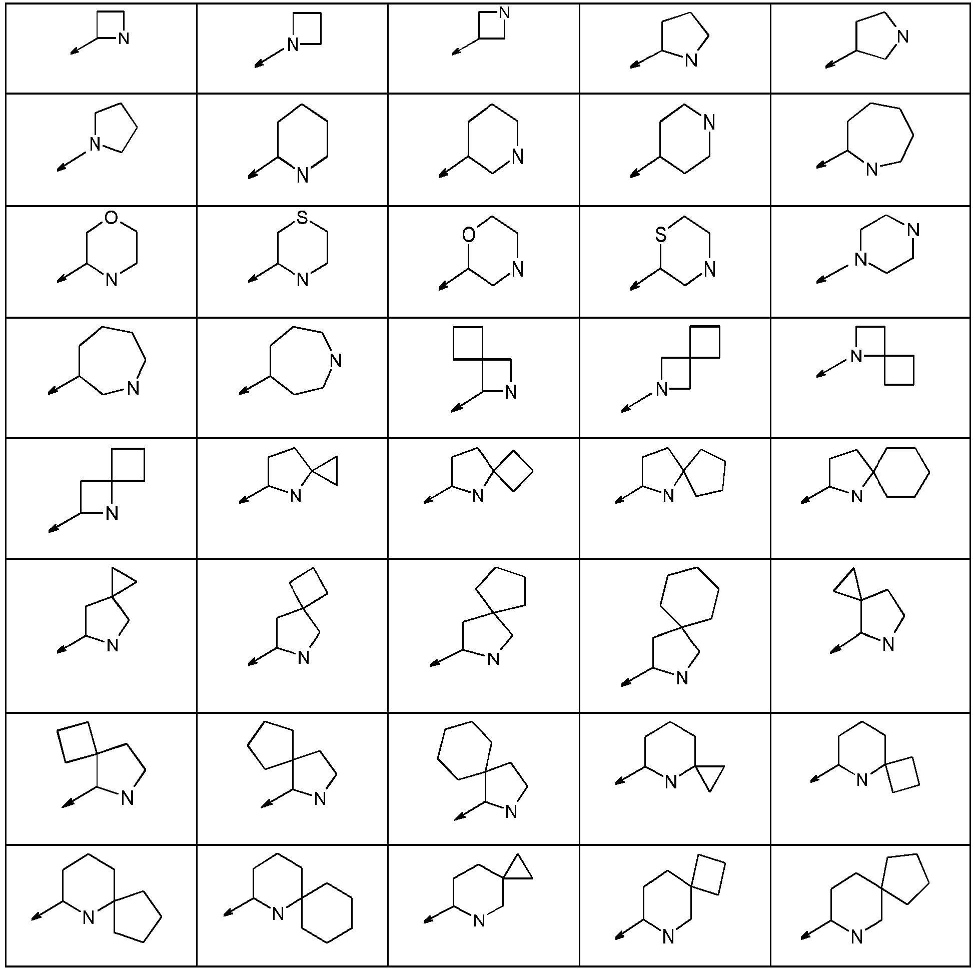

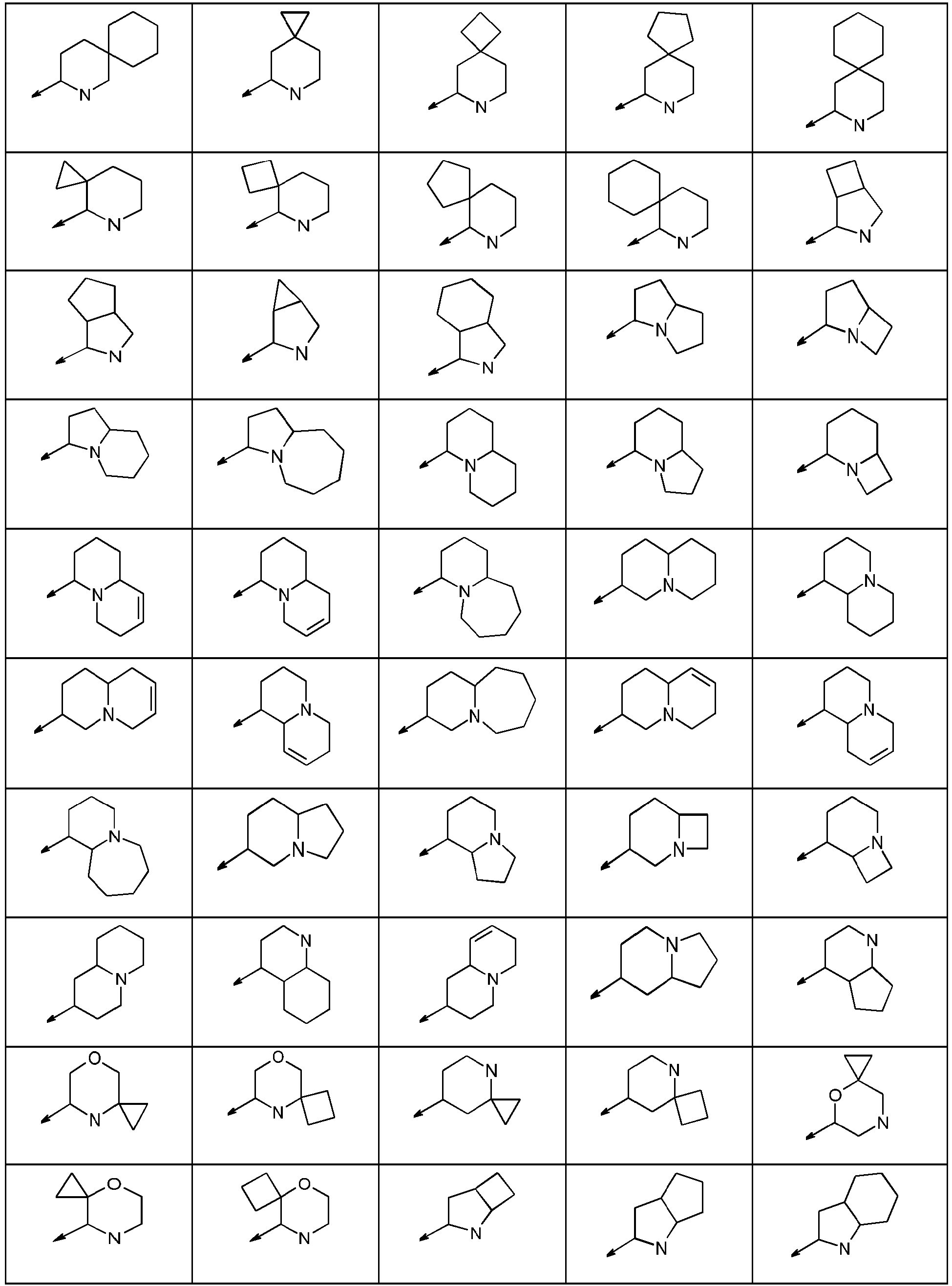

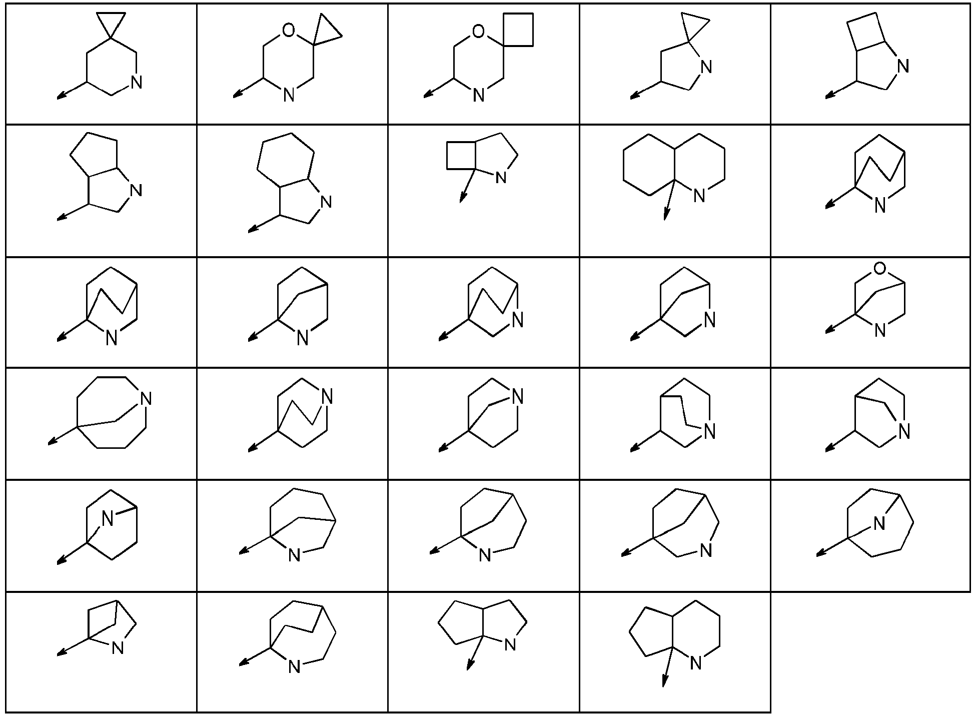

Die oben aufgeführten Heterocyclen sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Oxo, Heteroarylalkoxy, Arylalkoxy, Heterocyclylalkoxy, Heterocyclylalkylthio, Heterocyclyloxy, Heterocyclylthio, Heteroaryloxy, Bis-alkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bis-alkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich es sich um einen teilweise oder vollständig gesättigten Stickstoff-Heterocyclus, so kann dieser sowohl über Kohlenstoff als auch über den Stickstoff mit dem Rest des Moleküls verknüpft sein.

Als Substituenten für einen substituierten heterocyclischen Rest kommen die weiter unten genannten Substituenten in Frage, zusätzlich auch Oxo und Thioxo. Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen N(O), S(O) (auch kurz SO) und S(O)z (auch kurz SOz) im heterocyclischen Ring. Im Fall von -N(O)- und -S(O)-Gruppen sind jeweils beide Enantiomere umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4-Tetrazol-5-yl, 1,2,3,4-Oxatriazol-5-yl, 1,2,3,4-Thiatriazol-5-yl, 1,2,3,5-Oxatriazol-4-yl, 1,2,3,5-Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 2H-Indazol-3-yl, 2H-Indazol-4-yl, 2H-Indazol-5-yl, 2H-Indazol-6-yl, 2H-Indazol-7-yl, 2H-Isoindol-2-yl, 2H-Isoindol-1-yl, 2H-Isoindol-3-yl, 2H-Isoindol-4-yl, 2H-Isoindol-5-yl, 2H-Isoindol-6-yl; 2H-Isoindol-7-yl, 1H-Benzimidazol-1-yl, 1H-Benzimidazol-2-yl, 1H-Benzimidazol-4-yl, 1H-Benzimidazol-5-yl, 1H-Benzimidazol-6-yl, 1H-Benzimidazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, 1,3-Benzthiazol-2-yl, 1,3-Benzthiazol-4-yl, 1,3-Benzthiazol-5-yl, 1,3-Benzthiazol-6-yl, 1,3-Benzthiazol-7-yl, 1,2-Benzisoxazol-3-yl, 1,2-Benzisoxazol-4-yl, 1,2-Benzisoxazol-5-yl, 1,2-Benzisoxazol-6-yl, 1,2-Benzisoxazol-7-yl, 1,2-Benzisothiazol-3-yl, 1,2-Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist und im letzteren Falle als "substituiertes Alkyl" bezeichnet wird. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod. Die Vorsilbe "Bis" schließt auch die Kombination unterschiedlicher Alkylreste ein, z. B. Methyl(Ethyl) oder Ethyl(Methyl).

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z. B. CCl₃, CClF₂, CFCl₂, CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

"Haloalkoxy" ist z.B. OCF₃, OCHF₂, OCH₂F, OCF₂CF₃, OCH₂CF₃ und OCHzCHzCl; Entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkenyl bedeutet z.B. Vinyl, welches ggf. durch weitere Alkylreste substituiert sein kann, z B. (aber nicht beschränkt auf) (C₂-C₆)-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. (C₂-C₆)-Alkinyl bedeutet z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Di-methyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl, das gegebenenfalls weiter substituiert ist, bevorzugt durch Wasserstoff, Alkyl, Alkoxy, Cyano, Nitro, Alkylthio, Haloalkylthio, Halogen, Alkenyl, Alkinyl, Haloalkyl, AMino, Alkylamino, Bisalkylamino, Alkocycarbonyl, Hydroxycarbonyl, Arylalkoxycarbonyl, Aminocarbonyl, Alkylaminocarbonyl, Cycloalkylaminocarbonyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfasst, wie beispielsweise Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl und Adamantan-2-yl, aber auch Systeme wie z. B. 1,1'-Bi(cyclopropyl)-1-yl, 1,1'-Bi(cyclopropyl)-2-yl. Der Ausdruck "(C₃-C₇)-Cycloalkyl" bedeutet eine Kurzschreibweise für Cycloalkyl mit drei bis sieben Kohlenstoffatomen entsprechend der Bereichsangabe für C-Atome.

Im Falle von substituiertem Cycloalkyl werden auch spirocyclische aliphatische Systeme umfasst, wie beispielsweise Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl.

"Cycloalkenyl" bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅. Cycloalkyliden bedeutet ein carbocyclischer Rest, der über eine Zweifachbindung gebunden ist.

Der Begriff "Alkylen", z. B. auch in der Form (C₁-C₈)-Alkylen, bedeutet den Rest eines geradkettigen oder verzweigten offenkettigen Kohlenwasserstoffrests, der an zwei Positionen an weitere Gruppen gebunden ist.

"Alkoxyalkyl" steht für einen über eine Alkylgruppe gebundenen Alkoxyrest und "Alkoxyalkoxy" bedeutet einen über ein Sauerstoffatom gebundenen Alkoxyalkylrest, z.B. (aber nicht beschränkt auf) Methoxymethoxy, Methoxyethoxy, Ethoxyethoxy, Methoxy-n-propyloxy.

"Arylalkyl" steht für einen über eine Alkylgruppe gebundenen Arylrest, "Heteroarylalkyl" bedeutet einen über eine Alkylgruppe gebundenen Heteroarylrest, und "Heterocyclylalkyl" bedeutet einen über eine Alkylgruppe gebundenen Heterocyclylrest.

"Cycloalkylalkyl" steht für einen über eine Alkylgruppe gebundenen Cycloalkylrest, z. B. (aber nicht beschränkt auf) Cyclopropylmethyl, Cyclobutylmethyl, Cyclopentylmethyl, Cyclohexylmethyl, 1-Cyclopropyleth-1-yl, 2-Cyclopropyleth-1-yl, 1-Cyclopropylprop-1-yl, 3-Cyclopropylprop-1-yl.

"Arylalkenyl" steht für einen über eine Alkenylgruppe gebundenen Arylrest, "Heteroarylalkenyl" bedeutet einen über eine Alkenylgruppe gebundenen Heteroarylrest, und "Heterocyclylalkenyl" bedeutet einen über eine Alkenylgruppe gebundenen Heterocyclylrest.

"Arylalkinyl" steht für einen über eine Alkinylgruppe gebundenen Arylrest, "Heteroarylalkinyl" bedeutet einen über eine Alkinylgruppe gebundenen Heteroarylrest, und "Heterocyclylalkinyl" bedeutet einen über eine Alkinylgruppe gebundenen Heterocyclylrest.

Erfindungsgemäß steht "Haloalkylthio" - in Alleinstellung oder als Bestandteil einer chemischen Gruppe - für geradkettiges oder verzweigtes S-Halogenalkyl, vorzugsweise mit 1 bis 8, oder mit 1 bis 6 Kohlenstoffatomen, wie (C₁-C₈)-, (C₁-C₆)- oder (C₁-C₄)-Haloalkylthio, z.B. (aber nicht beschränkt auf) Trifluormethylthio, Pentafluorethylthio, Difluormethyl, 2,2-Difluoreth-1-ylthio, 2,2,2-Difluoreth-1-ylthio, 3,3,3-prop-1-ylthio.

"Halocycloalkyl" und "Halocycloalkenyl" bedeuten durch gleiche oder verschiedene Halogenatome, wie z. B. F, Cl und Br, oder durch Haloalkyl, wie z. B. Trifluormethyl oder Difluormethyl teilweise oder vollständig substituiertes Cycloalkyl oder Cycloalkenyl , z.B. 1-Fluorcycloprop-1-yl, 2-Fluorcycloprop-1-yl, 2,2-Difluorcycloprop-1-yl, 1-Fluorcyclobut-1-yl, 1-Trifluormethylcycloprop-1-yl, 2-Trifluormethylcycloprop-1-yl, 1-Chlor-cycloprop-1-yl, 2-Chlorcycloprop-1-yl, 2,2-Dichlorcycloprop-1-yl, 3,3-Difluorcyclobutyl.

Synthese von substituierten N-Phenyluracilen der allgemeinen Formel (I).

Die erfindungsgemäßen substituierten N-Phenyluracile der allgemeinen Formel (I) können ausgehend von bekannten Verfahren hergestellt werden. Die eingesetzten und untersuchten Syntheserouten gehen dabei von kommerziell erhältlichen oder leicht herstellbaren heteroaromatischen Aminen und von entsprechend substituierten Hydroxyestern aus. Die Gruppierungen G, Q, R¹, R², R³, R⁴, R⁵, R⁶, R⁷, X und Y der allgemeinen Formel (I) haben in den nachfolgenden Schemata die zuvor definierten Bedeutungen, sofern nicht beispielhafte, aber nicht einschränkende, Definitionen erfolgen. Als erstes Schlüsselintermediat für die Synthese der erfindungsgemäßen Verbindungen der allgemeinen Formel (Ia), bei denen X für Schwefel (S) und Y für Sauerstoff (O) steht, wird ein gegebenenfalls weiter substituiertes Mercaptophenyl-1H-pyrimidin-2,4-dion hergestellt. Beispielhaft, aber nicht einschränkend, wird dies an der Synthese von 3-(4-Chlor-2-fluor-5-mercaptophenyl)-1-methyl-6-trifluormethyl-1H-pyrimidin-2,4-dion (IIa) gezeigt (Schema 1). Dazu wird ein geeignetes substituiertes Anilin, beispielhaft, aber nicht einschränkend, 2-Fluor-4-Chloranilin, mit einem geeigneten Reagenz (z. B. Triphosgen) in einem geeigneten polar-aprotischen Lösemittel (z. B. Dichlormethan) in das entsprechende Isocyanat überführt, das im nächsten Schritt durch Umsetzung mit einem geeigneten Aminoacrylsäureester unter Verwendung einer geeigneten Base (z. B. Natriumhydrid oder Kalium-tert-butylat) in einem geeigneten polar-aprotischen Lösemittel (z. B. N,N-Dimethylformamid) in das entsprechende gegebenenfalls weiter substituierte Pyrimidin-2,4-dion, beispielhaft, aber nicht einschränkend, 3-(4-Chlor-2-fluorphenyl)-1-methyl-6-trifluormethyl-1H-pyrimidin-2,4-dion, überführt (Schema 1). Durch nachfolgende Sulfochlorierung mit einem geeigneten Reagenz (z. B. Chlorsulfonsäure) und anschließende Reduktion mit einem geeigneten Reduktionsmittel (z. B. Zn in EtOH und HCl, Zinn(II)chlorid-Hydrat oder Triphenylphosphin) kann das gewünschte Mercaptophenyl-1H-pyrimidin-2,4-dion, beispielhaft, aber nicht einschränkend, 3-(4-Chlor-2-fluor-5-mercaptophenyl)-1-methyl-6-trifluormethyl-1H-pyrimidin-2,4-dion (IIa), hergestellt werden (vgl. KR1345394; EP1122244; EP408382; WO 2003/029226; WO2010/038953; US2011/0224083; KR2011/110420). Im nachfolgenden Schema 1 stehen R² und R³ beispielhaft, aber nicht einschränkend für Fluor, R⁴ beispielhaft, aber nicht einschränkend für Chlor und X beispielhaft, aber nicht einschränkend für Schwefel.

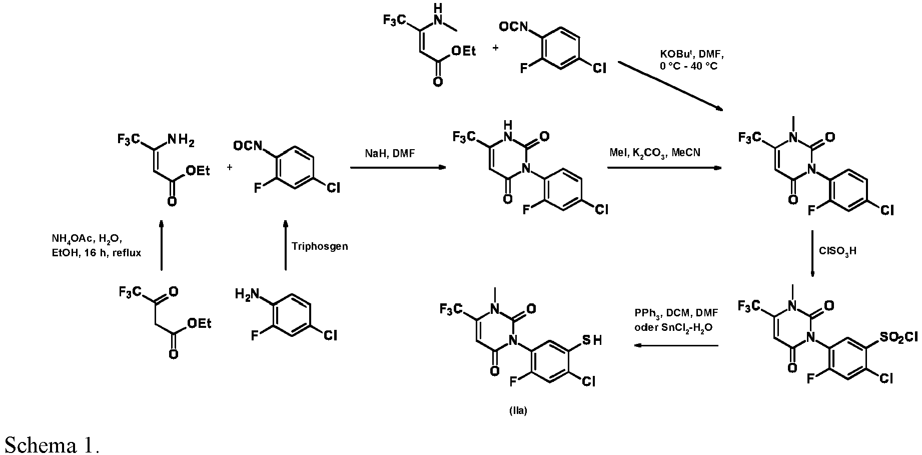

Die in Schema 1 beschriebene Synthese des Schlüsselintermediats (IIa) kann auch auf die Herstellung ähnlicher Intermediate angewendet werden. Die betreffenden intermediären weiter substituierten N-Methyl-5-Mercaptophenyl-1H-pyrimidin-2,4-dione (II) können daraufhin auf verschiedenen Wegen in die gewünschten erfindungsgemäßen Verbindungen der allgemeinen Formel (la), bei denen X für Schwefel (S) und Y für Sauerstoff (O) stehen, überführt werden (Schema 2), nachdem die Verbindungen (II) in einem ersten Schritt mit Hilfe eines geeigneten, gegebenenfalls weiter substituierten Iod-Phenols unter Verwendung einer geeigneten Base oder unter Verwendung eines geeigneten Übergangsmetallkatalysators (z. B. Tris(dibenzylidenaceton)dipalladium(0)) mit einem geeigneten Liganden (z. B. 4,5-Bis(diphenylphosphino)-9,9-dimethylxanthen) und einer geeigneten Base (z. B. Diispropyl(ethyl)amin) in einem geeigneten polar-aprotischen Lösemittel (z.B. Dioxan) zu Intermediaten (III) umgesetzt worden sind. Im nachfolgenden Schema 2 haben Q, R², R³ und R⁴ die obenstehenden erfindungsgemäßen Bedeutungen. Weiterhin stehen R¹, R⁵, R⁶, R⁷ beispielhaft, aber nicht einschränkend für Wasserstoff, X beispielhaft, aber nicht einschränkend für Schwefel, Y beispielhaft, aber nicht einschränkend für Sauerstoff und G beispielhaft, aber nicht einschränkend für CH₂. Das entsprechende beispielhaft, aber nicht einschränkend in Schema 2 beschriebene Intermediat (III) kann durch Umsetzung mit einem geeigneten, gegebenenfalls weiter substituierten Iodalkansäure-ester (in Schema 3 beispielhaft, aber nicht einschränkend ein Iodessigsäureester) unter Verwendung einer geeigneten Base (z.B. Silber(I)carbonat) in einem geeigneten polar-aprotischen Lösemittel (z.B. n-Hexan oder Cyclohexan) bei erhöhter Temperatur (z.B. unter Mikrowellenbedingungen) in einen entsprechenden intermediären Oxyalkansäureester (IVa, IVb) oder die gewünschten Zielverbindungen der allgemeinen Formel (Ia) überführt werden (vgl. Synthesis 2009, 2725). Die entsprechenden Iodalkansäureester sind über literaturbekannte Wege herstellbar (vgl. Eur. J. Org. Chem., 2006, 71, 8459; WO2012037573; Organometallics, 2009, 28, 132).

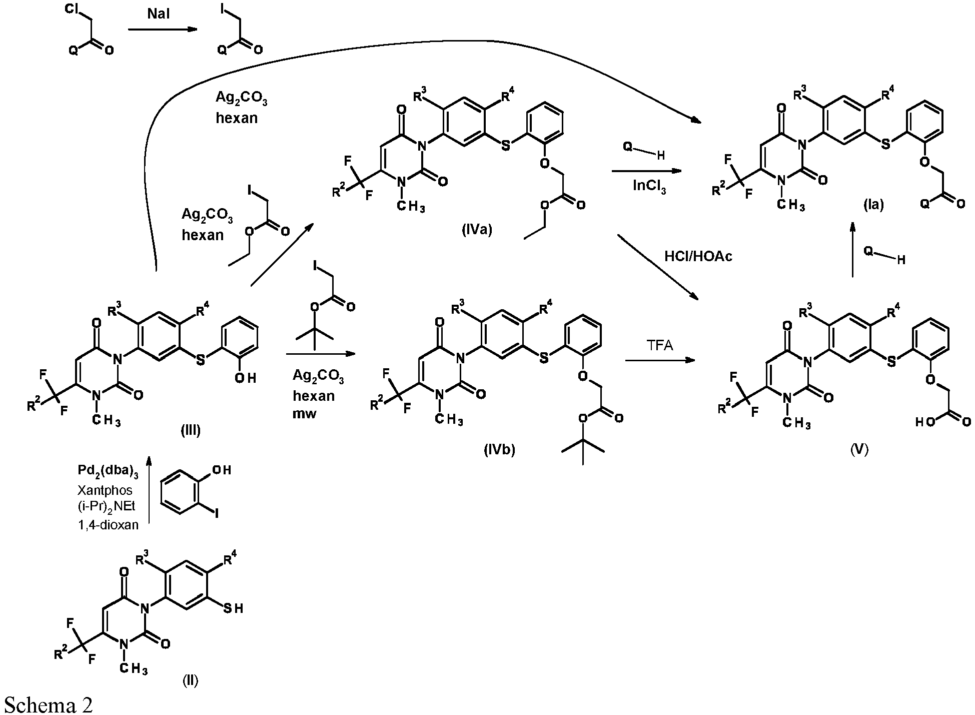

Die intermediären Ethylester (IVa) und tert-Butylester (IVb) können danach unter geeigneten Reaktionsbedingungen [Verwendung einer geeigneten Säure wie z. B. Salzsäure oder Essigsäure im Falle von (IVa) oder Trifluoressigsäure (TFA) im Falle von (IVb)] in die entsprechende freie Säure (V) überführt werden. Durch Reaktion der entsprechenden intermediären Säure (V) mit einer geeigneten Verbindung Q-H unter Vermittlung durch geeignete Kupplungsreagenzien (z. B. HOBt = 1-Hydroxybenzotriazol, EDC = 1-Ethyl-3-(3-dimethylaminopropyl)carbodiimid, HATU = O-(7-Azabenzotriazol-1-yl)-N,N,N',N'-tetramethyluronium-hexafluorphosphat, T3P = 2,4,6-Tripropyl-1,3,5,2,4,6-trioxa-triphosphorinane-2,4,6-trioxid) und geeignete Basen (z. B. Diisopropylethylamin, Triethylamin) in einem geeigneten polar-aprotischen Lösemittel (z. B. Dichlormethan, Chloroform) können die gewünschten substituierten N-Phenyluracile der allgemeinen Formel (Ia) hergestellt werden. Alternativ kann der Ethylester (IVa) durch Kupplung mit einer geeigneten Verbindung Q-H unter Vermittlung durch eine geeignete Lewissäure (z.B. Indium(III)chlorid) in das entsprechende gewünschte substituierte N-Phenyluracil der allgemeinen Formel (Ia) überführt werden (vgl. WO2011/1307088).

Die Herstellung der Verbindungen der allgemeinen Formel (I), bei denen X und Y beispielhaft, aber nicht einschränkend für Sauerstoff (O) stehen, verläuft über die Synthese von Schlüsselintermediaten (VI) mit Fluorsubstituent an Position 5, wie z. B. 3-(2,5-Difluor-4-nitro)-1-methyl-6-trifluormethyl-1H-pyrimidin-2,4-dion (VIa). Dazu wird ein geeignetes substituiertes Anilin, beispielhaft, aber nicht einschränkend, 2,5-Difluoranilin, mit einem geeigneten Reagenz (z. B. Triphosgen) in einem geeigneten polar-aprotischen Lösemittel (z. B. Dichlormethan) in das entsprechende Isocyanat überführt, das im nächsten Schritt durch Umsetzung mit einem geeigneten Aminoacrylsäureester unter Verwendung einer geeigneten Base (z. B. Natriumhydrid oder Kalium-tert-butylat) in einem geeigneten polar-aprotischen Lösemittel (z. B. N,N-Dimethylformamid) in das entsprechende gegebenenfalls weiter substituierte Pyrimidin-2,4-dion, hier beispielhaft, aber nicht einschränkend, 3-(2,5-Difluorphenyl)-6-trifluormethyl-1H-pyrimidin-2,4-dion, überführt wird (Schema 3). Durch Nitrierung mit einem geeigneten Nitrierungsreagenz und nachfolgende N-Methylierung mit einem geeigneten Methylierungsreagens erhält man das gewünschte Intermediat, hier beispielhaft, aber nicht einschränkend 3-(2,5-Difluor-4-nitro)-1-methyl-6-trifluormethyl-1H-pyrimidin-2,4-dion (VIa). Im nachfolgenden Schema 3 stehen R² und R³ beispielhaft, aber nicht einschränkend für Fluor und R⁴ beispielhaft, aber nicht einschränkend für Nitro.

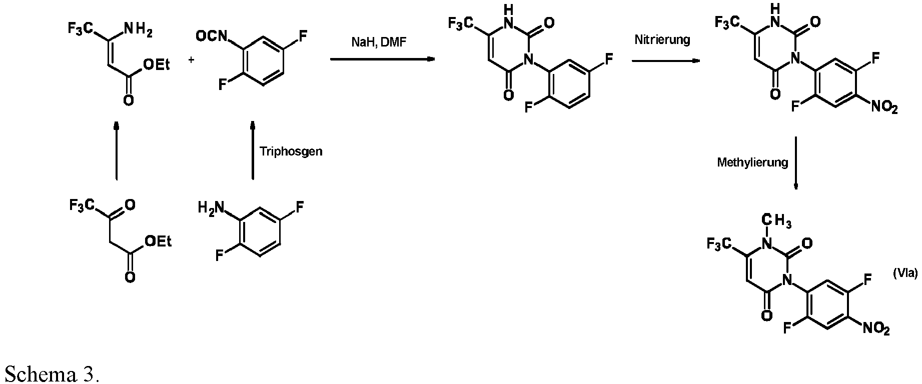

Das auf die oben beschriebene Weise erhaltene Intermediat (VI), z. B. Verbindung (VIa), kann danach mit einem geeigneten substituierten 2-Carbonylalkyloxy-1-hydroxybenzol (VII) unter Verwendung einer geeigneten Base (z. B. Kaliumcarbonat) in einem geeigneten polar-aprotischen Lösemittel (z. B. N,N-Dimethylformamid (DMF)) in ein gewünschtes substituiertes N-Phenyluracil (Ib, R⁴ = Nitro) überführt werden. Das dazu verwendete Intermediat (VII) kann durch mehrstufige Synthese ausgehend von kommerziell erhältlichem 1-Chlor-2-nitrobenzol über (i) basenvermittelte Kupplung (z.B. mit Natriumhydrid) mit einem geeigneten substituierten Hydroxyalkylcarbonylreagenz in einem geeigneten polar-aprotischen Lösemittel (z. B. Tetrahydrofuran oder Dioxan) oder alternativ durch Reaktion von 2-Nitrophenol mit einem geeigneten substituierten Chlormethylcarbonylreagenz, (ii) Reduktion der Nitrogruppe mit einem geeigneten Reduktionsmittel (z. B. Wasserstoff, Palladium auf Kohle in einem geeigneten polar-protischen Lösemittel), (iii) Diazotierung (mit einem geeigneten Diazotierungsreagenz, z.B. tert-Butylnitrit (t-BuONO), Bortrifluorid-Etherat (BF₃-OEt₂) in geeigneten polar-aprotischen Lösemitteln (z.B. Dichlormethan (DCM), Dimethoxyethan), (iv) Umsetzung mit Acetanhydrid und (v) Freisetzung der Hydroxygruppe durch Abspaltung der Acetylschutzgruppe (z.B. basenvermittelt mit Kaliumcarbonat in einem polar-protischen Lösemittel) erhalten werden. Die Nitrogruppe in Verbindung (Ib) kann danach durch Reduktion und anschließende Sandmeyer-Reaktion in einen Halogensubstituenten (z.B. Chlor, Brom) überführt werden, so daß auf diese Weise das gewünschte substituierte N-Phenyluracil (Ic) erhalten werden kann. Im nachfolgenden Schema 4 haben Q und R² die obenstehenden erfindungsgemäßen Bedeutungen. Weiterhin stehen R³ beispielhaft, aber nicht einschränkend für Fluor, R⁴ beispielhaft, aber nicht einschränkend für Chlor oder Nitro, R¹, R⁵, R⁶, R⁷ beispielhaft, aber nicht einschränkend für Wasserstoff, X und Y beispielhaft, aber nicht einschränkend für Sauerstoff und G beispielhaft, aber nicht einschränkend für CH₂.

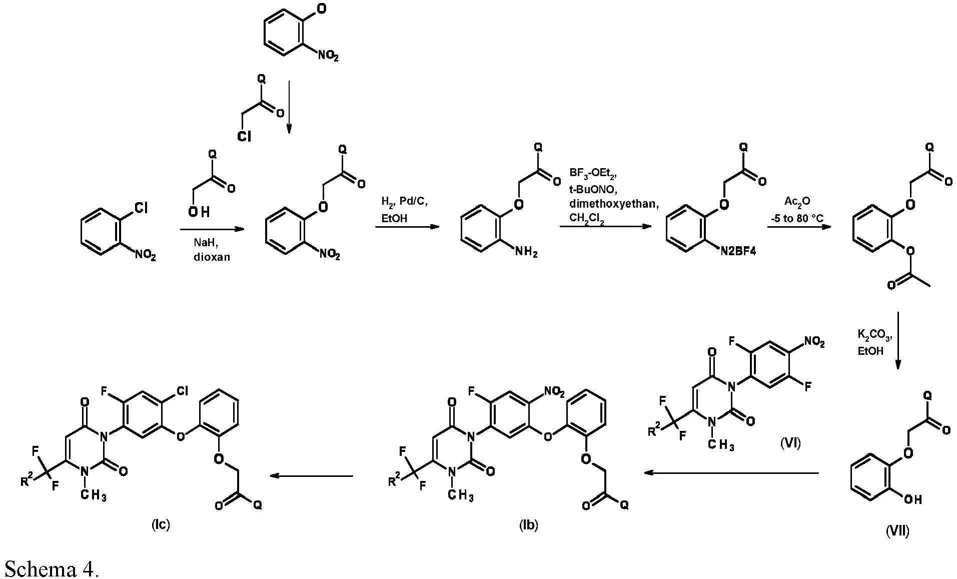

Das auf die oben beschriebene Weise erhaltene Intermediat (VI) kann entsprechend mit einem geeigneten substituierten 2-Carbonylalkylthio-1-hydroxybenzol (VIII) unter Verwendung einer geeigneten Base (z. B. Kaliumcarbonat) in einem geeigneten polar-aprotischen Lösemittel (z. B. N,N-Dimethylformamid (DMF)) in ein gewünschtes substituiertes N-Phenyluracil (Id, R⁴ = Nitro) mit X=O (Sauerstoff) und Y=S (Schwefel) überführt werden. Das dazu verwendete Intermediat (VIII) kann durch mehrstufige Synthese analog zur in Schema 4 beschriebenen Synthese des Intermediates (VII) ausgehend von kommerziell erhältlichem 1-Chlor-2-nitrobenzol oder 2-Nitrothiophenol hergestellt werden. Die Nitrogruppe in Verbindung (Id) kann danach durch Reduktion und anschließende Sandmeyer-Reaktion in einen Halogensubstituenten (z.B. Chlor, Brom) überführt werden, so dass auf diese Weise das gewünschte substituierte N-Phenyluracil (Ie) erhalten werden kann. Im nachfolgenden Schema 5 haben Q und R² die obenstehenden erfindungsgemäßen Bedeutungen. Weiterhin stehen R³ beispielhaft, aber nicht einschränkend für Fluor, R⁴ beispielhaft, aber nicht einschränkend für Chlor oder Nitro, R¹, R⁵, R⁶, R⁷ beispielhaft, aber nicht einschränkend für Wasserstoff, X steht beispielhaft, aber nicht einschränkend für Sauerstoff, Y steht beispielhaft, aber nicht einschränkend für Schwefel und G steht beispielhaft, aber nicht einschränkend für CH₂.

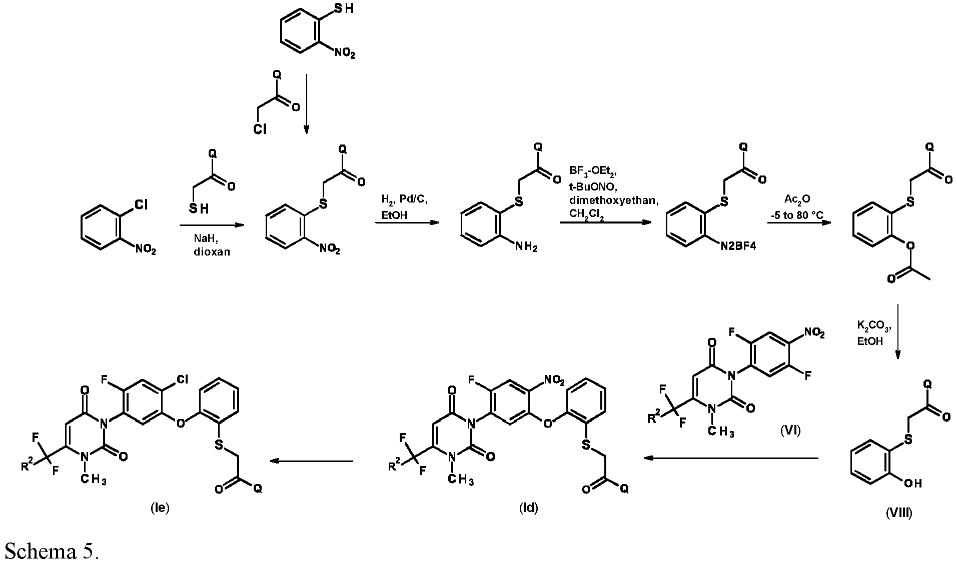

Die intermediären weiter substituierten N-Methyl-5-Mercaptophenyl-1H-pyrimidin-2,4-dione (II) können auch in die gewünschten erfindungsgemäßen Verbindungen der allgemeinen Formel (If), bei denen X und Y für Schwefel (S) stehen, überführt werden (Schema 6), nachdem die Verbindungen (III) in einem ersten Schritt mit Hilfe eines geeigneten gegebenenfalls weiter substituierten Iod-Thiophenols unter Verwendung einer geeigneten Base oder unter Verwendung eines geeigneten Übergangsmetallkatalysators (z. B. Tris(dibenzylidenaceton)dipalladium(0)) mit einem geeigneten Liganden (z. B. 4,5-Bis(diphenylphosphino)-9,9-dimethylxanthen) und einer geeigneten Base (z. B. Diispropyl(ethyl)amin) in einem geeigneten polar-aprotischen Lösemittel (z.B. Dioxan) zu Intermediaten des Typs (IX) umgesetzt worden sind. Gegebenenfalls ist die zwischenzeitliche Schützung der Thiolgruppe unter Verwendung einer geeigneten Schutzgruppe erforderlich. Die Intermediate (IX) können dann mit unterschiedlich substituierten Halogenalkancarbonsäuren unter Verwendung von geeigneten Basen zu den gewünschten Verbindungen der allgemeinen Formel (If) umgesetzt werden. Im nachfolgenden Schema 6 haben Q, R², R³, und R⁴ die obenstehenden erfindungsgemäßen Bedeutungen. Weiterhin stehen R¹, R⁵, R⁶, R⁷ beispielhaft, aber nicht einschränkend für Wasserstoff, X und Y beispielhaft, aber nicht einschränkend für Schwefel und G beispielhaft, aber nicht einschränkend für CH₂. Weiterhin werden die Reaktionswege im folgenden Schema 6 zur besseren Klarheit beispielhaft, aber nicht einschränkend unter Verwendung von Iodessigsäureestern beschrieben. Es eignen sich auch vergleichbare Halogenalkancarbonsäuren (Halogen = Brom oder Chlor) zur Kupplung mit Intermediat (IX).

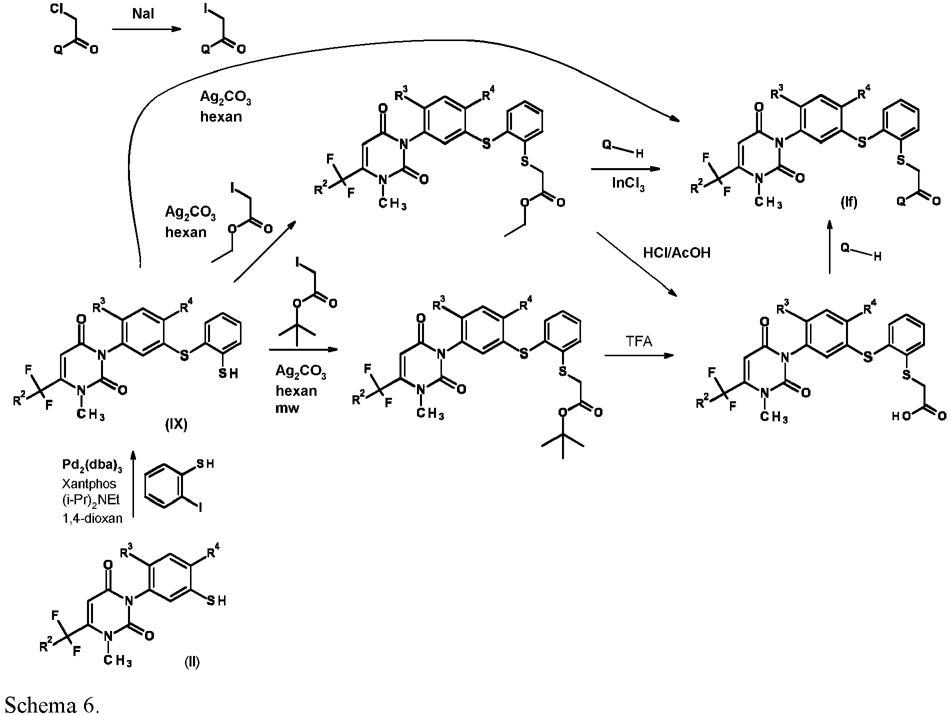

Ausgewählte detaillierte Synthesebeispiele für die erfindungsgemäßen Verbindungen der allgemeinen Formeln (I) sind im Folgenden aufgeführt. Die angegebenen Beispielnummern entsprechen den in den nachstehenden Tabellen I.1 bis 1.33 genannten Nummerierungen. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 MHz bei ¹H-NMR und 150 MHz bei ¹³C-NMR und 375 MHz bei ¹⁹F-NMR, Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben. Die verwendeten Abkürzungen für chemische Gruppen haben beispielsweise die nachfolgenden Bedeutungen: Me = CH₃, Et = CH₂CH₃, t-Hex = C(CH₃)₂CH(CH₃)₂, t-Bu = C(CH₃)₃, n-Bu = unverzweigtes Butyl, n-Pr = unverzweigtes Propyl, i-Pr = verzweigtes Propyl, c-Pr = Cyclopropyl, c-Hex = Cyclohexyl.

### Synthesebeispiele:

### Bspl. 1.1-1: 2-Methoxyethyl-[2-({2-chlor-4-fluor-5-[3-methyl-2,6-dioxo-4-(trifluormethyl)-3,6-dihydropyrimidin-1(2H)-yl]phenyl} sulfanyl)phenoxy]acetat

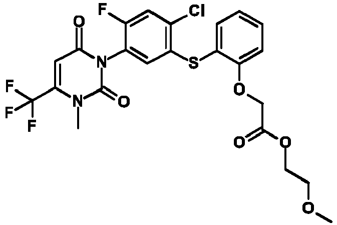

Zu einer Lösung von Methylglycol (0.012 g, 0.155 mmol) in 4 ml Dichlormethan gab man [2-({2-Chlor-4-fluor-5-[3-methyl-2,6-dioxo-4-(trifluormethyl)-3,6-dihydropyrimidin-1 (2H)-yl]phenyl}sulfanyl)phenoxy]essigsäure (0.060 g, 0.119 mmol) und anschließend nacheinander 1-Hydroxy-1H-benzotriazol Hydrat (0.024 g, 0.155 mmol), 1-(3-Dimethylaminopropyl)-3-ethylcarbodiimidhydrochlorid (0.030 g, 0.155 mmol) und 4-Dimethylaminopyridin (10 mol%) und rührte über Nacht bei Raumtemperatur. Das Reaktionsgemisch wurde säulenchromatographisch an Kieselgel mit n-Heptan / Essigsäureethylester-Gradient gereinigt. Man erhielt 0.044 g (64% der Theorie) 2-Methoxyethyl-[2-({2-chlor-4-fluor-5-[3-methyl-2,6-dioxo-4-(trifluormethyl)-3,6-dihydropyrimidin-1(2H)-yl]phenyl}sulfanyl)phenoxy]acetat. ¹H-NMR (CDCl₃ δ, ppm) 7.44-7.41 (m, 1H), 7.34-7.30 (m, 2H), 7.08 (d, 1H), 7.01-6.97 (m, 1H), 6.78-6.76 (m, 1H), 6.27 (s, 1H), 4.70 (s, 2H), 4.29-4.26 (m, 2H), 3.58-3.56 (m, 2H), 3.50-3,49 (m, 3H), 3.35 (s, 3H).

### Bspl. I.2-1: 2-Methoxyethyl-(2RS)-2-[2-({2-chlor-4-fluor-5-[3-methyl-2,6-dioxo-4-(trifluormethyl)-3,6-dihydropyrimidin-1(2H)-yl]phenyl}sulfanyl)phenoxy]propanoat

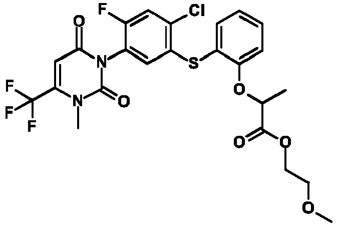

Zu einer Lösung von Methylglycol (0.011 g, 0.150 mmol) in 4 ml Dichlormethan gab man (2RS)-2-[2-({2-Chlor-4-fluor-5-[3-methyl-2,6-dioxo-4-(trifluormethyl)-3,6-dihydropyrimidin-1(2H)-yl]phenyl}sulfanyl)phenoxy]propansäure (0.060 g, 0.116 mmol) und anschließend nacheinander 1-Hydroxy-1H-benzotriazol Hydrat (0.023 g, 0.150 mmol), 1-(3-Dimethylaminopropyl)-3-ethylcarbodiimidhydrochlorid (0.029 g, 0.150 mmol) und 4-Dimethylaminopyridin (10 mol%) und rührte über Nacht bei Raumtemperatur. Das Reaktionsgemisch wurde säulenchromatographisch an Kieselgel mit *n*-Heptan / Essigsäureethylester-Gradient gereinigt. Man erhielt 0.056 g (80% der Theorie) 2- Methoxyethyl-(2RS)-2-[2-({2-chlor-4-fluor-5-[3-methyl-2,6-dioxo-4-(trifluormethyl)-3,6-dihydropyrimidin-1(2H)-yl]phenyl}sulfanyl)phenoxy]propanoat. ¹H-NMR (CDCl₃ δ, ppm) 7.43-7.40 (m, 1H), 7.32-7.28 (m, 2H), 7.10-6.95 (m, 2H), 6.76-6.72 (m, 1H), 6.27-6.26 (m, 1H), 4.79-4.73 (m, 1H), 4.32-4.22 (m, 2H), 3.56-3.53 (m, 2H), 3,49 (m, 3H), 3.33 (s, 3H), 1.51-1.50 (m, 3H).

### Bspl. I.7-176: Cyanmethyl-(2-{2-chlor-4-fluor-5-[3-methyl-2,6-dioxo-4-(trifluormethyl)-3,6-dihydropyrimidin-1(2H)-yl]phenoxy}phenoxy)acetat

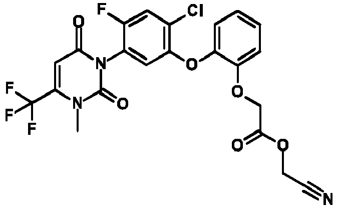

Zu einer Lösung von Glykolonitril (0.027 g, 0.286 mmol) in 10 ml Dichlormethan gab man (2-{2-Chlor-4-fluor-5-[3-methyl-2,6-dioxo-4-(trifluormethyl)-3,6-dihydropyrimidin-1 (2H)-yl]phenoxy}phenoxy)essigsäure (0.100 g, 0.205 mmol) und anschließend nacheinander 1-Hydroxy-1H-benzotriazol Hydrat (0.041 g, 0.266 mmol), 1-(3-Dimethylaminopropyl)-3-ethylcarbodiimidhydrochlorid (0.051 g, 0.266 mmol) und 4-Dimethylaminopyridin (10 mol%) und rührte 2 h bei Raumtemperatur. Man versetzte das Reaktionsgemisch mit Wasser und Dichlormethan, extrahierte die wässrige Phase mehrmals mit Dichlormethan, trocknete die vereinigten organischen Phasen über Natriumsulfat und entfernte das Lösungsmittel im Vakuum. Nach säulenchromatographischer Reinigung an Kieselgel mit einem n-Heptan / Essigsäureethylester-Gradienten erhielt man 0.095 g (79% der Theorie) eines farblosen Feststoffs. ¹H-NMR (CDCl₃ δ, ppm) 7.39 (d, 1H), 7.18-7.12 (m, 1H), 7.11-7.03 (m, 2H), 6.94 (d, 1H), 6.68 (d, 1H), 6.29 (s, 1H), 4.76 (s, 2H), 4.74 (s, 2H), 3.50 (s, 3H).

### Bspl. I.7-71: Tetrahydrofuran-2-ylmethyl-(2-{2-chlor-4-fluor-5-[3-methyl-2,6-dioxo-4-(trifluormethyl)-3,6-dihydropyrimidin-1(2H)-yl]phenoxy}phenoxy)acetat

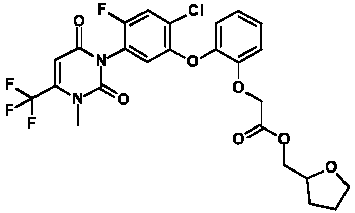

Zu einer Lösung von Tetrahydrofuran-2-ylmethanol (0.042 g, 0.412 mmol) in 15 ml Dichlormethan gab man (2-{2-Chlor-4-fluor-5-[3-methyl-2,6-dioxo-4-(trifluormethyl)-3,6-dihydropyrimidin-1(2H)-yl]phenoxy}phenoxy)essigsäure (0.160 g, 0.327 mmol) und anschließend nacheinander 1-Hydroxy-1H-benzotriazol Hydrat (0.059 g, 0.383 mmol), 1-(3-Dimethylaminopropyl)-3-ethylcarbodiimid-hydrochlorid (0.073 g, 0.383 mmol) und 4-Dimethylaminopyridin (10 mol%) und rührte 2 h bei Raumtemperatur. Man versetzte das Reaktionsgemisch mit Wasser und Dichlormethan, extrahierte die wässrige Phase mehrmals mit Dichlormethan, trocknete die vereinigten organischen Phasen über Natriumsulfat und entfernte das Lösungsmittel im Vakuum. Nach säulenchromatographischer Reinigung an Kieselgel mit einem n-Heptan / Essigsäureethylester-Gradienten erhielt man 0.129 g (73% der Theorie) eines farblosen Feststoffs. ¹H-NMR (CDCl₃ δ, ppm) 7.36 (d, 1H), 7.14-7.10 (m, 1H), 7.07-7.04 (m, 1H), 7.01-6.97 (m, 1H), 6.92 (d, 1H), 6.77 (d, 1H), 6.28 (s, 1H), 4.70 (s, 2H), 4.21-4.18 (m, 1H), 4,13-4.05 (m, 2H), 3.83-3.73 (m, 2H), 3.50 (s, 3H), 2.04-1.83 (m, 3H), 1.59-1.52 (m, 1H).

### Bspl. I.7-276: Pyridin-2-ylmethyl-(2-{2-chlor-4-fluor-5-[3-methyl-2,6-dioxo-4-(trifluormethyl)-3,6-dihydropyrimidin-1 (2H)-yl]phenoxy }phenoxy)acetat

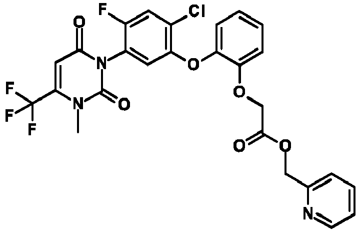

Zu einer Lösung von Pyridin-2-ylmethanol (0.028 g, 0.258 mmol) in 10 ml Dichlormethan gab man (2-{2-Chlor-4-fluor-5-[3-methyl-2,6-dioxo-4-(trifluormethyl)-3,6-dihydropyrimidin-1 (2H)-yl]phenoxy}phenoxy)essigsäure (0.100 g, 0.205 mmol) und anschließend nacheinander 1-Hydroxy-1H-benzotriazol Hydrat (0.037 g, 0.239 mmol), 1-(3-Dimethylaminopropyl)-3-ethylcarbodiimid-hydrochlorid (0.046 g, 0.239 mmol) und 4-Dimethylaminopyridin (10 mol%) und rührte 2 h bei Raumtemperatur. Man versetzte das Reaktionsgemisch mit Wasser und Dichlormethan, extrahierte die wässrige Phase mehrmals mit Dichlormethan, trocknete die vereinigten organischen Phasen über Natriumsulfat und entfernte das Lösungsmittel im Vakuum. Nach säulenchromatographischer Reinigung an Kieselgel mit einem n-Heptan / Essigsäureethylester-Gradienten erhielt man 0.096 g (81% der Theorie) eines farblosen Feststoffs. ¹H-NMR (CDCl₃ δ, ppm) 8.57-8.55 (m, 1H), 7.66 (t, 1H), 7.32 (d, 1H), 7.26-7.21 (m, 2H), 7.14-7.08 (m, 2H), 7.02-6.94 (m, 2H), 6.75 (d, 1H), 6.24 (s, 1H), 5.28 (s, 2H), 4.77 (s, 2H), 3.48 (s, 3H).

### Bspl. I.7-183: 2-Nitroethyl-(2-{2-chlor-4-fluor-5-[3-methyl-2,6-dioxo-4-(trifluormethyl)-3,6-dihydropyrimidin-1(2H)-yl]phenoxy}phenoxy)acetat

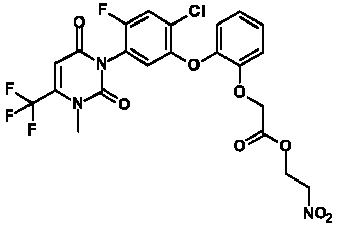

Zu einer Lösung von (2-{2-Chlor-4-fluor-5-[3-methyl-2,6-dioxo-4-(trifluormethyl)-3,6-dihydropyrimidin-1(2H)-yl]phenoxy}phenoxy)essigsäure (0.150 g, 0.306 mmol) in 1.0 ml Toluol gab man nacheinander 2-Nitroethanol (0.139 g, 1.531 mmol), konzentrierte Essigsäure (0.092 g, 1.531 mmol), konzentrierte Schwefelsäure (0.015 g, 0.153 mmol) und rührte 5 h unter Rückfluss. Man ließ das Reaktionsgemisch über Nacht stehen und rührte weitere 5 h unter Rückfluss. Man versetzte das Reaktionsgemisch mit Wasser und Dichlormethan, extrahierte die wässrige Phase mehrmals mit Dichlormethan, trocknete die vereinigten organischen Phasen über Natriumsulfat und entfernte das Lösungsmittel im Vakuum. Nach säulenchromatographischer Reinigung an Kieselgel mit einem n-Heptan/ Aceton-Gradienten gefolgt von einer weiteren säulenchromatographischer Reinigung an Kieselgel mit einem n-Heptan / Essigsäureethylester-Gradienten erhielt man 0.036 g (20% der Theorie) 2-Nitroethyl-(2-{2-chlor-4-fluor-5-[3-methyl-2,6-dioxo-4-(trifluormethyl)-3,6-dihydropyrimidin-1 (2H)-yl]phenoxy}phenoxy)acetat. ¹H-NMR (CDCl₃ δ, ppm) 7.37 (d, 1H), 7.16-7.12 (m, 1H), 7.09-7.07 (m, 1H), 7.04-7.00 (m, 1H), 6.90 (d, 1H), 6.67 (d, 1H), 6.28 (s, 1H), 4.67 (s, 2H), 4.66-4.64 (m, 2H), 4.59-4.56 (m, 2H), 3.50 (s, 3H).

### Bspl. I.7-177: 1-Cyanethyl-(2-{2-chlor-4-fluor-5-[3-methyl-2,6-dioxo-4-(trifluormethyl)-3,6-dihydropyrimidin-1 (2H)-yl]phenoxy}phenoxy)acetat

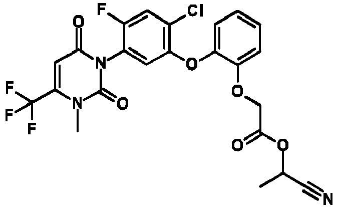

Zu einer Lösung von (2-{2-Chlor-4-fluor-5-[3-methyl-2,6-dioxo-4-(trifluormethyl)-3,6-dihydropyrimidin-1(2H)-yl]phenoxy}phenoxy)essigsäure (0.160 g, 0.327 mmol) in 4.0 ml Acetonitril gab man nacheinander 4-Dimethylaminopyridin (0.005 g, 0.039 mmol), Kaliumcarbonat (0.056 g, 0.404 mmol), 2-Brompropannitril (0.052 g, 0.389 mmol) und rührte 2h bei Raumtemperatur und anschließend 1 h unter Rückfluss. Man versetzte das Reaktionsgemisch mit Wasser und Dichlormethan, extrahierte die wässrige Phase mehrmals mit Dichlormethan, trocknete die vereinigten organischen Phasen über Natriumsulfat und entfernte das Lösungsmittel im Vakuum. Nach säulenchromatographischer Reinigung an Kieselgel mit einem n-Heptan / Essigsäureethylester-Gradienten und anschließender Trocknung des erhaltenen Produkts im Vakuum bei 40°C erhielt man 0.160 g (95% der Theorie) 1-Cyanethyl-(2-{2-chlor-4-fluor-5-[3-methyl-2,6-dioxo-4-(trifluormethyl)-3,6-dihydropyrimidin-1 (2H)-yl]phenoxy}phenoxy)acetat. ¹H-NMR (CDCl₃ δ, ppm) 7.38 (d, 1H), 7.17-7.13 (m, 1H), 7.10-7.02 (m, 2H), 6.95-6.93 (m, 1H), 6.72-6.68 (m, 1H), 6.29 (d, 1H), 5.45-5.42 (m, 1H), 4.72 (s, 2H), 3.50 (s, 3H), 1.64-1.61 (m, 3H).

### Bspl. I.7-1: 2-Methoxyethyl-(2-{2-chlor-4-fluor-5-[3-methyl-2,6-dioxo-4-(trifluormethyl)-3,6-dihydropyrimidin-1(2H)-yl]phenoxy}phenoxy)acetat

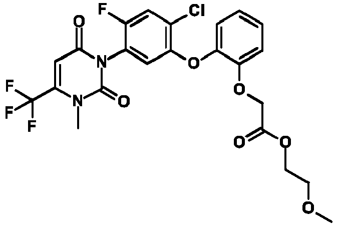

Unter Stickstoffatmosphäre gab man zu Ethyl-(2-{2-chlor-4-fluor-5-[3-methyl-2,6-dioxo-4-(trifluormethyl)-3,6-dihydropyrimidin-1(2H)-yl]phenoxy}phenoxy)acetat (5.520 g, 10.681 mmol) in 46.32 ml 2-Methoxyethanol Indium(III)chlorid (2.599 g, 11,749 mmol) und rührte 3 h bei 115°C. Nach dem Abkühlen auf Raumtemperatur goss man das Reaktionsgemisch auf Wasser, gab Essigsäureethylester zu, extrahierte die wässrige Phase mehrmals mit Essigsäureethylester, wusch die vereinigten organischen Phasen mit gesättigter wässriger Natriumchlorid-Lösung, trocknete die organische Phase über Natriumsulfat und entfernte das Lösungsmittel im Vakuum. Nach säulenchromatographischer Reinigung an Kieselgel mit einem n-Heptan / Essigsäureethylester-Gradienten erhielt man 5.150 g (88% der Theorie) eines farblosen Feststoffs. ¹H-NMR (CDCl₃ δ, ppm) 7.36 (d, 1H), 7.14-7.10 (m, 1H), 7.07-7.05 (m, 1H), 7.01-6.99 (m, 1H), 6.93-6.91 (m, 1H), 6.77 (d, 1H), 6.28 (s, 1H), 4.70 (s, 2H), 4.29-4.27 (m, 2H), 3.56-3.54 (m, 2H), 3.50 (s, 3H), 3.33 (s, 3H).

### Bspl. I.7-94: 1-Methoxypropan-2-yl-(2-{2-chlor-4-fluor-5-[3-methyl-2,6-dioxo-4-(trifluormethyl)-3,6-dihydropyrimidin-1(2H)-yl]phenoxy}phenoxy)acetat

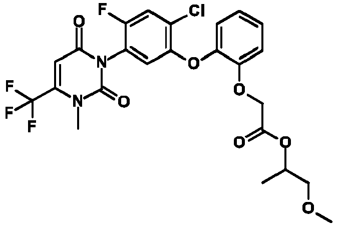

Unter Stickstoffatmosphäre gab man zu 2-Methoxyethyl-(2-{2-chlor-4-fluor-5-[3-methyl-2,6-dioxo-4-(trifluormethyl)-3,6-dihydropyrimidin-1(2H)-yl]phenoxy}phenoxy)acetat (0.160 g, 0.293 mmol) in 4.0 ml 1-Methoxypropan-2-ol Indium(III)chlorid (0.116 g, 0.527 mmol) und erhitzte das Reaktionsgemisch 1.5 h bei 125°C in einem Mikrowellenreaktor. Nach dem Abkühlen auf Raumtemperatur goss man das Reaktionsgemisch auf Wasser, gab Dichlormethan zu, extrahierte die wässrige Phase mehrmals mit Dichlormethan, trocknete die vereinigten organischen Phasen über Natriumsulfat und entfernte das Lösungsmittel im Vakuum. Nach säulenchromatographischer Reinigung an Kieselgel mit einem n-Heptan / Essigsäureethylester-Gradienten erhielt man 0.110 g (66% der Theorie) 1-Methoxypropan-2-yl-(2-{2-chlor-4-fluor-5-[3-methyl-2,6-dioxo-4-(trifluormethyl)-3,6-dihydropyrimidin-1 (2H)-yl]phenoxy}phenoxy)acetat. ¹H-NMR (CDCl₃ δ, ppm) 7.36 (d, 1H), 7.14-7.10 (m, 1H), 7.07-7.04 (m, 1H), 7.01-6.96 (m, 1H), 6.93-6.90 (m, 1H), 6.79-6.76 (m, 1H), 6.28 (d, 1H), 5.16-5.12 (m, 1H), 4.65 (s, 2H), 3.50 (s, 3H), 3.44-3.36 (m, 2H), 3.33 (s, 3H), 1.23 (d, 3H).

### Bspl. 1.6-1: 2-Methoxyethyl-(2-{4-fluor-5-[3-methyl-2,6-dioxo-4-(trifluormethyl)-3,6-dihydropyrimidin-1 (2H)-yl]-2-nitrophenoxy}phenoxy)acetat.

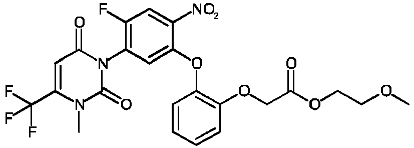

Ethyl-(2-{4-fluor-5-[3-methyl-2,6-dioxo-4-(trifluormethyl)-3,6-dihydropyrimidin-1 (2H)-yl]-2-nitrophenoxy}phenoxy)acetat (100 mg, 0.19 mmol) wurde in einem Mikrowellengefäß unter Argon mit 2-Methoxyethanol (505 mg, 6.63 mmol) und Indium(III)chlorid (51.2 mg, 0.23 mmol) versetzt. Das Reaktionsgemisch wurde 120 Minuten lang bei einer Temperatur von 115 °C unter Mikrowellenbedingungen gerührt. Nach dem Abkühlen auf Raumtemperatur wurde das Reaktionsgemisch nit 50 mL Wasser verrührt und mit 10 mL Dichlormethan versetzt. Die organische Phase wurde abgetrennt und eingeengt. Durch säulenchromatographische Reinigung des erhaltenen Rohproduktes an Kieselgel mit einem n-Heptan / Essigsäureethylester-Gradienten wurde 2-Methoxyethyl-(2-{4-fluor-5-[3-methyl-2,6-dioxo-4-(trifluormethyl)-3,6-dihydropyrimidin-1(2H)-yl]-2-nitrophenoxy}phenoxy)acetat. (78 mg, 73 % der Theorie) in Form eines farblosen Öls erhalten.

¹H-NMR (CDCl₃ δ, ppm) 7.87 (d, 1H), 7.19 (m, 2H), 7.03 (t, 1H), 6.95 (d, 1H), 6.88 (d, 1H), 6.28 (s, 1H), 4.67 (s, 2H), 4.25 (t, 2H), 3.53 (t, 2H), 3.50 (s, 3H), 3.31 (s, 3H).

### Bspl. 1.6-176: Cyanmethyl-(2-{4-fluor-5-[3-methyl-2,6-dioxo-4-(trifluormethyl)-3,6-dihydropyrimidin-1(2H)-yl]-2-nitrophenoxy}phenoxy)acetat.

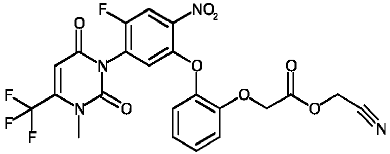

(2- {4-Fluor-5 - [3 -methyl-2,6-dioxo-4-(trifluormethyl)-3,6-dihydropyrimidin-1 (2H)-yl] -2-nitrophenoxy}phenoxy)essigsäure (100 mg, 0.20 mmol) wurde mit Bromacetonitril (38 mg, 0.30 mmol) und Triethylamin (30 mg, 0.30 mmol) unter Argon in 1 mL Aceton vorgelegt und 2.5 Stunden bei Raumtemperatur gerührt. Nach DC-Kontrolle wurde weiteres Bromacetonitril (13 mg, 0.10 mmol) zugesetzt und eine weitere Stunde bei Raumtemperatur nachgerührt. Das Reaktionsgemisch wurde eingeengt und mit 10 mL Wasser und 10 mL Dichlormethan versetzt. Die organische Phase wurde abgetrennt und eingeengt. Durch säulenchromatographische Reinigung des erhaltenen Rohproduktes an Kieselgel mit einem n-Heptan / Essigsäureethylester-Gradienten wurde Cyanmethyl-(2-{4-fluor-5-[3-methyl-2,6-dioxo-4-(trifluormethyl)-3,6-dihydropyrimidin-1 (2H)-yl]-2-nitrophenoxy}phenoxy)acetat (104 mg, 95 % der Theorie) in Form eines farblosen Öls erhalten.

¹H-NMR (CDCl₃ δ, ppm) 7.89 (d, 1H), 7.24-7.20 (m, 2H), 7.09 (dt, 1H), 6.92 (dd, 1H), 6.82 (d, 1H), 6.30 (s, 1H), 4.75 (s, 2H), 4.71 (s, 2H), 3.51 (s, 3H).

### Bspl. 1.8-176: Cyanmethyl-(2-{2-brom-4-fluor-5-[3-methyl-2,6-dioxo-4-(trifluormethyl)-3,6-dihydropyrimidin-1 (2H)-yl]phenoxy}phenoxy)acetat

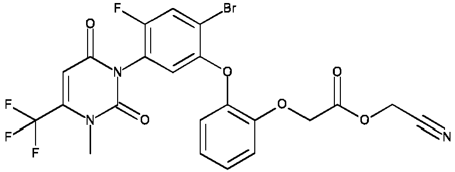

(2-{2-Brom-4-fluor-5-[3-methyl-2,6-dioxo-4-(trifluormethyl)-3,6-dihydropyrimidin-1 (2H)-yl]phenoxy}phenoxy)essigsäure (1.000 g, 1.86 mmol) wurde mit Bromacetonitril (337 mg, 2.81 mmol) und Triethylamin (380 mg, 3.75 mmol) unter Argon in 22 mL Dichlormethan vorgelegt und 5 Stunden bei Raumtemperatur gerührt. Der Ansatz stand über Nacht und wurde mit 10 mL Wasser versetzt und dann verrührt. Die organische Phase wurde abgetrennt und eingeengt. Durch säulenchromatographische Reinigung des erhaltenen Rohproduktes an Kieselgel mit einem n-Heptan / Essigsäureethylester-Gradienten wurde Cyanmethyl-(2-{2-brom-4-fluor-5-[3-methyl-2,6-dioxo-4-(trifluormethyl)-3,6-dihydropyrimidin-1(2H)-yl]phenoxy}phenoxy)acetat (598 mg, 55 % der Theorie) in Form eines farblosen Öls erhalten.

¹H-NMR (CDCl₃ δ, ppm) 7.55 (d, 1H), 7.18-7.03 (m, 3H), 6.95 (dd, 1H), 6.64 (d, 1H), 6.29 (s, 1H), 4.76 (s, 2H), 4.75 (s, 2H), 3.50 (s, 3H).

### Bspl. I.9-176: Cyanmethyl-(2-{2-cyan-4-fluor-5-[3-methyl-2,6-dioxo-4-(trifluormethyl)-3,6-dihydropyrimidin-1(2H)-yl]phenoxy}phenoxy)acetat

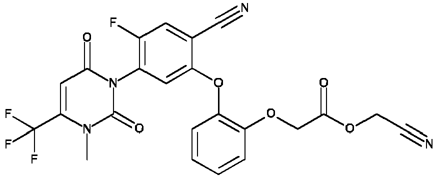

Cyanmethyl-(2-{2-brom-4-fluor-5-[3-methyl-2,6-dioxo-4-(trifluormethyl)-3,6-dihydropyrimidin-1 (2H)-yl]phenoxy}phenoxy)acetat (598 mg, 1.05 mmol) wurde mit Zinkcyanid (129 mg, 1.10 mmol) und Tetrakis(triphenylphosphin)palladium (0) (121 mg, 0.10 mmol) unter Argon in 15 mL N,N-Dimethylacetamid vorgelegt und 1 Stunde bei 180 grad Celsius gerührt. Nach DC-Kontrolle wurde das Reaktionsgemisch auf 10 mL Wasser gegeben und mit Essigester extrahiert. Die vereinigte organische Phase wurde zweimal mit gesättigter Kochsalzlösung gewaschen, nachfolgend mit Natriumsulfat getrocknet und nach Abfiltration im Vakuum eingeengt. Durch säulenchromatographische Reinigung des erhaltenen Rohproduktes an Kieselgel mit einem n-Heptan / Essigsäureethylester-Gradienten wurde Cyanmethyl-(2-{2-cyan-4-fluor-5-[3-methyl-2,6-dioxo-4-(trifluormethyl)-3,6-dihydropyrimidin-1 (2H)-yl]phenoxy}phenoxy)acetat (232 mg, 42 % der Theorie) in Form eines farblosen Öls erhalten. ¹H-NMR (CDCl₃ δ, ppm) 7.53 (d, 1H), 7.23 (m, 2H), 7.09 (dt, 1H), 6.90 (d, 1H), 6.68 (d, 1H), 6.29 (s, 1H), 4.75 (s, 2H), 4.71 (s, 2H), 3.50 (s, 3H).

In Analogie zu den oben angeführten und an entsprechender Stelle rezitierten Herstellungsbeispielen sowie unter Berücksichtigung der allgemeinen Angaben zur Herstellung von substituierten N-Heterocyclyl- und N-Heteroaryltetrahydropyrimidinonen erhält man die nachfolgend genannten Verbindungen:

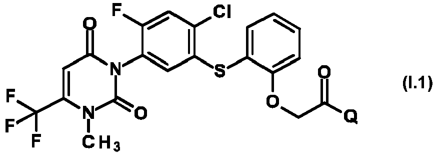

Tabelle 1.1: Bevorzugte Verbindungen der Formel (I.1) sind die Verbindungen 1.1-1 bis 1.1-406, worin Q die in der jeweiligen Zeile angegebenen Bedeutungen der Tabelle 1 hat. Die Verbindungen I.1-1 bis 1.1-406 der Tabelle 1.1 sind somit durch die Bedeutung der jeweiligen Einträge No. 1 bis 406 für Q der Tabelle 1 definiert.

**Tabelle 1:**

| No. | Q |
|---|---|
| 1 | Q-1 |
| 2 | Q-2 |
| 3 | Q-3 |
| 4 | Q-4 |
| 5 | Q-5 |
| 6 | Q-6 |
| 7 | Q-7 |
| 8 | Q-8 |
| 9 | Q-9 |
| 10 | Q-10 |
| 11 | Q-11 |
| 12 | Q-12 |
| 13 | Q-13 |
| 14 | Q-14 |
| 15 | Q-15 |
| 16 | Q-16 |
| 17 | Q-17 |
| 18 | Q-18 |
| 19 | Q-19 |
| 20 | Q-20 |
| 21 | Q-21 |
| 22 | Q-22 |
| 23 | Q-23 |
| 24 | Q-24 |
| 25 | Q-25 |
| 26 | Q-26 |
| 27 | Q-27 |
| 28 | Q-28 |
| 29 | Q-29 |
| 30 | Q-30 |
| 31 | Q-31 |
| 32 | Q-32 |
| 33 | Q-33 |
| 34 | Q-34 |
| 35 | Q-35 |
| 36 | Q-36 |
| 37 | Q-37 |
| 38 | Q-38 |
| 39 | Q-39 |
| 40 | Q-40 |
| 41 | Q-41 |
| 42 | Q-42 |
| 43 | Q-43 |
| 44 | Q-44 |
| 45 | Q-45 |
| 46 | Q-46 |
| 47 | Q-47 |
| 48 | Q-48 |
| 49 | Q-49 |
| 50 | Q-50 |
| 51 | Q-51 |
| 52 | Q-52 |
| 53 | Q-53 |
| 54 | Q-54 |
| 55 | Q-55 |
| 56 | Q-56 |
| 57 | Q-57 |
| 58 | Q-58 |
| 59 | Q-59 |
| 60 | Q-60 |
| 61 | Q-61 |
| 62 | Q-62 |
| 63 | Q-63 |
| 64 | Q-64 |
| 65 | Q-65 |
| 66 | Q-66 |
| 67 | Q-67 |
| 68 | Q-68 |
| 69 | Q-69 |
| 70 | Q-70 |
| 71 | Q-71 |
| 72 | Q-72 |
| 73 | Q-73 |
| 74 | Q-74 |
| 75 | Q-75 |
| 76 | Q-76 |
| 77 | Q-77 |
| 78 | Q-78 |
| 79 | Q-79 |
| 80 | Q-80 |
| 81 | Q-81 |
| 82 | Q-82 |
| 83 | Q-83 |
| 84 | Q-84 |
| 85 | Q-85 |
| 86 | Q-86 |
| 87 | Q-87 |
| 88 | Q-88 |
| 89 | Q-89 |
| 90 | Q-90 |
| 91 | Q-91 |
| 92 | Q-92 |
| 93 | Q-93 |
| 94 | Q-94 |
| 95 | Q-95 |
| 96 | Q-96 |
| 97 | Q-97 |
| 98 | Q-98 |
| 99 | Q-99 |
| 100 | Q-100 |
| 101 | Q-101 |
| 102 | Q-102 |
| 103 | Q-103 |
| 104 | Q-104 |
| 105 | Q-105 |
| 106 | Q-106 |
| 107 | Q-107 |
| 108 | Q-108 |
| 109 | Q-109 |
| 110 | Q-110 |
| 111 | Q-111 |
| 112 | Q-112 |
| 113 | Q-113 |
| 114 | Q-114 |
| 115 | Q-115 |
| 116 | Q-116 |
| 117 | Q-117 |
| 118 | Q-118 |
| 119 | Q-119 |
| 120 | Q-120 |
| 121 | Q-121 |
| 122 | Q-122 |
| 123 | Q-123 |
| 124 | Q-124 |
| 125 | Q-125 |
| 126 | Q-126 |
| 127 | Q-127 |
| 128 | Q-128 |
| 129 | Q-129 |
| 130 | Q-130 |
| 131 | Q-131 |
| 132 | Q-132 |
| 133 | Q-133 |
| 134 | Q-134 |
| 135 | Q-135 |
| 136 | Q-136 |
| 137 | Q-137 |
| 138 | Q-138 |
| 139 | Q-139 |
| 140 | Q-140 |
| 141 | Q-141 |
| 142 | Q-142 |
| 143 | Q-143 |
| 144 | Q-144 |
| 145 | Q-145 |
| 146 | Q-146 |
| 147 | Q-147 |
| 148 | Q-148 |
| 149 | Q-149 |
| 150 | Q-150 |
| 151 | Q-151 |
| 152 | Q-152 |
| 153 | Q-153 |
| 154 | Q-154 |
| 155 | Q-155 |
| 156 | Q-156 |
| 157 | Q-157 |
| 158 | Q-158 |
| 159 | Q-159 |
| 160 | Q-160 |
| 161 | Q-161 |
| 162 | Q-162 |
| 163 | Q-163 |
| 164 | Q-164 |
| 165 | Q-165 |
| 166 | Q-166 |
| 167 | Q-167 |
| 168 | Q-168 |
| 169 | Q-169 |
| 170 | Q-170 |
| 171 | Q-171 |
| 172 | Q-172 |
| 173 | Q-173 |
| 174 | Q-174 |
| 175 | Q-175 |
| 176 | Q-176 |
| 177 | Q-177 |
| 178 | Q-178 |
| 179 | Q-179 |
| 180 | Q-180 |
| 181 | Q-181 |
| 182 | Q-182 |
| 183 | Q-183 |
| 184 | Q-184 |
| 185 | Q-185 |
| 186 | Q-186 |
| 187 | Q-187 |
| 188 | Q-188 |
| 189 | Q-189 |
| 190 | Q-190 |
| 191 | Q-191 |
| 192 | Q-192 |
| 193 | Q-193 |
| 194 | Q-194 |
| 195 | Q-195 |
| 196 | Q-196 |
| 197 | Q-197 |
| 198 | Q-198 |
| 199 | Q-199 |
| 200 | Q-200 |
| 201 | Q-201 |
| 202 | Q-202 |
| 203 | Q-203 |
| 204 | Q-204 |
| 205 | Q-205 |
| 206 | Q-206 |
| 207 | Q-207 |
| 208 | Q-208 |
| 209 | Q-209 |
| 210 | Q-210 |
| 211 | Q-211 |
| 212 | Q-212 |
| 213 | Q-213 |
| 214 | Q-214 |
| 215 | Q-215 |
| 216 | Q-216 |
| 217 | Q-217 |
| 218 | Q-218 |
| 219 | Q-219 |
| 220 | Q-220 |
| 221 | Q-221 |
| 222 | Q-222 |
| 223 | Q-223 |
| 224 | Q-224 |
| 225 | Q-225 |
| 226 | Q-226 |
| 227 | Q-227 |
| 228 | Q-228 |
| 229 | Q-229 |
| 230 | Q-230 |
| 231 | Q-231 |
| 232 | Q-232 |
| 233 | Q-233 |
| 234 | Q-234 |
| 235 | Q-235 |
| 236 | Q-236 |
| 237 | Q-237 |
| 238 | Q-238 |
| 239 | Q-239 |
| 240 | Q-240 |
| 241 | Q-241 |
| 242 | Q-242 |
| 243 | Q-243 |
| 244 | Q-244 |
| 245 | Q-245 |
| 246 | Q-246 |
| 247 | Q-247 |
| 248 | Q-248 |
| 249 | Q-249 |
| 250 | Q-250 |
| 251 | Q-251 |
| 252 | Q-252 |
| 253 | Q-253 |
| 254 | Q-254 |
| 255 | Q-255 |
| 256 | Q-256 |
| 257 | Q-257 |
| 258 | Q-258 |
| 259 | Q-259 |
| 260 | Q-260 |
| 261 | Q-261 |
| 262 | Q-262 |
| 263 | Q-263 |
| 264 | Q-264 |
| 265 | Q-265 |
| 266 | Q-266 |
| 267 | Q-267 |
| 268 | Q-268 |
| 269 | Q-269 |
| 270 | Q-270 |
| 271 | Q-271 |
| 272 | Q-272 |
| 273 | Q-273 |
| 274 | Q-274 |
| 275 | Q-275 |
| 276 | Q-276 |
| 277 | Q-277 |
| 278 | Q-278 |
| 279 | Q-279 |
| 280 | Q-280 |
| 281 | Q-281 |
| 282 | Q-282 |
| 283 | Q-283 |
| 284 | Q-284 |
| 285 | Q-285 |
| 286 | Q-286 |
| 287 | Q-287 |
| 288 | Q-288 |
| 289 | Q-289 |
| 290 | Q-290 |
| 291 | Q-291 |
| 292 | Q-292 |
| 293 | Q-293 |
| 294 | Q-294 |
| 295 | Q-295 |
| 296 | Q-296 |
| 297 | Q-297 |
| 298 | Q-298 |
| 299 | Q-299 |
| 300 | Q-300 |
| 301 | Q-301 |
| 302 | Q-302 |
| 303 | Q-303 |
| 304 | Q-304 |
| 305 | Q-305 |
| 306 | Q-306 |
| 307 | Q-307 |
| 308 | Q-308 |
| 309 | Q-309 |
| 310 | Q-310 |
| 311 | Q-311 |
| 312 | Q-312 |
| 313 | Q-313 |
| 314 | Q-314 |
| 315 | Q-315 |
| 316 | Q-316 |
| 317 | Q-317 |
| 318 | Q-318 |
| 319 | Q-319 |
| 320 | Q-320 |
| 321 | Q-321 |
| 322 | Q-322 |
| 323 | Q-323 |
| 324 | Q-324 |
| 325 | Q-325 |
| 326 | Q-326 |
| 327 | Q-327 |
| 328 | Q-328 |
| 329 | Q-329 |
| 330 | Q-330 |
| 331 | Q-331 |
| 332 | Q-332 |
| 333 | Q-333 |
| 334 | Q-334 |
| 335 | Q-335 |
| 336 | Q-336 |
| 337 | Q-337 |
| 338 | Q-338 |
| 339 | Q-339 |
| 340 | Q-340 |
| 341 | Q-341 |
| 342 | Q-342 |
| 343 | Q-343 |
| 344 | Q-344 |
| 345 | Q-345 |
| 346 | Q-346 |
| 347 | Q-347 |
| 348 | Q-348 |
| 349 | Q-349 |
| 350 | Q-350 |
| 351 | Q-351 |
| 352 | Q-352 |
| 353 | Q-353 |
| 354 | Q-354 |
| 355 | Q-355 |
| 356 | Q-356 |
| 357 | Q-357 |
| 358 | Q-358 |
| 359 | Q-359 |
| 360 | Q-360 |
| 361 | Q-361 |
| 362 | Q-362 |
| 363 | Q-363 |
| 364 | Q-364 |
| 365 | Q-365 |
| 366 | Q-366 |
| 367 | Q-367 |
| 368 | Q-368 |
| 369 | Q-369 |
| 370 | Q-370 |
| 371 | Q-371 |
| 372 | Q-372 |
| 373 | Q-373 |
| 374 | Q-374 |
| 375 | Q-375 |
| 376 | Q-376 |
| 377 | Q-377 |
| 378 | Q-378 |
| 379 | Q-379 |
| 380 | Q-380 |
| 381 | Q-381 |
| 382 | Q-382 |
| 383 | Q-383 |
| 384 | Q-384 |
| 385 | Q-385 |
| 386 | Q-386 |
| 387 | Q-387 |
| 388 | Q-388 |
| 389 | Q-389 |
| 390 | Q-390 |
| 391 | Q-391 |
| 392 | Q-392 |
| 393 | Q-393 |
| 394 | Q-394 |
| 395 | Q-395 |
| 396 | Q-396 |
| 397 | Q-397 |
| 398 | Q-398 |
| 399 | Q-399 |
| 400 | Q-400 |
| 401 | Q-401 |
| 402 | Q-402 |
| 403 | Q-403 |
| 404 | Q-404 |
| 405 | Q-405 |
| 406 | Q-406 |

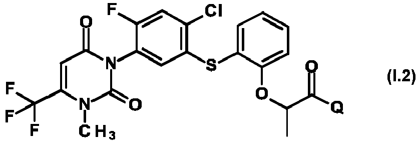

Tabelle I.2: Bevorzugte Verbindungen der Formel (I.2) sind die Verbindungen I2-1 bis I2-40₆, worin Q die in der jeweiligen Zeile angegebenen Bedeutungen der Tabelle 1 hat. Die Verbindungen 1.2-1 bis I.2-406 der Tabelle I.2 sind somit durch die Bedeutung der jeweiligen Einträge No. 1 bis 406 für Q der Tabelle 1 definiert.

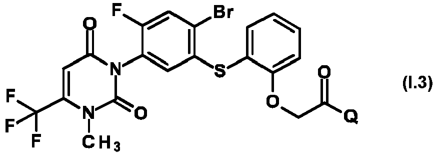

Tabelle I.3: Bevorzugte Verbindungen der Formel (I.3) sind die Verbindungen I.3-1 bis I.3-406, worin Q die in der jeweiligen Zeile angegebenen Bedeutungen der Tabelle 1 hat. Die Verbindungen I.3-1 bis I.3-406 der Tabelle I.3 sind somit durch die Bedeutung der jeweiligen Einträge No. 1 bis 406 für Q der Tabelle 1 definiert.

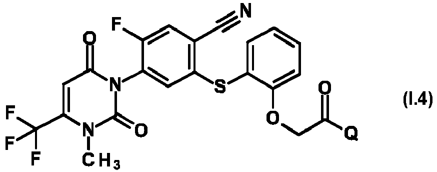

Tabelle I.4: Bevorzugte Verbindungen der Formel (I.4) sind die Verbindungen I.4-1 bis I.4-406, worin Q die in der jeweiligen Zeile angegebenen Bedeutungen der Tabelle 1 hat. Die Verbindungen 1.4-1 bis I.4-406 der Tabelle I.4 sind somit durch die Bedeutung der jeweiligen Einträge No. 1 bis 406 für Q der Tabelle 1 definiert.

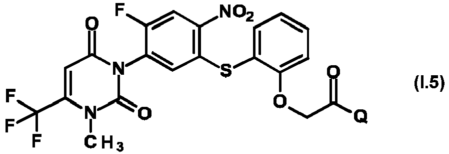

Tabelle I.5: Bevorzugte Verbindungen der Formel (I.5) sind die Verbindungen I.5-1 bis I.5-406, worin Q die in der jeweiligen Zeile angegebenen Bedeutungen der Tabelle 1 hat. Die Verbindungen 1.5-1 bis I.5-406 der Tabelle I.5 sind somit durch die Bedeutung der jeweiligen Einträge No. 1 bis 406 für Q der Tabelle 1 definiert.

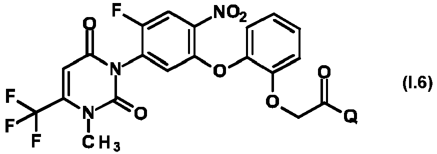

Tabelle I.6: Bevorzugte Verbindungen der Formel (I.6) sind die Verbindungen I.6-1 bis I.6-406, worin Q die in der jeweiligen Zeile angegebenen Bedeutungen der Tabelle 1 hat. Die Verbindungen I. 6-1 bis I.6-406 der Tabelle I.6 sind somit durch die Bedeutung der jeweiligen Einträge No. 1 bis 406 für Q der Tabelle 1 definiert.

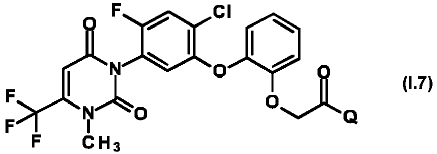

Tabelle I.7: Bevorzugte Verbindungen der Formel (I.7) sind die Verbindungen I.7-1 bis I.7-406, worin Q die in der jeweiligen Zeile angegebenen Bedeutungen der Tabelle 1 hat. Die Verbindungen I.7-1 bis I.7-406 der Tabelle I.7 sind somit durch die Bedeutung der jeweiligen Einträge No. 1 bis 406 für Q der Tabelle 1 definiert.

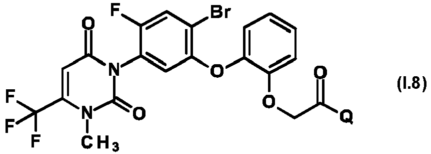

Tabelle I.8: Bevorzugte Verbindungen der Formel (I.8) sind die Verbindungen I.8-1 bis I.8-406, worin Q die in der jeweiligen Zeile angegebenen Bedeutungen der Tabelle 1 hat. Die Verbindungen 1.8-1 bis I.8-406 der Tabelle I.8 sind somit durch die Bedeutung der jeweiligen Einträge No. 1 bis 406 für Q der Tabelle 1 definiert.

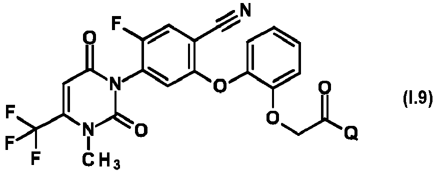

Tabelle I.9: Bevorzugte Verbindungen der Formel (I.9) sind die Verbindungen I.9-1 bis I.9-406, worin Q die in der jeweiligen Zeile angegebenen Bedeutungen der Tabelle 1 hat. Die Verbindungen I.9-1 bis I.9-406 der Tabelle I.9 sind somit durch die Bedeutung der jeweiligen Einträge No. 1 bis 406 für Q der Tabelle 1 definiert.

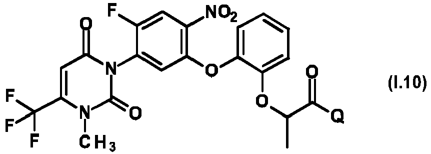

Tabelle I.10: Bevorzugte Verbindungen der Formel (I.10) sind die Verbindungen I.10-1 bis I.10-406, worin Q die in der jeweiligen Zeile angegebenen Bedeutungen der Tabelle 1 hat. Die Verbindungen I.10-1 bis I.10-406 der Tabelle I.10 sind somit durch die Bedeutung der jeweiligen Einträge No. 1 bis 406 für Q der Tabelle 1 definiert.

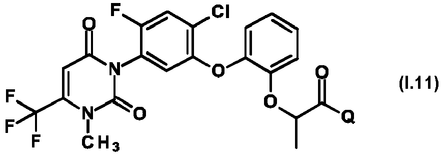

Tabelle I.11: Bevorzugte Verbindungen der Formel (I.11) sind die Verbindungen I.11-1 bis I.11-406, worin Q die in der jeweiligen Zeile angegebenen Bedeutungen der Tabelle 1 hat. Die Verbindungen I.11-1 bis 1. 11-406 der Tabelle 1.11 sind somit durch die Bedeutung der jeweiligen Einträge No. 1 bis 406 für Q der Tabelle 1 definiert.

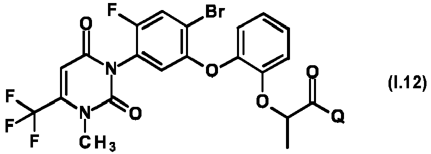

Tabelle 1.12: Bevorzugte Verbindungen der Formel (I.12) sind die Verbindungen 1.12-1 bis 1.12-406, worin Q die in der jeweiligen Zeile angegebenen Bedeutungen der Tabelle 1 hat. Die Verbindungen I.12-1 bis 1.12-406 der Tabelle 1.12 sind somit durch die Bedeutung der jeweiligen Einträge No. 1 bis 406 für Q der Tabelle 1 definiert.

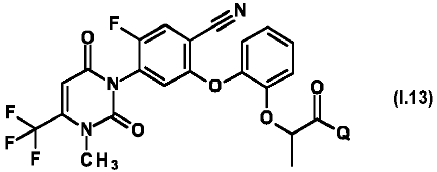

Tabelle I.13: Bevorzugte Verbindungen der Formel (I.13) sind die Verbindungen I.13-1 bis I.13-406, worin Q die in der jeweiligen Zeile angegebenen Bedeutungen der Tabelle 1 hat. Die Verbindungen I.13-1 bis 1.13-406 der Tabelle 1.13 sind somit durch die Bedeutung der jeweiligen Einträge No. 1 bis 406 für Q der Tabelle 1 definiert.

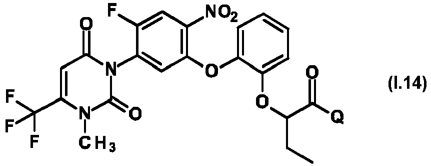

Tabelle 1.14: Bevorzugte Verbindungen der Formel (I.14) sind die Verbindungen 1.14-1 bis 1.14-406, worin Q die in der jeweiligen Zeile angegebenen Bedeutungen der Tabelle 1 hat. Die Verbindungen I.14-1 bis 1.14-406 der Tabelle 1.14 sind somit durch die Bedeutung der jeweiligen Einträge No. 1 bis 406 für Q der Tabelle 1 definiert.

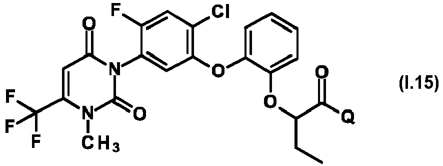

Tabelle 1.15: Bevorzugte Verbindungen der Formel (I.15) sind die Verbindungen 1.15-1 bis 1.15-406, worin Q die in der jeweiligen Zeile angegebenen Bedeutungen der Tabelle 1 hat. Die Verbindungen I.15-1 bis 1.15-406 der Tabelle 1.15 sind somit durch die Bedeutung der jeweiligen Einträge No. 1 bis 406 für Q der Tabelle 1 definiert.

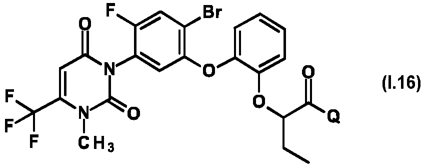

Tabelle 1.16: Bevorzugte Verbindungen der Formel (I.16) sind die Verbindungen 1.16-1 bis 1.16-406, worin Q die in der jeweiligen Zeile angegebenen Bedeutungen der Tabelle 1 hat. Die Verbindungen I.16-1 bis 1.16-406 der Tabelle 1.16 sind somit durch die Bedeutung der jeweiligen Einträge No. 1 bis 406 für Q der Tabelle 1 definiert.

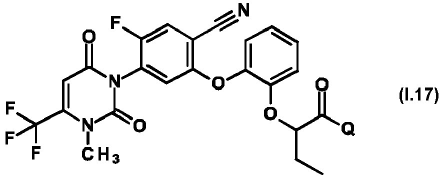

Tabelle 1.17: Bevorzugte Verbindungen der Formel (I.17) sind die Verbindungen 1.17-1 bis 1.17-406, worin Q die in der jeweiligen Zeile angegebenen Bedeutungen der Tabelle 1 hat. Die Verbindungen I.17-1 bis 1.17-406 der Tabelle 1.17 sind somit durch die Bedeutung der jeweiligen Einträge No. 1 bis 406 für Q der Tabelle 1 definiert.

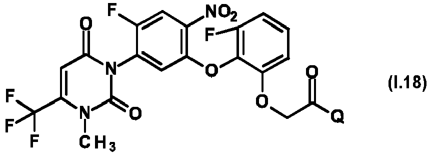

Tabelle 1.18: Bevorzugte Verbindungen der Formel (I.18) sind die Verbindungen I.18-1 bis 1.18-406, worin Q die in der jeweiligen Zeile angegebenen Bedeutungen der Tabelle 1 hat. Die Verbindungen I.18-1 bis 1.18-406 der Tabelle 1.18 sind somit durch die Bedeutung der jeweiligen Einträge No. 1 bis 406 für Q der Tabelle 1 definiert.

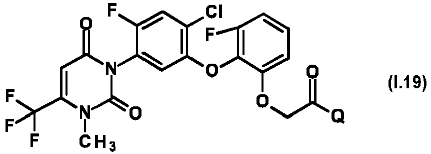

Tabelle 1.19: Bevorzugte Verbindungen der Formel (I.19) sind die Verbindungen 1.19-1 bis 1.19-406, worin Q die in der jeweiligen Zeile angegebenen Bedeutungen der Tabelle 1 hat. Die Verbindungen I.19-1 bis 1.19-406 der Tabelle 1.19 sind somit durch die Bedeutung der jeweiligen Einträge No. 1 bis 406 für Q der Tabelle 1 definiert.

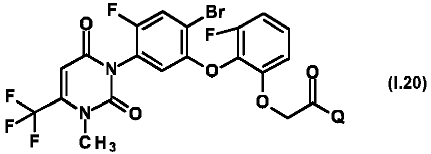

Tabelle I.20: Bevorzugte Verbindungen der Formel (I.20) sind die Verbindungen I.20-1 bis I.20-406, worin Q die in der jeweiligen Zeile angegebenen Bedeutungen der Tabelle 1 hat. Die Verbindungen I.20-1 bis I.20-406 der Tabelle I.20 sind somit durch die Bedeutung der jeweiligen Einträge No. 1 bis 406 für Q der Tabelle 1 definiert.

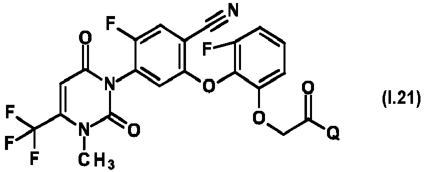

Tabelle I.21: Bevorzugte Verbindungen der Formel (I.21) sind die Verbindungen I.21-1 bis I.21-406, worin Q die in der jeweiligen Zeile angegebenen Bedeutungen der Tabelle 1 hat. Die Verbindungen I.21-1 bis I.21-406 der Tabelle I.21 sind somit durch die Bedeutung der jeweiligen Einträge No. 1 bis 406 für Q der Tabelle 1 definiert.

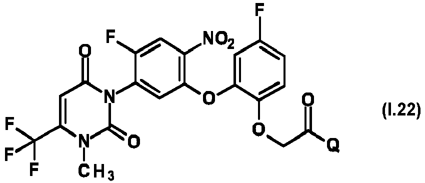

Tabelle I.22: Bevorzugte Verbindungen der Formel (I.22) sind die Verbindungen I.22-1 bis I22-406, worin Q die in der jeweiligen Zeile angegebenen Bedeutungen der Tabelle 1 hat. Die Verbindungen I.22-1 bis I.22-406 der Tabelle I.22 sind somit durch die Bedeutung der jeweiligen Einträge No. 1 bis 406 für Q der Tabelle 1 definiert.

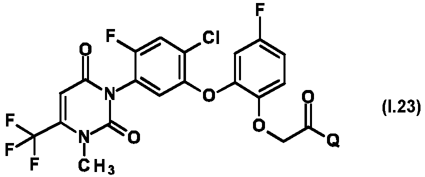

Tabelle I.23: Bevorzugte Verbindungen der Formel (I.23) sind die Verbindungen I.23-1 bis I.23-406, worin Q die in der jeweiligen Zeile angegebenen Bedeutungen der Tabelle 1 hat. Die Verbindungen I.23-1 bis I.23-406 der Tabelle I.23 sind somit durch die Bedeutung der jeweiligen Einträge No. 1 bis 406 für Q der Tabelle 1 definiert.

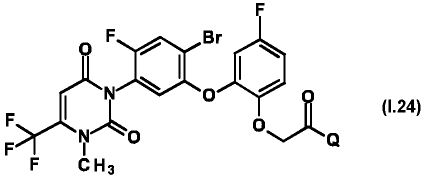

Tabelle I.24: Bevorzugte Verbindungen der Formel (I.24) sind die Verbindungen I.24-1 bis I24-406, worin Q die in der jeweiligen Zeile angegebenen Bedeutungen der Tabelle 1 hat. Die Verbindungen I.24-1 bis I.24-406 der Tabelle I.24 sind somit durch die Bedeutung der jeweiligen Einträge No. 1 bis 406 für Q der Tabelle 1 definiert.

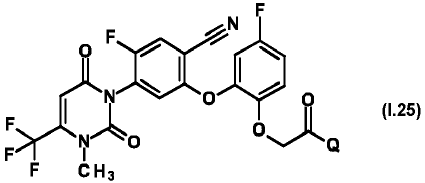

Tabelle I.25: Bevorzugte Verbindungen der Formel (I.25) sind die Verbindungen I.25-1 bis I25-406, worin Q die in der jeweiligen Zeile angegebenen Bedeutungen der Tabelle 1 hat. Die Verbindungen I.25-1 bis I.25-406 der Tabelle I.25 sind somit durch die Bedeutung der jeweiligen Einträge No. 1 bis 406 für Q der Tabelle 1 definiert.

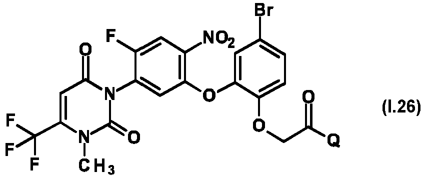

Tabelle I.26: Bevorzugte Verbindungen der Formel (I.26) sind die Verbindungen I26-1 bis I26-406, worin Q die in der jeweiligen Zeile angegebenen Bedeutungen der Tabelle 1 hat. Die Verbindungen I.26-1 bis I.22-406 der Tabelle I.26 sind somit durch die Bedeutung der jeweiligen Einträge No. 1 bis 406 für Q der Tabelle 1 definiert.

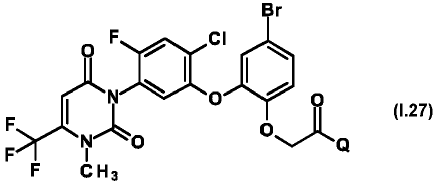

Tabelle I.27: Bevorzugte Verbindungen der Formel (I.27) sind die Verbindungen I.27-1 bis I27-406, worin Q die in der jeweiligen Zeile angegebenen Bedeutungen der Tabelle 1 hat. Die Verbindungen I.27-1 bis I.27-406 der Tabelle I.27 sind somit durch die Bedeutung der jeweiligen Einträge No. 1 bis 406 für Q der Tabelle 1 definiert.

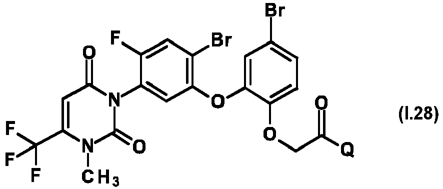

Tabelle I.28: Bevorzugte Verbindungen der Formel (I.28) sind die Verbindungen I.28-1 bis I28-406, worin Q die in der jeweiligen Zeile angegebenen Bedeutungen der Tabelle 1 hat. Die Verbindungen I.28-1 bis I.28-406 der Tabelle I.28 sind somit durch die Bedeutung der jeweiligen Einträge No. 1 bis 406 für Q der Tabelle 1 definiert.

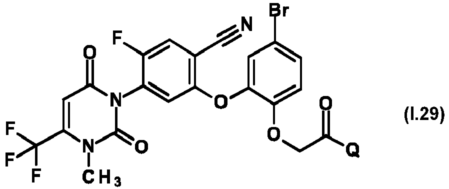

Tabelle I.29: Bevorzugte Verbindungen der Formel (I.29) sind die Verbindungen I.29-1 bis I29-406, worin Q die in der jeweiligen Zeile angegebenen Bedeutungen der Tabelle 1 hat. Die Verbindungen I.29-1 bis I.29-406 der Tabelle I.29 sind somit durch die Bedeutung der jeweiligen Einträge No. 1 bis 406 für Q der Tabelle 1 definiert.

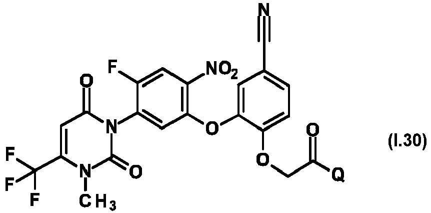

Tabelle I.30: Bevorzugte Verbindungen der Formel (I.30) sind die Verbindungen I.30-1 bis I.30-406, worin Q die in der jeweiligen Zeile angegebenen Bedeutungen der Tabelle 1 hat. Die Verbindungen I.30-1 bis I.30-406 der Tabelle I.30 sind somit durch die Bedeutung der jeweiligen Einträge No. 1 bis 406 für Q der Tabelle 1 definiert.

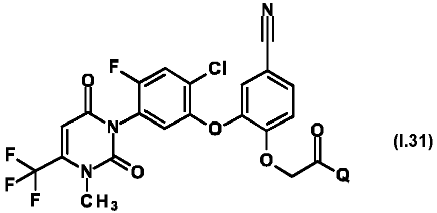

Tabelle I.31: Bevorzugte Verbindungen der Formel (I.31) sind die Verbindungen I.31-1 bis I.31-406, worin Q die in der jeweiligen Zeile angegebenen Bedeutungen der Tabelle 1 hat. Die Verbindungen I.31-1 bis I.31-406 der Tabelle I.31 sind somit durch die Bedeutung der jeweiligen Einträge No. 1 bis 406 für Q der Tabelle 1 definiert.

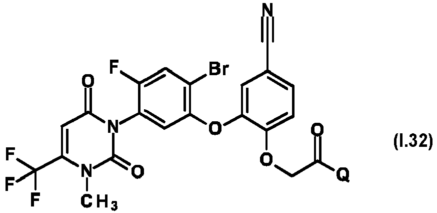

Tabelle I.32: Bevorzugte Verbindungen der Formel (I.32) sind die Verbindungen I.32-1 bis I.32-406, worin Q die in der jeweiligen Zeile angegebenen Bedeutungen der Tabelle 1 hat. Die Verbindungen I.32-1 bis I.32-406 der Tabelle I.32 sind somit durch die Bedeutung der jeweiligen Einträge No. 1 bis 406 für Q der Tabelle 1 definiert.

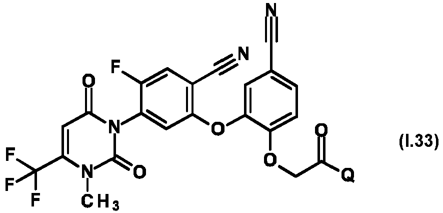

Tabelle I.33: Bevorzugte Verbindungen der Formel (I.33) sind die Verbindungen I.33-1 bis I.33-406, worin Q die in der jeweiligen Zeile angegebenen Bedeutungen der Tabelle 1 hat. Die Verbindungen I.33-1 bis I.33-406 der Tabelle I.33 sind somit durch die Bedeutung der jeweiligen Einträge No. 1 bis 406 für Q der Tabelle 1 definiert.

NMR-Daten ausgewählter Beispiele: Die ¹H-NMR-Daten ausgewählter Beispiele von Verbindungen der allgemeinen Formel (I) werden auf zwei verschiedene Weisen angegeben, und zwar (a) klassische NMR-Auswertung und Interpretation oder (b) in Form von 1H-NMR-Peaklisten nach der weiter unten beschriebenen Methode.

### a) klassische NMR-Interpretation

Bspl. I.7-23: ¹H-NMR (CDCl₃ δ, ppm): 7.36 (d, 1H), 7.14-7.10 (m, 1H), 7.07-7.04 (m, 1H), 7.01-6.99 (m, 1H), 6.94-6.91 (m, 1H), 6.77 (d, 1H), 6.28 (s, 1H), 4.69 (s, 2H), 4.31-4.29 (m, 2H), 3.69-3.67 (m, 2H), 3.61-3.59 (m, 2H), 3.52-3.50 (m, 2H), 3.37 (s, 3H).

Bspl. 1.7-121: ¹H-NMR (CDCl₃ δ, ppm): 7.37 (d, 1H), 7.14-7.10 (m, 1H), 7.07-7.05 (m, 1H), 7.02-6.98 (m, 1H), 6.91 (d, 1H), 6.74-6.71 (m, 1H), 6.28 (s, 1H), 5.36-5.33 (m, 1H), 4.65 (s, 2H), 3.88-3.77 (m, 4H), 3.50 (s, 3H), 2.17-2.10 (m, 1H), 1.99-1.95 (m, 1H).

Bspl. I.7-286: ¹H-NMR (CDCl₃ δ, ppm): 8.47 (d, 1H), 7.69 (d, 1H), 7.33 (d, 1H), 7.26-7.22 (m, 1H), 7.13-6.96 (m, 4H), 6.80 (d, 1H), 6.26 (s, 1H), 5.30 (s, 2H), 4.82 (s, 2H), 3.48 (s, 3H).

Bspl. I.7-335: ¹H-NMR (CDCl₃ δ, ppm): 8.65 (s, 1H), 7.75 (d, 1H), 7.67 (d, 1H), 7.32 (d, 1H), 7.12 (t, 1H), 7.07 (d, 1H), 7.03 (t, 1H), 6.93 (d, 1H), 6.68 (d, 1H), 6.28 (s, 1H), 5.24 (s, 2H), 4.71 (s, 2H), 3.50 (s, 3H).

Bspl. I.8-335: ¹H-NMR (CDCl₃ δ, ppm): 8.65 (s, 1H), 7.75 (d, 1H), 7.66 (d, 1H), 7.47 (d, 1H), 7.13 (t, 1H), 7.08 (d, 1H), 7.04 (t, 1H), 6.93 (d, 1H), 6.65 (d, 1H), 6.29 (s, 1H), 5.24 (s, 2H), 4.71 (s, 2H), 3.50 (s, 3H).

Bspl. I.9-367: ¹H-NMR (CDCl₃ δ, ppm): 7.46 (d, 1H), 7.22 (d, 1H), 7.15 (t, 1H), 7.07 (s, 1H), 7.03 (t, 1H), 6.85 (d, 1H), 6.80 (d, 1H), 6.25 (s, 1H), 5.19 (s, 2H), 4.68 (s, 2H), 3.48 (s, 3H), 2.69 (s, 3H).

Bspl. I.23-365: ¹H-NMR (CDCl₃ δ, ppm): 7.86 (s, 1H), 7.69 (s, 1H), 7.37 (d, 1H), 6.94-6.90 (m, 1H), 6.84 (d, 1H), 6.81-6.73 (m, 2H), 6.30 (s, 1H), 5.13 (s, 2H), 4.67 (s, 2H), 3.52 (s, 3H).

Bspl. I.23-367: ¹H-NMR (CDCl₃ δ, ppm): 7.36 (d, 1H), 7.11 (s, 1H), 6.95-6.90 (m, 1H), 6.86 (d, 1H), 6.79-6.74 (m, 2H), 6.29 (s, 1H), 5.23 (s, 2H), 4.68 (s, 2H), 3.51 (s, 3H), 2.71 (s, 3H).

Bspl. I.20-176: ¹H-NMR (CDCl₃ δ, ppm): 7.55 (d, 1H), 7.16-7.12 (m, 1H), 6.94-6.89 (m, 1H), 6.70-6.68 (m, 1H), 6.57 (d, 1H), 6.28 (s, 1H), 4.75 (s, 2H), 4.73 (s, 2H), 3.50 (s, 3H).

Bspl. I.20-1: ¹H-NMR (CDCl₃ δ, ppm): 7.52 (d, 1H), 7.11-7.08 (m, 1H), 6.88-6.85 (m, 1H), 6.69 (d, 1H), 6.68-6.66 (m, 1H), 6.26 (s, 1H), 4.69 (s, 2H), 4-28-4.25 (m, 2H), 3.55-3.52 (m, 2H), 3.49 (s, 3H), 3.32 (s, 3H).

### b) NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispiels hat daher die Form:
δ₁ (Intensität₁); δ₂ (Intensität₂);........; δᵢ (Intensitätᵢ);...... δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| 1.1-72: ¹H-NMR(400.0 MHz, CDCl3): |
| δ= 7.5190 (1.5); 7.4207 (1.6); 7.4169 (2.3); 7.4142 (1.8); 7.4015 (1.8); 7.3983 (2.4); 7.3949 (1.8); 7.3405 (1.7); 7.3335 (5.3); 7.3198 (2.2); 7.3108 (5.3); 7.3013 (2.1); 7.2969 (1.8); 7.2601 (268.7); 7.0646 (2.8); 7.0579 (2.9); 7.0460 (2.9); 7.0392 (2.8); 7.0209 (1.8); 7.0181 (1.9); 7.0019 (3.1); 6.9992 (3.2); 6.9962 (1.7); 6.9830 (1.6); 6.9803 (1.5); 6.7737 (2.9); 6.7531 (2.7); 6.2969 (4.7); 6.2768 (4.6); 4.6694 (16.0); 4.1622 (1.0); 4.1457 (1.1); 4.1403 (1.0); 4.1353 (1.5); 4.1309 (1.8); 4.1239 (1.0); 4.1190 (1.5); 4.1132 (3.0); 4.0968 (1.6); 4.0500 (1.6); 4.0295 (3.0); 4.0231 (1.0); 4.0090 (1.6); 4.0027 (2.0); 3.9822 (1.1); 3.8550 (0.9); 3.8413 (1.0); 3.8340 (2.0); 3.8205 (2.1); 3.8137 (1.5); 3.7998 (1.4); 3.7678 (1.2); 3.7645 (1.2); 3.7457 (3.7); 3.7276 (3.6); 3.7082 (1.9); 3.6871 (1.1); 3.5296 (1.3); 3.5167 (1.5); 3.5016 (12.4); 3.4987 (12.9); 3.4875 (1.3); 3.4742 (1.1); 2.5594 (0.8); 2.5426 (1.0); 2.5246 (0.8); 2.0450 (7.2); 2.0196 (0.8); 2.0064 (1.0); 1.9876 (1.1); 1.9743 (0.8); 1.5985 (0.6); 1.5798 (1.0); 1.5668 (1.4); 1.5411 (10.0); 1.5160 (0.7); 1.3037 (0.8); 1.2773 (3.0); 1.2595 (6.0); 1.2416 (2.2); 0.8991 (1.9); 0.8821 (6.5); 0.8643 (2.4); 0.1459 (0.6); 0.0080 (4.7); -0.0002 (160.7); -0.0086 (4.6); -0.1496 (0.6) |
| 1.2-72: ¹H-NMR(400.0 MHz, CDCl3): |
| δ= 7.3961 (0.6); 7.3926 (0.5); 7.3849 (0.8); 7.3802 (0.9); 7.3765 (0.7); 7.3735 (0.6); 7.3658 (0.8); 7.3369 (1.3); 7.3311 (1.4); 7.3143 (1.3); 7.3085 (1.4); 7.2970 (0.5); 7.2930 (0.6); 7.2781 (1.0); 7.2738 (1.2); 7.2600 (35.9); 7.2549 (0.9); 7.1025 (0.8); 7.0913 (0.8); 7.0839 (0.7); 7.0727 (0.8); 7.0105 (0.7); 6.9969 (0.8); 6.9918 (0.8); 6.9881 (1.0); 6.9854 (1.0); 6.9785 (0.8); 6.9691 (1.6); 6.9664 (1.6); 6.9502 (0.8); 6.9475 (0.7); 6.7229 (0.7); 6.7091 (0.8); 6.7013 (0.7); 6.6886 (0.7); 6.2969 (1.3); 6.2825 (2.2); 6.2685 (1.2); 4.7637 (1.0); 4.7467 (1.1); 4.7323 (0.9); 4.1304 (0.7); 4.1126 (0.6); 4.0548 (0.6); 4.0495 (0.7); 4.0380 (0.6); 4.0298 (0.7); 4.0061 (0.8); 3.9791 (0.5); 3.8202 (0.7); 3.8072 (0.6); 3.7992 (0.7); 3.7287 (0.8); 3.7235 (1.1); 3.7114 (1.1); 3.7057 (1.1); 3.7009 (1.0); 3.6921 (0.9); 3.6827 (0.7); 3.4952 (5.7); 3.4829 (0.8); 3.4694 (0.7); 3.4601 (0.9); 3.4468 (0.9); 2.0449 (1.7); 1.5412 (16.0); 1.5152 (4.7); 1.5109 (4.3); 1.4981 (4.5); 1.4940 (4.1); 1.2771 (1.0); 1.2647 (1.9); 1.2594 (2.0); 1.2415 (0.6); 0.8990 (1.0); 0.8820 (3.6); 0.8643 (1.4); 0.0079 (1.4); -0.0002 (46.0); -0.0085 (1.3) |
| 1.2-71: ¹H-NMR(400.0 MHz, CDCl3): |
| δ= 7.4207 (0.5); 7.4095 (0.6); 7.4051 (0.7); 7.4014 (0.6); 7.3904 (0.6); 7.3234 (1.1); 7.3183 (1.1); 7.3008 (1.6); 7.2959 (1.2); 7.2813 (0.8); 7.2600 (37.4); 7.1072 (0.6); 7.0972 (0.6); 7.0885 (0.6); 7.0785 (0.6); 7.0224 (0.6); 7.0186 (0.6); 7.0037 (0.6); 6.9999 (0.6); 6.9792 (0.6); 6.9614 (1.1); 6.9423 (0.5); 6.7456 (0.8); 6.7254 (0.5); 6.2736 (1.7); 6.2666 (1.0); 6.2610 (1.0); 4.7857 (0.6); 4.7695 (0.6); 4.7570 (0.5); 4.1854 (0.5); 4.1308 (0.7); 4.1135 (0.6); 4.0803 (0.6); 4.0682 (0.8); 4.0633 (0.9); 4.0440 (0.9); 3.7892 (0.7); 3.7726 (0.8); 3.7601 (0.5); 3.7557 (0.5); 3.4894 (3.1); 3.4864 (3.4); 2.0452 (1.3); 1.8965 (0.6); 1.8910 (0.6); 1.8837 (0.6); 1.8774 (0.7); 1.8716 (0.8); 1.8605 (0.5); 1.8545 (0.5); 1.5387 (16.0); 1.5148 (3.3); 1.4977 (3.2); 1.2773 (0.8); 1.2644 (1.7); 1.2597 (1.8); 1.2417 (0.5); 0.8990 (0.9); 0.8820 (3.1); 0.8643 (1.2); 0.0080 (1.5); -0.0002 (47.9); -0.0085 (1.2) |
| 1.2-23: ¹H-NMR(400.0 MHz, CDCl3): |
| δ= 7.4172 (1.0); 7.4130 (1.1); 7.4071 (1.0); 7.4029 (1.1); 7.3980 (1.1); 7.3938 (1.1); 7.3879 (1.0); 7.3837 (1.1); 7.3265 (1.9); 7.3209 (1.9); 7.3038 (2.4); 7.2985 (2.4); 7.2845 (1.3); 7.2826 (1.4); 7.2806 (1.3); 7.2602 (32.9); 7.0934 (1.8); 7.0748 (1.8); 7.0059 (1.7); 6.9873 (1.8); 6.9828 (1.1); 6.9803 (1.1); 6.9639 (1.9); 6.9614 (1.9); 6.9451 (0.9); 6.9425 (0.9); 6.7504 (1.0); 6.7377 (1.0); 6.7323 (1.0); 6.7194 (0.9); 6.2753 (5.0); 4.7680 (1.2); 4.7547 (1.2); 4.7509 (1.2); 4.7377 (1.2); 4.2900 (0.7); 4.2784 (2.4); 4.2658 (2.8); 4.2563 (1.9); 3.6811 (2.9); 3.6691 (3.7); 3.6571 (2.6); 3.6049 (0.8); 3.6009 (1.0); 3.5991 (1.1); 3.5946 (2.3); 3.5887 (2.5); 3.5847 (1.4); 3.5812 (2.8); 3.5745 (2.1); 3.5202 (2.5); 3.5125 (3.5); 3.5071 (2.4); 3.4907 (7.0); 3.4887 (6.7); 3.3717 (16.0); 3.3690 (16.0); 1.5447 (13.3); 1.5076 (8.9); 1.4906 (8.8); 1.2648 (0.8); 0.8820 (1.4); 0.8642 (0.6); 0.0080 (1.2); -0.0002 (42.0); -0.0085 (1.2) |
| 1.2-2: ¹H-NMR(400.0 MHz, CDCl3): |
| δ= 7.4253 (0.8); 7.4210 (0.8); 7.4179 (0.8); 7.4135 (0.8); 7.4061 (0.8); 7.4020 (0.9); 7.3985 (0.8); 7.3945 (0.8); 7.3237 (1.5); 7.3191 (1.5); 7.3010 (1.9); 7.2966 (2.0); 7.2778 (1.2); 7.2600 (44.7); 7.0984 (1.4); 7.0798 (1.4); 7.0118 (1.4); 6.9932 (1.4); 6.9804 (0.9); 6.9638 (1.5); 6.9612 (1.5); 6.9425 (0.7); 6.7493 (0.8); 6.7401 (0.8); 6.7311 (0.7); 6.7192 (0.8); 6.2735 (2.3); 6.2657 (2.3); 4.7719 (1.0); 4.7579 (1.0); 4.7548 (1.0); 4.7410 (1.0); 4.2748 (0.6); 4.2635 (1.8); 4.2570 (1.2); 4.2512 (2.4); 4.2415 (1.2); 3.6034 (2.4); 3.5914 (2.8); 3.5793 (2.1); 3.5096 (0.8); 3.5045 (0.9); 3.4921 (5.8); 3.4870 (7.0); 3.4746 (2.6); 3.4695 (2.6); 3.4571 (0.8); 3.4519 (0.9); 1.5378 (16.0); 1.5110 (7.3); 1.4939 (7.1); 1.2647 (1.3); 1.1993 (2.6); 1.1936 (2.7); 1.1818 (5.4); 1.1761 (5.4); 1.1643 (2.6); 1.1586 (2.6); 0.8989 (0.7); 0.8820 (2.2); 0.8643 (0.9); 0.0080 (1.8); -0.0002 (58.2); -0.0085 (1.6) |
| 1.2-58: ¹H-NMR(400.0 MHz, CDCI3): |
| δ= 7.5189 (0.8); 7.4260 (1.6); 7.4218 (1.9); 7.4163 (1.6); 7.4122 (1.8); 7.4068 (2.0); 7.4027 (2.0); 7.3971 (1.8); 7.3930 (1.9); 7.3435 (3.2); 7.3365 (3.3); 7.3206 (4.0); 7.3141 (4.2); 7.2996 (2.4); 7.2975 (2.3); 7.2954 (2.2); 7.2792 (1.6); 7.2749 (1.6); 7.2601 (152.7); 7.0526 (3.0); 7.0340 (3.1); 7.0079 (1.3); 7.0050 (2.4); 7.0020 (1.3); 6.9937 (3.1); 6.9890 (2.2); 6.9861 (3.8); 6.9832 (2.1); 6.9751 (3.1); 6.9700 (1.1); 6.9672 (1.9); 6.9643 (1.0); 6.7996 (1.6); 6.7750 (2.2); 6.7532 (1.6); 6.2920 (5.2); 6.2876 (4.9); 4.8190 (0.6); 4.8021 (1.9); 4.7850 (2.0); 4.7797 (2.0); 4.7626 (2.0); 4.7456 (0.6); 4.2560 (0.6); 4.2360 (1.3); 4.2266 (2.2); 4.2224 (2.5); 4.2182 (2.0); 4.2133 (4.7); 4.2089 (1.9); 4.2049 (2.6); 4.2027 (2.1); 4.1994 (1.8); 4.1932 (1.5); 4.1729 (0.6); 4.1486 (0.6); 4.1308 (1.8); 4.1131 (1.8); 4.0950 (0.6); 3.7542 (1.7); 3.7448 (3.2); 3.7352 (2.5); 3.5010 (13.1); 3.4981 (13.4); 2.0449 (8.6); 1.5454 (16.0); 1.5420 (15.1); 1.5284 (11.0); 1.5250 (10.3); 1.2843 (1.4); 1.2772 (3.6); 1.2594 (7.5); 1.2415 (2.8); 0.8990 (2.1); 0.8820 (7.3); 0.8643 (2.8); 0.0080 (4.6); -0.0002 (153.4); -0.0085 (4.3); -0.1496 (0.5) |
| 1.1-26: ¹H-NMR(400.0 MHz, CDCI3): |
| δ= 7.4316 (1.0); 7.4275 (1.1); 7.4125 (1.1); 7.4083 (1.1); 7.3405 (0.5); 7.3362 (0.5); 7.3253 (2.0); 7.3218 (0.8); 7.3199 (0.8); 7.3175 (0.8); 7.3158 (0.7); 7.3025 (2.1); 7.2969 (0.6); 7.2600 (24.5); 7.0896 (1.8); 7.0710 (1.8); 7.0140 (0.6); 7.0113 (0.7); 6.9952 (1.2); 6.9923 (1.2); 6.9762 (0.5); 6.9734 (0.6); 6.7728 (1.0); 6.7703 (1.0); 6.7521 (0.9); 6.7496 (0.9); 6.2872 (3.0); 4.6610 (5.8); 4.2291 (0.7); 4.2235 (0.8); 4.2127 (1.6); 4.2073 (1.6); 4.1965 (0.8); 4.1911 (0.8); 3.5000 (4.1); 3.4971 (4.2); 3.3932 (1.7); 3.3779 (3.6); 3.3626 (1.8); 3.2982 (16.0); 2.0069 (0.6); 1.8938 (1.5); 1.8781 (2.2); 1.8624 (1.5); 1.5395 (9.6); 0.0080 (0.9); -0.0002 (32.4); -0.0085 (0.9) |
| 1.1-23: ¹H-NMR(400.0 MHz, CDCI3): |
| δ= 7.4227 (1.0); 7.4186 (1.1); 7.4036 (1.1); 7.3994 (1.1); 7.3379 (0.6); 7.3336 (0.6); 7.3253 (2.0); 7.3191 (0.8); 7.3173 (0.8); 7.3150 (0.8); 7.3131 (0.7); 7.3027 (2.0); 7.2986 (0.8); 7.2943 (0.6); 7.2606 (7.8); 7.0826 (1.8); 7.0639 (1.8); 7.0115 (0.7); 7.0086 (0.7); 6.9925 (1.2); 6.9897 (1.2); 6.9736 (0.6); 6.9708 (0.6); 6.7872 (1.0); 6.7847 (1.0); 6.7665 (1.0); 6.7640 (0.9); 6.2781 (3.0); 4.6947 (5.7); 4.3023 (1.5); 4.2935 (1.1); 4.2904 (1.6); 4.2867 (1.0); 4.2782 (1.6); 3.7020 (1.8); 3.6933 (1.1); 3.6899 (1.8); 3.6870 (1.1); 3.6780 (1.6); 3.6251 (1.2); 3.6172 (1.4); 3.6144 (1.6); 3.6093 (1.4); 3.6026 (2.4); 3.5366 (2.5); 3.5300 (1.4); 3.5244 (1.6); 3.5222 (1.5); 3.5135 (1.2); 3.4979 (4.2); 3.4951 (4.3); 3.3763 (16.0); 1.2844 (0.5); 1.2550 (1.5); -0.0002 (10.2) |
| 1.1-71: ¹H-NMR(400.0 MHz, CDCl3): |
| δ= 7.4740 (16.0); 7.4228 (0.8); 7.4186 (0.9); 7.4036 (0.9); 7.3995 (1.0); 7.3473 (0.7); 7.3453 (0.7); 7.3431 (0.7); 7.3412 (0.6); 7.3351 (1.7); 7.3267 (0.6); 7.3224 (0.6); 7.3126 (1.6); 7.1128 (0.9); 7.1103 (0.9); 7.0943 (0.9); 7.0916 (0.9); 7.0228 (0.6); 7.0200 (0.6); 7.0039 (1.0); 7.0011 (1.0); 6.9849 (0.5); 6.8135 (0.9); 6.7928 (0.8); 6.2879 (1.5); 6.2823 (1.4); 4.7135 (3.6); 4.1625 (0.6); 4.0841 (1.0); 4.0806 (0.7); 4.0775 (0.6); 4.0678 (0.9); 4.0534 (0.5); 3.8216 (0.6); 3.8165 (0.5); 3.7678 (0.6); 3.4989 (3.5); 2.7526 (7.9); 2.5968 (2.3); 2.5921 (4.7); 2.5874 (6.5); 2.5827 (4.5); 2.5780 (2.0); 1.9141 (0.7); 1.9107 (0.8); 1.8944 (0.9); 1.8778 (0.6); 0.0080 (0.8); -0.0002 (21.7); -0.0085 (0.6) |
| 1.7-276: ¹H-NMR(400.6 MHz, CDCl3): |
| δ= 8.5693 (1.3); 8.5653 (1.7); 8.5623 (1.3); 8.5577 (1.2); 8.5547 (1.6); 8.5505 (1.4); 7.6829 (1.2); 7.6785 (1.2); 7.6636 (2.1); 7.6592 (2.3); 7.6444 (1.4); 7.6400 (1.4); 7.3311 (4.6); 7.3089 (4.6); 7.2654 (0.9); 7.2611 (74.1); 7.2554 (0.9); 7.2396 (3.5); 7.2382 (3.7); 7.2262 (1.6); 7.2249 (1.5); 7.2195 (3.8); 7.2074 (1.3); 7.1368 (1.1); 7.1323 (1.3); 7.1183 (1.6); 7.1166 (1.7); 7.1139 (2.0); 7.1122 (2.0); 7.0983 (1.8); 7.0938 (2.3); 7.0860 (2.1); 7.0816 (2.1); 7.0659 (3.9); 7.0616 (2.8); 7.0194 (2.1); 7.0157 (2.6); 7.0010 (2.0); 6.9995 (1.7); 6.9975 (2.5); 6.9957 (1.9); 6.9810 (1.1); 6.9774 (1.1); 6.9599 (2.9); 6.9563 (2.5); 6.9396 (2.3); 6.9361 (2.0); 6.7627 (4.3); 6.7463 (4.5); 6.2439 (7.6); 5.2819 (12.6); 4.7754 (16.0); 4.7671 (0.8); 3.4786 (10.2); 3.4756 (10.6); 2.0451 (2.3); 1.5909 (1.2); 1.2772 (1.0); 1.2641 (1.1); 1.2594 (2.1); 1.2415 (0.8); 0.8988 (0.6); 0.8819 (2.2); 0.8643 (0.8); 0.0080 (1.3); 0.0041 (0.6); -0.0002 (46.0); -0.0058 (0.7); -0.0085 (1.3) |
| 1.7-176: ¹H-NMR(400.6 MHz, CDCI3): |
| δ= 7.3991 (3.0); 7.3770 (3.1); 7.2607 (35.1); 7.1781 (0.8); 7.1734 (0.9); 7.1599 (1.2); 7.1579 (1.2); 7.1553 (1.4); 7.1533 (1.2); 7.1398 (1.2); 7.1351 (1.5); 7.1160 (1.0); 7.1114 (1.3); 7.0960 (2.7); 7.0914 (2.0); 7.0672 (1.7); 7.0636 (1.7); 7.0490 (1.4); 7.0471 (1.2); 7.0455 (1.4); 7.0435 (1.0); 7.0290 (0.7); 7.0253 (0.6); 6.9524 (1.8); 6.9488 (1.7); 6.9321 (1.6); 6.9286 (1.4); 6.6854 (2.9); 6.6691 (2.9); 6.2899 (5.0); 4.7595 (16.0); 4.7450 (12.5); 4.1305 (0.6); 4.1127 (0.6); 3.5050 (7.2); 3.5020 (7.2); 2.0450 (3.0); 1.5507 (6.0); 1.2772 (1.1); 1.2594 (2.1); 1.2415 (0.9); 0.8820 (1.2); 0.0080 (0.6); -0.0002 (22.3); -0.0084 (0.7) |
| 1.7-2: ¹H-NMR(400.2 MHz, d₆-DMSO): |
| δ= 7.8200 (3.9); 7.7969 (3.9); 7.2054 (0.8); 7.2006 (0.9); 7.1878 (1.1); 7.1843 (1.7); 7.1801 (1.6); 7.1672 (1.6); 7.1625 (1.7); 7.1165 (2.5); 7.1133 (2.8); 7.0959 (1.7); 7.0924 (1.6); 7.0844 (1.3); 7.0797 (1.6); 7.0645 (3.7); 7.0598 (3.0); 7.0458 (2.1); 7.0424 (1.9); 7.0282 (1.8); 7.0249 (2.0); 7.0084 (0.8); 7.0047 (0.7); 6.9635 (3.7); 6.9468 (3.8); 6.5141 (7.5); 5.7551 (4.4); 4.7852 (11.1); 4.1967 (3.0); 4.1884 (2.7); 4.1850 (3.4); 4.1811 (2.6); 4.1731 (3.3); 3.5366 (3.6); 3.5283 (2.5); 3.5247 (3.6); 3.5130 (3.2); 3.4239 (2.3); 3.4064 (7.4); 3.3889 (7.6); 3.3714 (2.8); 3.3558 (10.5); 3.3270 (108.8); 2.6753 (0.5); 2.6707 (0.7); 2.6661 (0.5); 2.5242 (1.8); 2.5195 (2.8); 2.5108 (43.0); 2.5063 (87.8); 2.5018 (115.2); 2.4971 (81.0); 2.4926 (37.6); 2.3332 (0.5); 2.3286 (0.7); 2.3240 (0.5); 1.9796 (1.0); 1.2353 (0.4); 1.0801 (7.8); 1.0627 (16.0); 1.0451 (7.5); -0.0002 (2.7) |
| 1.9-1: ¹H-NMR(400.6 MHz, CDCl3): |
| δ= 7.5023 (1.5); 7.4812 (1.5); 7.2605 (20.7); 7.2282 (0.7); 7.2241 (1.0); 7.2152 (0.6); 7.2084 (0.8); 7.2046 (1.3); 7.1959 (1.0); 7.1908 (0.6); 7.1758 (0.8); 7.1717 (0.6); 7.0552 (0.7); 7.0516 (0.8); 7.0358 (1.1); 7.0322 (1.0); 7.0165 (0.5); 6.8821 (0.9); 6.8786 (0.9); 6.8616 (0.8); 6.8583 (0.8); 6.8085 (1.4); 6.7940 (1.5); 6.2670 (2.6); 4.6660 (4.2); 4.2621 (0.9); 4.2596 (0.9); 4.2527 (1.0); 4.2510 (1.0); 4.2476 (1.0); 4.2454 (1.0); 4.2388 (1.0); 4.2362 (0.9); 3.5452 (1.8); 3.5335 (1.8); 3.5218 (1.7); 3.4892 (3.7); 3.4862 (3.8); 3.3156 (16.0); 1.5412 (8.4); 0.0080 (0.9); -0.0002 (33.1); -0.0085 (0.9) |
| 1.7-41: ¹H-NMR(400.6 MHz, CDCl3): |
| δ= 7.3781 (4.2); 7.3559 (4.2); 7.2602 (38.3); 7.1490 (1.0); 7.1444 (1.1); 7.1306 (1.5); 7.1287 (1.5); 7.1261 (1.8); 7.1242 (1.6); 7.1105 (1.6); 7.1060 (1.9); 7.0834 (1.5); 7.0788 (1.8); 7.0633 (3.6); 7.0588 (2.8); 7.0303 (2.1); 7.0267 (2.3); 7.0120 (1.9); 7.0101 (1.6); 7.0085 (1.9); 7.0067 (1.4); 6.9921 (0.9); 6.9884 (0.9); 6.9363 (2.6); 6.9328 (2.4); 6.9160 (2.2); 6.9125 (1.9); 6.7255 (4.1); 6.7091 (4.1); 6.2769 (7.1); 4.7037 (16.0); 4.6945 (0.6); 4.3814 (2.2); 4.3701 (2.9); 4.3582 (2.7); 4.1411 (4.9); 4.1360 (1.2); 4.1318 (2.8); 4.1292 (4.7); 4.1231 (1.1); 4.1176 (4.0); 4.1128 (0.6); 3.4984 (9.9); 3.4955 (9.9); 2.0448 (1.9); 1.5500 (1.3); 1.2770 (0.9); 1.2728 (0.7); 1.2646 (1.2); 1.2593 (1.9); 1.2551 (0.8); 1.2414 (0.6); 0.8990 (0.6); 0.8820 (2.2); 0.8644 (0.8); 0.0079 (0.7); -0.0002 (24.0); -0.0085 (0.7) |
| 1.7-278: ¹H-NMR(400.6 MHz, CDCl3): |
| δ= 8.5830 (2.1); 8.5700 (2.1); 7.5193 (0.6); 7.3136 (4.2); 7.2916 (4.2); 7.2674 (0.7); 7.2666 (0.7); 7.2607 (106.8); 7.1909 (3.2); 7.1762 (3.1); 7.1503 (1.0); 7.1455 (1.2); 7.1322 (1.5); 7.1301 (1.5); 7.1275 (1.9); 7.1254 (1.7); 7.1121 (1.6); 7.1073 (2.1); 7.0927 (1.4); 7.0881 (1.7); 7.0726 (3.8); 7.0680 (2.8); 7.0469 (2.2); 7.0432 (2.6); 7.0288 (1.8); 7.0268 (1.5); 7.0253 (1.9); 7.0232 (1.5); 7.0088 (0.9); 7.0051 (0.9); 6.9972 (0.6); 6.9502 (2.5); 6.9466 (2.4); 6.9299 (2.1); 6.9264 (2.0); 6.7028 (3.9); 6.6864 (4.0); 6.2559 (7.0); 5.1861 (7.2); 4.7523 (16.0); 3.4885 (9.6); 3.4855 (10.2); 2.1716 (3.8); 2.0452 (1.3); 1.2773 (0.7); 1.2639 (1.2); 1.2595 (1.5); 0.8989 (0.6); 0.8820 (2.2); 0.8642 (0.8); 0.0080 (1.9); -0.0002 (68.0); -0.0085 (2.1) |
| 1.7-277: ¹H-NMR(400.2 MHz, d₆-DMSO): |
| δ= 8.5668 (4.0); 8.5624 (4.0); 8.5424 (2.9); 8.5384 (3.0); 8.5305 (3.1); 8.5264 (3.0); 8.3150 (0.5); 7.7915 (6.0); 7.7684 (6.0); 7.7509 (1.6); 7.7457 (2.4); 7.7410 (1.5); 7.7312 (1.8); 7.7259 (2.7); 7.7215 (1.7); 7.4052 (2.4); 7.4035 (2.4); 7.3931 (2.4); 7.3914 (2.4); 7.3856 (2.2); 7.3839 (2.2); 7.3736 (2.1); 7.3719 (2.1); 7.1993 (0.9); 7.1946 (1.0); 7.1821 (1.1); 7.1784 (2.8); 7.1738 (2.4); 7.1617 (2.8); 7.1572 (3.2); 7.1466 (3.9); 7.1423 (4.6); 7.1258 (1.9); 7.1218 (1.3); 7.0877 (1.6); 7.0831 (2.3); 7.0676 (5.1); 7.0634 (4.0); 7.0497 (3.0); 7.0455 (2.8); 7.0326 (2.4); 7.0287 (2.8); 7.0254 (1.2); 7.0130 (1.1); 7.0085 (1.1); 6.9453 (5.7); 6.9286 (5.8); 6.5024 (11.5); 5.7552 (12.8); 5.1930 (16.0); 4.8511 (14.8); 3.3411 (16.8); 3.3293 (276.4); 3.1752 (0.4); 3.1620 (0.4); 2.6799 (0.3); 2.6754 (0.7); 2.6709 (1.1); 2.6663 (0.8); 2.6616 (0.3); 2.5244 (2.7); 2.5197 (4.2); 2.5110 (65.0); 2.5065 (134.8); 2.5019 (178.0); 2.4973 (125.5); 2.4927 (58.4); 2.3378 (0.4); 2.3333 (0.8); 2.3288 (1.0); 2.3241 (0.8); 2.3197 (0.4); -0.0002 (3.9) |
| 1.7-291: ¹H-NMR(400.6 MHz, CDCl3): |
| δ= 9.1487 (1.2); 9.1423 (1.2); 7.5195 (0.6); 7.4664 (1.8); 7.4547 (2.2); 7.4508 (2.7); 7.4458 (2.6); 7.4297 (0.7); 7.3276 (4.3); 7.3057 (4.3); 7.2882 (0.5); 7.2610 (105.3); 7.2529 (0.9); 7.2512 (0.8); 7.1562 (1.0); 7.1516 (1.2); 7.1379 (1.5); 7.1360 (1.6); 7.1333 (1.9); 7.1315 (1.8); 7.1178 (1.7); 7.1132 (2.1); 7.0885 (1.6); 7.0840 (1.9); 7.0685 (3.7); 7.0640 (2.9); 7.0362 (2.1); 7.0326 (2.5); 7.0180 (1.9); 7.0163 (1.5); 7.0144 (2.0); 7.0127 (1.6); 6.9977 (1.3); 6.9944 (1.0); 6.9717 (2.7); 6.9682 (2.4); 6.9515 (2.2); 6.9480 (2.0); 6.6995 (4.1); 6.6832 (4.1); 6.2643 (7.3); 5.5141 (7.7); 5.5114 (8.0); 4.7744 (16.0); 4.7157 (0.5); 3.4946 (10.0); 3.4917 (10.4); 2.1777 (1.1); 2.1717 (5.7); 2.0452 (0.8); 1.2643 (0.9); 1.2596 (1.1); 0.8819 (1.8); 0.8643 (0.7); 0.0079 (1.9); -0.0002 (64.1); -0.0085 (2.0) |
| 1.7-115: ¹H-NMR(400.6 MHz, CDCl3): |
| δ= 7.3890 (4.1); 7.3669 (4.1); 7.2606 (39.0); 7.1594 (0.9); 7.1548 (1.1); 7.1411 (1.5); 7.1392 (1.5); 7.1365 (1.8); 7.1347 (1.5); 7.1210 (1.4); 7.1164 (1.7); 7.0956 (1.4); 7.0911 (1.7); 7.0755 (3.5); 7.0710 (2.6); 7.0433 (1.9); 7.0396 (2.1); 7.0250 (1.8); 7.0230 (1.6); 7.0214 (1.8); 7.0198 (1.3); 7.0050 (0.8); 7.0014 (0.8); 6.9456 (2.4); 6.9420 (2.3); 6.9253 (2.1); 6.9218 (1.9); 6.7145 (3.9); 6.6981 (3.9); 6.2819 (6.9); 5.4984 (1.1); 5.4958 (1.1); 5.4826 (2.2); 5.4694 (1.2); 5.4668 (1.2); 5.4537 (0.5); 4.8761 (2.5); 4.8577 (4.0); 4.8550 (3.1); 4.8389 (2.7); 4.6917 (16.0); 4.6268 (1.6); 4.6230 (1.8); 4.6201 (1.4); 4.6138 (1.6); 4.6074 (2.6); 4.6026 (1.4); 4.6012 (1.4); 4.5945 (1.4); 4.5921 (1.5); 4.5881 (1.2); 3.5011 (9.7); 3.4984 (9.6); 2.1711 (1.4); 1.5530 (5.1); 1.2650 (1.0); 0.8990 (0.5); 0.8821 (1.7); 0.8644 (0.7); 0.0080 (0.7); -0.0002 (23.5); -0.0085 (0.6) |
| 1.7-356: ¹H-NMR(400.2 MHz, d₆-DMSO): |
| δ= 7.7947 (2.6); 7.7716 (2.6); 7.5769 (4.4); 7.1853 (0.6); 7.1798 (0.6); 7.1686 (0.7); 7.1637 (1.2); 7.1595 (1.0); 7.1477 (1.0); 7.1423 (1.1); 7.0728 (1.9); 7.0652 (0.6); 7.0598 (1.0); 7.0550 (1.3); 7.0519 (1.3); 7.0455 (2.6); 7.0398 (2.5); 7.0348 (1.6); 7.0207 (1.2); 7.0176 (1.4); 7.0009 (0.4); 6.9976 (0.4); 6.9548 (2.5); 6.9381 (2.6); 6.5087 (5.0); 5.7533 (7.5); 4.9652 (8.4); 4.7377 (7.3); 3.7087 (16.0); 3.3478 (328.3); 2.6766 (0.4); 2.6720 (0.6); 2.6673 (0.4); 2.5254 (1.4); 2.5204 (2.1); 2.5119 (34.8); 2.5075 (72.7); 2.5030 (96.8); 2.4985 (69.9); 2.4940 (33.8); 2.3343 (0.4); 2.3298 (0.6); 2.3254 (0.4); 2.0741 (15.8); -0.0002 (0.5) |
| 1.7-394: ¹H-NMR(400.2 MHz, d₆-DMSO): |
| δ= 8.3127 (0.3); 7.8198 (5.4); 7.7967 (5.5); 7.2189 (1.2); 7.2140 (1.4); 7.1975 (2.8); 7.1936 (2.4); 7.1806 (2.2); 7.1759 (2.4); 7.1150 (4.4); 7.0970 (3.0); 7.0942 (2.8); 7.0861 (1.8); 7.0814 (2.2); 7.0662 (5.2); 7.0616 (4.6); 7.0496 (3.2); 7.0465 (3.1); 7.0319 (2.9); 7.0291 (3.2); 7.0123 (1.2); 7.0089 (1.1); 6.9526 (5.4); 6.9359 (5.4); 6.5432 (0.3); 6.5118 (10.7); 5.7534 (7.2); 5.0458 (0.4); 5.0366 (3.3); 5.0277 (6.9); 5.0189 (3.3); 4.8538 (0.6); 4.8170 (16.0); 4.1382 (0.8); 4.1292 (0.8); 4.1237 (0.5); 4.1088 (5.7); 4.1006 (9.7); 4.0924 (5.5); 4.0718 (0.7); 4.0629 (0.7); 3.8509 (1.0); 3.8460 (1.5); 3.8409 (1.4); 3.8346 (2.3); 3.8292 (4.7); 3.8220 (4.8); 3.8160 (5.7); 3.8112 (3.1); 3.8062 (3.6); 3.7995 (2.8); 3.7912 (5.3); 3.7858 (5.3); 3.7813 (5.0); 3.7768 (4.0); 3.7730 (4.0); 3.7654 (1.2); 3.7568 (1.1); 3.3922 (1.5); 3.3436 (666.4); 3.1755 (0.5); 3.1624 (0.4); 2.6762 (0.8); 2.6718 (1.1); 2.6675 (0.8); 2.5112 (74.2); 2.5072 (141.9); 2.5028 (182.1); 2.4983 (134.0); 2.3339 (0.8); 2.3296 (1.1); 2.3251 (0.8); -0.0002 (1.5) |
| 1.7-72: ¹H-NMR(400.2 MHz, d₆-DMSO): |
| δ= 8.3126 (0.4); 7.8184 (6.1); 7.7953 (6.1); 7.2183 (1.2); 7.2138 (1.3); 7.1971 (2.9); 7.1934 (2.6); 7.1800 (2.5); 7.1755 (2.8); 7.1381 (4.0); 7.1347 (4.6); 7.1175 (2.6); 7.1140 (2.4); 7.0947 (2.0); 7.0903 (2.5); 7.0747 (5.0); 7.0704 (4.3); 7.0490 (3.0); 7.0456 (2.9); 7.0311 (2.8); 7.0281 (3.2); 7.0115 (1.4); 7.0079 (1.3); 6.9472 (6.0); 6.9305 (6.0); 6.5072 (9.2); 5.7535 (8.8); 4.8306 (0.6); 4.7867 (16.0); 4.0760 (1.1); 4.0661 (1.0); 4.0594 (1.2); 4.0493 (2.8); 4.0393 (2.1); 4.0326 (2.0); 4.0227 (2.0); 3.9972 (2.1); 3.9882 (2.0); 3.9773 (2.1); 3.9688 (2.4); 3.9615 (1.2); 3.9504 (1.1); 3.9415 (1.1); 3.7074 (1.2); 3.6933 (1.4); 3.6870 (3.0); 3.6729 (3.1); 3.6668 (2.1); 3.6528 (2.0); 3.6435 (2.5); 3.6222 (3.4); 3.6105 (2.1); 3.6040 (3.1); 3.5914 (3.4); 3.5735 (3.0); 3.5534 (1.3); 3.3870 (3.3); 3.3809 (4.0); 3.3730 (5.3); 3.3462 (870.4); 3.1756 (0.6); 3.1625 (0.5); 2.6809 (0.4); 2.6766 (0.9); 2.6720 (1.2); 2.6675 (0.9); 2.6631 (0.4); 2.5253 (3.5); 2.5204 (5.6); 2.5120 (75.1); 2.5075 (152.8); 2.5030 (200.4); 2.4984 (142.5); 2.4940 (67.6); 2.4741 (1.6); 2.4556 (1.7); 2.4385 (1.2); 2.4207 (0.6); 2.3389 (0.4); 2.3343 (0.9); 2.3298 (1.2); 2.3252 (0.8); 1.9371 (0.7); 1.9230 (0.8); 1.9168 (1.2); 1.9045 (1.4); 1.8965 (0.9); 1.8921 (1.0); 1.8854 (1.5); 1.8717 (1.3); 1.8656 (0.9); 1.8514 (0.7); 1.5429 (0.8); 1.5264 (1.3); 1.5113 (1.7); 1.4921 (1.7); 1.4773 (1.1); 1.4606 (0.7); -0.0002 (0.8) |
| 1.7-179: ¹H-NMR(400.2 MHz, d₆-DMSO): |
| δ= 8.3122 (0.4); 7.8187 (5.4); 7.7956 (5.5); 7.2124 (0.9); 7.2078 (1.0); 7.1915 (2.7); 7.1871 (2.3); 7.1748 (2.7); 7.1704 (3.0); 7.1598 (4.0); 7.1556 (4.6); 7.1391 (1.8); 7.1351 (1.4); 7.0978 (1.7); 7.0932 (2.2); 7.0777 (4.8); 7.0735 (4.0); 7.0585 (2.8); 7.0544 (2.6); 7.0414 (2.3); 7.0376 (2.7); 7.0219 (1.1); 7.0174 (1.1); 6.9510 (5.4); 6.9343 (5.4); 6.5443 (0.3); 6.5140 (10.5); 5.7532 (7.3); 4.8551 (0.6); 4.8103 (16.0); 4.2862 (5.1); 4.2712 (10.2); 4.2561 (5.2); 3.4082 (0.5); 3.3924 (1.4); 3.3467 (706.0); 3.1758 (0.8); 3.1627 (0.7); 2.8933 (5.6); 2.8783 (10.4); 2.8633 (5.3); 2.6811 (0.4); 2.6766 (0.7); 2.6720 (1.0); 2.6675 (0.7); 2.6631 (0.3); 2.5254 (2.7); 2.5206 (4.1); 2.5120 (63.6); 2.5076 (129.7); 2.5030 (169.6); 2.4985 (120.3); 2.4940 (56.8); 2.3388 (0.4); 2.3344 (0.7); 2.3298 (1.0); 2.3252 (0.8); 2.3207 (0.4); -0.0001 (1.0) |
| 1.6-41: ¹H-NMR(400.6 MHz, CDCl3): |
| δ= 7.8876 (1.8); 7.8661 (1.8); 7.2606 (86.1); 7.2177 (0.7); 7.2138 (1.6); 7.1952 (2.0); 7.1933 (2.5); 7.1886 (0.7); 7.1736 (1.0); 7.1695 (0.6); 7.0718 (0.9); 7.0682 (0.9); 7.0524 (1.1); 7.0488 (1.2); 7.0330 (0.6); 7.0295 (0.5); 6.9065 (1.1); 6.9033 (0.9); 6.8861 (1.1); 6.8810 (2.0); 6.8658 (1.8); 6.2766 (3.4); 4.6734 (6.6); 4.3621 (1.0); 4.3509 (1.4); 4.3390 (1.3); 4.1362 (2.3); 4.1310 (0.6); 4.1271 (1.3); 4.1242 (2.2); 4.1183 (0.5); 4.1128 (1.9); 3.5007 (4.8); 3.4978 (4.8); 1.5426 (16.0); 0.0080 (1.3); -0.0002 (53.5); -0.0085 (1.5) |
| 1.6-276: ¹H-NMR(400.6 MHz, CDCl3): |
| δ= 8.5648 (1.9); 8.5605 (2.5); 8.5505 (2.5); 8.5466 (1.8); 7.8353 (5.1); 7.8138 (5.0); 7.7077 (1.2); 7.7032 (1.2); 7.6884 (2.6); 7.6841 (2.3); 7.6692 (1.4); 7.6647 (1.3); 7.5196 (0.6); 7.3962 (0.6); 7.2611 (104.1); 7.2522 (2.4); 7.2413 (4.9); 7.2326 (1.9); 7.2220 (4.6); 7.2149 (3.0); 7.2109 (3.5); 7.1949 (4.2); 7.1911 (4.9); 7.1752 (3.2); 7.1710 (2.2); 7.1550 (2.6); 7.1508 (1.9); 7.0549 (2.4); 7.0513 (2.5); 7.0355 (3.4); 7.0320 (3.1); 7.0161 (1.6); 7.0126 (1.5); 6.9975 (0.6); 6.9453 (5.0); 6.9304 (8.2); 6.9104 (3.0); 6.9071 (2.5); 6.2323 (9.6); 5.6502 (1.6); 5.2532 (15.0); 4.7542 (16.0); 4.1308 (0.5); 4.1130 (0.6); 3.4747 (13.9); 3.4719 (13.3); 2.0452 (2.5); 1.6105 (0.5); 1.2773 (0.8); 1.2595 (1.7); 1.2416 (0.8); 0.0080 (1.8); 0.0063 (0.6); -0.0002 (63.7); -0.0085 (2.1) |
| 1.6-23: ¹H-NMR(400.6 MHz, CDCl3): |
| δ= 7.8800 (1.6); 7.8584 (1.6); 7.2609 (47.9); 7.2040 (1.6); 7.1850 (2.9); 7.1661 (0.9); 7.1620 (0.5); 7.0526 (0.7); 7.0491 (0.8); 7.0342 (0.7); 7.0297 (1.1); 6.9504 (1.6); 6.9354 (1.6); 6.9034 (1.0); 6.8832 (1.0); 6.8799 (0.6); 6.2812 (3.2); 4.6639 (5.0); 4.2800 (1.4); 4.2682 (1.5); 4.2644 (1.1); 4.2560 (1.6); 3.6798 (1.8); 3.6677 (1.8); 3.6558 (1.7); 3.6048 (1.1); 3.5959 (1.6); 3.5941 (1.6); 3.5881 (1.5); 3.5817 (2.6); 3.5217 (2.6); 3.5153 (1.5); 3.5091 (1.7); 3.5074 (1.6); 3.4994 (5.4); 3.3672 (16.0); 1.5495 (11.9); 0.0079 (0.8); -0.0002 (28.0); -0.0085 (0.8) |
| 1.6-2: ¹H-NMR(400.6 MHz, CDCl3): |
| δ= 7.8796 (0.8); 7.8580 (0.8); 7.2606 (87.6); 7.2052 (0.6); 7.1860 (0.7); 7.1822 (0.7); 7.0326 (0.6); 6.9970 (0.5); 6.9606 (0.8); 6.9455 (0.8); 6.2777 (1.5); 4.6715 (2.4); 4.2627 (0.7); 4.2511 (0.7); 4.2387 (0.8); 3.5981 (1.0); 3.5889 (0.6); 3.5861 (0.9); 3.5833 (0.5); 3.5741 (0.9); 3.5032 (1.3); 3.4994 (2.1); 3.4965 (2.1); 3.4859 (1.8); 3.4685 (1.8); 3.4510 (0.6); 1.5434 (16.0); 1.2195 (0.6); 1.1910 (1.9); 1.1735 (3.8); 1.1560 (1.8); 0.0079 (1.4); -0.0002 (53.4); - 0.0085 (1.5) |
| 1.7-339: ¹H-NMR(400.6 MHz, CDCl3): |
| δ= 7.5189 (0.5); 7.3854 (4.2); 7.3633 (4.2); 7.2605 (93.0); 7.1661 (1.0); 7.1616 (1.2); 7.1477 (1.6); 7.1458 (1.6); 7.1432 (1.9); 7.1414 (1.6); 7.1275 (1.6); 7.1230 (1.9); 7.1003 (1.7); 7.0959 (1.8); 7.0803 (3.5); 7.0759 (2.7); 7.0411 (2.0); 7.0374 (2.2); 7.0227 (1.9); 7.0210 (1.6); 7.0191 (1.9); 7.0174 (1.4); 7.0027 (1.0); 6.9990 (1.0); 6.9969 (0.6); 6.9378 (2.5); 6.9342 (2.3); 6.9174 (2.2); 6.9139 (1.9); 6.7112 (4.0); 6.6949 (4.1); 6.2808 (7.0); 4.6550 (16.0); 4.3722 (4.3); 4.3553 (9.2); 4.3385 (4.5); 4.2666 (3.8); 4.2519 (6.3); 4.2369 (4.0); 3.4996 (9.9); 3.4967 (9.7); 2.3435 (1.0); 2.3270 (2.5); 2.3118 (3.3); 2.2968 (2.6); 2.2801 (0.9); 1.5432 (8.3); 0.0080 (1.6); -0.0002 (55.1); -0.0085 (1.4) |
| 1.8-23: ¹H-NMR(400.6 MHz, CDCI3): |
| δ= 7.5334 (1.8); 7.5119 (1.8); 7.2607 (38.9); 7.1243 (0.6); 7.1224 (0.6); 7.1198 (0.7); 7.1180 (0.7); 7.1041 (0.6); 7.0996 (0.8); 7.0716 (0.7); 7.0672 (0.8); 7.0516 (1.4); 7.0472 (1.1); 7.0128 (0.8); 7.0091 (0.9); 6.9944 (0.7); 6.9908 (0.8); 6.9410 (1.1); 6.9374 (1.0); 6.9206 (0.9); 6.9171 (0.8); 6.7414 (1.7); 6.7252 (1.7); 6.2774 (2.8); 4.6901 (5.7); 4.3106 (1.4); 4.3017 (1.0); 4.2987 (1.4); 4.2950 (1.0); 4.2865 (1.5); 3.6936 (1.7); 3.6846 (1.0); 3.6815 (1.7); 3.6788 (1.0); 3.6695 (1.6); 3.6100 (1.1); 3.6023 (1.4); 3.5995 (1.5); 3.5944 (1.5); 3.5878 (2.5); 3.5231 (2.4); 3.5165 (1.4); 3.5108 (1.5); 3.5086 (1.5); 3.4970 (4.0); 3.4941 (4.0); 3.3683 (16.0); 2.0454 (1.2); 1.5508 (14.1); 1.2774 (0.5); 1.2596 (1.1); 0.8820 (1.1); 0.0079 (0.8); -0.0002 (26.8); -0.0085 (0.8) |
| 1.8-41: ¹H-NMR(400.6 MHz, CDCl3): |
| δ= 7.5409 (2.4); 7.5192 (2.5); 7.2657 (0.6); 7.2600 (48.9); 7.2552 (0.7); 7.1519 (0.5); 7.1474 (0.6); 7.1336 (0.8); 7.1317 (0.8); 7.1291 (1.0); 7.1271 (0.9); 7.1134 (0.8); 7.1089 (1.0); 7.0889 (0.9); 7.0844 (1.0); 7.0688 (1.9); 7.0644 (1.4); 7.0326 (1.0); 7.0290 (1.2); 7.0143 (1.0); 7.0126 (0.8); 7.0108 (1.0); 7.0091 (0.8); 6.9944 (0.5); 6.9396 (1.4); 6.9360 (1.3); 6.9193 (1.2); 6.9158 (1.0); 6.6887 (2.3); 6.6726 (2.4); 6.2729 (3.8); 4.7046 (8.4); 4.3800 (1.2); 4.3687 (1.5); 4.3567 (1.4); 4.1413 (2.6); 4.1362 (0.7); 4.1321 (1.6); 4.1294 (2.4); 4.1233 (0.6); 4.1178 (2.1); 3.4960 (5.2); 3.4931 (5.2); 2.7789 (0.9); 2.5515 (2.0); 2.0452 (1.4); 1.5454 (16.0); 1.2597 (1.0); 0.0080 (0.9); -0.0002 (33.8); - 0.0085 (1.0) |
| 1.8-176: ¹H-NMR(400.6 MHz, CDCl3): |
| δ= 7.2600 (61.4); 6.2864 (0.7); 4.7584 (2.5); 4.7467 (1.8); 3.5033 (1.0); 3.5003 (1.0); 1.5335 (16.0); 0.0080 (2.5); 0.0055 (0.5); -0.0002 (90.9); -0.0067 (0.7); -0.0085 (2.5) |
| 1.9-23: ¹H-NMR(400.6 MHz, CDCl3): |
| δ= 7.7003 (0.5); 7.6971 (0.7); 7.6798 (0.9); 7.6762 (0.8); 7.6703 (0.5); 7.6670 (0.7); 7.6498 (0.8); 7.6462 (0.8); 7.5367 (0.5); 7.5098 (1.5); 7.4891 (2.0); 7.4825 (0.7); 7.4742 (0.5); 7.4709 (0.9); 7.4670 (0.7); 7.4637 (0.9); 7.3525 (0.5); 7.3479 (0.9); 7.3428 (1.1); 7.3354 (1.5); 7.3311 (1.3); 7.3255 (1.1); 7.3072 (0.7); 7.3004 (0.6); 7.2626 (47.5); 7.2262 (0.7); 7.2224 (1.1); 7.2193 (0.7); 7.2062 (0.7); 7.2031 (1.3); 7.1996 (1.3); 7.1948 (0.6); 7.1798 (0.8); 7.1757 (0.5); 7.0565 (0.7); 7.0530 (0.7); 7.0372 (1.0); 7.0337 (1.0); 6.8886 (0.9); 6.8852 (0.8); 6.8680 (0.8); 6.8653 (0.6); 6.8010 (1.5); 6.7864 (1.5); 6.2750 (2.8); 4.6624 (3.4); 4.2847 (1.2); 4.2729 (1.2); 4.2691 (0.9); 4.2608 (1.3); 3.6858 (1.6); 3.6767 (1.0); 3.6738 (1.6); 3.6619 (1.4); 3.6097 (1.0); 3.6010 (1.4); 3.5989 (1.4); 3.5929 (1.4); 3.5866 (2.4); 3.5267 (2.4); 3.5204 (1.4); 3.5143 (1.4); 3.5124 (1.3); 3.5047 (1.1); 3.4917 (3.8); 3.4890 (3.8); 3.3712 (16.0); 3.3216 (0.5); 3.1281 (0.5); 3.0165 (15.4); 2.9442 (9.5); 2.0870 (9.8); 1.5729 (1.1); 1.2539 (0.7); 0.0080 (2.0); -0.0002 (70.5); -0.0085 (2.0) |
| 1.9-41: ¹H-NMR(400.6 MHz, CDCl3): |
| δ= 7.5186 (0.6); 7.5127 (5.1); 7.4917 (5.1); 7.2601 (86.1); 7.2321 (2.2); 7.2282 (3.7); 7.2245 (2.2); 7.2203 (1.3); 7.2122 (2.3); 7.2090 (4.4); 7.2049 (4.4); 7.2001 (2.2); 7.1852 (2.6); 7.1810 (1.8); 7.0736 (2.3); 7.0700 (2.5); 7.0542 (3.6); 7.0507 (3.3); 7.0349 (1.6); 7.0313 (1.5); 6.8939 (3.0); 6.8905 (2.5); 6.8735 (2.8); 6.8705 (2.2); 6.7282 (5.0); 6.7137 (5.1); 6.2665 (9.3); 4.6709 (16.0); 4.3676 (2.6); 4.3564 (3.5); 4.3443 (3.4); 4.1395 (6.5); 4.1347 (1.6); 4.1304 (3.6); 4.1276 (5.9); 4.1214 (1.5); 4.1162 (5.1); 3.4914 (12.8); 3.4885 (13.1); 3.1263 (1.1); 2.4064 (0.8); 2.0453 (0.6); 1.5376 (9.0); 1.2510 (0.9); 0.0079 (3.7); -0.0002 (129.1); -0.0085 (3.7) |
| 1.6-183: ¹H-NMR(400.2 MHz, d₆-DMSO): |
| δ= 13.0005 (0.4); 8.3149 (0.6); 8.2785 (0.7); 8.2726 (5.6); 8.2627 (0.4); 8.2561 (0.8); 8.2502 (5.6); 7.5164 (2.0); 7.4980 (2.1); 7.4798 (2.2); 7.4613 (2.2); 7.2627 (1.3); 7.2587 (1.7); 7.2403 (3.1); 7.2383 (3.0); 7.2260 (4.5); 7.2222 (4.5); 7.2060 (4.8); 7.2022 (3.2); 7.1736 (5.3); 7.1584 (5.8); 7.1235 (0.7); 7.1171 (3.3); 7.1139 (3.8); 7.0963 (2.8); 7.0934 (2.8); 7.0706 (0.5); 7.0651 (2.4); 7.0617 (2.1); 7.0552 (0.4); 7.0453 (3.1); 7.0267 (1.6); 7.0232 (1.4); 6.6432 (2.5); 6.6370 (2.5); 6.6065 (2.3); 6.6003 (2.3); 6.5659 (0.3); 6.5441 (11.8); 6.1897 (1.2); 6.1849 (1.3); 6.1719 (1.3); 6.1670 (1.2); 5.7551 (13.5); 4.8430 (0.3); 4.8339 (0.4); 4.8188 (0.5); 4.7440 (1.0); 4.7300 (0.8); 4.7203 (0.5); 4.7179 (0.8); 4.7149 (0.5); 4.7056 (0.8); 4.6474 (0.8); 4.6342 (16.0); 4.5637 (0.6); 4.5519 (0.4); 4.5403 (0.4); 3.9594 (0.7); 3.9471 (0.6); 3.9350 (0.6); 3.6223 (0.7); 3.3584 (17.5); 3.3280 (105.6); 3.2698 (0.6); 3.1748 (0.4); 2.6798 (0.4); 2.6754 (1.0); 2.6708 (1.3); 2.6663 (1.0); 2.6616 (0.4); 2.5243 (3.6); 2.5195 (5.9); 2.5109 (87.6); 2.5065 (177.7); 2.5019 (231.9); 2.4973 (163.4); 2.4927 (76.2); 2.3380 (0.5); 2.3332 (1.0); 2.3287 (1.4); 2.3241 (1.0); 2.3197 (0.5); 1.2982 (0.4); 1.2588 (0.7); 1.2353 (1.1); -0.0002 (6.1) |
| 1.8-2: ¹H-NMR(400.6 MHz, CDCI3): |
| δ= 7.5323 (4.1); 7.5108 (4.1); 7.2605 (34.2); 7.1395 (0.9); 7.1351 (1.1); 7.1211 (1.4); 7.1192 (1.4); 7.1167 (1.6); 7.1149 (1.5); 7.1009 (1.4); 7.0965 (1.8); 7.0748 (1.6); 7.0704 (1.8); 7.0547 (3.3); 7.0504 (2.5); 7.0119 (1.8); 7.0082 (2.1); 6.9935 (1.6); 6.9919 (1.4); 6.9899 (1.7); 6.9883 (1.5); 6.9735 (0.8); 6.9699 (0.9); 6.9377 (2.4); 6.9341 (2.2); 6.9173 (2.0); 6.9138 (1.8); 6.7480 (3.8); 6.7319 (3.9); 6.2812 (0.6); 6.2749 (6.4); 4.6967 (13.1); 4.6903 (1.3); 4.6831 (0.6); 4.2926 (3.2); 4.2838 (2.1); 4.2807 (3.2); 4.2771 (2.1); 4.2687 (3.6); 3.6128 (4.2); 3.6057 (1.5); 3.6038 (2.2); 3.6007 (4.0); 3.5980 (2.3); 3.5888 (3.9); 3.5097 (2.5); 3.4963 (9.8); 3.4925 (14.4); 3.4747 (7.7); 3.4572 (2.5); 2.0302 (1.8); 1.5499 (9.7); 1.1949 (7.9); 1.1826 (1.0); 1.1774 (16.0); 1.1651 (0.5); 1.1599 (7.6); 0.0080 (0.9); -0.0002 (33.9); -0.0085 (1.0) |
| 1.8-1: ¹H-NMR(400.6 MHz, CDCl3): |
| δ= 7.5322 (2.4); 7.5107 (2.3); 7.2608 (10.9); 7.1432 (0.9); 7.1389 (0.9); 7.1205 (1.6); 7.1046 (1.3); 7.1003 (1.3); 7.0796 (1.4); 7.0754 (1.4); 7.0596 (2.3); 7.0555 (1.9); 7.0139 (1.5); 7.0104 (1.5); 6.9922 (1.6); 6.9755 (0.8); 6.9721 (0.8); 6.9330 (2.0); 6.9297 (1.8); 6.9127 (1.6); 6.9094 (1.4); 6.7448 (2.2); 6.7287 (2.2); 6.2738 (4.0); 4.6977 (8.4); 4.2895 (2.5); 4.2783 (3.0); 4.2663 (2.5); 3.5625 (3.2); 3.5508 (3.8); 3.5392 (3.1); 3.4949 (8.2); 3.3398 (1.8); 3.3269 (16.0); 1.5588 (2.4); -0.0002 (7.9) |
| 1.8-276: ¹H-NMR(400.6 MHz, CDCl3): |
| δ= 8.5684 (1.4); 8.5643 (1.9); 8.5611 (1.3); 8.5569 (1.2); 8.5535 (1.7); 8.5495 (1.5); 7.6827 (1.2); 7.6783 (1.2); 7.6634 (2.2); 7.6590 (2.2); 7.6442 (1.4); 7.6397 (1.4); 7.4937 (4.9); 7.4722 (4.9); 7.2613 (16.8); 7.2366 (4.3); 7.2248 (1.5); 7.2220 (1.3); 7.2177 (4.5); 7.2060 (1.2); 7.1384 (1.1); 7.1340 (1.4); 7.1198 (1.7); 7.1182 (1.7); 7.1154 (2.1); 7.1138 (2.2); 7.0998 (1.8); 7.0954 (2.5); 7.0906 (2.1); 7.0862 (2.1); 7.0705 (3.9); 7.0662 (2.8); 7.0201 (2.2); 7.0164 (2.6); 7.0016 (1.9); 7.0002 (1.7); 6.9980 (2.1); 6.9964 (1.9); 6.9817 (1.1); 6.9780 (1.1); 6.9622 (3.0); 6.9585 (2.6); 6.9419 (2.4); 6.9383 (2.1); 6.7293 (4.6); 6.7132 (4.6); 6.2384 (7.7); 5.2809 (12.8); 4.7759 (16.0); 4.7626 (0.6); 4.6490 (0.5); 3.4749 (10.4); 3.4720 (10.8); 2.1681 (0.5); 1.2539 (1.2); 0.0080 (0.6); -0.0002 (21.0); -0.0085 (0.6) |
| 1.9-2: ¹H-NMR(400.2 MHz, d₆-DMSO): |
| δ= 8.1472 (4.0); 8.1245 (4.0); 8.1171 (0.4); 7.3039 (2.2); 7.3001 (2.7); 7.2843 (3.3); 7.2806 (3.7); 7.2662 (2.3); 7.2468 (2.0); 7.2429 (1.6); 7.1690 (2.7); 7.1659 (3.0); 7.1483 (2.0); 7.1453 (1.9); 7.1014 (1.7); 7.0981 (1.6); 7.0822 (2.7); 7.0792 (2.4); 7.0630 (1.3); 7.0597 (1.1); 6.9792 (3.9); 6.9645 (4.0); 6.5342 (8.2); 5.7554 (5.2); 4.8341 (0.4); 4.7614 (12.2); 4.1781 (3.5); 4.1666 (4.1); 4.1546 (3.7); 3.5236 (4.0); 3.5117 (4.3); 3.5000 (3.5); 3.4197 (2.5); 3.4022 (7.8); 3.3847 (7.9); 3.3672 (2.8); 3.3470 (13.3); 3.3270 (149.5); 2.6752 (0.6); 2.6708 (0.8); 2.6663 (0.6); 2.5240 (2.5); 2.5105 (56.5); 2.5063 (111.7); 2.5018 (143.9); 2.4973 (103.4); 2.4931 (50.1); 2.3332 (0.6); 2.3287 (0.8); 2.3243 (0.6); 1.9841 (1.6); 1.2983 (0.4); 1.2588 (0.7); 1.2352 (0.9); 1.0788 (7.9); 1.0613 (16.0); 1.0438 (7.6); - 0.0002 (2.9) |
| 1.9-276: ¹H-NMR(400.6 MHz, CDCl3): |
| δ= 8.5790 (2.2); 8.5745 (2.5); 8.5655 (2.6); 7.7554 (0.9); 7.7357 (1.6); 7.7166 (1.0); 7.5261 (0.6); 7.5190 (0.9); 7.4480 (5.9); 7.4269 (5.9); 7.2805 (3.2); 7.2606 (137.6); 7.2429 (0.9); 7.2307 (3.4); 7.2266 (4.0); 7.2107 (4.5); 7.2069 (5.6); 7.1900 (3.5); 7.1851 (2.8); 7.1701 (2.9); 7.1659 (2.3); 7.0584 (2.7); 7.0550 (3.1); 7.0391 (3.9); 7.0356 (4.0); 7.0199 (1.9); 7.0163 (1.8); 6.9969 (0.8); 6.9319 (3.3); 6.9287 (3.4); 6.9116 (2.9); 6.9084 (2.8); 6.7843 (5.6); 6.7698 (5.6); 6.2943 (0.8); 6.2237 (10.8); 5.3194 (0.5); 5.2799 (11.2); 4.7564 (12.3); 4.7545 (12.5); 4.7128 (0.6); 4.6431 (1.6); 3.5271 (0.5); 3.4955 (1.2); 3.4675 (15.7); 3.4648 (16.0); 3.3193 (2.2); 2.9660 (0.8); 2.8924 (0.6); 2.0499 (2.0); 2.0453 (1.9); 1.5717 (0.9); 1.3330 (2.4); 1.2843 (3.3); 1.2773 (1.7); 1.2545 (4.6); 1.2416 (1.5); 1.1745 (1.1); 1.1590 (1.0); 0.8803 (1.3); 0.8627 (0.7); 0.1456 (0.6); 0.0079 (5.5); -0.0002 (181.1); -0.0085 (6.8); -0.1497 (0.6) |
| 1.8-183: ¹H-NMR(400.6 MHz, CDCl3): |
| δ= 7.5498 (4.0); 7.5283 (4.0); 7.2606 (33.7); 7.1661 (1.0); 7.1616 (1.2); 7.1478 (1.4); 7.1459 (1.4); 7.1433 (1.7); 7.1414 (1.5); 7.1276 (1.5); 7.1231 (1.8); 7.0989 (1.5); 7.0944 (1.6); 7.0789 (3.4); 7.0744 (2.6); 7.0444 (2.1); 7.0407 (2.3); 7.0261 (1.7); 7.0244 (1.4); 7.0225 (1.8); 7.0208 (1.4); 7.0061 (0.9); 7.0025 (0.9); 6.9177 (2.3); 6.9141 (2.2); 6.8973 (2.0); 6.8937 (1.8); 6.6412 (3.7); 6.6251 (3.8); 6.2790 (6.2); 4.6717 (16.0); 4.6646 (1.2); 4.6594 (3.8); 4.6534 (1.9); 4.6482 (2.5); 4.6456 (2.5); 4.6399 (1.8); 4.5932 (1.8); 4.5874 (3.1); 4.5848 (2.5); 4.5798 (2.3); 4.5758 (2.5); 4.5736 (3.6); 4.5688 (0.9); 4.5643 (1.9); 4.5624 (1.8); 3.4990 (8.6); 3.4960 (8.8); 3.3192 (0.8); 2.0497 (0.7); 2.0451 (1.9); 1.2771 (0.7); 1.2593 (1.4); 1.2414 (0.6); 0.0080 (1.0); -0.0002 (32.5); -0.0085 (0.9) |
| 1.8-291: ¹H-NMR(400.6 MHz, CDCl3): |
| δ= 9.1478 (2.0); 9.1430 (2.1); 9.1363 (2.0); 9.1314 (2.0); 7.5537 (1.2); 7.5325 (1.4); 7.5185 (0.6); 7.4864 (4.8); 7.4725 (1.2); 7.4647 (5.5); 7.4514 (4.3); 7.4449 (3.6); 7.4400 (4.2); 7.4236 (1.4); 7.4187 (1.4); 7.2600 (81.1); 7.1571 (1.0); 7.1527 (1.3); 7.1495 (0.7); 7.1387 (1.5); 7.1369 (1.6); 7.1343 (1.8); 7.1325 (1.7); 7.1287 (0.6); 7.1240 (0.6); 7.1187 (1.6); 7.1141 (1.9); 7.1086 (1.0); 7.1042 (0.8); 7.0925 (1.7); 7.0880 (1.9); 7.0724 (4.0); 7.0681 (3.3); 7.0488 (0.6); 7.0364 (2.0); 7.0328 (3.0); 7.0181 (1.8); 7.0130 (2.1); 6.9981 (1.0); 6.9946 (1.0); 6.9731 (2.8); 6.9695 (2.4); 6.9529 (2.3); 6.9493 (2.0); 6.6697 (1.2); 6.6624 (4.5); 6.6537 (1.2); 6.6463 (4.5); 6.3007 (1.8); 6.2590 (7.4); 5.5090 (7.7); 5.5062 (7.8); 5.4536 (1.8); 4.7710 (16.0); 4.6626 (4.0); 3.5068 (2.6); 3.5040 (2.8); 3.4905 (10.1); 3.4878 (10.2); 2.7757 (1.4); 2.6225 (0.5); 2.5515 (1.3); 2.1772 (4.5); 1.2556 (0.6); 0.0080 (3.3); -0.0002 (116.1); -0.0085 (3.5) |
| 1.9-291: ¹H-NMR(400.6 MHz, CDCl3): |
| δ= 9.1797 (2.2); 9.1751 (2.4); 9.1678 (2.4); 9.1632 (2.4); 7.5628 (1.6); 7.5508 (1.4); 7.5416 (4.1); 7.5296 (4.3); 7.5162 (3.7); 7.5115 (4.1); 7.4949 (1.5); 7.4903 (1.3); 7.4614 (5.1); 7.4404 (5.0); 7.2606 (83.8); 7.2264 (5.4); 7.2070 (8.1); 7.2029 (2.1); 7.1879 (2.6); 7.1837 (1.7); 7.0738 (2.1); 7.0702 (2.4); 7.0536 (2.2); 7.0508 (3.4); 7.0351 (1.6); 7.0315 (1.5); 6.9969 (0.6); 6.9408 (2.8); 6.9381 (2.1); 6.9203 (2.6); 6.9169 (1.7); 6.6968 (4.8); 6.6823 (4.8); 6.2522 (9.0); 5.5236 (1.2); 5.4892 (7.8); 5.4812 (7.8); 5.4472 (0.9); 4.7661 (0.6); 4.7475 (16.0); 3.4875 (11.8); 3.4846 (12.2); 0.0079 (3.6); -0.0002 (126.7); -0.0085 (3.7) |
| 1.8-291: ¹H-NMR(400.6 MHz, CDCl3): |
| δ= 9.1478 (2.0); 9.1429 (2.0); 9.1362 (2.0); 9.1313 (2.0); 7.4894 (4.5); 7.4749 (1.3); 7.4679 (4.6); 7.4634 (1.1); 7.4537 (3.8); 7.4421 (4.2); 7.4355 (3.4); 7.4306 (3.7); 7.4143 (1.1); 7.4093 (0.8); 7.2613 (35.0); 7.1581 (1.0); 7.1536 (1.2); 7.1397 (1.5); 7.1380 (1.6); 7.1353 (1.8); 7.1335 (1.7); 7.1197 (1.6); 7.1151 (2.0); 7.0944 (1.7); 7.0900 (1.9); 7.0744 (3.7); 7.0700 (2.8); 7.0378 (2.0); 7.0341 (2.4); 7.0194 (1.8); 7.0178 (1.5); 7.0158 (2.0); 7.0142 (1.6); 6.9995 (0.9); 6.9958 (1.0); 6.9742 (2.8); 6.9706 (2.4); 6.9539 (2.2); 6.9504 (2.0); 6.6634 (4.4); 6.6473 (4.4); 6.2602 (7.2); 5.5118 (7.6); 5.5089 (7.7); 4.7741 (16.0); 4.1308 (1.1); 4.1129 (1.0); 3.4917 (9.9); 3.4888 (10.1); 3.3194 (0.8); 2.0499 (0.8); 2.0453 (5.0); 1.5781 (9.0); 1.2774 (1.4); 1.2595 (3.1); 1.2417 (1.4); 0.0079 (1.5); -0.0002 (52.1); - 0.0085 (1.4) |
| 1.9-291: ¹H-NMR(400.6 MHz, CDCl3): |
| δ= 9.1592 (2.3); 9.1545 (2.4); 9.1474 (2.4); 9.1426 (2.4); 7.7008 (2.9); 7.6976 (4.1); 7.6939 (1.7); 7.6855 (1.2); 7.6803 (5.5); 7.6767 (4.6); 7.6709 (2.9); 7.6676 (4.2); 7.6640 (1.8); 7.6556 (1.2); 7.6504 (5.4); 7.6468 (4.5); 7.5736 (0.6); 7.5700 (1.2); 7.5662 (1.3); 7.5627 (0.6); 7.5575 (0.7); 7.5518 (2.6); 7.5476 (2.6); 7.5422 (0.9); 7.5366 (1.4); 7.5329 (3.1); 7.5291 (3.0); 7.5254 (1.3); 7.5197 (0.6); 7.5169 (1.7); 7.5050 (1.4); 7.4957 (4.5); 7.4888 (2.7); 7.4869 (3.9); 7.4838 (6.9); 7.4817 (3.4); 7.4797 (4.0); 7.4763 (2.0); 7.4717 (6.6); 7.4674 (8.3); 7.4641 (4.6); 7.4606 (9.8); 7.4570 (1.9); 7.4542 (1.3); 7.4505 (3.8); 7.4465 (2.6); 7.4430 (2.7); 7.4393 (5.8); 7.2613 (69.0); 7.2285 (2.0); 7.2244 (5.3); 7.2199 (1.0); 7.2086 (2.2); 7.2061 (4.7); 7.2043 (7.7); 7.1998 (2.1); 7.1848 (2.7); 7.1807 (1.8); 7.0713 (2.3); 7.0677 (2.5); 7.0518 (2.9); 7.0483 (3.4); 7.0326 (1.6); 7.0291 (1.6); 6.9379 (2.9); 6.9348 (2.2); 6.9174 (2.6); 6.6980 (4.7); 6.6835 (4.8); 6.2524 (9.1); 5.5183 (1.1); 5.4844 (7.9); 5.4756 (8.0); 5.4576 (0.9); 5.4419 (1.0); 4.7656 (0.8); 4.7469 (16.0); 3.4875 (12.1); 3.4845 (12.7); 2.1800 (2.3); 2.0453 (1.6); 1.5901 (3.0); 1.3332 (0.6); 1.2844 (0.9); 1.2773 (0.6); 1.2594 (1.4); 1.2562 (1.6); 1.2416 (0.5); 0.0080 (3.1); -0.0002 (104.0); -0.0058 (1.4); -0.0085 (3.1) |
| 1.19-276: ¹H-NMR(400.0 MHz, CDCl3): |
| δ= 8.5628 (1.0); 8.5581 (1.0); 8.5494 (1.0); 8.5450 (0.8); 7.6964 (0.7); 7.6920 (0.7); 7.6771 (1.5); 7.6726 (1.3); 7.6578 (0.8); 7.6534 (0.8); 7.3225 (2.6); 7.3004 (2.6); 7.2613 (7.0); 7.2450 (1.2); 7.2406 (1.3); 7.2364 (1.7); 7.2260 (1.0); 7.2167 (1.7); 7.1197 (0.7); 7.1047 (0.7); 7.0983 (1.4); 7.0835 (1.3); 7.0771 (0.8); 7.0623 (0.7); 6.8814 (0.8); 6.8781 (0.9); 6.8575 (1.3); 6.8368 (0.7); 6.8335 (0.6); 6.7192 (1.9); 6.7124 (1.0); 6.7088 (1.5); 6.7037 (2.0); 6.6912 (0.8); 6.6875 (1.2); 6.2209 (4.3); 5.2986 (16.0); 5.2901 (0.7); 5.2642 (7.1); 4.7605 (8.7); 3.4659 (6.0); 3.4632 (6.0); 2.0445 (0.9); 1.2589 (0.8); -0.0002 (10.0) |
| 1.19-176: ¹H-NMR(400.0 MHz, CDCl3): |
| δ= 7.3919 (3.5); 7.3699 (3.4); 7.2606 (8.0); 7.1737 (0.8); 7.1589 (0.9); 7.1524 (1.8); 7.1377 (1.8); 7.1312 (1.1); 7.1165 (1.0); 6.9356 (1.1); 6.9325 (1.2); 6.9116 (1.8); 6.8910 (0.9); 6.8879 (0.9); 6.7009 (1.2); 6.6975 (2.0); 6.6797 (1.1); 6.6763 (1.7); 6.6076 (2.4); 6.5916 (2.3); 6.2789 (6.0); 5.2989 (14.6); 4.7544 (2.0); 4.7484 (16.0); 4.7319 (13.5); 4.7225 (1.2); 4.1299 (0.5); 4.1120 (0.5); 3.5337 (0.7); 3.4998 (8.6); 3.4972 (8.7); 2.0442 (2.4); 1.5500 (2.3); 1.2767 (0.7); 1.2589 (1.4); 1.2410 (0.6); -0.0002 (11.4) |
| 1.23-176: ¹H-NMR(400.0 MHz, CDCl3): |
| δ= 7.4170 (3.4); 7.3949 (3.3); 7.2606 (12.4); 6.9813 (1.4); 6.9685 (1.5); 6.9589 (2.0); 6.9460 (1.9); 6.8642 (1.2); 6.8568 (1.4); 6.8452 (1.3); 6.8418 (1.0); 6.8377 (1.5); 6.8345 (1.2); 6.8227 (0.8); 6.8152 (1.1); 6.8079 (2.2); 6.7985 (3.6); 6.7861 (2.4); 6.7821 (3.4); 6.7790 (1.6); 6.3117 (5.7); 5.2995 (11.5); 4.7712 (16.0); 4.7655 (1.3); 4.7221 (13.9); 4.6879 (1.0); 3.5431 (0.7); 3.5234 (8.2); 3.5206 (8.2); 2.0448 (1.8); 1.5462 (4.8); 1.2771 (0.6); 1.2592 (1.2); 0.0079 (0.7); -0.0002 (17.8); -0.0084 (0.6) |
| 1.23-1: ¹H-NMR(400.0 MHz, CDCl3): |
| δ= 7.3903 (2.1); 7.3681 (2.0); 7.2611 (5.9); 6.9572 (0.9); 6.9443 (0.9); 6.9347 (1.2); 6.9219 (1.1); 6.8684 (2.0); 6.8520 (2.0); 6.8288 (0.6); 6.8214 (0.8); 6.8095 (0.7); 6.8065 (0.5); 6.8020 (0.8); 6.7990 (0.7); 6.7795 (0.7); 6.7702 (1.2); 6.7628 (0.9); 6.7483 (1.3); 6.7409 (0.9); 6.3017 (3.5); 5.2992 (6.3); 4.6717 (7.8); 4.6510 (0.5); 4.3027 (2.0); 4.2942 (1.2); 4.2911 (1.9); 4.2873 (1.1); 4.2793 (2.0); 3.5791 (2.3); 3.5707 (1.2); 3.5673 (2.2); 3.5644 (1.2); 3.5557 (2.1); 3.5177 (4.9); 3.5150 (5.0); 3.3523 (1.5); 3.3463 (16.0); -0.0002 (8.3) |
| 1.19-1: ¹H-NMR(400.0 MHz, CDCl3): |
| δ= 7.3607 (2.2); 7.3386 (2.2); 7.2610 (4.8); 7.1336 (0.5); 7.1186 (0.6); 7.1124 (1.1); 7.0975 (1.1); 7.0911 (0.7); |
| 7.0763 (0.6); 6.8806 (0.8); 6.8774 (0.8); 6.8566 (1.2); 6.8359 (0.6); 6.8329 (0.6); 6.7243 (1.7); 6.7081 (1.6); 6.6752 (1.2); 6.6540 (1.1); 6.2630 (3.9); 5.2988 (12.2); 4.6853 (8.1); 4.2734 (1.3); 4.2654 (1.7); 4.2602 (1.5); 4.2521 (1.3); 3.5476 (2.5); 3.5360 (2.9); 3.5293 (1.0); 3.5244 (2.3); 3.4899 (5.8); 3.4876 (5.8); 3.3370 (1.3); 3.3171 (16.0); 1.5622 (1.3); -0.0002 (6.8) |
| 1.19-23: ¹H-NMR(400.6 MHz, CDCl3): |
| δ= 7.3611 (1.6); 7.3390 (1.6); 7.2614 (5.8); 7.1139 (0.9); 7.0990 (0.9); 7.0927 (0.5); 6.8797 (0.5); 6.8764 (0.5); 6.8583 (0.5); 6.8562 (0.7); 6.8531 (0.6); 6.7148 (1.0); 6.6986 (1.0); 6.6833 (0.8); 6.6620 (0.8); 6.2666 (2.6); 5.2990 (8.8); 4.6768 (5.3); 4.2950 (1.2); 4.2864 (0.9); 4.2832 (1.2); 4.2794 (0.8); 4.2711 (1.3); 3.6802 (1.6); 3.6733 (0.6); 3.6711 (0.9); 3.6682 (1.6); 3.6656 (1.0); 3.6635 (0.6); 3.6563 (1.4); 3.6022 (0.9); 3.6009 (0.9); 3.5933 (1.3); 3.5912 (1.3); 3.5903 (1.3); 3.5853 (1.4); 3.5789 (2.3); 3.5182 (2.3); 3.5117 (1.4); 3.5068 (1.3); 3.5057 (1.3); 3.5038 (1.3); 3.4945 (2.2); 3.4915 (3.5); 3.4885 (3.5); 3.3683 (1.9); 3.3660 (16.0); 1.5679 (1.8); -0.0002 (8.7) |
| 1.23-23: ¹H-NMR(400.6 MHz, CDCl3): |
| δ= 7.3919 (1.8); 7.3698 (1.8); 7.2627 (3.6); 6.9669 (0.8); 6.9540 (0.8); 6.9444 (1.0); 6.9315 (1.0); 6.8704 (1.8); 6.8540 (1.8); 6.8294 (0.6); 6.8219 (0.7); 6.8100 (0.6); 6.8025 (0.7); 6.7994 (0.7); 6.7800 (0.6); 6.7630 (1.1); 6.7556 (0.8); 6.7411 (1.1); 6.7337 (0.8); 6.3040 (3.1); 5.2991 (12.5); 4.6645 (6.7); 4.3206 (1.6); 4.3119 (1.0); 4.3087 (1.7); 4.3049 (1.0); 4.2966 (1.7); 3.7012 (1.9); 3.6928 (1.1); 3.6892 (1.9); 3.6860 (1.1); 3.6773 (1.7); 3.6199 (1.3); 3.6114 (1.5); 3.6091 (1.8); 3.6036 (1.5); 3.5970 (2.6); 3.5297 (2.7); 3.5227 (2.6); 3.5182 (5.9); 3.5156 (5.7); 3.5072 (1.4); 3.3700 (16.0); 1.5867 (0.9); -0.0002 (5.1) |
| 1.23-276: ¹H-NMR(400.6 MHz, CDCl3): |
| δ= 8.6265 (1.6); 8.6142 (1.6); 7.7696 (1.1); 7.7652 (1.1); 7.7503 (2.2); 7.7459 (2.1); 7.7310 (1.2); 7.7266 (1.2); 7.3548 (4.4); 7.3326 (6.4); 7.3122 (2.3); 7.3064 (1.4); 7.2997 (1.2); 7.2872 (1.1); 7.2612 (18.1); 6.9784 (1.8); 6.9657 (1.8); 6.9561 (2.2); 6.9433 (2.2); 6.8537 (4.2); 6.8374 (4.2); 6.8231 (1.3); 6.8156 (1.6); 6.8038 (1.4); 6.8009 (1.0); 6.7963 (1.7); 6.7934 (1.6); 6.7814 (1.0); 6.7783 (2.9); 6.7740 (1.6); 6.7710 (1.8); 6.7564 (2.7); 6.7491 (1.6); 6.2783 (7.5); 5.3150 (12.4); 5.2991 (11.1); 4.7495 (16.0); 4.6352 (0.8); 3.5034 (10.0); 3.5006 (10.3); 0.0080 (0.8); -0.0002 (27.4); -0.0085 (0.7) |
| 1.8-278: ¹H-NMR(400.6 MHz, CDCl3): |
| δ= 8.5803 (3.6); 8.5766 (2.5); 8.5689 (2.6); 8.5653 (3.5); 7.4781 (4.6); 7.4565 (4.5); 7.2610 (24.0); 7.2138 (0.5); 7.1798 (0.5); 7.1653 (4.6); 7.1613 (2.6); 7.1540 (3.1); 7.1515 (4.5); 7.1503 (4.4); 7.1332 (1.7); 7.1312 (1.6); 7.1286 (1.9); 7.1266 (1.6); 7.1132 (1.7); 7.1085 (2.2); 7.0970 (1.6); 7.0924 (1.8); 7.0770 (3.8); 7.0724 (2.7); 7.0466 (2.3); 7.0428 (2.5); 7.0284 (1.9); 7.0264 (1.6); 7.0248 (1.9); 7.0228 (1.5); 7.0084 (0.9); 7.0047 (0.9); 6.9519 (2.6); 6.9482 (2.4); 6.9316 (2.2); 6.9280 (1.9); 6.6679 (4.4); 6.6518 (4.5); 6.2706 (0.6); 6.2497 (7.2); 5.2988 (7.6); 5.1755 (6.6); 4.7510 (16.0); 4.7439 (0.9); 4.7352 (0.6); 4.7212 (1.2); 3.4992 (1.0); 3.4839 (10.0); 3.4810 (10.0); 2.1154 (1.1); 2.0932 (0.6); 2.0447 (0.6); 1.4365 (0.6); 1.4224 (0.6); 1.2590 (0.8); 1.2547 (0.7); 0.0080 (0.9); 0.0040 (0.8); -0.0002 (34.3); -0.0085 (1.0) |
| 1.8-356: ¹H-NMR(400.6 MHz, CDCI3): |
| δ= 7.4988 (2.8); 7.4772 (2.8); 7.2928 (3.7); 7.2610 (14.5); 7.1134 (0.7); 7.1086 (0.7); 7.0953 (1.0); 7.0932 (1.0); 7.0906 (1.2); 7.0885 (1.0); 7.0752 (1.0); 7.0704 (1.3); 7.0532 (0.9); 7.0486 (1.1); 7.0332 (2.4); 7.0285 (1.8); 7.0076 (1.4); 7.0039 (1.6); 6.9896 (1.1); 6.9859 (1.2); 6.9840 (0.9); 6.9695 (0.6); 6.9658 (0.5); 6.8875 (1.6); 6.8839 (1.5); 6.8672 (1.4); 6.8637 (1.2); 6.6953 (2.7); 6.6792 (2.6); 6.2774 (4.6); 5.2988 (11.1); 5.0117 (5.6); 4.6215 (9.5); 4.5971 (0.7); 3.7941 (16.0); 3.5135 (0.6); 3.4996 (6.3); 3.4968 (6.4); 2.2141 (1.6); 2.2078 (15.0); 2.1979 (0.6); 2.1207 (0.9); 1.2590 (0.5); 0.0079 (0.7); -0.0002 (21.4); -0.0085 (0.6) |
| 1.7-17: ¹H-NMR(400.6 MHz, CDCI3): |
| δ= 7.3747 (4.3); 7.3526 (4.3); 7.2601 (11.6); 7.1456 (0.9); 7.1411 (1.1); 7.1253 (1.7); 7.1226 (2.0); 7.1071 (1.5); 7.1026 (1.7); 7.0758 (1.6); 7.0714 (1.8); 7.0558 (3.6); 7.0514 (2.8); 7.0209 (1.9); 7.0174 (2.1); 6.9990 (2.1); 6.9826 (0.8); 6.9791 (0.8); 6.9375 (2.7); 6.9342 (2.6); 6.9171 (2.3); 6.9139 (2.1); 6.7555 (4.1); 6.7390 (4.2); 6.2779 (7.7); 5.9527 (0.6); 5.9426 (1.2); 5.9326 (0.6); 5.8147 (1.2); 5.8046 (2.5); 5.7945 (1.2); 5.6766 (0.6); 5.6666 (1.2); 5.6565 (0.6); 5.2985 (14.4); 4.6913 (16.0); 4.3081 (2.9); 4.2975 (3.3); 4.2932 (2.8); 4.2849 (3.3); 3.7434 (3.9); 3.7315 (4.8); 3.7200 (3.6); 3.6784 (2.4); 3.6683 (2.4); 3.6436 (4.9); 3.6335 (4.8); 3.6087 (2.5); 3.5986 (2.4); 3.4976 (11.2); 3.4954 (11.7); 1.5496 (3.4); -0.0002 (15.3) |
| 1.7-3: ¹H-NMR(400.6 MHz, CDCl3): |
| δ= 7.3685 (3.7); 7.3464 (3.7); 7.2606 (9.4); 7.1337 (1.0); 7.1292 (1.0); 7.1153 (1.4); 7.1134 (1.4); 7.1108 (1.6); 7.1089 (1.4); 7.0951 (1.4); 7.0906 (1.7); 7.0665 (1.5); 7.0620 (1.6); 7.0464 (3.2); 7.0420 (2.4); 7.0087 (1.8); 7.0050 (2.0); 6.9904 (1.6); 6.9887 (1.3); 6.9868 (1.7); 6.9850 (1.3); 6.9704 (0.8); 6.9667 (0.8); 6.9339 (2.3); 6.9303 (2.0); 6.9135 (1.9); 6.9100 (1.6); 6.7822 (3.6); 6.7657 (3.8); 6.2780 (6.2); 5.2977 (16.0); 4.6911 (12.6); 4.2946 (3.2); 4.2857 (2.1); 4.2827 (3.2); 4.2790 (2.0); 4.2705 (3.4); 3.6140 (3.9); 3.6051 (2.1); 3.6019 (3.7); 3.5989 (2.1); 3.5899 (3.6); 3.4980 (8.3); 3.4950 (8.4); 3.3969 (4.4); 3.3800 (9.0); 3.3632 (4.5); 1.5926 (1.7); 1.5756 (3.7); 1.5571 (3.7); 1.5401 (1.9); 1.5219 (0.5); 0.9161 (6.9); 0.8976 (14.1); 0.8790 (6.2); -0.0002 (12.9) |
| 1.7-18: ¹H-NMR(400.6 MHz, CDCl3): |
| δ= 7.3744 (4.3); 7.3522 (4.3); 7.2600 (12.8); 7.1457 (1.0); 7.1412 (1.2); 7.1273 (1.6); 7.1254 (1.5); 7.1228 (1.8); 7.1210 (1.6); 7.1072 (1.6); 7.1026 (1.9); 7.0783 (1.7); 7.0739 (1.8); 7.0583 (3.6); 7.0539 (2.8); 7.0223 (2.1); 7.0187 (2.3); 7.0040 (1.8); 7.0023 (1.5); 7.0004 (1.9); 6.9988 (1.4); 6.9841 (0.9); 6.9804 (0.9); 6.9358 (2.6); 6.9322 (2.3); 6.9155 (2.2); 6.9119 (1.9); 6.7488 (4.2); 6.7324 (4.2); 6.2759 (7.1); 5.2981 (16.0); 4.6933 (15.7); 4.3195 (2.3); 4.3175 (2.3); 4.3106 (2.7); 4.3079 (2.7); 4.3061 (2.6); 4.3027 (2.5); 4.2960 (2.5); 4.2944 (2.4); 3.8290 (2.6); 3.8074 (10.3); 3.7970 (4.4); 3.7857 (10.6); 3.7640 (2.8); 3.4971 (9.7); 3.4942 (9.8); 1.5506 (2.4); -0.0002 (17.5) |
| 1.7-8: ¹H-NMR(400.6 MHz, CDCl3): |
| δ= 7.3708 (3.8); 7.3487 (3.9); 7.2605 (10.2); 7.1443 (0.9); 7.1399 (1.0); 7.1258 (1.4); 7.1240 (1.4); 7.1215 (1.6); 7.1197 (1.4); 7.1057 (1.4); 7.1013 (1.6); 7.0767 (1.6); 7.0723 (1.7); 7.0566 (3.2); 7.0523 (2.4); 7.0136 (1.7); 7.0099 (1.9); 6.9950 (1.6); 6.9936 (1.4); 6.9915 (1.7); 6.9752 (0.8); 6.9716 (0.8); 6.9317 (2.3); 6.9281 (2.1); 6.9114 (2.0); 6.9078 (1.7); 6.7793 (3.7); 6.7628 (3.8); 6.2812 (6.5); 5.2983 (16.0); 4.6971 (13.5); 4.3229 (1.6); 4.3139 (2.6); 4.3106 (2.1); 4.3043 (2.1); 4.3003 (2.9); 4.2915 (1.7); 4.1155 (5.9); 4.1137 (5.7); 4.1096 (5.9); 4.1077 (5.4); 3.7076 (2.2); 3.7042 (2.3); 3.6958 (3.6); 3.6928 (3.3); 3.6845 (1.9); 3.6803 (1.8); 3.4996 (9.1); 3.4968 (8.9); 2.4400 (2.6); 2.4341 (5.2); 2.4281 (2.4); 1.5566 (1.9); -0.0002 (13.0) |
| 1.7-7: ¹H-NMR(400.6 MHz, CDCl3): |
| δ= 7.3688 (4.6); 7.3467 (4.7); 7.2603 (11.8); 7.1364 (1.0); 7.1319 (1.2); 7.1179 (1.7); 7.1161 (1.6); 7.1135 (2.0); 7.1117 (1.7); 7.0978 (1.6); 7.0934 (1.9); 7.0713 (1.8); 7.0669 (2.0); 7.0512 (3.8); 7.0469 (2.9); 7.0113 (2.1); 7.0076 (2.3); 6.9928 (2.0); 6.9910 (1.7); 6.9892 (2.0); 6.9729 (0.9); 6.9693 (1.0); 6.9323 (2.8); 6.9288 (2.5); 6.9120 (2.4); 6.9085 (2.0); 6.7791 (4.5); 6.7626 (4.5); 6.2764 (7.8); 5.8987 (0.6); 5.8845 (1.3); 5.8727 (0.8); 5.8704 (0.7); 5.8586 (1.5); 5.8557 (0.8); 5.8443 (0.8); 5.8414 (1.5); 5.8297 (0.9); 5.8273 (0.8); 5.8155 (1.6); 5.8014 (0.8); 5.2980 (13.2); 5.2813 (0.9); 5.2773 (2.4); 5.2732 (2.4); 5.2692 (0.9); 5.2383 (0.8); 5.2343 (2.0); 5.2302 (2.1); 5.2262 (0.8); 5.2046 (1.0); 5.2015 (2.2); 5.1980 (1.8); 5.1943 (0.8); 5.1787 (0.9); 5.1756 (2.0); 5.1721 (1.7); 5.1683 (0.7); 4.6942 (16.0); 4.3068 (3.6); 4.2978 (2.7); 4.2951 (3.6); 4.2911 (2.5); 4.2829 (3.9); 3.9768 (3.3); 3.9735 (5.4); 3.9699 (3.2); 3.9627 (3.3); 3.9594 (5.2); 3.9557 (3.0); 3.6219 (4.8); 3.6154 (1.6); 3.6124 (2.9); 3.6100 (4.8); 3.6046 (1.4); 3.5981 (4.3); 3.4959 (10.8); 3.4931 (10.5); 1.5595 (1.7); 0.0079 (0.5); -0.0002 (15.6) |
| 1.7-27: ¹H-NMR(400.6 MHz, CDCl3): |
| δ= 7.3706 (4.6); 7.3484 (4.6); 7.2605 (12.1); 7.1398 (1.0); 7.1354 (1.2); 7.1194 (2.0); 7.1169 (2.3); 7.1012 (1.6); 7.0968 (1.8); 7.0698 (1.7); 7.0655 (2.0); 7.0498 (3.7); 7.0455 (3.0); 7.0108 (2.0); 7.0073 (2.2); 6.9889 (2.4); 6.9724 (0.9); 6.9690 (0.9); 6.9219 (2.9); 6.9188 (2.8); 6.9016 (2.4); 6.8985 (2.2); 6.7717 (4.4); 6.7553 (4.4); 6.2836 (8.2); 5.2984 (8.8); 4.6524 (15.8); 4.2493 (3.8); 4.2332 (8.0); 4.2171 (3.9); 3.4999 (12.6); 3.4980 (12.8); 3.4571 (2.5); 3.4396 (7.7); 3.4329 (4.4); 3.4221 (8.3); 3.4174 (9.1); 3.4021 (4.8); 1.9129 (1.1); 1.8972 (4.1); 1.8814 (6.0); 1.8656 (3.9); 1.8497 (1.0); 1.5598 (2.0); 1.1867 (8.0); 1.1692 (16.0); 1.1517 (7.6); 0.0079 (0.6); -0.0002 (15.0) |
| 1.7-24: ¹H-NMR(400.6 MHz, CDCl3): |
| δ= 7.3699 (4.4); 7.3478 (4.4); 7.2608 (12.9); 7.1389 (1.0); 7.1345 (1.1); 7.1162 (2.2); 7.1004 (1.6); 7.0960 (1.8); 7.0650 (1.8); 7.0607 (1.9); 7.0450 (3.8); 7.0408 (2.9); 7.0095 (2.2); 7.0061 (2.2); 6.9899 (2.3); 6.9880 (2.3); 6.9713 (1.0); 6.9678 (0.9); 6.9374 (3.2); 6.9171 (2.6); 6.7773 (4.4); 6.7609 (4.4); 6.2802 (8.3); 5.2987 (5.8); 4.6874 (15.9); 4.3094 (4.0); 4.2975 (4.6); 4.2853 (4.2); 3.7000 (4.5); 3.6878 (4.9); 3.6760 (4.1); 3.6185 (1.9); 3.6161 (1.9); 3.6080 (4.8); 3.6010 (3.4); 3.5944 (5.6); 3.5668 (5.7); 3.5591 (3.6); 3.5521 (4.6); 3.5417 (4.1); 3.5242 (7.8); 3.5066 (9.0); 3.4987 (13.8); 3.4894 (3.3); 1.5659 (2.8); 1.2231 (8.0); 1.2056 (16.0); 1.1881 (7.8); 0.0079 (0.6); -0.0002 (17.3); - 0.0085 (0.6) |
| 1.7-4: ¹H-NMR(400.6 MHz, CDCI3): |
| δ= 7.3681 (2.0); 7.3459 (2.0); 7.2605 (5.9); 7.1296 (0.5); 7.1157 (0.7); 7.1138 (0.7); 7.1113 (0.8); 7.1094 (0.7); 7.0956 (0.7); 7.0911 (0.8); 7.0659 (0.8); 7.0614 (0.8); 7.0458 (1.7); 7.0414 (1.2); 7.0080 (0.9); 7.0043 (1.0); 6.9896 (0.9); 6.9879 (0.7); 6.9860 (0.9); 6.9845 (0.7); 6.9348 (1.2); 6.9312 (1.1); 6.9145 (1.0); 6.9109 (0.9); 6.7864 (1.9); 6.7699 (2.0); 6.2794 (3.3); 5.2983 (5.2); 4.6894 (6.6); 4.2766 (1.6); 4.2645 (1.7); 4.2613 (1.1); 4.2520 (1.8); 3.6077 (2.1); 3.5978 (1.4); 3.5953 (2.1); 3.5908 (0.7); 3.5834 (2.6); 3.5686 (1.4); 3.5533 (1.0); 3.4989 (4.6); 3.4960 (4.5); 1.5618 (0.8); 1.1430 (16.0); 1.1366 (0.5); 1.1277 (16.0); -0.0002 (7.9) |
| 1.8-277: ¹H-NMR(400.6 MHz, CDCl3): |
| δ= 8.7665 (1.9); 8.7508 (1.0); 8.1424 (1.0); 8.1226 (1.0); 7.7385 (0.8); 7.7246 (0.9); 7.7187 (0.8); 7.7048 (0.7); 7.4650 (3.0); 7.4436 (3.0); 7.2609 (17.2); 7.1381 (0.6); 7.1334 (0.7); 7.1199 (1.1); 7.1180 (1.0); 7.1153 (1.3); 7.1133 (1.0); 7.1011 (1.5); 7.0960 (2.0); 7.0813 (2.6); 7.0768 (1.5); 7.0502 (1.5); 7.0463 (1.6); 7.0320 (1.2); 7.0286 (1.1); 7.0261 (0.8); 7.0120 (0.6); 7.0083 (0.5); 6.9286 (1.7); 6.9249 (1.5); 6.9084 (1.4); 6.9050 (1.2); 6.6521 (2.9); 6.6361 (2.9); 6.2720 (4.8); 5.2994 (16.0); 5.2917 (6.8); 4.7088 (6.0); 4.7062 (5.6); 3.4912 (6.6); 3.4884 (6.4); 0.0080 (0.9); - 0.0002 (24.1); -0.0086 (0.6) |
| 1.9-278: ¹H-NMR(400.6 MHz, CDCl3): |
| δ= 8.8366 (3.0); 8.8332 (1.6); 8.8233 (1.7); 8.8201 (3.0); 7.6770 (2.5); 7.6606 (2.4); 7.4601 (2.6); 7.4392 (2.6); 7.2610 (19.6); 7.2564 (0.7); 7.2520 (1.3); 7.2498 (1.7); 7.2455 (1.5); 7.2340 (1.6); 7.2296 (2.3); 7.2257 (1.8); 7.2163 (1.5); 7.2121 (0.6); 7.1255 (1.1); 7.1219 (1.2); 7.1073 (1.0); 7.1048 (1.0); 7.1026 (1.7); 7.0868 (0.7); 7.0832 (0.6); 6.9864 (1.6); 6.9833 (1.4); 6.9656 (1.4); 6.9615 (1.1); 6.6828 (2.5); 6.6684 (2.6); 6.2711 (4.7); 5.3773 (5.6); 5.2997 (16.0); 4.7614 (6.6); 3.4992 (6.3); 3.4966 (6.2); 0.0080 (0.8); -0.0002 (26.4); -0.0085 (0.8) |
| 1.9-277: ¹H-NMR(400.6 MHz, CDCl3): |
| δ= 8.5953 (1.9); 8.5917 (1.9); 8.5832 (2.0); 8.5796 (1.9); 8.5544 (2.6); 8.5498 (2.6); 7.6475 (1.2); 7.6431 (1.9); 7.6386 (1.1); 7.6280 (1.3); 7.6235 (2.1); 7.6190 (1.1); 7.4407 (4.9); 7.4197 (4.9); 7.3156 (1.8); 7.3032 (1.8); 7.2961 (1.6); 7.2840 (1.7); 7.2613 (18.2); 7.2226 (2.3); 7.2186 (2.8); 7.2029 (2.8); 7.1990 (4.6); 7.1800 (2.7); 7.1757 (2.0); 7.1602 (1.9); 7.1560 (1.5); 7.0674 (1.9); 7.0639 (2.0); 7.0481 (2.8); 7.0446 (2.7); 7.0287 (1.2); 7.0253 (1.2); 6.8720 (2.8); 6.8686 (2.7); 6.8516 (2.6); 6.8482 (2.4); 6.7039 (4.7); 6.6893 (4.7); 6.2618 (8.7); 5.2991 (9.6); 5.1686 (10.9); 4.6586 (16.0); 3.4825 (11.8); 3.4800 (11.9); 2.1182 (1.1); 2.0447 (1.1); 1.4367 (0.6); 1.4224 (0.6); 1.2592 (1.0); 0.0084 (0.8); -0.0002 (26.3); -0.0078 (0.7) |
| 1.9-356: ¹H-NMR(400.6 MHz, CDCl3): |
| δ= 7.4347 (2.6); 7.4135 (2.6); 7.3124 (3.7); 7.2616 (12.6); 7.2126 (1.3); 7.2085 (1.6); 7.1929 (1.6); 7.1890 (2.6); 7.1853 (0.8); 7.1703 (1.4); 7.1659 (1.0); 7.1501 (1.2); 7.1459 (0.9); 7.0529 (1.1); 7.0493 (1.2); 7.0336 (1.6); 7.0300 (1.5); 7.0143 (0.7); 7.0107 (0.7); 6.8429 (1.5); 6.8394 (1.5); 6.8224 (1.4); 6.8190 (1.3); 6.7170 (2.5); 6.7024 (2.6); 6.2694 (4.7); 5.2989 (12.6); 4.9953 (4.6); 4.9917 (4.6); 4.5763 (8.4); 3.8108 (16.0); 3.4931 (6.1); 3.4903 (6.2); 2.2034 (15.3); 2.1191 (1.0); 2.0444 (1.6); 1.4352 (0.5); 1.4211 (0.5); 1.2769 (0.6); 1.2591 (1.3); 1.2413 (0.5); 0.0079 (0.5); -0.0002 (18.1); -0.0085 (0.5) |
| 1.7-43: ¹H-NMR(400.6 MHz, CDCl3): |
| δ= 7.3750 (4.5); 7.3529 (4.5); 7.2600 (20.5); 7.1494 (1.1); 7.1449 (1.3); 7.1311 (1.6); 7.1292 (1.6); 7.1266 (1.9); 7.1247 (1.8); 7.1109 (1.8); 7.1064 (2.1); 7.0831 (1.8); 7.0786 (2.0); 7.0630 (3.9); 7.0586 (3.0); 7.0263 (2.2); 7.0226 (2.5); 7.0079 (1.9); 7.0062 (1.6); 7.0043 (2.0); 7.0026 (1.6); 6.9879 (1.0); 6.9843 (1.0); 6.9244 (2.7); 6.9208 (2.5); 6.9041 (2.3); 6.9005 (2.0); 6.7336 (4.3); 6.7172 (4.5); 6.2775 (7.2); 5.2988 (6.5); 4.6625 (16.0); 4.2628 (3.5); 4.2474 (7.5); 4.2319 (3.7); 3.9891 (4.2); 3.9737 (8.8); 3.9584 (4.4); 3.4989 (9.7); 3.4959 (10.1); 2.0383 (0.6); 2.0232 (2.4); 2.0079 (3.5); 1.9926 (2.3); 1.9772 (0.6); 1.5456 (1.8); 0.0080 (0.8); -0.0002 (30.6); -0.0084 (1.0) |
| 1.7-9: ¹H-NMR(400.6 MHz, CDCl3): |
| δ= 7.3814 (3.3); 7.3593 (3.4); 7.2604 (12.6); 7.1560 (0.8); 7.1514 (0.9); 7.1377 (1.4); 7.1357 (1.4); 7.1331 (1.5); 7.1312 (1.2); 7.1175 (1.2); 7.1129 (1.4); 7.0780 (1.2); 7.0736 (1.4); 7.0581 (2.9); 7.0536 (2.2); 7.0279 (1.7); 7.0243 (1.8); 7.0096 (1.6); 7.0077 (1.4); 7.0061 (1.6); 6.9897 (0.7); 6.9861 (0.7); 6.9467 (2.2); 6.9433 (1.9); 6.9264 (1.8); 6.9230 (1.5); 6.7492 (3.3); 6.7328 (3.4); 6.2844 (5.8); 5.2990 (10.3); 4.6995 (12.5); 4.3412 (2.0); 4.3389 (1.8); 4.3326 (2.3); 4.3298 (2.4); 4.3277 (2.2); 4.3244 (2.0); 4.3182 (2.2); 4.2275 (16.0); 3.7797 (3.2); 3.7685 (4.4); 3.7572 (2.7); 3.5032 (8.8); 3.5006 (8.2); 1.5497 (2.2); 0.0080 (0.6); -0.0002 (19.0); -0.0085 (0.6) |
| 1.7-42: ¹H-NMR(400.6 MHz, CDCl3): |
| δ= 7.3781 (4.5); 7.3559 (4.5); 7.2599 (18.4); 7.1409 (1.0); 7.1362 (1.1); 7.1226 (1.6); 7.1206 (1.5); 7.1180 (1.8); 7.1161 (1.5); 7.1025 (1.5); 7.0979 (1.8); 7.0779 (1.6); 7.0733 (1.8); 7.0579 (3.8); 7.0533 (2.8); 7.0278 (2.1); 7.0241 (2.2); 7.0095 (1.8); 7.0059 (1.8); 6.9896 (0.8); 6.9859 (0.8); 6.9292 (2.7); 6.9256 (2.4); 6.9089 (2.2); 6.9053 (2.0); 6.7264 (4.3); 6.7099 (4.3); 6.2764 (7.4); 5.2988 (4.6); 4.6998 (16.0); 4.3979 (3.3); 4.3865 (4.0); 4.3827 (2.3); 4.3743 (4.3); 4.2099 (3.9); 4.1977 (3.9); 4.1863 (2.9); 3.4983 (10.2); 3.4955 (10.1); 1.5426 (3.2); 0.0079 (0.8); -0.0002 (26.8); -0.0085 (0.8) |
| 1.7-303: ¹H-NMR(400.6 MHz, CDCl3): |
| δ= 8.4246 (1.5); 8.4193 (1.5); 7.7194 (1.6); 7.7142 (1.6); 7.3442 (1.3); 7.3221 (1.4); 7.2607 (1.4); 7.1166 (0.5); 7.1141 (0.5); 7.1005 (0.5); 7.0961 (0.6); 7.0700 (0.7); 7.0535 (0.8); 7.0495 (0.9); 7.0078 (1.9); 6.9881 (1.6); 6.9852 (0.6); 6.7857 (1.3); 6.7693 (1.3); 6.2609 (2.2); 5.3547 (4.6); 5.2951 (16.0); 4.7924 (4.1); 3.4840 (3.0); 3.4812 (3.0); 2.1676 (1.2); -0.0002 (2.1) |
| 1.7-309: ¹H-NMR(400.6 MHz, CDCI3): |
| δ= 8.0746 (1.0); 8.0728 (1.0); 8.0552 (1.2); 7.8496 (1.2); 7.8301 (2.3); 7.8106 (1.2); 7.4580 (1.4); 7.4382 (1.1); 7.4363 (1.1); 7.3314 (2.1); 7.3093 (2.1); 7.2622 (5.9); 7.1336 (0.5); 7.1195 (0.7); 7.1180 (0.7); 7.1150 (0.9); 7.0997 (0.7); 7.0952 (1.0); 7.0832 (0.8); 7.0788 (0.9); 7.0632 (1.7); 7.0588 (1.2); 7.0246 (0.9); 7.0209 (1.1); 7.0062 (0.8); 7.0026 (0.9); 6.9807 (1.4); 6.9770 (1.1); 6.9604 (1.0); 6.9569 (0.8); 6.7582 (2.0); 6.7417 (2.0); 6.2447 (3.5); 5.3906 (4.8); 5.2988 (16.0); 4.7815 (6.7); 3.9993 (16.0); 3.4804 (4.7); 3.4777 (4.7); 2.0447 (0.9); 1.2587 (0.8); -0.0002 (8.7) |
| 1.7-301: ¹H-NMR(400.6 MHz, CDCl3): |
| δ= 8.3203 (1.8); 8.3144 (1.8); 7.3445 (2.3); 7.3223 (2.3); 7.2606 (4.8); 7.2451 (0.6); 7.2392 (0.6); 7.2250 (0.7); 7.2223 (0.7); 7.2192 (0.7); 7.2164 (0.6); 7.2023 (0.6); 7.1963 (0.6); 7.1211 (0.5); 7.1165 (0.6); 7.1026 (0.8); 7.1009 (0.8); 7.0982 (1.0); 7.0965 (0.9); 7.0826 (0.8); 7.0781 (1.0); 7.0612 (0.9); 7.0568 (1.0); 7.0411 (1.9); 7.0367 (1.4); 7.0033 (1.0); 6.9996 (1.2); 6.9849 (0.9); 6.9834 (0.8); 6.9813 (1.0); 6.9797 (0.9); 6.9449 (1.4); 6.9413 (1.2); 6.9246 (1.1); 6.9210 (1.0); 6.7653 (2.2); 6.7489 (2.2); 6.2790 (3.8); 5.3230 (3.2); 5.3181 (3.1); 5.2977 (16.0); 4.7390 (7.7); 3.4966 (5.2); 3.4938 (5.3); -0.0002 (7.0) |
| 1.7-302: ¹H-NMR(400.6 MHz, CDCl3): |
| δ= 8.3761 (1.4); 8.3714 (1.4); 7.4775 (1.1); 7.4726 (1.0); 7.4552 (1.0); 7.4503 (1.0); 7.3427 (2.0); 7.3205 (2.0); 7.2613 (1.8); 7.1048 (0.7); 7.1003 (0.8); 7.0851 (0.6); 7.0806 (0.8); 7.0642 (0.8); 7.0597 (0.8); 7.0442 (1.6); 7.0397 (1.2); 7.0056 (0.8); 7.0020 (0.9); 6.9870 (0.8); 6.9833 (0.9); 6.9509 (1.2); 6.9473 (1.0); 6.9306 (1.0); 6.9271 (0.8); 6.7651 (1.9); 6.7486 (1.9); 6.2744 (3.4); 5.3193 (3.7); 5.3142 (3.6); 5.2959 (16.0); 4.7451 (6.6); 3.4927 (4.6); 3.4902 (4.6); -0.0002 (2.7) |
| 1.7-308: ¹H-NMR(400.6 MHz, CDCl3): |
| δ= 9.1442 (0.9); 9.1423 (0.9); 9.1389 (0.9); 9.1371 (0.8); 8.2643 (0.8); 8.2590 (0.8); 8.2440 (0.8); 8.2386 (0.8); 7.3285 (1.3); 7.3061 (1.7); 7.2837 (0.9); 7.2819 (0.8); 7.2640 (1.9); 7.1332 (0.5); 7.1315 (0.5); 7.1287 (0.6); 7.1271 (0.5); 7.1087 (0.6); 7.0956 (0.5); 7.0912 (0.6); 7.0756 (1.1); 7.0712 (0.8); 7.0350 (0.6); 7.0313 (0.7); 7.0166 (0.6); 7.0149 (0.5); 7.0129 (0.6); 6.9801 (0.9); 6.9766 (0.7); 6.9598 (0.7); 6.9563 (0.6); 6.7401 (1.3); 6.7238 (1.3); 6.2429 (2.3); 5.3351 (3.7); 5.2973 (16.0); 4.7933 (4.6); 3.9580 (10.0); 3.4795 (3.2); 3.4767 (3.1); -0.0002 (2.6) |
| 1.7-327: ¹H-NMR(400.6 MHz, CDCl3): |
| δ= 8.1776 (1.0); 8.1721 (1.0); 7.7344 (0.7); 7.7282 (0.6); 7.3379 (2.0); 7.3158 (2.0); 7.2625 (2.6); 7.1177 (0.5); 7.1047 (0.7); 7.1024 (0.7); 7.0998 (0.8); 7.0976 (0.7); 7.0846 (0.7); 7.0797 (0.9); 7.0636 (0.6); 7.0587 (0.8); 7.0435 (1.9); 7.0387 (1.3); 7.0239 (1.1); 7.0203 (1.1); 7.0061 (0.8); 7.0027 (0.8); 7.0005 (0.5); 6.9203 (0.9); 6.9098 (1.3); 6.9061 (1.2); 6.8993 (0.8); 6.8899 (1.3); 6.8859 (0.9); 6.6944 (1.9); 6.6780 (1.9); 6.2764 (3.4); 5.2975 (16.0); 5.1583 (4.7); 4.6825 (7.4); 3.4984 (4.8); 3.4956 (4.6); -0.0002 (3.8) |
| 1.7-321: ¹H-NMR(400.6 MHz, CDCl3): |
| δ= 9.1882 (2.7); 8.7020 (6.3); 7.3460 (2.2); 7.3239 (2.2); 7.2628 (5.7); 7.1322 (0.5); 7.1273 (0.6); 7.1142 (0.8); 7.1120 (0.7); 7.1094 (1.0); 7.1072 (0.8); 7.0942 (0.8); 7.0893 (1.1); 7.0802 (0.7); 7.0755 (0.8); 7.0601 (2.0); 7.0554 (1.3); 7.0360 (1.2); 7.0323 (1.3); 7.0180 (0.9); 7.0159 (0.7); 7.0145 (0.9); 7.0122 (0.6); 6.9193 (1.3); 6.9156 (1.2); 6.8990 (1.1); 6.8955 (1.0); 6.6873 (2.1); 6.6709 (2.1); 6.2822 (3.6); 5.2987 (16.0); 5.1788 (5.8); 4.6983 (8.2); 3.4974 (4.8); 3.4945 (4.8); -0.0002 (8.0) |
| 1.7-275: ¹H-NMR(400.6 MHz, CDCl3): |
| δ= 7.3510 (4.6); 7.3288 (4.7); 7.3222 (4.4); 7.3166 (4.3); 7.2606 (16.2); 7.1109 (1.0); 7.1064 (1.2); 7.0924 (1.6); 7.0907 (1.6); 7.0881 (1.9); 7.0863 (1.7); 7.0724 (1.5); 7.0679 (2.0); 7.0601 (1.9); 7.0557 (2.0); 7.0400 (3.8); 7.0357 (2.7); 6.9959 (2.0); 6.9922 (2.2); 6.9774 (1.9); 6.9758 (1.8); 6.9739 (2.0); 6.9575 (1.0); 6.9539 (0.9); 6.9002 (2.7); 6.8966 (2.5); 6.8799 (2.3); 6.8764 (2.1); 6.7910 (4.4); 6.7745 (4.5); 6.2809 (7.8); 6.2216 (4.8); 6.2160 (4.7); 5.2984 (4.0); 5.1674 (14.8); 4.6860 (14.6); 4.0606 (4.3); 4.0429 (6.1); 4.0249 (4.4); 3.4972 (10.6); 3.4944 (10.6); 1.9123 (0.5); 1.8938 (2.3); 1.8756 (4.2); 1.8577 (4.2); 1.8395 (2.5); 1.8213 (0.6); 1.6035 (0.6); 0.9285 (7.7); 0.9101 (16.0); 0.8915 (7.1); 0.0079 (0.7); -0.0002 (23.8); -0.0085 (0.7) |
| 1.7-282: ¹H-NMR(400.6 MHz, CDCI3): |
| δ= 8.5353 (1.6); 8.5216 (1.6); 8.5142 (1.5); 8.5006 (1.5); 7.3386 (4.5); 7.3164 (4.5); 7.2607 (18.9); 7.1491 (1.0); 7.1447 (1.2); 7.1307 (1.5); 7.1290 (1.6); 7.1262 (2.0); 7.1245 (1.9); 7.1106 (1.7); 7.1062 (2.2); 7.0959 (1.9); 7.0916 (2.0); 7.0758 (3.8); 7.0715 (2.8); 7.0333 (2.1); 7.0295 (2.4); 7.0149 (1.9); 7.0134 (1.6); 7.0113 (2.0); 7.0096 (1.8); 6.9949 (1.1); 6.9911 (2.0); 6.9849 (2.0); 6.9837 (2.0); 6.9761 (1.6); 6.9704 (3.5); 6.9669 (3.6); 6.9617 (3.1); 6.9553 (2.3); 6.9503 (2.7); 6.9469 (2.2); 6.9416 (1.4); 6.9354 (0.8); 6.7443 (4.4); 6.7280 (4.4); 6.2554 (7.5); 5.2987 (6.5); 5.2765 (12.6); 4.7837 (16.0); 3.4857 (10.1); 3.4828 (10.5); 2.0446 (0.9); 1.2591 (0.9); 0.8819 (0.5); 0.0080 (0.7); - 0.0002 (28.3); -0.0085 (0.8) |
| 1.7-283: ¹H-NMR(400.6 MHz, CDCl3): |
| δ= 8.4132 (3.2); 8.4060 (3.1); 7.3993 (1.0); 7.3921 (1.0); 7.3783 (1.9); 7.3711 (1.9); 7.3576 (1.5); 7.3504 (1.4); 7.3347 (4.4); 7.3125 (4.6); 7.2753 (2.0); 7.2607 (15.1); 7.2538 (1.4); 7.2429 (1.3); 7.1340 (1.0); 7.1295 (1.2); 7.1157 (1.6); 7.1139 (1.6); 7.1112 (1.9); 7.1094 (1.6); 7.0957 (1.6); 7.0911 (2.0); 7.0802 (1.7); 7.0757 (1.8); 7.0601 (3.8); 7.0557 (2.7); 7.0234 (2.1); 7.0197 (2.3); 7.0051 (1.9); 7.0034 (1.6); 7.0016 (1.9); 6.9998 (1.5); 6.9852 (0.9); 6.9815 (0.9); 6.9488 (2.8); 6.9452 (2.4); 6.9286 (2.3); 6.9250 (1.9); 6.7450 (4.3); 6.7286 (4.4); 6.2580 (7.4); 5.2987 (6.0); 5.2560 (9.9); 4.7531 (16.0); 3.4885 (10.3); 3.4856 (10.2); 0.0079 (0.7); -0.0002 (21.9); -0.0085 (0.6) |
| 1.7-272: ¹H-NMR(400.6 MHz, CDCl3): |
| δ= 7.3497 (2.5); 7.3275 (2.5); 7.2987 (2.4); 7.2932 (2.3); 7.2606 (7.4); 7.2592 (6.0); 7.1135 (0.5); 7.1091 (0.6); 7.0936 (1.1); 7.0911 (1.1); 7.0893 (1.2); 7.0750 (0.8); 7.0706 (1.0); 7.0620 (1.0); 7.0577 (1.0); 7.0419 (2.0); 7.0376 (1.4); 6.9984 (1.0); 6.9949 (1.2); 6.9788 (1.2); 6.9769 (1.2); 6.9751 (1.2); 6.9010 (1.6); 6.8976 (1.4); 6.8808 (1.3); 6.8774 (1.1); 6.7889 (2.3); 6.7724 (2.3); 6.2783 (4.5); 6.2196 (2.6); 6.2141 (2.5); 5.2985 (1.9); 5.2971 (1.5); 5.1564 (8.6); 4.6843 (8.6); 3.8733 (16.0); 3.4954 (6.6); 3.4939 (6.6); 1.5823 (1.7); -0.0002 (10.3); -0.0017 (8.6) |
| 1.7-281: ¹H-NMR(400.6 MHz, CDCl3): |
| δ= 8.4081 (1.4); 8.4047 (2.6); 8.4013 (1.5); 8.3966 (1.5); 8.3931 (2.6); 8.3898 (1.4); 7.4353 (1.2); 7.4320 (1.2); 7.4142 (1.8); 7.4113 (2.6); 7.3908 (1.7); 7.3875 (1.7); 7.3390 (5.1); 7.3243 (1.6); 7.3168 (5.4); 7.3134 (2.9); 7.3027 (2.3); 7.2924 (1.8); 7.2814 (1.0); 7.2607 (10.8); 7.1198 (1.0); 7.1155 (1.2); 7.1004 (1.9); 7.0961 (2.7); 7.0812 (1.6); 7.0768 (2.1); 7.0642 (2.0); 7.0601 (2.2); 7.0445 (3.6); 7.0401 (2.9); 6.9937 (1.8); 6.9901 (2.5); 6.9749 (2.0); 6.9709 (3.1); 6.9655 (3.6); 6.9624 (2.2); 6.9553 (1.0); 6.9517 (1.3); 6.9455 (2.6); 6.9420 (1.9); 6.7865 (4.9); 6.7700 (4.9); 6.2739 (8.6); 5.3626 (8.4); 5.3576 (8.4); 5.2979 (8.6); 4.7639 (16.0); 3.4916 (11.6); 3.4890 (11.9); 1.5990 (0.5); 1.2583 (0.6); -0.0002 (15.5) |
| 1.7-274: ¹H-NMR(400.6 MHz, CDCl3): |
| δ= 7.3503 (4.5); 7.3409 (4.2); 7.3354 (4.2); 7.3281 (4.4); 7.2609 (10.3); 7.1130 (1.0); 7.1085 (1.1); 7.0943 (1.6); 7.0927 (1.6); 7.0900 (2.0); 7.0745 (1.4); 7.0700 (1.9); 7.0612 (1.8); 7.0568 (1.9); 7.0411 (3.6); 7.0368 (2.6); 6.9967 (1.9); 6.9931 (2.0); 6.9781 (1.9); 6.9764 (1.9); 6.9746 (2.0); 6.9583 (0.8); 6.9547 (0.9); 6.9027 (2.6); 6.8993 (2.5); 6.8824 (2.2); 6.8790 (2.0); 6.7927 (4.2); 6.7762 (4.2); 6.2806 (7.7); 6.2278 (4.5); 6.2221 (4.4); 5.2978 (5.0); 5.1687 (14.9); 4.6864 (14.5); 4.6540 (1.2); 4.1711 (2.2); 4.1528 (6.7); 4.1345 (6.8); 4.1162 (2.3); 3.4965 (10.8); 3.4943 (10.9); 1.4903 (7.8); 1.4720 (16.0); 1.4538 (7.6); 1.3176 (0.6); 1.2998 (1.1); 1.2819 (0.6); 1.2587 (0.5); 0.0079 (0.6); -0.0002 (14.7) |
| 1.28-1: ¹H-NMR(400.6 MHz, CDCl3): |
| δ= 7.5410 (1.9); 7.5196 (2.0); 7.2611 (5.0); 7.2457 (1.0); 7.2398 (1.3); 7.2241 (1.0); 7.2182 (1.6); 7.2019 (2.7); 7.1961 (1.8); 6.8176 (2.1); 6.7959 (1.9); 6.7884 (1.9); 6.7724 (1.9); 6.2910 (3.1); 5.2991 (7.9); 4.6734 (6.5); 4.2890 (1.5); 4.2807 (1.0); 4.2775 (1.4); 4.2737 (1.0); 4.2657 (1.7); 3.5648 (2.0); 3.5589 (0.6); 3.5561 (1.1); 3.5531 (1.9); 3.5504 (1.1); 3.5475 (0.6); 3.5415 (1.9); 3.5111 (4.3); 3.5083 (4.3); 3.3374 (16.0); 1.5621 (1.6); -0.0002 (7.3) |
| 1.28-23: ¹H-NMR(400.6 MHz, CDCl3): |
| δ= 7.5421 (1.8); 7.5206 (1.8); 7.2622 (3.9); 7.2457 (1.0); 7.2398 (1.2); 7.2240 (1.0); 7.2181 (1.5); 7.1937 (2.5); 7.1878 (1.8); 6.8275 (2.0); 6.8058 (1.8); 6.7877 (1.8); 6.7717 (1.8); 6.2939 (3.0); 5.2990 (9.0); 4.6669 (5.8); 4.3077 (1.4); 4.2990 (1.1); 4.2959 (1.5); 4.2920 (1.0); 4.2837 (1.5); 3.6901 (1.7); 3.6813 (1.1); 3.6781 (1.7); 3.6750 (1.0); 3.6662 (1.5); 3.6097 (1.2); 3.6008 (1.5); 3.5989 (1.5); 3.5929 (1.4); 3.5865 (2.4); 3.5234 (2.5); 3.5165 (2.3); 3.5118 (5.6); 3.5090 (5.2); 3.5013 (1.2); 3.3704 (16.0); 1.5840 (0.5); -0.0002 (5.8) |
| 1.28-335: ¹H-NMR(400.6 MHz, CDCl3): |
| δ= 8.6662 (1.8); 8.6628 (1.8); 7.7710 (0.9); 7.7657 (0.9); 7.7511 (1.4); 7.7457 (1.4); 7.6918 (2.1); 7.6900 (2.2); 7.6719 (1.3); 7.6700 (1.3); 7.5005 (2.3); 7.4792 (2.3); 7.2615 (7.8); 7.2472 (1.3); 7.2413 (1.6); 7.2257 (1.2); 7.2198 (2.0); 7.2030 (3.2); 7.1973 (2.3); 6.8286 (2.6); 6.8070 (2.3); 6.7010 (2.2); 6.6852 (2.2); 6.2907 (4.1); 5.2998 (16.0); 5.2346 (6.8); 4.6836 (8.4); 3.5174 (6.6); 3.5150 (6.8); 2.0447 (0.8); 1.5586 (3.6); 1.2593 (0.6); -0.0002 (11.4) |
| 1.28-277: ¹H-NMR(400.6 MHz, CDCl3): |
| δ= 8.5959 (0.9); 8.5922 (1.0); 8.5842 (2.0); 8.5804 (1.9); 7.6400 (0.6); 7.6357 (0.8); 7.6302 (0.5); 7.6204 (0.6); 7.6160 (0.9); 7.6106 (0.5); 7.5027 (2.3); 7.4813 (2.3); 7.3050 (0.8); 7.2930 (0.8); 7.2855 (0.7); 7.2734 (0.7); 7.2624 (5.4); 7.2241 (1.2); 7.2182 (1.7); 7.2028 (1.0); 7.1968 (2.4); 7.1900 (3.3); 7.1845 (1.7); 6.7955 (2.3); 6.7741 (2.1); 6.7217 (2.2); 6.7058 (2.2); 6.2901 (3.8); 5.2986 (16.0); 5.1798 (6.2); 4.6679 (8.1); 3.5074 (5.5); 3.5046 (5.3); 2.0442 (0.8); 1.2587 (0.5); -0.0002 (7.9) |
| 1.28-329: ¹H-NMR(400.6 MHz, CDCl3): |
| δ= 8.4757 (0.9); 8.4715 (0.9); 8.4635 (0.9); 8.4593 (0.9); 7.5548 (0.8); 7.5510 (0.8); 7.5358 (0.8); 7.5318 (0.8); 7.4923 (2.2); 7.4708 (2.3); 7.2617 (9.2); 7.2268 (1.3); 7.2209 (1.7); 7.2054 (1.1); 7.1995 (2.4); 7.1902 (3.3); 7.1845 (1.9); 7.1380 (0.7); 7.1257 (0.8); 7.1191 (0.7); 7.1065 (0.7); 6.7987 (2.3); 6.7772 (2.1); 6.7238 (2.2); 6.7078 (2.2); 6.2784 (3.6); 5.2989 (16.0); 5.1863 (5.5); 4.6794 (7.6); 3.5048 (4.7); 3.5019 (4.8); 2.5385 (0.6); 2.5314 (9.6); - 0.0002 (13.7) |
| 1.28-321: ¹H-NMR(400.6 MHz, CDCl3): |
| δ= 9.2019 (5.7); 8.7214 (10.2); 7.5180 (4.4); 7.4966 (4.4); 7.2616 (18.5); 7.2408 (2.1); 7.2349 (3.0); 7.2195 (1.6); 7.2136 (4.2); 7.2073 (6.3); 7.2017 (3.3); 6.8113 (4.5); 6.7900 (4.0); 6.6988 (4.3); 6.6830 (4.3); 6.2974 (7.7); 5.2997 (13.6); 5.1755 (13.1); 4.6772 (0.8); 4.6664 (16.0); 3.5116 (10.7); 3.5092 (11.1); 2.1263 (1.0); 2.0448 (1.2); 1.5804 (2.0); 1.2592 (0.9); 0.0080 (0.9); -0.0002 (26.7); -0.0085 (0.8) |
| 1.28-356: ¹H-NMR(400.6 MHz, CDCl3): |
| δ= 7.5095 (2.6); 7.4880 (2.6); 7.2845 (3.5); 7.2620 (7.3); 7.2138 (1.3); 7.2079 (1.7); 7.1922 (1.3); 7.1863 (2.1); 7.1684 (3.5); 7.1626 (2.4); 6.7658 (2.7); 6.7441 (2.6); 6.7372 (2.4); 6.7212 (2.4); 6.2954 (4.3); 5.2987 (16.0); 5.0093 (6.2); 4.5969 (8.7); 3.8818 (0.6); 3.8037 (0.8); 3.7959 (14.4); 3.5158 (5.7); 3.5133 (6.1); 2.4816 (0.6); 2.2231 (0.6); 2.2132 (14.0); 2.0443 (0.6); 1.2590 (0.6); 0.8817 (0.5); -0.0002 (10.5) |
| 1.28-176: ¹H-NMR(400.6 MHz, CDCl3): |
| δ= 7.5695 (3.3); 7.5481 (3.3); 7.2844 (1.8); 7.2785 (2.4); 7.2604 (13.8); 7.2571 (3.3); 7.2440 (4.5); 7.2383 (2.9); 6.8418 (3.6); 6.8202 (3.3); 6.6984 (3.2); 6.6826 (3.3); 6.3033 (5.7); 5.2995 (9.4); 4.7599 (16.0); 4.7197 (12.8); 3.8618 (1.0); 3.5179 (8.3); 3.5153 (8.3); 2.0448 (0.7); 1.6761 (1.4); 1.5460 (1.7); 1.2592 (0.7); 0.0079 (0.5); -0.0002 (19.1); -0.0085 (0.5) |
| 1.33-1: ¹H-NMR(400.6 MHz, CDCl3): |
| δ= 7.5437 (1.4); 7.5373 (4.6); 7.5320 (0.6); 7.5164 (3.2); 7.5113 (1.0); 7.2616 (8.2); 6.9190 (1.6); 6.8982 (1.5); 6.8153 (1.7); 6.8011 (1.7); 6.2799 (3.3); 5.3000 (6.8); 4.7354 (5.7); 4.2711 (1.6); 4.2633 (1.0); 4.2596 (1.6); 4.2557 (1.0); 4.2480 (1.8); 3.5549 (1.9); 3.5469 (1.2); 3.5433 (2.0); 3.5402 (1.2); 3.5318 (1.9); 3.5053 (4.3); 3.5027 (4.6); 3.3558 (0.5); 3.3509 (0.9); 3.3327 (16.0); 3.3145 (0.6); 2.0447 (0.6); 1.5581 (2.1); -0.0002 (12.0) |
| 1.7-367: ¹H-NMR(400.6 MHz, CDCl3): |
| δ= 7.3490 (2.0); 7.3269 (2.0); 7.2604 (12.8); 7.1098 (0.5); 7.0959 (0.7); 7.0941 (0.7); 7.0915 (0.9); 7.0897 (0.8); 7.0759 (0.8); 7.0711 (1.4); 7.0679 (2.5); 7.0663 (3.9); 7.0496 (1.7); 7.0453 (1.1); 7.0068 (0.9); 7.0031 (1.0); 6.9885 (0.9); 6.9867 (0.8); 6.9850 (0.9); 6.9831 (0.7); 6.9106 (1.2); 6.9069 (1.1); 6.8902 (1.0); 6.8868 (0.9); 6.7768 (1.9); 6.7604 (2.0); 6.2667 (3.4); 5.2992 (0.6); 5.2201 (5.7); 4.7124 (6.9); 3.4909 (4.7); 3.4882 (4.6); 2.6900 (16.0); 1.5681 (0.6); 0.0079 (0.6); -0.0002 (18.1) |
| 1.7-366: ¹H-NMR(400.6 MHz, CDCl3): |
| δ= 8.7702 (4.4); 8.7652 (4.3); 7.3467 (4.4); 7.3246 (4.4); 7.2823 (3.7); 7.2805 (2.7); 7.2773 (3.6); 7.2606 (16.3); 7.1197 (0.9); 7.1152 (1.1); 7.1011 (1.7); 7.0967 (2.1); 7.0813 (1.5); 7.0768 (2.1); 7.0733 (1.9); 7.0688 (1.9); 7.0532 (3.7); 7.0489 (2.6); 7.0112 (2.0); 7.0075 (2.1); 6.9927 (2.0); 6.9892 (2.0); 6.9729 (0.9); 6.9692 (0.9); 6.9157 (2.7); 6.9122 (2.5); 6.8954 (2.3); 6.8920 (2.0); 6.7514 (4.2); 6.7349 (4.3); 6.2679 (7.7); 5.7139 (0.6); 5.3390 (13.4); 5.2989 (0.8); 4.7238 (16.0); 3.4906 (11.2); 3.4882 (11.0); 1.5757 (1.7); 0.0078 (0.8); -0.0002 (23.0); -0.0085 (0.7) |
| 1.7-371: ¹H-NMR(400.6 MHz, CDCl3): |
| δ= 7.8613 (5.2); 7.3599 (3.0); 7.3377 (3.0); 7.2612 (11.0); 7.1251 (4.0); 7.1099 (1.1); 7.1076 (1.0); 7.1050 (1.3); 7.1028 (1.1); 7.0897 (1.1); 7.0848 (1.4); 7.0655 (0.9); 7.0608 (1.2); 7.0455 (2.7); 7.0408 (1.9); 7.0240 (1.6); 7.0204 (1.8); 7.0061 (1.3); 7.0025 (1.3); 7.0003 (0.8); 6.9861 (0.6); 6.9824 (0.6); 6.9163 (1.8); 6.9128 (1.6); 6.8961 (1.5); 6.8925 (1.3); 6.7124 (2.9); 6.6960 (2.9); 6.2843 (4.9); 5.2988 (16.0); 5.1864 (8.4); 4.6786 (11.3); 3.5021 (6.7); 3.4992 (6.8); 2.0444 (1.4); 1.2589 (0.9); -0.0002 (15.3) |
| 1.7-365: ¹H-NMR(400.6 MHz, CDCl3): |
| δ= 7.8420 (2.0); 7.6585 (2.3); 7.6564 (2.1); 7.3567 (2.1); 7.3345 (2.1); 7.2623 (3.1); 7.1153 (0.5); 7.1016 (0.7); 7.0997 (0.7); 7.0970 (0.9); 7.0951 (0.7); 7.0815 (0.7); 7.0769 (0.9); 7.0637 (0.8); 7.0591 (0.8); 7.0436 (1.8); 7.0391 (1.3); 7.0104 (1.0); 7.0067 (1.0); 6.9921 (0.8); 6.9901 (0.8); 6.9885 (0.8); 6.9868 (0.6); 6.9106 (1.2); 6.9070 (1.1); 6.8903 (1.0); 6.8868 (0.9); 6.7496 (2.0); 6.7331 (2.0); 6.2801 (3.4); 5.2977 (16.0); 5.1215 (5.2); 4.6918 (7.4); 3.4973 (4.6); 3.4945 (4.6); 2.0437 (0.8); 1.2584 (0.5); -0.0002 (4.5) |
| 1.7-328: ¹H-NMR(400.6 MHz, CDCl3): |
| δ= 8.4421 (2.8); 8.4353 (2.8); 8.3820 (2.8); 7.3698 (0.9); 7.3638 (1.2); 7.3587 (0.9); 7.3477 (1.0); 7.3416 (1.3); 7.3342 (4.6); 7.3120 (4.4); 7.2610 (14.5); 7.1350 (0.9); 7.1303 (1.1); 7.1168 (1.4); 7.1149 (1.4); 7.1122 (1.7); 7.1103 (1.5); 7.0968 (1.4); 7.0921 (1.9); 7.0819 (1.4); 7.0772 (1.7); 7.0618 (3.8); 7.0572 (2.6); 7.0354 (2.1); 7.0317 (2.2); 7.0172 (1.7); 7.0140 (1.7); 6.9973 (0.8); 6.9936 (0.7); 6.9242 (2.6); 6.9206 (2.4); 6.9040 (2.2); 6.9004 (1.9); 6.6968 (4.2); 6.6804 (4.2); 6.2770 (7.3); 5.2990 (10.6); 5.1953 (11.7); 4.7058 (16.0); 3.4963 (9.9); 3.4936 (10.0); 0.0079 (0.7); -0.0002 (21.0); -0.0085 (0.6) |
| 1.7-296: ¹H-NMR(400.6 MHz, CDCl3): |
| δ= 8.4873 (1.6); 8.4775 (1.6); 7.5620 (1.4); 7.5445 (1.6); 7.3720 (0.7); 7.3498 (0.7); 7.3185 (4.0); 7.2963 (4.0); 7.2607 (13.9); 7.2563 (1.7); 7.2438 (1.6); 7.2368 (1.4); 7.2246 (1.4); 7.1189 (1.0); 7.1145 (1.1); 7.0996 (1.8); 7.0969 (1.5); 7.0936 (1.4); 7.0804 (1.4); 7.0759 (1.8); 7.0656 (0.5); 7.0577 (1.5); 7.0529 (1.7); 7.0366 (3.3); 7.0326 (2.5); 7.0072 (0.8); 6.9955 (1.3); 6.9919 (2.6); 6.9832 (2.9); 6.9797 (2.9); 6.9770 (1.8); 6.9740 (2.5); 6.9630 (1.9); 6.9591 (2.0); 6.9530 (0.5); 6.7818 (3.8); 6.7653 (3.8); 6.7415 (0.6); 6.7250 (0.6); 6.2709 (1.2); 6.2583 (6.8); 5.3176 (12.0); 5.2983 (14.0); 4.8921 (0.6); 4.7658 (9.3); 4.6549 (1.6); 3.4828 (10.4); 3.4800 (9.9); 2.4083 (1.0); 2.3277 (16.0); 0.0080 (0.7); -0.0002 (20.0); -0.0085 (0.6) |
| 1.7-284: ¹H-NMR(400.6 MHz, CDCl3): |
| δ= 8.3421 (2.8); 8.3360 (2.9); 7.5983 (1.9); 7.5922 (1.8); 7.5779 (2.2); 7.5716 (2.1); 7.3366 (4.3); 7.3143 (5.1); 7.3118 (4.0); 7.2912 (3.1); 7.2609 (16.2); 7.1270 (0.9); 7.1221 (1.1); 7.1091 (1.6); 7.1067 (1.5); 7.1042 (1.9); 7.1019 (1.5); 7.0891 (1.5); 7.0841 (2.0); 7.0696 (1.2); 7.0648 (1.6); 7.0496 (3.9); 7.0448 (2.7); 7.0292 (2.3); 7.0255 (2.4); 7.0113 (1.9); 7.0077 (1.8); 7.0055 (1.1); 6.9912 (0.8); 6.9876 (0.7); 6.9120 (2.6); 6.9086 (2.4); 6.8918 (2.2); 6.8884 (1.9); 6.6922 (4.2); 6.6758 (4.2); 6.2749 (7.3); 5.2989 (7.3); 5.1512 (12.0); 4.6840 (16.0); 3.9833 (0.5); 3.4973 (10.6); 3.4945 (10.1); 1.4107 (1.6); 1.2937 (0.6); 1.2555 (1.4); 0.0080 (0.6); -0.0002 (23.6); -0.0085 (0.7) |
| 1.19-365: ¹H-NMR(400.6 MHz, CDCl3): |
| δ= 7.8368 (4.6); 7.6722 (0.6); 7.6701 (0.6); 7.6644 (4.9); 7.6622 (5.2); 7.3495 (4.4); 7.3274 (4.4); 7.2619 (11.4); 7.1100 (1.0); 7.0952 (1.1); 7.0888 (2.3); 7.0740 (2.4); 7.0676 (1.4); 7.0527 (1.3); 6.8777 (1.3); 6.8744 (1.6); 6.8534 (2.2); 6.8512 (2.1); 6.8331 (1.2); 6.8298 (1.2); 6.6845 (2.9); 6.6684 (3.0); 6.6540 (1.3); 6.6503 (2.3); 6.6469 (1.4); 6.6328 (1.2); 6.6291 (2.1); 6.6258 (1.2); 6.3230 (0.6); 6.2666 (7.4); 5.2988 (16.0); 5.1279 (0.9); 5.1054 (11.5); 4.6768 (15.9); 3.5313 (0.8); 3.5285 (0.8); 3.4898 (10.0); 3.4870 (10.5); 2.0443 (1.6); 1.5868 (6.9); 1.2767 (0.5); 1.2589 (1.2); -0.0002 (9.9) |
| 1.19-89: ¹H-NMR(400.6 MHz, CDCl3): |
| δ= 7.3737 (4.6); 7.3516 (4.6); 7.2611 (15.7); 7.1447 (1.4); 7.1299 (1.2); 7.1235 (2.5); 7.1088 (2.4); 7.1023 (1.5); 7.0876 (1.3); 6.8975 (1.4); 6.8942 (1.4); 6.8739 (2.1); 6.8710 (1.8); 6.8529 (1.4); 6.8496 (1.2); 6.6832 (1.5); 6.6796 (2.4); 6.6762 (1.4); 6.6594 (4.2); 6.6551 (1.4); 6.6444 (2.8); 6.3291 (0.6); 6.2758 (7.6); 5.2990 (13.7); 4.7661 (3.8); 4.7500 (4.4); 4.7468 (4.5); 4.7306 (4.1); 4.6583 (16.0); 4.3980 (2.5); 4.3892 (2.7); 4.3830 (4.4); 4.3739 (4.3); 4.3677 (7.4); 4.3586 (2.5); 4.3510 (5.1); 3.5369 (0.8); 3.5341 (0.8); 3.4972 (10.5); 3.4943 (10.5); 3.2693 (0.9); 3.2666 (0.9); 3.2647 (0.8); 3.2497 (1.4); 3.2349 (0.8); 3.2329 (0.9); 3.2303 (0.7); 2.0445 (1.5); 1.5667 (5.5); 1.5633 (6.1); 1.2768 (0.5); 1.2590 (1.1); -0.0002 (16.0) |
| 1.8-329: ¹H-NMR(400.6 MHz, CDCl3): |
| δ= 8.4643 (0.9); 8.4601 (0.9); 8.4521 (0.9); 8.4478 (0.8); 7.5504 (0.8); 7.5465 (0.7); 7.5312 (0.8); 7.5274 (0.8); 7.4805 (2.2); 7.4590 (2.2); 7.2613 (8.2); 7.1240 (1.1); 7.1190 (0.7); 7.1129 (0.8); 7.1056 (1.4); 7.1038 (1.0); 7.1009 (1.0); 7.0988 (0.9); 7.0938 (0.7); 7.0856 (0.8); 7.0808 (1.1); 7.0730 (0.8); 7.0684 (0.9); 7.0529 (1.9); 7.0484 (1.3); 7.0248 (1.1); 7.0210 (1.2); 7.0067 (0.9); 7.0046 (0.8); 7.0031 (0.9); 7.0011 (0.7); 6.9146 (1.2); 6.9110 (1.2); 6.8944 (1.1); 6.8908 (0.9); 6.6774 (2.1); 6.6613 (2.2); 6.2586 (3.5); 5.2982 (16.0); 5.1874 (5.4); 4.7082 (7.5); 3.4869 (4.7); 3.4840 (4.8); 2.5169 (9.6); 2.0442 (0.6); 1.2588 (0.9); -0.0002 (11.5) |
| 1.7-329: ¹H-NMR(400.6 MHz, CDCl3): |
| δ= 8.4641 (0.8); 8.4599 (0.9); 8.4519 (0.9); 8.4477 (0.8); 7.5509 (0.8); 7.5469 (0.8); 7.5317 (0.8); 7.5277 (0.8); 7.3168 (2.2); 7.2946 (2.2); 7.2615 (6.5); 7.1234 (0.8); 7.1201 (0.7); 7.1151 (0.7); 7.1113 (0.8); 7.1022 (1.1); 7.1000 (0.8); 7.0972 (1.0); 7.0950 (1.0); 7.0819 (0.8); 7.0771 (1.0); 7.0674 (0.7); 7.0626 (0.9); 7.0473 (2.0); 7.0426 (1.3); 7.0224 (1.1); 7.0187 (1.2); 7.0044 (0.8); 7.0009 (0.9); 6.9988 (0.7); 6.9098 (1.3); 6.9061 (1.2); 6.8895 (1.1); 6.8860 (1.0); 6.7134 (2.1); 6.6971 (2.0); 6.2628 (3.6); 5.2980 (16.0); 5.1883 (5.5); 4.7070 (7.6); 3.4895 (4.8); 3.4867 (5.0); 2.5155 (9.7); 2.0440 (0.6); 1.2586 (0.8); -0.0002 (9.3) |
| 1.8-321: ¹H-NMR(400.6 MHz, CDCl3): |
| δ= 9.1886 (2.6); 8.7036 (5.9); 7.5061 (2.0); 7.4846 (2.0); 7.2617 (5.9); 7.1179 (0.6); 7.1161 (0.7); 7.1134 (0.8); 7.1116 (0.8); 7.0980 (0.6); 7.0933 (0.9); 7.0867 (0.7); 7.0820 (0.8); 7.0666 (1.7); 7.0621 (1.2); 7.0386 (0.9); 7.0350 (1.0); 7.0205 (0.8); 7.0171 (0.8); 6.9239 (1.2); 6.9203 (1.1); 6.9036 (1.0); 6.9002 (0.9); 6.6470 (2.0); 6.6310 (2.0); 6.2777 (3.4); 5.2989 (16.0); 5.1759 (5.8); 4.6975 (7.4); 3.4945 (4.7); 3.4919 (4.9); 1.2580 (0.8); -0.0002 (8.2) |
| 1.8-367: ¹H-NMR(400.6 MHz, CDCl3): |
| δ= 7.5108 (2.0); 7.4892 (2.0); 7.2614 (4.9); 7.1113 (0.5); 7.0974 (0.7); 7.0956 (0.7); 7.0930 (0.8); 7.0912 (0.7); 7.0774 (0.7); 7.0732 (1.6); 7.0693 (1.1); 7.0649 (3.2); 7.0536 (1.6); 7.0494 (1.0); 7.0074 (0.9); 7.0037 (1.0); 6.9890 (0.8); 6.9873 (0.7); 6.9856 (0.8); 6.9837 (0.6); 6.9135 (1.2); 6.9099 (1.0); 6.8932 (1.0); 6.8898 (0.8); 6.7433 (1.9); 6.7271 (1.9); 6.2620 (3.2); 5.2979 (16.0); 5.2182 (5.1); 4.7129 (6.5); 3.4875 (4.3); 3.4847 (4.4); 2.6988 (0.6); 2.6884 (15.2); 2.0440 (0.6); 1.2586 (0.5); -0.0002 (6.9) |
| 1.19-367: ¹H-NMR(400.6 MHz, CDCl3): |
| δ= 7.3414 (2.0); 7.3193 (1.9); 7.2612 (7.5); 7.0981 (0.5); 7.0832 (0.7); 7.0769 (1.2); 7.0706 (3.4); 7.0620 (1.1); 7.0557 (0.6); 7.0409 (0.6); 6.8707 (0.6); 6.8675 (0.7); 6.8467 (0.9); 6.8443 (0.8); 6.8262 (0.5); 6.8230 (0.5); 6.7339 (1.2); 6.7178 (1.2); 6.6532 (0.6); 6.6496 (1.1); 6.6461 (0.6); 6.6321 (0.6); 6.6284 (0.9); 6.2500 (3.3); 5.2987 (0.9); 5.2258 (0.5); 5.1993 (5.2); 4.6972 (6.8); 3.4813 (4.4); 3.4784 (4.4); 2.6971 (1.4); 2.6830 (16.0); 1.5873 (1.5); - 0.0002 (7.3) |
| 1.19-71: ¹H-NMR(400.6 MHz, CDCl3): |
| δ= 7.3585 (3.6); 7.3364 (3.6); 7.2617 (8.4); 7.1322 (0.9); 7.1173 (1.0); 7.1110 (1.9); 7.0961 (2.0); 7.0897 (1.1); 7.0749 (1.1); 6.8779 (1.2); 6.8746 (1.2); 6.8564 (1.3); 6.8543 (1.7); 6.8513 (1.4); 6.8333 (1.0); 6.8300 (0.9); 6.7228 (2.1); 6.7067 (2.1); 6.6825 (1.0); 6.6792 (1.7); 6.6760 (0.9); 6.6613 (1.0); 6.6580 (1.6); 6.2654 (3.3); 6.2611 (3.1); 5.2984 (16.0); 4.6905 (10.2); 4.1880 (1.0); 4.1815 (0.8); 4.1689 (1.2); 4.1624 (1.4); 4.1543 (1.0); 4.1047 (0.9); 4.0893 (1.9); 4.0742 (1.7); 4.0685 (1.0); 4.0620 (1.0); 4.0574 (2.6); 4.0501 (0.8); 4.0412 (0.9); 3.8169 (1.0); 3.8124 (0.7); 3.8004 (1.1); 3.7959 (1.7); 3.7838 (0.6); 3.7794 (1.7); 3.7713 (0.6); 3.7631 (0.7); 3.7595 (0.6); 3.7544 (1.2); 3.7427 (1.1); 3.7379 (0.9); 3.7337 (0.6); 3.7262 (0.6); 3.7220 (0.5); 3.5321 (0.6); 3.4884 (7.9); 2.0441 (0.5); 1.9711 (0.6); 1.9540 (0.8); 1.9372 (0.5); 1.9016 (0.6); 1.8931 (0.6); 1.8855 (1.3); 1.8767 (1.3); 1.8665 (1.8); 1.8580 (1.6); 1.8497 (1.4); 1.8412 (1.2); 1.5827 (2.6); 1.5802 (2.4); 1.5542 (0.6); 1.5398 (0.5); 1.5362 (0.6); 1.5259 (0.5); 1.5196 (0.5); -0.0002 (8.4) |
| 1.19-72: ¹H-NMR(400.6 MHz, CDCl3): |
| δ= 7.3676 (4.6); 7.3455 (4.6); 7.2614 (13.6); 7.1443 (1.1); 7.1295 (1.1); 7.1231 (2.3); 7.1083 (2.2); 7.1019 (1.4); 7.0871 (1.2); 6.8928 (1.4); 6.8896 (1.5); 6.8690 (2.2); 6.8664 (1.9); 6.8483 (1.3); 6.8450 (1.2); 6.6725 (3.8); 6.6576 (2.5); 6.6553 (2.5); 6.6507 (2.0); 6.2834 (3.9); 6.2645 (3.9); 5.2988 (15.7); 4.6476 (16.0); 4.1663 (0.8); 4.1499 (0.9); 4.1396 (1.8); 4.1234 (1.9); 4.1130 (1.4); 4.0968 (1.3); 4.0482 (1.4); 4.0280 (1.5); 4.0227 (1.7); 4.0021 (1.7); 3.9961 (0.9); 3.9757 (0.9); 3.8465 (0.8); 3.8330 (0.9); 3.8256 (1.8); 3.8120 (1.8); 3.8052 (1.3); 3.7916 (1.2); 3.7564 (1.1); 3.7535 (1.0); 3.7384 (2.2); 3.7313 (1.4); 3.7173 (2.6); 3.7002 (1.6); 3.6800 (0.7); 3.5342 (0.7); 3.5314 (0.8); 3.5194 (1.1); 3.5063 (1.6); 3.4931 (10.5); 3.4906 (10.5); 3.4785 (1.1); 3.4654 (1.0); 2.5513 (0.7); 2.5343 (0.9); 2.5168 (0.7); 2.0443 (0.9); 2.0149 (0.6); 2.0020 (0.9); 1.9923 (0.6); 1.9830 (0.9); 1.9699 (0.7); 1.5696 (4.7); 1.5604 (1.2); 1.5423 (1.0); 1.5282 (0.7); 1.2589 (0.7); -0.0002 (13.9) |
| I.19-41: ¹H-NMR(400.6 MHz, CDCl3): |
| δ= 7.3695 (4.2); 7.3474 (4.2); 7.2606 (7.4); 7.1430 (1.3); 7.1281 (1.2); 7.1217 (2.4); 7.1069 (2.3); 7.1005 (1.4); 7.0858 (1.2); 6.8983 (1.5); 6.8950 (1.4); 6.8748 (2.4); 6.8719 (1.7); 6.8537 (1.4); 6.8504 (1.2); 6.6826 (1.7); 6.6791 (2.4); 6.6756 (1.3); 6.6614 (1.7); 6.6579 (2.3); 6.6542 (1.4); 6.6485 (3.0); 6.6324 (2.9); 6.3194 (0.6); 6.2610 (7.2); 5.2979 (11.5); 4.6900 (16.0); 4.3688 (2.5); 4.3590 (3.4); 4.3457 (2.8); 4.1293 (5.0); 4.1246 (1.8); 4.1198 (4.0); 4.1174 (5.0); 4.1105 (1.7); 4.1060 (3.8); 3.5314 (1.0); 3.4909 (11.8); 3.4882 (10.6); 1.5608 (3.2); -0.0002 (7.3) |
| 1.19-356: ¹H-NMR(400.6 MHz, CDCl3): |
| δ= 7.3264 (2.7); 7.3036 (4.2); 7.3018 (4.0); 7.2621 (7.2); 7.1054 (0.6); 7.0905 (0.8); 7.0842 (1.4); 7.0694 (1.4); 7.0630 (0.9); 7.0482 (0.8); 6.8779 (0.8); 6.8746 (0.9); 6.8542 (1.3); 6.8515 (1.1); 6.8333 (0.7); 6.8300 (0.7); 6.6562 (1.7); 6.6400 (1.7); 6.6260 (0.9); 6.6224 (1.5); 6.6190 (0.9); 6.6048 (0.8); 6.6012 (1.4); 6.5978 (0.8); 6.2647 (4.6); 5.2982 (10.8); 5.0219 (0.6); 5.0032 (4.8); 4.9999 (4.7); 4.6046 (1.0); 4.5971 (9.5); 3.7989 (16.0); 3.4934 (6.2); 3.4907 (6.3); 2.2045 (15.4); 2.1957 (1.3); 2.0441 (1.6); 1.6165 (0.7); 1.2766 (0.5); 1.2588 (1.3); -0.0002 (7.9) |
| 1.19-278: ¹H-NMR(400.6 MHz, CDCl3): |
| δ= 8.5908 (3.1); 8.5758 (3.2); 7.3045 (3.6); 7.2823 (3.6); 7.2622 (10.0); 7.1647 (3.9); 7.1607 (2.4); 7.1537 (2.6); 7.1497 (3.6); 7.1421 (1.0); 7.1272 (1.0); 7.1208 (1.9); 7.1061 (2.0); 7.0996 (1.2); 7.0849 (1.1); 6.9141 (1.1); 6.9108 (1.2); 6.8906 (1.7); 6.8877 (1.4); 6.8695 (1.0); 6.8662 (0.9); 6.6965 (1.2); 6.6930 (2.0); 6.6895 (1.1); 6.6754 (1.2); 6.6718 (1.8); 6.6684 (1.0); 6.6335 (2.3); 6.6175 (2.3); 6.2289 (6.2); 5.2983 (16.0); 5.1763 (0.8); 5.1640 (5.8); 4.7277 (13.4); 3.5208 (0.6); 3.5180 (0.6); 3.4734 (8.4); 3.4705 (8.5); 2.0440 (1.5); 1.2765 (0.5); 1.2586 (1.5); 1.2408 (0.5); - 0.0002 (10.2) |
| 1.19-321: ¹H-NMR(400.6 MHz, CDCl3): |
| δ= 9.1915 (6.2); 8.6951 (14.7); 7.3396 (4.0); 7.3176 (4.1); 7.2621 (11.5); 7.1265 (0.9); 7.1194 (0.5); 7.1116 (1.0); 7.1052 (2.2); 7.0905 (2.2); 7.0843 (1.6); 7.0693 (1.2); 6.9045 (1.9); 6.8815 (2.9); 6.8600 (1.5); 6.6615 (2.8); 6.6403 (2.6); 6.5965 (3.3); 6.5805 (3.4); 6.3217 (0.6); 6.2683 (7.9); 5.2993 (11.2); 5.1770 (1.2); 5.1659 (14.4); 4.6747 (16.0); 3.5259 (1.2); 3.4877 (15.0); 2.1254 (0.8); 2.0443 (1.1); 1.5879 (8.5); 1.2767 (0.5); 1.2562 (1.8); 1.2412 (0.6); -0.0002 (12.3); -0.0021 (7.5); -0.0081 (0.8) |
| 1.19-277: ¹H-NMR(400.6 MHz, CDCl3): |
| δ= 8.5914 (1.6); 8.5878 (1.7); 8.5794 (1.7); 8.5757 (1.7); 8.5610 (2.2); 8.5562 (2.2); 7.6358 (1.0); 7.6303 (1.6); 7.6259 (1.1); 7.6162 (1.2); 7.6108 (1.8); 7.6063 (1.2); 7.3208 (4.4); 7.2988 (5.1); 7.2901 (1.6); 7.2826 (1.5); 7.2705 (1.3); 7.2688 (1.3); 7.2617 (14.3); 7.1162 (1.1); 7.1014 (1.1); 7.0950 (2.4); 7.0802 (2.4); 7.0738 (1.5); 7.0590 (1.3); 6.8933 (1.3); 6.8900 (1.4); 6.8692 (2.0); 6.8668 (1.8); 6.8487 (1.3); 6.8454 (1.2); 6.6563 (1.4); 6.6526 (2.4); 6.6492 (1.3); 6.6316 (4.9); 6.6157 (2.7); 6.3175 (0.6); 6.2567 (7.4); 5.2985 (15.0); 5.1873 (1.0); 5.1718 (10.9); 4.6738 (16.0); 3.5267 (0.8); 3.5238 (0.9); 3.4834 (9.8); 3.4805 (10.2); 2.0441 (1.3); 1.6578 (0.8); 1.2588 (1.3); -0.0002 (14.2) |
| 1.28-276: ¹H-NMR(400.6 MHz, CDCl3): |
| δ= 8.5994 (1.0); 8.5950 (1.3); 8.5905 (0.6); 8.5852 (1.3); 8.5811 (0.9); 7.7361 (0.8); 7.7316 (0.8); 7.7168 (1.6); 7.7124 (1.3); 7.6975 (0.9); 7.6931 (0.9); 7.5037 (3.1); 7.4822 (3.1); 7.2866 (1.1); 7.2765 (2.6); 7.2673 (1.2); 7.2605 (19.9); 7.2571 (2.8); 7.2314 (1.5); 7.2255 (2.4); 7.2072 (3.6); 7.2053 (8.6); 7.2016 (1.7); 6.8495 (0.6); 6.8456 (2.1); 6.8434 (1.6); 6.8255 (1.5); 6.8233 (1.8); 6.7760 (3.0); 6.7600 (3.0); 6.2617 (5.0); 5.2992 (16.0); 5.2877 (8.1); 4.7507 (10.0); 3.4939 (6.7); 3.4910 (6.6); 0.0079 (0.8); -0.0002 (26.9); -0.0085 (0.7) |
| 1.28-331: ¹H-NMR(400.6 MHz, CDCl3): |
| δ= 8.3728 (0.9); 8.3680 (0.9); 8.3608 (0.9); 8.3560 (0.8); 7.6663 (0.9); 7.6615 (0.9); 7.6474 (1.0); 7.6426 (0.9); 7.5070 (2.1); 7.4856 (2.1); 7.2607 (10.8); 7.2489 (1.3); 7.2432 (1.8); 7.2384 (1.1); 7.2271 (1.6); 7.2216 (1.9); 7.2110 (3.0); 7.2053 (1.7); 6.8330 (2.2); 6.8114 (2.0); 6.7375 (2.0); 6.7216 (2.0); 6.2751 (3.6); 5.2994 (16.0); 5.2623 (5.5); 4.7243 (7.1); 3.5053 (5.0); 3.5027 (5.1); 0.0079 (0.6); -0.0002 (15.5) |
| 1.28-278: ¹H-NMR(400.6 MHz, CDCl3): |
| δ= 8.6615 (1.8); 7.4808 (4.5); 7.4594 (4.5); 7.3411 (3.2); 7.3284 (3.0); 7.2607 (34.2); 7.2442 (2.0); 7.2383 (3.7); 7.2257 (6.1); 7.2200 (3.9); 6.8507 (4.9); 6.8292 (4.4); 6.7067 (4.3); 6.6908 (4.4); 6.2779 (8.1); 5.2996 (14.3); 5.2322 (11.2); 4.7403 (0.6); 4.7290 (16.0); 3.5080 (12.2); 1.2548 (0.9); 0.0079 (1.3); -0.0002 (47.4); -0.0084 (1.7) |
| 1.33-176: ¹H-NMR(400.6 MHz, CDCl3): |
| δ= 7.5787 (0.8); 7.5739 (3.4); 7.5673 (2.6); 7.5625 (2.7); 7.5562 (2.0); 7.5534 (2.2); 7.2610 (10.4); 6.9575 (1.8); 6.9350 (1.7); 6.7064 (1.8); 6.6923 (1.8); 6.3030 (3.4); 5.3000 (16.0); 4.8011 (0.5); 4.7816 (6.8); 4.7641 (9.1); 3.5165 (4.7); 3.5139 (4.9); 0.0080 (0.5); -0.0002 (15.0) |
| 1.33-276: ¹H-NMR(400.6 MHz, CDCl3): |
| δ= 8.5751 (1.7); 8.5713 (2.2); 8.5685 (1.7); 8.5633 (1.7); 8.5593 (2.1); 8.5567 (1.7); 7.7441 (1.4); 7.7397 (1.4); 7.7249 (3.1); 7.7205 (3.0); 7.7056 (1.9); 7.7012 (1.8); 7.5346 (5.7); 7.5296 (7.4); 7.5229 (0.8); 7.5199 (0.6); 7.5063 (3.8); 7.5012 (2.7); 7.4899 (5.3); 7.4849 (4.0); 7.4798 (3.3); 7.4691 (5.1); 7.2901 (2.0); 7.2777 (4.2); 7.2753 (4.3); 7.2616 (26.1); 6.9698 (5.2); 6.9484 (4.9); 6.8246 (4.9); 6.8103 (5.0); 6.7850 (0.5); 6.2396 (9.4); 6.2222 (0.9); 5.3041 (0.6); 5.2480 (11.8); 4.8297 (0.7); 4.8111 (15.6); 4.7169 (1.0); 3.5329 (0.5); 3.4815 (12.4); 3.4789 (12.8); 2.0068 (16.0); 0.0080 (1.0); -0.0002 (36.2); -0.0085 (1.1) |
| 1.23-89: ¹H-NMR(400.6 MHz, CDCl3): |
| δ= 7.4027 (3.1); 7.3807 (3.1); 7.2613 (12.8); 6.9584 (1.2); 6.9455 (1.4); 6.9359 (1.8); 6.9231 (1.8); 6.8450 (3.0); 6.8346 (1.2); 6.8286 (3.4); 6.8154 (1.2); 6.8122 (0.8); 6.8079 (1.4); 6.8048 (1.1); 6.7930 (0.8); 6.7855 (1.0); 6.7682 (1.8); 6.7609 (1.4); 6.7463 (1.9); 6.7390 (1.4); 6.3106 (5.2); 5.2995 (16.0); 4.7838 (3.5); 4.7679 (4.0); 4.7642 (3.9); 4.7483 (3.8); 4.6525 (11.8); 4.6313 (0.7); 4.4265 (1.7); 4.4211 (1.6); 4.4112 (2.9); 4.4059 (2.7); 4.3957 (1.8); 4.3892 (5.8); 4.3722 (5.4); 3.5230 (7.0); 3.5201 (7.0); 3.2919 (0.6); 3.2891 (0.6); 3.2872 (0.6); 3.2723 (1.0); 3.2575 (0.6); 3.2555 (0.6); 3.2528 (0.5); 1.5639 (2.5); 1.2592 (0.5); 0.0079 (0.5); -0.0002 (19.5); -0.0085 (0.5) |
| 1.23-356: ¹H-NMR(400.6 MHz, CDCl3): |
| δ= 7.3589 (2.6); 7.3368 (2.6); 7.3039 (3.5); 7.2620 (9.5); 6.9067 (1.0); 6.8939 (1.1); 6.8843 (1.5); 6.8714 (1.5); 6.8260 (2.5); 6.8096 (2.6); 6.7994 (0.9); 6.7920 (1.1); 6.7802 (0.9); 6.7770 (0.6); 6.7727 (1.1); 6.7697 (1.0); 6.7578 (0.6); 6.7503 (0.8); 6.7424 (1.5); 6.7351 (1.1); 6.7205 (1.6); 6.7131 (1.1); 6.3052 (4.3); 5.2990 (16.0); 5.0302 (0.7); 5.0223 (7.7); 4.5958 (9.9); 4.5769 (0.7); 3.8026 (0.5); 3.7952 (15.3); 3.5214 (5.8); 3.5185 (5.9); 2.2193 (14.8); 2.2150 (1.4); -0.0002 (14.0) |
| 1.8-331: ¹H-NMR(400.6 MHz, CDCl3): |
| δ= 8.3728 (0.6); 7.6768 (1.2); 7.6721 (1.1); 7.6578 (1.3); 7.6557 (1.0); 7.6531 (1.2); 7.4953 (3.2); 7.4738 (3.2); 7.2611 (13.8); 7.2525 (1.0); 7.2455 (0.9); 7.2335 (0.8); 7.1500 (0.7); 7.1454 (0.8); 7.1316 (1.1); 7.1297 (1.1); 7.1271 (1.4); 7.1253 (1.1); 7.1116 (1.1); 7.1071 (1.4); 7.0969 (1.2); 7.0924 (1.3); 7.0768 (2.6); 7.0723 (1.9); 7.0420 (1.4); 7.0383 (1.6); 7.0237 (1.3); 7.0217 (1.2); 7.0201 (1.3); 7.0183 (1.0); 7.0037 (0.6); 7.0000 (0.6); 6.9528 (1.8); 6.9492 (1.7); 6.9326 (1.5); 6.9290 (1.3); 6.6888 (3.1); 6.6727 (3.1); 6.2592 (5.2); 5.2993 (16.0); 5.2691 (7.9); 4.7544 (10.8); 3.4888 (7.2); 3.4860 (7.0); 0.0078 (0.6); -0.0002 (20.9); -0.0021 (1.1); -0.0029 (0.7); -0.0085 (0.6) |
| 1.23-41: ¹H-NMR(400.6 MHz, CDCl3): |
| δ= 7.3988 (4.1); 7.3767 (4.0); 7.2606 (12.5); 6.9624 (1.6); 6.9496 (1.7); 6.9399 (2.3); 6.9271 (2.3); 6.8355 (1.4); 6.8297 (4.2); 6.8163 (1.8); 6.8134 (4.8); 6.8089 (1.9); 6.8058 (1.5); 6.7939 (1.0); 6.7864 (1.3); 6.7780 (2.4); 6.7707 (1.7); 6.7561 (2.4); 6.7488 (1.7); 6.3014 (6.6); 5.2989 (13.5); 4.6813 (16.0); 4.6568 (1.0); 4.3906 (2.1); 4.3794 (2.8); 4.3677 (2.6); 4.1536 (4.8); 4.1483 (1.6); 4.1453 (2.2); 4.1418 (4.2); 4.1393 (2.4); 4.1362 (1.4); 4.1304 (4.0); 3.5423 (0.6); 3.5393 (0.6); 3.5184 (9.0); 3.5154 (9.2); 1.5519 (2.6); 0.0079 (0.5); -0.0002 (18.7); -0.0027 (0.8); -0.0085 (0.5) |
| 1.23-71: ¹H-NMR(400.6 MHz, CDCl3): |
| δ= 7.3889 (3.1); 7.3668 (3.2); 7.2618 (10.7); 6.9580 (1.2); 6.9451 (1.2); 6.9354 (1.7); 6.9226 (1.6); 6.8708 (2.3); 6.8544 (2.3); 6.8273 (1.1); 6.8198 (1.3); 6.8079 (1.2); 6.8048 (0.9); 6.8004 (1.3); 6.7973 (1.2); 6.7855 (0.7); 6.7780 (1.1); 6.7648 (1.9); 6.7574 (1.4); 6.7428 (1.9); 6.7354 (1.4); 6.3028 (3.1); 6.3008 (3.1); 5.2994 (16.0); 4.6765 (11.1); 4.6550 (0.6); 4.2193 (0.9); 4.2172 (1.0); 4.2002 (1.2); 4.1981 (1.2); 4.1836 (0.6); 4.1024 (0.7); 4.0981 (1.5); 4.0962 (1.8); 4.0907 (1.2); 4.0860 (1.7); 4.0841 (1.4); 4.0782 (1.0); 4.0716 (1.9); 3.8467 (0.6); 3.8425 (0.7); 3.8405 (0.7); 3.8364 (0.6); 3.8257 (1.1); 3.8195 (1.1); 3.8095 (0.6); 3.8032 (0.5); 3.7743 (0.9); 3.7692 (1.1); 3.7576 (0.6); 3.7525 (0.9); 3.7484 (0.5); 3.5175 (6.6); 3.5152 (6.8); 1.9663 (0.6); 1.9183 (0.6); 1.9145 (0.6); 1.9019 (1.4); 1.8982 (1.6); 1.8821 (1.8); 1.8789 (1.5); 1.8655 (1.1); 1.8620 (1.1); 1.5824 (0.6); 1.5731 (1.1); 1.5620 (0.6); 1.5510 (0.6); -0.0002 (15.1) |
| 1.7-288: ¹H-NMR(400.6 MHz, CDCl3): |
| δ= 8.5218 (0.7); 7.6636 (1.4); 7.6577 (1.4); 7.6428 (1.6); 7.6368 (1.5); 7.3329 (3.7); 7.3108 (3.7); 7.2608 (18.0); 7.2138 (0.8); 7.1931 (0.7); 7.1407 (0.8); 7.1361 (1.0); 7.1224 (1.3); 7.1204 (1.2); 7.1178 (1.5); 7.1159 (1.4); 7.1023 (1.4); 7.0977 (1.8); 7.0864 (1.4); 7.0820 (1.6); 7.0664 (3.2); 7.0619 (2.3); 7.0301 (1.8); 7.0264 (2.0); 7.0118 (1.5); 7.0100 (1.2); 7.0082 (1.6); 7.0063 (1.2); 6.9918 (0.8); 6.9881 (0.8); 6.9536 (2.1); 6.9500 (1.9); 6.9334 (1.8); 6.9298 (1.5); 6.7376 (3.5); 6.7212 (3.6); 6.2551 (5.9); 5.2992 (16.0); 5.2524 (7.7); 4.7596 (12.0); 3.4865 (7.8); 3.4836 (8.0); 0.0080 (0.8); -0.0002 (27.0); -0.0085 (0.7) |
| 1.23-278: ¹H-NMR(400.6 MHz, CDCl3): |
| δ= 8.5963 (4.5); 8.5923 (2.8); 8.5852 (2.8); 8.5812 (4.7); 7.3460 (3.7); 7.3239 (3.7); 7.2620 (12.4); 7.1945 (4.1); 7.1905 (2.5); 7.1834 (2.6); 7.1794 (4.1); 6.9662 (1.5); 6.9533 (1.6); 6.9438 (2.0); 6.9309 (2.1); 6.8334 (1.1); 6.8259 (1.5); 6.8142 (1.4); 6.8106 (4.2); 6.8068 (1.9); 6.8037 (1.7); 6.7942 (3.8); 6.7843 (1.4); 6.7815 (2.3); 6.7742 (1.6); 6.7596 (2.3); 6.7523 (1.6); 6.2867 (6.3); 5.2989 (16.0); 5.1832 (9.3); 4.7238 (14.3); 4.7002 (1.0); 3.5368 (0.6); 3.5340 (0.6); 3.5108 (8.5); 3.5079 (8.9); 2.0445 (0.8); 1.2589 (1.5); 1.2569 (1.3); -0.0002 (17.4); -0.0085 (0.6) |
| 1.23-277: ¹H-NMR(400.6 MHz, CDCl3): |
| δ= 8.5926 (3.1); 8.5887 (2.9); 8.5809 (1.7); 8.5767 (1.5); 7.6570 (0.8); 7.6525 (1.1); 7.6515 (1.1); 7.6470 (0.8); 7.6374 (0.9); 7.6330 (1.2); 7.6318 (1.3); 7.6274 (0.9); 7.3553 (3.2); 7.3331 (3.3); 7.3039 (1.1); 7.3020 (1.1); 7.2918 (1.1); 7.2898 (1.2); 7.2844 (1.1); 7.2823 (1.1); 7.2722 (1.0); 7.2702 (1.1); 7.2619 (13.4); 6.9332 (1.3); 6.9204 (1.4); 6.9109 (1.8); 6.8980 (1.9); 6.8142 (3.2); 6.8089 (1.3); 6.8014 (1.7); 6.7979 (3.4); 6.7897 (1.2); 6.7866 (0.8); 6.7823 (1.8); 6.7791 (1.3); 6.7673 (0.7); 6.7597 (3.0); 6.7522 (1.3); 6.7375 (2.0); 6.7303 (1.3); 6.3005 (5.5); 5.2990 (16.0); 5.1926 (8.8); 4.6698 (13.2); 4.6475 (0.9); 3.5357 (0.5); 3.5143 (7.4); 3.5113 (7.6); 2.0444 (0.5); 1.2589 (0.9); 1.2566 (0.8); 0.0079 (0.5); -0.0002 (19.1); -0.0085 (0.6) |
| 1.7-331: ¹H-NMR(400.6 MHz, CDCl3): |
| δ= 7.6719 (1.0); 7.6530 (1.1); 7.3331 (2.6); 7.3110 (2.6); 7.2612 (10.3); 7.1461 (0.6); 7.1414 (0.7); 7.1278 (0.9); 7.1259 (0.9); 7.1232 (1.1); 7.1213 (1.0); 7.1078 (0.9); 7.1031 (1.2); 7.0900 (1.0); 7.0855 (1.1); 7.0700 (2.3); 7.0655 (1.7); 7.0387 (1.2); 7.0351 (1.4); 7.0205 (1.0); 7.0186 (0.9); 7.0170 (1.1); 7.0151 (0.8); 7.0005 (0.5); 6.9970 (0.5); 6.9485 (1.4); 6.9449 (1.4); 6.9283 (1.2); 6.9247 (1.1); 6.7264 (2.5); 6.7100 (2.6); 6.2623 (4.3); 5.2991 (16.0); 5.2717 (5.1); 4.7541 (9.0); 3.4911 (5.8); 3.4883 (6.0); -0.0002 (14.4) |
| 1.23-72: ¹H-NMR(400.6 MHz, CDCl3): |
| δ= 7.3975 (3.6); 7.3755 (3.6); 7.2615 (13.1); 6.9469 (1.0); 6.9348 (1.0); 6.9245 (1.5); 6.9117 (1.5); 6.8496 (3.4); 6.8333 (3.6); 6.8281 (1.6); 6.8164 (1.4); 6.8132 (1.0); 6.8089 (1.6); 6.8057 (1.3); 6.7940 (0.9); 6.7865 (1.1); 6.7722 (1.4); 6.7711 (1.3); 6.7638 (1.0); 6.7504 (1.4); 6.7491 (1.4); 6.7430 (1.0); 6.7419 (1.0); 6.3151 (3.1); 6.3040 (3.1); 5.2994 (16.0); 4.6400 (13.7); 4.6172 (0.9); 4.1871 (0.6); 4.1714 (0.9); 4.1602 (1.0); 4.1565 (0.7); 4.1448 (1.4); 4.1296 (1.0); 4.0670 (1.1); 4.0546 (1.0); 4.0469 (1.2); 4.0401 (0.7); 4.0345 (1.1); 4.0277 (0.7); 4.0199 (0.7); 4.0075 (0.7); 3.8603 (0.7); 3.8467 (0.7); 3.8393 (1.4); 3.8256 (1.4); 3.8190 (1.1); 3.8053 (1.0); 3.7900 (0.9); 3.7687 (1.3); 3.7514 (1.9); 3.7332 (1.6); 3.7149 (1.3); 3.6938 (0.8); 3.5446 (1.4); 3.5324 (1.4); 3.5205 (9.2); 3.5176 (8.6); 3.4980 (0.8); 2.5736 (0.5); 2.5565 (0.7); 2.5392 (0.5); 2.0447 (0.6); 2.0311 (0.5); 2.0187 (0.7); 1.9990 (0.7); 1.9861 (0.6); 1.6001 (0.6); 1.5864 (0.9); 1.5668 (2.4); 1.5540 (0.6); 1.2591 (0.7); 0.0080 (0.5); -0.0002 (19.0) |
| 1.23-321: ¹H-NMR(400.6 MHz, CDCl3): |
| δ= 9.1982 (3.5); 8.7287 (8.0); 7.3716 (2.9); 7.3495 (2.9); 7.2627 (9.8); 6.9473 (1.1); 6.9345 (1.2); 6.9250 (1.6); 6.9121 (1.6); 6.8231 (0.9); 6.8156 (1.1); 6.8039 (1.0); 6.7973 (3.5); 6.7934 (1.2); 6.7813 (3.4); 6.7741 (2.6); 6.7668 (1.2); 6.7523 (1.7); 6.7449 (1.1); 6.3069 (4.8); 5.2998 (16.0); 5.2972 (0.5); 5.1888 (8.2); 4.6717 (11.2); 4.6483 (0.8); 3.5181 (6.4); 3.5153 (6.6); 1.2568 (0.8); -0.0002 (14.5); -0.0029 (0.6) |

Gegenstand der vorliegenden Erfindung ist weiterhin die Verwendung einer oder mehrerer erfindungsgemäßer Verbindungen der allgemeinen Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (I.1) bis (I.33) und/oder deren Salze, jeweils wie oben definiert, als Herbizid und/oder Pflanzenwachstumsregulator, vorzugsweise in Kulturen von Nutz- und/oder Zierpflanzen.

Gegenstand der vorliegenden Erfindung ist ferner ein Verfahren zur Bekämpfung von Schadpflanzen und/oder zur Wachstumsregulierung von Pflanzen, dadurch gekennzeichnet, dass eine wirksame Menge
- einer oder mehrerer erfindungsgemäßer Verbindungen der allgemeinen Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (I.1) bis (I.33) und/oder deren Salze, jeweils wie oben definiert, oder
- eines erfindungsgemäßen Mittels, wie nachstehend definiert,
auf die (Schad)Pflanzen, (Schad)Pflanzensamen, den Boden, in dem oder auf dem die (Schad)Pflanzen wachsen, oder die Anbaufläche appliziert wird.

Gegenstand der vorliegenden Erfindung ist auch ein Verfahren zur Bekämpfung von unerwünschten Pflanzen, vorzugsweise in Nutzpflanzenkulturen, dadurch gekennzeichnet, dass eine wirksame Menge
- einer oder mehrerer Verbindungen der allgemeinen Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (I.1) bis (I.33) und/oder deren Salze, jeweils wie oben definiert, oder
- eines erfindungsgemäßen Mittels, wie nachstehend definiert,
auf unerwünschte Pflanzen (z.B. Schadpflanzen wie mono- oder dikotyle Unkräuter oder unerwünschte Kulturpflanzen), das Saatgut der unerwünschten Pflanzen (d.h. Pflanzensamen, z.B. Körner, Samen oder vegetative Vermehrungsorgane wie Knollen oder Sprossteile mit Knospen), den Boden, in dem oder auf dem die unerwünschte Pflanzen wachsen, (z.B. den Boden von Kulturland oder Nicht-Kulturland) oder die Anbaufläche (d.h. Fläche, auf der die unerwünschte Pflanzen wachsen werden) appliziert wird.

Gegenstand der vorliegenden Erfindung ist ferner auch Verfahren zur Bekämpfung zur Wachstumsregulierung von Pflanzen, vorzugsweise von Nutzpflanzen, dadurch gekennzeichnet, dass eine wirksame Menge
- einer oder mehrerer Verbindungen der allgmeinenen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (I.1) bis (I.33) und/oder deren Salze, jeweils wie oben definiert, oder
- eines erfindungsgemäßen Mittels, wie nachstehend definiert,
die Pflanze, das Saatgut der Pflanze (d.h. Pflanzensamen, z.B. Körner, Samen oder vegetative Vermehrungsorgane wie Knollen oder Sprossteile mit Knospen), den Boden, in dem oder auf dem die Pflanzen wachsen, (z.B. den Boden von Kulturland oder Nicht-Kulturland) oder die Anbaufläche (d.h. Fläche, auf der die Pflanzen wachsen werden) appliziert wird.

Dabei können die erfindungsgemäßen Verbindungen der allgemeinen Formel (I) bzw. die erfindungsgemäßen Mittel z.B. im Vorsaat- (ggf. auch durch Einarbeitung in den Boden), Vorauflauf- und/oder Nachauflaufverfahren ausgebracht werden. Im Einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Vorzugsweise werden in einem erfindungsgemäßen Verfahren zur Bekämpfung von Schadpflanzen oder zur Wachstumsregulierung von Pflanzen eine oder mehrere Verbindungen der allgemeinen Formel (I) und/oder deren Salze zur Bekämpfung von Schadpflanzen oder zur Wachstumsregulierung in Kulturen von Nutzpflanzen oder Zierpflanzen eingesetzt, wobei die Nutzpflanzen oder Zierpflanzen in einer bevorzugten Ausgestaltung transgene Pflanzen sind.

Die erfindungsgemäßen Verbindungen der allgemeinen Formel (I) und/oder deren Salze eignen sich zur Bekämpfung der folgenden Gattungen von monokotylen und dikotylen Schadpflanzen:
Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen der allgemeinen Formel (I) vor dem Keimen der Schadpflanzen (Ungräser und/oder Unkräuter) auf die Erdoberfläche appliziert (Vorauflaufverfahren), so wird entweder das Auflaufen der Ungras- bzw. Unkrautkeimlinge vollständig verhindert oder diese wachsen bis zum Keimblattstadium heran, stellen jedoch dann ihr Wachstum ein und sterben schließlich nach Ablauf von drei bis vier Wochen vollkommen ab.

Bei Applikation der erfindungsgemäßen Verbindungen der allgemeinen Formel (I) auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen der allgemeinen Formel (I) (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die erfindungsgemäßen Verbindungen der allgemeinen Formel (I) auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen der allgemeinen Formel (I) und/oder deren Salze in wirtschaftlich bedeutenden transgenen Kulturen von Nutz und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen der allgemeinen Formel (I) auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die erfindungsgemäßen Verbindungen der allgemeinen Formel (I) auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz-und Zierpflanzen, z.B. von Getreide wie Weizen, Gerste, Roggen, Hafer, Triticale, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden.

Zahlreiche molekularbiologische Techniken, mit denen neue transgene Pflanzen mit veränderten Eigenschaften hergestellt werden können, sind dem Fachmann bekannt. Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z.B. Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Verbindungen der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung in Getreide, dabei vorzugsweise Mais, Weizen, Gerste, Roggen, Hafer, Hirse, oder Reis, im Vor- oder Nachauflauf.

Bevorzugt ist auch die Verwendung in Soja im Vor- oder Nachauflauf.

Die erfindungsgemäße Verwendung zur Bekämpfung von Schadpflanzen oder zur Wachstumsregulierung von Pflanzen schließt auch den Fall ein, bei dem eine Verbindung der allgemeinen Formel (I) oder deren Salz erst nach der Ausbringung auf der Pflanze, in der Pflanze oder im Boden aus einer Vorläufersubstanz ("Prodrug") gebildet wird.

Gegenstand der Erfindung ist auch die Verwendung einer oder mehrerer Verbindungen der allgemeinen Formel (I) oder deren Salzen bzw. eines erfindungsgemäßen Mittels (wie nachstehend definiert) (in einem Verfahren) zur Bekämpfung von Schadpflanzen oder zur Wachstumsregulierung von Pflanzen, dadurch gekennzeichnet, dass man eine wirksame Menge einer oder mehreren Verbindungen der allgemeinen Formel (1) oder deren Salzen auf die Pflanzen (Schadpflanzen, ggf. zusammen mit den Nutzpflanzen) Pflanzensamen, den Boden, in dem oder auf dem die Pflanzen wachsen, oder die Anbaufläche appliziert.

### Gegenstand der Erfindung ist auch ein herbizides und/oder pflanzenwachstumsregulierendes Mittel, dadurch gekennzeichnet, dass das Mittel

(a) eine oder mehrere Verbindungen der allgemeinen Formel (I) und/oder deren Salze enthält wie oben definiert, vorzugsweise in einer der als bevorzugt bzw. besonders bevorzugt gekennzeichneten Ausgestaltung, insbesondere eine oder mehrere Verbindungen der Formeln (1.1) bis (I.33) und/oder deren Salze, jeweils wie oben definiert,
   und
(b) ein oder mehrere weitere Stoffe ausgewählt aus den Gruppen (i) und/oder (ii):
   (i) ein oder mehrere weitere agrochemisch wirksame Stoffe, vorzugsweise ausgewählt aus der Gruppe bestehend aus Insektiziden, Akariziden, Nematiziden, weiteren Herbiziden (d.h. solche, die nicht der oben definierten allgemeinen Formel (I) entsprechen), Fungiziden, Safenern, Düngemitteln und/oder weiteren Wachstumsregulatoren,
   (ii) ein oder mehrere im Pflanzenschutz übliche Formulierungshilfsmittel.

Die weiteren agrochemischen wirksamen Stoffe des Bestandteils (i) eines erfindungsgemäßen Mittels sind dabei vorzugsweise ausgewählt aus der Gruppe der Stoffe, die in "The Pesticide Manual", 16th edition, The British Crop Protection Council und the Royal Soc. of Chemistry, 2012 genannt sind.

Ein erfindungsgemäßes herbizides oder pflanzenwachstumsregulierendes Mittel, umfasst vorzugsweise ein, zwei, drei oder mehr im Pflanzenschutz übliche Formulierungshilfsmittel (ii) ausgewählt aus der Gruppe bestehend aus Tensiden, Emulgatoren, Dispergiermitteln, Filmbildnern, Verdickungsmitteln, anorganischen Salzen, Stäubemitteln, bei 25 °C und 1013 mbar festen Trägerstoffen, vorzugsweise adsorptionsfähigen, granulierten Inertmaterialien, Netzmitteln, Antioxidationsmitteln, Stabilisatoren, Puffersubstanzen, Antischaummitteln, Wasser, organischen Lösungsmitteln, vorzugsweise bei 25 °C und 1013 mbar mit Wasser in jedem beliebigen Verhältnis mischbare organische Lösungsmittel.

Die erfindungsgemäßen Verbindungen der allgemeinen Formel (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der allgemeinen Formel (I) und/oder deren Salze enthalten.

Die erfindungsgemäßen Verbindungen der allgemeinen Formel (1) und/oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen und die Formulierungshilfsmittel wie Inertmaterialien, Tenside, Lösungsmittel und weitere Zusatzstoffe sind dem Fachmann bekannt, und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Nass-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wässrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen, vorzugsweise herbizide oder pflanzenwachstumsregulierende Mittel der vorliegenden Erfindung enthalten vorzugsweise eine Gesamtmenge von 0,1 bis 99 Gew.-%, bevorzugt 0,5 bis 95 Gew.-%, weiter bevorzugt 1 bis 90 Gew.-%, insbesondere bevorzugt 2 bis 80 Gew.-%, an Wirkstoffen der allgemeinen Formel (I) und deren Salzen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die erfindungsgemäßen Verbindungen der allgemeinen Formel (I) oder deren Salze können als solche oder in Form ihrer Zubereitungen (Formulierungen) mit anderen pestizid wirksamen Stoffen, wie z.B. Insektiziden, Akariziden, Nematiziden, Herbiziden, Fungiziden, Safenern, Düngemitteln und/oder Wachstumsregulatoren kombiniert eingesetzt werden, z.B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen der allgemeinen Formel (1) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. in Weed Research 26 (1986) 441-445 oder "The Pesticide Manual", 16th edition, The British Crop Protection Council and the Royal Soc. of Chemistry, 2012 und der dort zitierten Literatur beschrieben sind.

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die Verbindungen (1) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z.B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200: 1 bis 1:200, vorzugsweise 100: 1 bis 1: 100, insbesondere 20: 1 bis 1:20. Die Safener können analog den Verbindungen der allgemeinen Formel (1) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Äußere Bedingungen wie Temperatur, Feuchtigkeit etc. beeinflussen zu einem gewissen Teil die Aufwandmenge der erfindungsgemäßen Verbindungen der allgemeinen Formel (I) und/oder deren Salze. Die Aufwandmenge kann dabei innerhalb weiter Grenzen variieren. Für die Anwendung als Herbizid zur Bekämpfung von Schadpflanzen liegt die Gesamtmenge an erfindungsgemäßen Verbindungen der allgemeinen Formel (I) und deren Salze vorzugsweise im Bereich von 0,001 bis 10,0 kg/ha, bevorzugt im Bereich von 0,005 bis 5 kg/ha, weiter bevorzugt im Bereich von 0,01 bis 1,5 kg/ha, insbesondere bevorzugt im Bereich von 0,05 bis 1 kg/ha. Dies gilt sowohl für die Anwendung im Vorauflauf oder im Nachauflauf.

Bei der Anwendung von erfindungsgemäßen Verbindungen der allgemeinen Formel (1) und/oder deren Salzen als Pflanzenwachstumsregulator, beispielsweise als Halmverkürzer bei Kulturpflanzen, wie sie oben genannt worden sind, vorzugsweise bei Getreidepflanzen wie Weizen, Gerste, Roggen, Triticale, Hirse, Reis oder Mais, liegt die Gesamt-Aufwandmenge vorzugsweise im Bereich von 0,001 bis 2 kg/ha, vorzugsweise im Bereich von 0,005 bis 1 kg/ha, insbesondere im Bereich von 10 bis 500 g/ha, ganz besonders bevorzugt im Bereich von 20 bis 250 g/ha. Dies gilt sowohl für die Anwendung im Vorauflauf oder im Nachauflauf.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Als Kombinationspartner für die erfindungsgemäßen Verbindungen der allgemeinen Formel (1) in erfindungsgemäßen Mitteln (z.B. Mischungsformulierungen oder im Tank-Mix) sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2012 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfasst, auch wenn diese nicht explizit erwähnt sind.

Beispiele für solche herbiziden Mischungspartner sind:
Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenacsodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -triisopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyrsodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, - potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquinammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl } sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, - isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuronethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, , SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

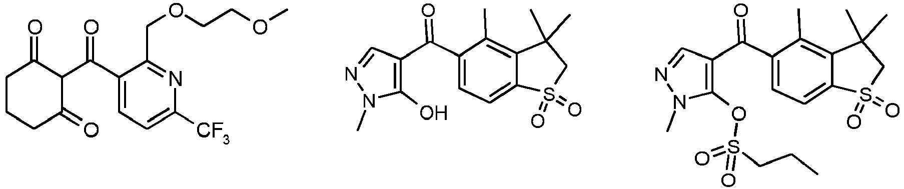

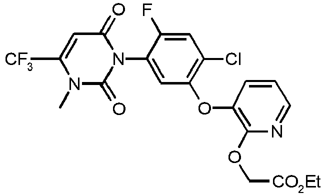

Beispiele für Pflanzenwachstumsregulatoren als mögliche Mischungspartner sind:
Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothaldipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2-naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Ebenfalls als Kombinationspartner für die erfindungsgemäßen Verbindungen der allgemeinen Formel (I) kommen beispielsweise die folgenden Safener in Frage:
S1) Verbindungen aus der Gruppe heterocyclischer Carbonsäurederivate:
   S1^{a}) Verbindungen vom Typ der Dichlorphenylpyrazolin-3-carbonsäure (S1^{a}), vorzugsweise Verbindungen wie
      1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
   S1^{b}) Derivate der Dichlorphenylpyrazolcarbonsäure (S1^{b}), vorzugsweise Verbindungen wie 1-(2,4-Dichlorphenyl)-5-methylpyrazol-3-carbonsäureethylester (S1-2), 1-(2,4-Dichlorphenyl)-5-isopropylpyrazol-3-carbonsäureethylester (S1-3), 1-(2,4-Dichlorphenyl)-5-(1,1-dimethyl-ethyl)pyrazol-3-carbonsäureethylester (S1-4) und verwandte Verbindungen, wie sie in EP-A-333131 und EP-A-269806 beschrieben sind;
   S1^{c}) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   S1^{d}) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen, wie sie in EP-A-174562 und EP-A-346620 beschrieben sind;
   S1^{e}) Verbindungen vom Typ der 5-Benzyl- oder 5-Phenyl-2-isoxazolin-3- carbonsäure, oder der 5,5-Diphenyl-2-isoxazolin-3-carbonsäure(S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-3-carbonsäureethylester (S1-11) ("Isoxadifen-ethyl") oder -n-propylester (S1-12) oder 5-(4-Fluorphenyl)-5-phenyl-2-isoxazolin-3-carbon-säureethylester (S 1-13), wie sie in der Patentanmeldung WO-A-95/07897 beschrieben sind.
S2) Verbindungen aus der Gruppe der 8-Chinolinoxyderivate (S2):
   S2^{a}) Verbindungen vom Typ der 8-Chinolinoxyessigsäure (S2^{a}), vorzugsweise (5-Chlor-8-chinolinoxy)essigsäure-(1-methylhexyl)-ester ("Cloquintocet-mexyl") (S2-1), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86750, EP-A-94349 und EP-A-191736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   S2^{b}) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Wirkstoffe vom Typ der Dichloracetamide (S3), die häufig als Vorauflaufsafener (bodenwirksame Safener) angewendet werden, wie z. B. "Dichlormid" (N,N-Diallyl-2,2-dichloracetamid) (S3-1), "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5), "DKA-24" (N-Allyl-N-[(allylaminocarbonyl)methyl]-dichloracetamid) der Firma Sagro-Chem (S3-6), "AD-67" oder "MON 4660" (3-Dichloracetyl-1-oxa-3-aza-spiro[4,5]decan) der Firma Nitrokemia bzw. Monsanto (S3-7), "TI-35" (1-Dichloracetyl-azepan) der Firma TRI-Chemical RT (S3-8), "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin) (S3-10), sowie dessen (R)-Isomer (S3-11).
S4) Verbindungen aus der Klasse der Acylsulfonamide (S4):
   S4^{a}) N-Acylsulfonamide der Formel (S4^{a}) und deren Salze wie sie in der WO-A-97/45016 beschrieben sind, 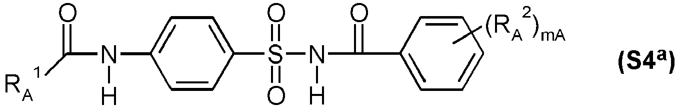 worin
      - R_{A}¹: (C₁-C₆)Alkyl, (C₃-C₆)Cycloalkyl, wobei die 2 letztgenannten Reste durch v_{A} Substituenten aus der Gruppe Halogen, (C₁-C₄)Alkoxy, (C₁-C₆)Haloalkoxy und (C₁-C₄)Alkylthio und im Falle cyclischer Reste auch durch (C₁-C₄)Alkyl und (C₁-C₄)Haloalkyl substituiert sind;
      - R_{A}²: Halogen, (C₁-C₄)Alkyl, (C₁-C₄)Alkoxy, CF₃,
      - m_{A}: 1 oder 2;
      - v_{A}: ist 0, 1, 2 oder 3 bedeuten;
   S4^{b}) Verbindungen vom Typ der 4-(Benzoylsulfamoyl)benzamide der Formel (S4^{b}) und deren Salze, wie sie in der WO-A-99/16744 beschrieben sind, 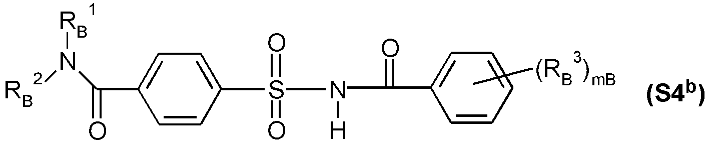 worin
      R_{B}¹, R_{B}² unabhängig voneinander Wasserstoff, (C₁-C₆)Alkyl, (C₃-C₆)Cycloalkyl, (C₃-C₆)Alkenyl, (C₃-C₆)Alkinyl,
      R_{B}³ Halogen, (C₁-C₄)Alkyl, (C₁-C₄)Haloalkyl oder (C₁-C₄)Alkoxy und
      m_{B} 1 oder 2 bedeuten,
      z.B. solche worin
      R_{B}¹ = Cyclopropyl, R_{B}² = Wasserstoff und (R_{B}³) = 2-OMe ist ("Cyprosulfamide", S4-1),
      R_{B}¹ = Cyclopropyl, R_{B}² = Wasserstoff und (R_{B}³) = 5-Cl-2-OMe ist (S4-2),
      R_{B}¹ = Ethyl, R_{B}² = Wasserstoff und (R_{B}³) = 2-OMe ist (S4-3),
      R_{B}¹ = Isopropyl, R_{B}² = Wasserstoff und (R_{B}³) = 5-Cl-2-OMe ist (S4-4) und
      R_{B}¹ = Isopropyl, R_{B}² = Wasserstoff und (R_{B}³) = 2-OMe ist (S4-5);
   S4^{c}) Verbindungen aus der Klasse der Benzoylsulfamoylphenylharnstoffe der Formel (S4^{c}), wie sie in der EP-A-365484 beschrieben sind, 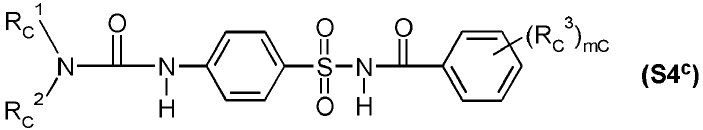 worin
      - R_{C}¹, R_{C}²: unabhängig voneinander Wasserstoff, (C₁-C₈)Alkyl, (C₃-C₈)Cycloalkyl, (C₃-C₆)Alkenyl, (C₃-C₆)Alkinyl,
      - R_{C}³: Halogen, (C₁-C₄)Alkyl, (C₁-C₄)Alkoxy, CF₃ und
      - m_{C}: 1 oder 2 bedeuten;
      beispielsweise
      1-[4-(N-2-Methoxybenzoylsulfamoyl)phenyl]-3-methylharnstoff,
      1-[4-(N-2-Methoxybenzoylsulfamoyl)phenyl]-3,3-dimethylharnstoff,
      1-[4-(N-4,5-Dimethylbenzoylsulfamoyl)phenyl]-3-methylharnstoff;
   S4^{d}) Verbindungen vom Typ der N-Phenylsulfonylterephthalamide der Formel (S4^{d}) und deren Salze, die z.B. bekannt sind aus CN 101838227, 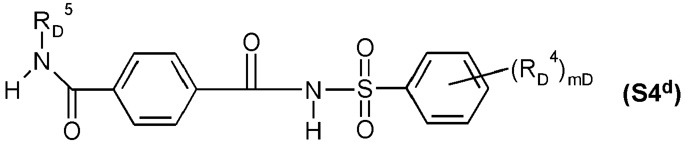 worin
      - R_{D}⁴: Halogen, (C₁-C₄)Alkyl, (C₁-C₄)Alkoxy, CF₃,
      - m_{D}: 1 oder 2;
      - R_{D}⁵: Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydro-chinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen aus der Klasse der Diphenylmethoxyessigsäurederivate (S7), z.B. Diphenylmethoxyessigsäuremethylester (CAS-Reg.Nr. 41858-19-9) (S7-1), Diphenylmethoxyessigsäureethylester oder Diphenylmethoxyessigsäure wie sie in der WO-A-98/38856 beschrieben sind.
S8) Verbindungen der Formel (S8), wie sie in der WO-A-98/27049 beschrieben sind, 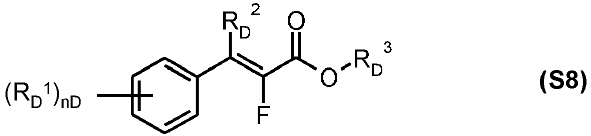 worin die Symbole und Indizes folgende Bedeutungen haben:
   - R_{D}¹: ist Halogen, (C₁-C₄)Alkyl, (C₁-C₄)Haloalkyl, (C₁-C₄)Alkoxy, (C₁-C₄)Haloalkoxy,
   - R_{D}²: ist Wasserstoff oder (C₁-C₄)Alkyl,
   - R_{D}³: ist Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze,
   - n_{D}: ist eine ganze Zahl von 0 bis 2.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr.: 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b}), wie sie in der WO-A-2007/023719 und WO-A-2007/023764 beschrieben sind, 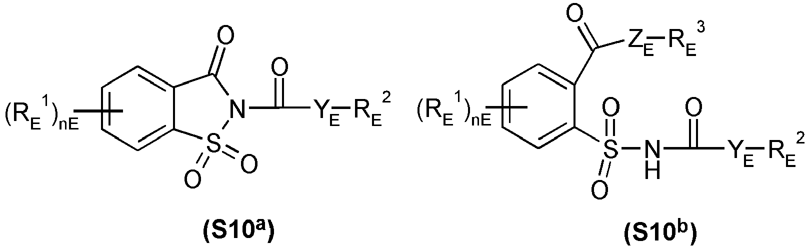 worin
   - R_{E}¹: Halogen, (C₁-C₄)Alkyl, Methoxy, Nitro, Cyano, CF₃, OCF₃
   - Y_{E}, Z_{E}: unabhängig voneinander O oder S,
   - n_{E}: eine ganze Zahl von 0 bis 4,
   - R_{E}²: (C₁-C₁₆)Alkyl, (C₂-C₆)Alkenyl, (C₃-C₆)Cycloalkyl, Aryl; Benzyl, Halogenbenzyl,
   - R_{E}³: Wasserstoff oder (C₁-C₆)Alkyl bedeuten.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5) (2-propenyl 1-oxa-4-azaspiro[4.5]decan-4-carbodithioat) (S13-6) der Firma Nitrokemia "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere, 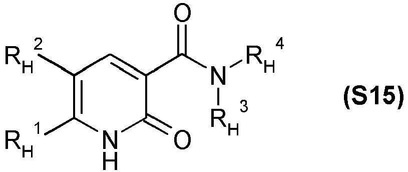 wie sie in der WO-A-2008/131861 und WO-A-2008/131860 beschrieben sind,
   worin
   - R_{H}¹: einen (C₁-C₆)Haloalkylrest bedeutet und
   - R_{H}²: Wasserstoff oder Halogen bedeutet und
   - R_{H}³, R_{H}⁴: unabhängig voneinander Wasserstoff, (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet oder
   - R_{H}³: (C₁-C₄)-Alkoxy, (C₂-C₄)Alkenyloxy, (C₂-C₆)Alkinyloxy oder (C₂-C₄)Haloalkoxy bedeutet und
   - R_{H}⁴: Wasserstoff oder (C₁-C₄)-Alkyl bedeutet oder
   - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z. B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Bevorzugte Safener in Kombination mit den erfindungsgemäßen Verbindungend der allgemeinen Formel (I) und/oder deren Salze, insbesondere mit den Verbindungen der Formeln (I.1) bis (I.34) und/oder deren Salze sind: Cloquintocet-mexyl, Cyprosulfamid, Fenchlorazol-ethylester, Isoxadifen-ethyl, Mefenpyr-diethyl, Fenclorim, Cumyluron, S4-1 und S4-5, und besonders bevorzugte Safener sind: Cloquintocet-mexyl, Cyprosulfamid, Isoxadifen-ethyl und Mefenpyr-diethyl.

Biologische Beispiele:

### A. Herbizide Wirkung und Kulturverträglichkeit im Nachauflauf

Samen von mono- bzw. dikotylen Unkraut- bzw. Kulturpflanzen wurden in Kunststoff- oder Holzfasertöpfen in sandigem Lehmboden ausgelegt, mit Erde abgedeckt und im Gewächshaus unter kontrollierten Wachstumsbedingungen angezogen. 2 bis 3 Wochen nach der Aussaat wurden die Versuchspflanzen im Einblattstadium behandelt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 l/ha auf die grünen Pflanzenteile gesprüht. Nach ca. 3 Wochen Standzeit der Versuchspflanzen im Gewächshaus, unter optimalen Wachstumsbedingungen, wurde die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen.

In den nachstehenden Tabellen A1 bis A14 sind die Wirkungen ausgewählter Verbindungen der allgemeinen Formel (1) gemäß der Tabellen 1.1 bis 1.33 auf verschiedene Schadpflanzen und einer Aufwandmenge entsprechend 20 g/ha und niedriger, die gemäß zuvor genannter Versuchsvorschrift erhalten wurden, dargestellt. Die Appendices "a", "b" und "c" differenzieren nach verwendeten Dosierungen bei ansonsten gleich geprüften Schadpflanzen.

**Tabelle A1a: Nachauflaufwirkung bei 1.25g/ha gegen ABUTH in %**

| Beispielnummer | Dosierung [g/ha] | ABUTH |
|---|---|---|
| I.1-72 | 1,25 | 100 |
| I.1-1 | 1,25 | 100 |
| I.2-23 | 1,25 | 100 |
| I.2-2 | 1,25 | 80 |
| I.2-58 | 1,25 | 100 |
| I.1-26 | 1,25 | 100 |
| I.1-23 | 1,25 | 100 |
| I.1-71 | 1,25 | 100 |
| I.7-23 | 1,25 | 100 |
| I.7-276 | 1,25 | 100 |
| I.7-2 | 1,25 | 100 |
| I.9-1 | 1,25 | 100 |
| I.7-41 | 1,25 | 100 |
| I.7-278 | 1,25 | 100 |
| I.7-277 | 1,25 | 90 |
| I.7-291 | 1,25 | 100 |
| I.7-71 | 1,25 | 100 |
| I.7-115 | 1,25 | 80 |
| I.7-356 | 1,25 | 90 |
| I.7-394 | 1,25 | 100 |
| I.7-72 | 1,25 | 100 |
| I.7-179 | 1,25 | 100 |
| I.7-177 | 1,25 | 100 |
| I.7-94 | 1,25 | 100 |
| I.6-1 | 1,25 | 100 |
| I.6-176 | 1,25 | 100 |
| I.6-41 | 1,25 | 100 |
| I.6-276 | 1,25 | 100 |
| I.6-23 | 1,25 | 100 |
| I.6-2 | 1,25 | 100 |
| I.7-339 | 1,25 | 90 |
| I.8-23 | 1,25 | 100 |
| I.8-41 | 1,25 | 80 |
| I.8-176 | 1,25 | 100 |
| I.9-23 | 1,25 | 80 |
| I.9-41 | 1,25 | 100 |
| I.8-2 | 1,25 | 100 |
| I.8-1 | 1,25 | 100 |
| I.8-276 | 1,25 | 100 |
| I.7-183 | 1,25 | 100 |
| I.9-2 | 1,25 | 100 |
| I.9-176 | 1,25 | 100 |
| I.9-276 | 1,25 | 100 |
| I.8-291 | 1,25 | 100 |
| I.9-291 | 1,25 | 100 |
| I.19-276 | 1,25 | 100 |
| I.19-176 | 1,25 | 100 |
| I.23-176 | 1,25 | 80 |
| I.23-1 | 1,25 | 100 |
| I.19-1 | 1,25 | 100 |
| I.23-276 | 1,25 | 90 |

**Tabelle A1b: Nachauflaufwirkung bei 5g/ha gegen ABUTH in %**

| Beispielnummer | Dosierung [g/ha] | ABUTH |
|---|---|---|
| I.1-72 | 5 | 100 |
| I.1-1 | 5 | 100 |
| I.2-72 | 5 | 100 |
| I.2-71 | 5 | 100 |
| I.2-1 | 5 | 100 |
| I.2-23 | 5 | 100 |
| I.2-2 | 5 | 100 |
| I.2-58 | 5 | 100 |
| I.1-26 | 5 | 100 |
| I.1-23 | 5 | 100 |
| I.1-71 | 5 | 100 |
| I.7-1 | 5 | 100 |
| I.7-23 | 5 | 100 |
| I.7-276 | 5 | 100 |
| I.7-176 | 5 | 100 |
| I.7-2 | 5 | 100 |
| I.9-1 | 5 | 100 |
| I.7-41 | 5 | 100 |
| I.7-278 | 5 | 100 |
| I.7-277 | 5 | 100 |
| I.7-291 | 5 | 100 |
| I.7-71 | 5 | 100 |
| I.7-115 | 5 | 100 |
| I.7-356 | 5 | 100 |
| I.7-394 | 5 | 100 |
| I.7-72 | 5 | 100 |
| I.7-179 | 5 | 100 |
| I.7-177 | 5 | 100 |
| I.7-94 | 5 | 100 |
| I.6-1 | 5 | 100 |
| I.6-176 | 5 | 100 |
| I.6-41 | 5 | 100 |
| I.6-276 | 5 | 100 |
| I.6-23 | 5 | 100 |
| I.6-2 | 5 | 100 |
| I.7-339 | 5 | 100 |
| I.8-23 | 5 | 100 |
| I.8-41 | 5 | 100 |
| I.8-176 | 5 | 100 |
| I.9-23 | 5 | 100 |
| I.9-41 | 5 | 100 |
| I.6-183 | 5 | 100 |
| I.8-2 | 5 | 100 |
| I.8-1 | 5 | 100 |
| I.8-276 | 5 | 100 |
| I.7-183 | 5 | 100 |
| I.9-2 | 5 | 100 |
| I.9-176 | 5 | 100 |
| I.9-276 | 5 | 100 |
| I.8-183 | 5 | 100 |
| I.8-291 | 5 | 100 |
| I.9-291 | 5 | 100 |
| I.19-276 | 5 | 100 |
| I.19-176 | 5 | 100 |
| I.23-176 | 5 | 100 |
| I.23-1 | 5 | 100 |
| I.19-1 | 5 | 100 |
| I.19-23 | 5 | 100 |
| I.23-23 | 5 | 100 |
| I.23-276 | 5 | 100 |

**Tabelle A1c: Nachauflaufwirkung bei 20g/ha gegen ABUTH in %**

| Beispielnummer | Dosierung [g/ha] | ABUTH |
|---|---|---|
| I.1-72 | 20 | 100 |
| I.1-1 | 20 | 100 |
| I.2-72 | 20 | 100 |
| I.2-71 | 20 | 100 |
| I.2-1 | 20 | 100 |
| I.2-23 | 20 | 100 |
| I.2-2 | 20 | 100 |
| I.2-58 | 20 | 100 |
| I.1-26 | 20 | 100 |
| I.1-23 | 20 | 100 |
| I.1-71 | 20 | 100 |
| I.7-1 | 20 | 100 |
| I.7-276 | 20 | 100 |
| I.7-176 | 20 | 100 |
| I.7-2 | 20 | 100 |
| I.9-1 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 100 |
| I.7-115 | 20 | 100 |
| I.7-356 | 20 | 100 |
| I.7-394 | 20 | 100 |
| I.7-72 | 20 | 100 |
| I.7-179 | 20 | 100 |
| I.7-177 | 20 | 100 |
| I.7-94 | 20 | 100 |
| I.6-1 | 20 | 100 |
| I.6-176 | 20 | 100 |
| I.6-41 | 20 | 100 |
| I.-276 | 20 | 100 |
| I.6-23 | 20 | 100 |
| I.6-2 | 20 | 100 |
| I.7-339 | 20 | 100 |
| I.8-23 | 20 | 100 |
| I.8-41 | 20 | 100 |
| I.8-176 | 20 | 100 |
| I.9-23 | 20 | 100 |
| I.9-41 | 20 | 100 |
| I.6-183 | 20 | 100 |
| I.8-2 | 20 | 100 |
| I.8-1 | 20 | 100 |
| I.8-276 | 20 | 100 |
| I.7-183 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 100 |
| I.19-176 | 20 | 100 |
| I.23-176 | 20 | 100 |
| I.23-1 | 20 | 100 |
| I.19-1 | 20 | 100 |
| I.19-23 | 20 | 100 |
| I.23-23 | 20 | 100 |
| I.23-276 | 20 | 100 |

**Tabelle A2a: Nachauflaufwirkung bei 1.25g/ha gegen ALOMY in %**

| Beispielnummer | Dosierung [g/ha] | ALOMY |
|---|---|---|
| I.7-23 | 1,25 | 80 |
| I.7-276 | 1,25 | 90 |
| I.7-41 | 1,25 | 100 |
| I.8-2 | 1,25 | 80 |
| I.8-291 | 1,25 | 80 |
| I.9-291 | 1,25 | 80 |

**Tabelle A2b: Nachauflaufwirkung bei 5g/ha gegen ALOMY in %**

| Beispielnummer | Dosierung [g/ha] | ALOMY |
|---|---|---|
| I.1-26 | 5 | 80 |
| I.1-23 | 5 | 90 |
| I.1-71 | 5 | 80 |
| I.7-23 | 5 | 100 |
| I.7-276 | 5 | 100 |
| I.7-176 | 5 | 100 |
| I.7-1 | 5 | 80 |
| I.7-2 | 5 | 80 |
| I.7-41 | 5 | 100 |
| I.7-278 | 5 | 90 |
| I.7-291 | 5 | 80 |
| I.7-71 | 5 | 80 |
| I.7-115 | 5 | 100 |
| I.7-356 | 5 | 100 |
| I.7-394 | 5 | 80 |
| I.7-177 | 5 | 90 |
| I.7-94 | 5 | 80 |
| I.6-1 | 5 | 80 |
| I.6-176 | 5 | 80 |
| I.6-41 | 5 | 80 |
| I.6-276 | 5 | 80 |
| I.6-23 | 5 | 80 |
| I.7-339 | 5 | 100 |
| I.8-23 | 5 | 80 |
| I.8-41 | 5 | 80 |
| I.8-176 | 5 | 80 |
| I.8-2 | 5 | 100 |
| I.8-1 | 5 | 100 |
| I.8-276 | 5 | 80 |
| I.7-183 | 5 | 80 |
| I.9-2 | 5 | 80 |
| I.9-176 | 5 | 100 |
| I.9-276 | 5 | 100 |
| I.8-183 | 5 | 80 |
| I.8-291 | 5 | 90 |
| I.9-291 | 5 | 90 |
| I.19-23 | 5 | 80 |

**Tabelle A2c: Nachauflaufwirkung bei 20g/ha gegen ALOMY in %**

| Beispielnummer | Dosierung [g/ha] | ALOMY |
|---|---|---|
| I.1-72 | 20 | 80 |
| I.1-1 | 20 | 80 |
| I.2-72 | 20 | 90 |
| I.2-71 | 20 | 80 |
| I.2-1 | 20 | 80 |
| I.2-23 | 20 | 80 |
| I.2-2 | 20 | 80 |
| I.2-58 | 20 | 80 |
| I.1-26 | 20 | 90 |
| I.1-23 | 20 | 90 |
| I.1-71 | 20 | 90 |
| I.7-1 | 20 | 100 |
| I.7-276 | 20 | 100 |
| I.7-176 | 20 | 100 |
| I.7-2 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 80 |
| I.7-115 | 20 | 100 |
| I.7-356 | 20 | 100 |
| I.7-394 | 20 | 100 |
| I.7-72 | 20 | 100 |
| I.7-179 | 20 | 100 |
| I.7-177 | 20 | 100 |
| I.7-94 | 20 | 80 |
| I.6-1 | 20 | 100 |
| I.6-176 | 20 | 90 |
| I.6-41 | 20 | 100 |
| I.6-276 | 20 | 100 |
| I.6-23 | 20 | 90 |
| I.6-2 | 20 | 90 |
| I.7-339 | 20 | 100 |
| I.8-23 | 20 | 100 |
| I.8-41 | 20 | 100 |
| I.8-176 | 20 | 100 |
| I.9-23 | 20 | 80 |
| I.9-41 | 20 | 80 |
| I.6-183 | 20 | 90 |
| I.8-2 | 20 | 100 |
| I.8-1 | 20 | 100 |
| I.8-276 | 20 | 100 |
| I.7-183 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 90 |
| I.19-176 | 20 | 80 |
| I.23-176 | 20 | 100 |
| I.23-1 | 20 | 90 |
| I.19-1 | 20 | 100 |
| I.19-23 | 20 | 100 |
| I.23-23 | 20 | 100 |
| I.23-276 | 20 | 100 |

**Tabelle A3a: Nachauflaufwirkung bei 1,25g/ha gegen AMARE in %**

| Beispielnummer | Dosierung [g/ha] | AMARE |
|---|---|---|
| I.1-72 | 1,25 | 80 |
| I.1-1 | 1,25 | 90 |
| I.2-72 | 1,25 | 90 |
| I.2-71 | 1,25 | 100 |
| I.2-1 | 1,25 | 100 |
| I.2-23 | 1,25 | 80 |
| I.2-2 | 1,25 | 100 |
| I.2-58 | 1,25 | 90 |
| I.1-26 | 1,25 | 100 |
| I.1-23 | 1,25 | 100 |
| I.1-71 | 1,25 | 100 |
| I.7-1 | 1,25 | 100 |
| I.7-23 | 1,25 | 100 |
| I.7-276 | 1,25 | 100 |
| I.7-2 | 1,25 | 100 |
| I.9-1 | 1,25 | 100 |
| I.7-41 | 1,25 | 100 |
| I.7-278 | 1,25 | 100 |
| I.7-277 | 1,25 | 100 |
| I.7-291 | 1,25 | 100 |
| I.7-71 | 1,25 | 100 |
| I.7-115 | 1,25 | 100 |
| I.7-356 | 1,25 | 100 |
| I.7-394 | 1,25 | 100 |
| I.7-72 | 1,25 | 100 |
| I.7-179 | 1,25 | 100 |
| I.7-177 | 1,25 | 100 |
| I.7-94 | 1,25 | 100 |
| I.6-1 | 1,25 | 100 |
| I.6-176 | 1,25 | 100 |
| I.6-41 | 1,25 | 100 |
| I.6-276 | 1,25 | 100 |
| I.6-23 | 1,25 | 100 |
| I.6-2 | 1,25 | 100 |
| I.7-339 | 1,25 | 100 |
| I.8-23 | 1,25 | 100 |
| I.8-41 | 1,25 | 100 |
| I.8-176 | 1,25 | 100 |
| I.9-23 | 1,25 | 100 |
| I.9-41 | 1,25 | 100 |
| I.6-183 | 1,25 | 100 |
| I.8-2 | 1,25 | 100 |
| I.8-1 | 1,25 | 100 |
| I.8-276 | 1,25 | 100 |
| I.7-183 | 1,25 | 100 |
| I.9-2 | 1,25 | 100 |
| I.9-176 | 1,25 | 100 |
| I.9-276 | 1,25 | 100 |
| I.8-183 | 1,25 | 100 |
| I.8-291 | 1,25 | 100 |
| I.9-291 | 1,25 | 100 |
| I.19-276 | 1,25 | 100 |
| I.19-176 | 1,25 | 100 |
| I.23-176 | 1,25 | 100 |
| I.19-1 | 1,25 | 100 |
| I.19-23 | 1,25 | 100 |
| I.23-23 | 1,25 | 100 |
| I.23-276 | 1,25 | 90 |

**Tabelle A3b: Nachauflaufwirkung bei 5g/ha gegen AMARE in %**

| Beispielnummer | Dosierung [g/ha] | AMARE |
|---|---|---|
| I.1-72 | 5 | 100 |
| I.1-1 | 5 | 100 |
| I.2-72 | 5 | 100 |
| I.2-71 | 5 | 100 |
| I.2-23 | 5 | 100 |
| I.2-2 | 5 | 100 |
| I.2-58 | 5 | 100 |
| I.1-26 | 5 | 100 |
| I.1-23 | 5 | 100 |
| I.1-71 | 5 | 100 |
| I.7-1 | 5 | 100 |
| I.7-23 | 5 | 100 |
| I.7-276 | 5 | 100 |
| I.7-176 | 5 | 100 |
| I.7-2 | 5 | 100 |
| I.9-1 | 5 | 100 |
| I.7-41 | 5 | 100 |
| I.7-278 | 5 | 100 |
| I.7-277 | 5 | 100 |
| I.7-291 | 5 | 100 |
| I.7-71 | 5 | 100 |
| I.7-115 | 5 | 100 |
| I.7-356 | 5 | 100 |
| I.7-394 | 5 | 100 |
| I.7-72 | 5 | 100 |
| I.7-179 | 5 | 100 |
| I.7-177 | 5 | 100 |
| I.7-94 | 5 | 100 |
| I.6-1 | 5 | 100 |
| I.6-176 | 5 | 100 |
| I.6-41 | 5 | 100 |
| I.6-276 | 5 | 100 |
| I.6-23 | 5 | 100 |
| I.6-2 | 5 | 100 |
| I.7-339 | 5 | 100 |
| I.8-23 | 5 | 100 |
| I.8-41 | 5 | 100 |
| I.8-176 | 5 | 100 |
| I.9-23 | 5 | 100 |
| I.9-41 | 5 | 100 |
| I.6-183 | 5 | 100 |
| I.8-2 | 5 | 100 |
| I.8-1 | 5 | 100 |
| I.8-276 | 5 | 100 |
| I.7-183 | 5 | 100 |
| I.9-2 | 5 | 100 |
| I.9-176 | 5 | 100 |
| I.9-276 | 5 | 100 |
| I.8-183 | 5 | 100 |
| I.8-291 | 5 | 100 |
| I.9-291 | 5 | 100 |
| I.19-276 | 5 | 100 |
| I.19-176 | 5 | 100 |
| I.23-176 | 5 | 100 |
| I.23-1 | 5 | 100 |
| I.19-1 | 5 | 100 |
| I.19-23 | 5 | 100 |
| I.23-23 | 5 | 100 |
| I.23-276 | 5 | 100 |

**Tabelle A3c: Nachauflaufwirkung bei 20g/ha gegen AMARE in %**

| Beispielnummer | Dosierung [g/ha] | AMARE |
|---|---|---|
| I.1-72 | 20 | 100 |
| I.1-1 | 20 | 100 |
| I.2-72 | 20 | 100 |
| I.2-71 | 20 | 100 |
| I.2-1 | 20 | 100 |
| I.2-23 | 20 | 100 |
| I.2-2 | 20 | 100 |
| I.2-58 | 20 | 100 |
| I.1-26 | 20 | 100 |
| I.1-23 | 20 | 100 |
| I.1-71 | 20 | 100 |
| I.7-1 | 20 | 100 |
| I.7-276 | 20 | 100 |
| I.7-176 | 20 | 100 |
| I.7-2 | 20 | 100 |
| I.9-1 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 100 |
| I.7-115 | 20 | 100 |
| I.7-356 | 20 | 100 |
| I.7-394 | 20 | 100 |
| I.7-72 | 20 | 100 |
| I.7-179 | 20 | 100 |
| I.7-177 | 20 | 100 |
| I.7-94 | 20 | 100 |
| I.6-1 | 20 | 100 |
| I.6-176 | 20 | 100 |
| I.6-41 | 20 | 100 |
| I.6-276 | 20 | 100 |
| I.6-23 | 20 | 100 |
| I.6-2 | 20 | 100 |
| I.7-339 | 20 | 100 |
| I.8-23 | 20 | 100 |
| I.8-41 | 20 | 100 |
| I.8-176 | 20 | 100 |
| I.9-23 | 20 | 100 |
| I.9-41 | 20 | 100 |
| I.6-183 | 20 | 100 |
| I.8-2 | 20 | 100 |
| I.8-1 | 20 | 100 |
| I.8-276 | 20 | 100 |
| I.7-183 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 100 |
| I.19-176 | 20 | 100 |
| I.23-176 | 20 | 100 |
| I.23-1 | 20 | 100 |
| I.19-1 | 20 | 100 |
| I.19-23 | 20 | 100 |
| I.23-23 | 20 | 100 |
| I.23-276 | 20 | 100 |

**Tabelle A4a: Nachauflaufwirkung bei 5g/ha gegen AVEFA in %**

| Beispielnummer | Dosierung [g/ha] | AVEFA |
|---|---|---|
| I.7-176 | 5 | 80 |
| I.7-41 | 5 | 80 |
| I.9-176 | 5 | 80 |
| I.9-276 | 5 | 80 |
| I.9-291 | 5 | 80 |

**Tabelle A4b: Nachauflaufwirkung bei 20g/ha gegen AVEFA in %**

| Beispielnummer | Dosierung [g/ha] | AVEFA |
|---|---|---|
| I.1-1 | 20 | 80 |
| I.1-26 | 20 | 80 |
| I.1-23 | 20 | 80 |
| I.1-71 | 20 | 80 |
| I.7-1 | 20 | 80 |
| I.7-276 | 20 | 90 |
| I.7-176 | 20 | 100 |
| I.7-2 | 20 | 90 |
| I.7-41 | 20 | 80 |
| I.7-278 | 20 | 80 |
| I.7-291 | 20 | 100 |
| I.7-71 | 20 | 90 |
| I.7-115 | 20 | 100 |
| I.7-356 | 20 | 80 |
| I.7-72 | 20 | 100 |
| I.7-179 | 20 | 80 |
| I.7-177 | 20 | 80 |
| I.7-94 | 20 | 80 |
| I.6-1 | 20 | 100 |
| I.6-176 | 20 | 80 |
| I.6-41 | 20 | 80 |
| I.6-276 | 20 | 80 |
| I.6-23 | 20 | 90 |
| I.8-23 | 20 | 80 |
| I.8-41 | 20 | 80 |
| I.8-176 | 20 | 80 |
| I.9-41 | 20 | 80 |
| I.8-2 | 20 | 100 |
| I.8-1 | 20 | 80 |
| I.8-276 | 20 | 80 |
| I.7-183 | 20 | 90 |
| I.9-2 | 20 | 90 |
| I.9-176 | 20 | 80 |
| I.9-276 | 20 | 90 |
| I.8-183 | 20 | 100 |
| I.8-291 | 20 | 90 |
| I.9-291 | 20 | 80 |

**Tabelle A5a: Nachauflaufwirkung bei 1.25g/ha gegen ECHCG in %**

| Beispielnummer | Dosierung [g/ha] | ECHCG |
|---|---|---|
| I.1-26 | 1,25 | 90 |
| I.1-23 | 1,25 | 90 |
| I.1-71 | 1,25 | 100 |
| I.7-1 | 1.25 | 90 |
| I.7-276 | 1,25 | 90 |
| I.7-41 | 1,25 | 90 |
| I.7-278 | 1,25 | 80 |
| I.7-71 | 1,25 | 100 |
| I.7-115 | 1,25 | 100 |
| I.7-356 | 1,25 | 90 |
| I.7-394 | 1,25 | 100 |
| I.7-72 | 1,25 | 80 |
| I.6-1 | 1,25 | 90 |
| I.6-176 | 1,25 | 100 |
| I.6-23 | 1,25 | 80 |
| I.8-1 | 1,25 | 80 |
| I.9-2 | 1,25 | 80 |
| I.9-176 | 1,25 | 100 |
| I.8-291 | 1,25 | 100 |
| I.19-1 | 1,25 | 100 |
| I.23-276 | 1,25 | 100 |

**Tabelle A5b: Nachauflaufwirkung bei 5g/ha gegen ECHCG in %**

| Beispielnummer | Dosierung [g/ha] | ECHCG |
|---|---|---|
| I.2-72 | 5 | 80 |
| I.2-23 | 5 | 100 |
| I.1-26 | 5 | 90 |
| I.1-23 | 5 | 100 |
| I.7-1 | 5 | 100 |
| I.7-23 | 5 | 100 |
| I.7-276 | 5 | 100 |
| I.7-176 | 5 | 100 |
| I.7-2 | 5 | 100 |
| I.9-1 | 5 | 90 |
| I.7-41 | 5 | 100 |
| I.7-278 | 5 | 100 |
| I.7-277 | 5 | 100 |
| I.7-291 | 5 | 100 |
| I.7-71 | 5 | 100 |
| I.7-115 | 5 | 100 |
| I.7-356 | 5 | 100 |
| I.7-394 | 5 | 100 |
| I.7-72 | 5 | 100 |
| I.7-179 | 5 | 100 |
| I.7-177 | 5 | 100 |
| I.7-94 | 5 | 100 |
| I.6-1 | 5 | 90 |
| I.6-176 | 5 | 100 |
| I.6-41 | 5 | 100 |
| I.6-276 | 5 | 90 |
| I.6-23 | 5 | 90 |
| I.6-2 | 5 | 90 |
| I.7-339 | 5 | 100 |
| I.8-23 | 5 | 100 |
| I.8-41 | 5 | 90 |
| I.8-176 | 5 | 100 |
| I.9-41 | 5 | 80 |
| I.8-2 | 5 | 100 |
| I.8-1 | 5 | 100 |
| I.8-276 | 5 | 100 |
| I.7-183 | 5 | 100 |
| I.9-2 | 5 | 100 |
| I.9-176 | 5 | 100 |
| I.9-276 | 5 | 100 |
| I.8-183 | 5 | 100 |
| I.8-291 | 5 | 100 |
| I.9-291 | 5 | 90 |
| I.19-276 | 5 | 100 |
| I.19-176 | 5 | 100 |
| I.23-176 | 5 | 100 |
| I.23-1 | 5 | 100 |
| I.19-1 | 5 | 100 |
| I.19-23 | 5 | 100 |
| I.23-23 | 5 | 100 |
| I.23-276 | 5 | 100 |

**Tabelle A5c: Nachauflaufwirkung bei 20g/ha gegen ECHCG in %**

| Beispielnummer | Dosierung [g/ha] | ECHCG |
|---|---|---|
| I.1-72 | 20 | 100 |
| I.1-1 | 20 | 80 |
| I.2-72 | 20 | 80 |
| I.2-71 | 20 | 80 |
| I.2-1 | 20 | 100 |
| I.2-23 | 20 | 100 |
| I.2-2 | 20 | 90 |
| I.1-26 | 20 | 100 |
| I.1-23 | 20 | 100 |
| I.1-71 | 20 | 100 |
| I.7-1 | 20 | 90 |
| I.7-276 | 20 | 100 |
| I.7-176 | 20 | 100 |
| I.7-2 | 20 | 100 |
| I.9-1 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 100 |
| I.7-115 | 20 | 100 |
| I.7-356 | 20 | 100 |
| I.7-394 | 20 | 100 |
| I.7-72 | 20 | 100 |
| I.7-179 | 20 | 100 |
| I.7-177 | 20 | 100 |
| I.7-94 | 20 | 100 |
| I.6-1 | 20 | 100 |
| I.6-176 | 20 | 100 |
| I.6-41 | 20 | 100 |
| I.6-276 | 20 | 100 |
| I.6-23 | 20 | 100 |
| I.6-2 | 20 | 100 |
| I.7-339 | 20 | 100 |
| I.8-23 | 20 | 100 |
| I.8-41 | 20 | 100 |
| I.8-176 | 20 | 100 |
| I.9-23 | 20 | 100 |
| I.9-41 | 20 | 100 |
| I.6-183 | 20 | 80 |
| I.8-2 | 20 | 100 |
| I.8-1 | 20 | 100 |
| I.8-276 | 20 | 100 |
| I.7-183 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 100 |
| I.19-176 | 20 | 100 |
| I.23-176 | 20 | 100 |
| I.23-1 | 20 | 100 |
| I.19-1 | 20 | 100 |
| I.19-23 | 20 | 100 |
| I.23-23 | 20 | 100 |
| I.23-276 | 20 | 100 |

**Tabelle A6a: Nachauflaufwirkung bei 5g/ha gegen LOLRI in %**

| Beispielnummer | Dosierung [g/ha] | LOLRI |
|---|---|---|
| I1-26 | 5 | 80 |
| I.7-276 | 5 | 80 |

**Tabelle A6b: Nachauflaufwirkung bei 20g/ha gegen LOLRI in %**

| Beispielnummer | Dosierung [g/ha] | LOLRI |
|---|---|---|
| I.1-23 | 20 | 80 |
| I.1-26 | 20 | 90 |
| I.6-1 | 20 | 80 |
| I.6-176 | 20 | 90 |
| I.6-2 | 20 | 90 |
| I.6-23 | 20 | 90 |
| I.6-276 | 20 | 90 |
| I.7-1 | 20 | 80 |
| I.7-176 | 20 | 80 |
| I.7-177 | 20 | 80 |
| I.7-179 | 20 | 100 |
| I.7-183 | 20 | 80 |
| I.7-2 | 20 | 80 |
| I.7-276 | 20 | 100 |
| I.7-278 | 20 | 80 |
| I.7-41 | 20 | 80 |
| I.7-71 | 20 | 80 |
| I.7-72 | 20 | 80 |
| I.8-1 | 20 | 80 |
| I.8-2 | 20 | 90 |
| I.8-276 | 20 | 100 |
| I.8-291 | 20 | 80 |
| I.9-291 | 20 | 90 |

**Tabelle A7a: Nachauflaufwirkung bei 1.25g/ha gegen MATIN in %**

| Beispielnummer | Dosierung [g/ha] | MATIN |
|---|---|---|
| I.1-26 | 1,25 | 80 |
| I.1-23 | 1,25 | 80 |
| I.1-71 | 1,25 | 80 |
| I.7-1 | 1,25 | 100 |
| I.7-23 | 1,25 | 90 |
| I.7-276 | 1,25 | 100 |
| I.7-2 | 1,25 | 80 |
| I.7-41 | 1,25 | 90 |
| I.7-71 | 1,25 | 80 |
| I.7-115 | 1,25 | 100 |
| I.7-356 | 1,25 | 80 |
| I.7-179 | 1,25 | 80 |
| I.7-177 | 1,25 | 80 |
| I.7-94 | 1,25 | 80 |
| I.6-23 | 1,25 | 80 |
| I.8-41 | 1,25 | 90 |
| I.9-41 | 1,25 | 100 |
| I.8-2 | 1,25 | 100 |
| I.8-1 | 1,25 | 100 |
| I.8-276 | 1,25 | 80 |
| I.7-183 | 1,25 | 80 |

**Tabelle A7b: Nachauflaufwirkung bei 5g/ha gegen MATIN in %**

| Beispielnummer | Dosierung [g/ha] | MATIN |
|---|---|---|
| I.1-72 | 5 | 90 |
| I.1-1 | 5 | 100 |
| I.2-71 | 5 | 80 |
| I.2-1 | 5 | 80 |
| I.2-23 | 5 | 80 |
| I.2-2 | 5 | 80 |
| I.2-58 | 5 | 80 |
| I.1-26 | 5 | 80 |
| I.1-23 | 5 | 100 |
| I.1-71 | 5 | 90 |
| I.7-1 | 5 | 100 |
| I.7-23 | 5 | 100 |
| I.7-276 | 5 | 100 |
| I.7-176 | 5 | 90 |
| I.7-2 | 5 | 80 |
| I.9-1 | 5 | 80 |
| I.7-41 | 5 | 90 |
| I.7-278 | 5 | 80 |
| I.7-277 | 5 | 80 |
| I.7-291 | 5 | 100 |
| I.7-71 | 5 | 100 |
| I.7-115 | 5 | 100 |
| I.7-356 | 5 | 100 |
| I.7-394 | 5 | 80 |
| I.7-72 | 5 | 100 |
| I.7-179 | 5 | 100 |
| I.7-177 | 5 | 80 |
| I.7-94 | 5 | 80 |
| I.6-1 | 5 | 100 |
| I.6-176 | 5 | 80 |
| I.6-41 | 5 | 80 |
| I.6-276 | 5 | 100 |
| I.6-23 | 5 | 100 |
| I.6-2 | 5 | 90 |
| I.7-339 | 5 | 100 |
| I.8-23 | 5 | 100 |
| I.8-41 | 5 | 90 |
| I.8-176 | 5 | 100 |
| I.9-23 | 5 | 80 |
| I.9-41 | 5 | 100 |
| I.6-183 | 5 | 80 |
| I.8-2 | 5 | 100 |
| I.8-1 | 5 | 100 |
| I.8-276 | 5 | 80 |
| I.7-183 | 5 | 100 |
| I.9-2 | 5 | 100 |
| I.9-176 | 5 | 100 |
| I.9-276 | 5 | 100 |
| I.8-183 | 5 | 80 |
| I.9-291 | 5 | 80 |
| I.19-276 | 5 | 100 |
| I.19-176 | 5 | 100 |
| I.23-1 | 5 | 80 |
| I.19-1 | 5 | 90 |
| I.19-23 | 5 | 100 |

**Tabelle A7c: Nachauflaufwirkung bei 20g/ha gegen MATIN in %**

| Beispielnummer | Dosierung [g/ha] | MATIN |
|---|---|---|
| I.1-72 | 20 | 100 |
| I.1-1 | 20 | 100 |
| I.2-72 | 20 | 100 |
| I.2-71 | 20 | 90 |
| I.2-1 | 20 | 100 |
| I.2-23 | 20 | 100 |
| I.2-2 | 20 | 100 |
| I.2-58 | 20 | 90 |
| I.1-26 | 20 | 100 |
| I.1-23 | 20 | 100 |
| I.1-71 | 20 | 100 |
| I.7-1 | 20 | 100 |
| I.7-276 | 20 | 100 |
| I.7-176 | 20 | 100 |
| I.7-2 | 20 | 100 |
| I.9-1 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 100 |
| I.7-115 | 20 | 100 |
| I.7-356 | 20 | 100 |
| I.7-394 | 20 | 90 |
| I.7-72 | 20 | 100 |
| I.7-179 | 20 | 100 |
| I.7-177 | 20 | 80 |
| I.7-94 | 20 | 100 |
| I.6-1 | 20 | 100 |
| I.6-176 | 20 | 100 |
| I.6-41 | 20 | 100 |
| I.6-276 | 20 | 100 |
| I.6-23 | 20 | 100 |
| I.6-2 | 20 | 100 |
| I.7-339 | 20 | 100 |
| I.8-23 | 20 | 100 |
| I.8-41 | 20 | 100 |
| I.8-176 | 20 | 100 |
| I.9-23 | 20 | 100 |
| I.9-41 | 20 | 100 |
| I.6-183 | 20 | 100 |
| I.8-2 | 20 | 100 |
| I.8-1 | 20 | 100 |
| I.8-276 | 20 | 100 |
| I.7-183 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 100 |
| I.19-176 | 20 | 100 |
| I.23-176 | 20 | 80 |
| I.23-1 | 20 | 100 |
| I.19-1 | 20 | 100 |
| I.19-23 | 20 | 100 |
| I.23-23 | 20 | 100 |
| I.23-276 | 20 | 90 |

**Tabelle A8a: Nachauflaufwirkung bei 1.25g/ha gegen PHBPU in %**

| Beispielnummer | Dosierung [g/ha] | PHBPU |
|---|---|---|
| I.1-72 | 1,25 | 80 |
| I.1-1 | 1,25 | 100 |
| I.2-72 | 1,25 | 100 |
| I.2-71 | 1,25 | 80 |
| I.2-1 | 1,25 | 100 |
| I.2-23 | 1,25 | 100 |
| I.2-2 | 1,25 | 100 |
| I.1-26 | 1,25 | 100 |
| I.1-23 | 1,25 | 100 |
| I.1-71 | 1,25 | 100 |
| I.7-1 | 1,25 | 100 |
| I.7-276 | 1,25 | 100 |
| I.7-2 | 1,25 | 100 |
| I.7-41 | 1,25 | 100 |
| I.7-278 | 1,25 | 100 |
| I.7-291 | 1,25 | 100 |
| I.7-71 | 1,25 | 100 |
| I.7-115 | 1,25 | 100 |
| I.7-394 | 1,25 | 100 |
| I.7-72 | 1,25 | 100 |
| I.7-179 | 1,25 | 100 |
| I.7-177 | 1,25 | 90 |
| I.7-94 | 1,25 | 100 |
| I.6-176 | 1,25 | 80 |
| I.6-41 | 1,25 | 100 |
| I.6-276 | 1,25 | 100 |
| I.6-23 | 1,25 | 80 |
| I.6-2 | 1,25 | 100 |
| I.7-339 | 1,25 | 100 |
| I.8-23 | 1,25 | 100 |
| I.8-41 | 1,25 | 100 |
| I.8-176 | 1,25 | 80 |
| I.9-23 | 1,25 | 80 |
| I.9-41 | 1,25 | 100 |
| I.6-183 | 1,25 | 100 |
| I.8-2 | 1,25 | 100 |
| I.8-1 | 1,25 | 100 |
| I.8-276 | 1,25 | 100 |
| I.7-183 | 1,25 | 80 |
| I.9-2 | 1,25 | 100 |
| I.9-276 | 1,25 | 100 |
| I.8-291 | 1,25 | 100 |
| I.9-291 | 1,25 | 100 |
| I.19-176 | 1,25 | 100 |
| I.23-176 | 1,25 | 90 |
| I.19-1 | 1,25 | 100 |
| I.19-23 | 1,25 | 100 |
| I.23-23 | 1,25 | 100 |
| I.23-276 | 1,25 | 90 |

**Tabelle A8b: Nachauflaufwirkung bei 5g/ha gegen PHBPU in %**

| Beispielnummer | Dosierung [g/ha] | PHBPU |
|---|---|---|
| I.1-72 | 5 | 100 |
| I.1-1 | 5 | 100 |
| I.2-72 | 5 | 100 |
| I.2-71 | 5 | 100 |
| I.2-1 | 5 | 100 |
| I.2-23 | 5 | 100 |
| I.2-2 | 5 | 100 |
| I.2-58 | 5 | 80 |
| I.1-26 | 5 | 100 |
| I.1-23 | 5 | 100 |
| I.1-71 | 5 | 100 |
| I.7-1 | 5 | 100 |
| I.7-23 | 5 | 100 |
| I.7-276 | 5 | 100 |
| I.7-176 | 5 | 100 |
| I.7-2 | 5 | 100 |
| I.9-1 | 5 | 100 |
| I.7-41 | 5 | 100 |
| I.7-278 | 5 | 100 |
| I.7-277 | 5 | 100 |
| I.7-291 | 5 | 100 |
| I.7-71 | 5 | 100 |
| I.7-115 | 5 | 100 |
| I.7-356 | 5 | 100 |
| I.7-394 | 5 | 100 |
| I.7-72 | 5 | 100 |
| I.7-179 | 5 | 100 |
| I.7-177 | 5 | 100 |
| I.7-94 | 5 | 100 |
| I.6-1 | 5 | 100 |
| I.6-176 | 5 | 100 |
| I.6-41 | 5 | 100 |
| I.6-276 | 5 | 100 |
| I.6-23 | 5 | 90 |
| I.6-2 | 5 | 100 |
| I.7-339 | 5 | 100 |
| I.8-23 | 5 | 100 |
| I.8-41 | 5 | 100 |
| I.8-176 | 5 | 80 |
| I.9-23 | 5 | 100 |
| I.9-41 | 5 | 100 |
| I.6-183 | 5 | 100 |
| I.8-2 | 5 | 100 |
| I.8-1 | 5 | 100 |
| I.8-276 | 5 | 100 |
| I.7-183 | 5 | 100 |
| I.9-2 | 5 | 100 |
| I.9-176 | 5 | 100 |
| I.9-276 | 5 | 100 |
| I.8-183 | 5 | 100 |
| I.8-291 | 5 | 100 |
| I.9-291 | 5 | 100 |
| I.19-276 | 5 | 100 |
| I.19-176 | 5 | 100 |
| I.23-176 | 5 | 90 |
| I.23-1 | 5 | 80 |
| I.19-1 | 5 | 100 |
| I.19-23 | 5 | 100 |
| I.23-23 | 5 | 100 |
| I.23-276 | 5 | 100 |

**Tabelle A8c: Nachauflaufwirkung bei 20g/ha gegen PHBPU in %**

| Beispielnummer | Dosierung [g/ha] | PHBPU |
|---|---|---|
| I.1-72 | 20 | 100 |
| I.1-1 | 20 | 100 |
| I.2-72 | 20 | 100 |
| I.2-71 | 20 | 100 |
| I.2-1 | 20 | 100 |
| I.2-23 | 20 | 100 |
| I.2-2 | 20 | 100 |
| I.2-58 | 20 | 100 |
| I.1-26 | 20 | 100 |
| I.1-23 | 20 | 100 |
| I.1-71 | 20 | 100 |
| I.7-1 | 20 | 100 |
| I.7-276 | 20 | 100 |
| I.7-176 | 20 | 100 |
| I.7-2 | 20 | 100 |
| I.9-1 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 100 |
| I.7-115 | 20 | 100 |
| I.7-356 | 20 | 100 |
| I.7-394 | 20 | 100 |
| I.7-72 | 20 | 100 |
| I.7-179 | 20 | 100 |
| I.7-177 | 20 | 100 |
| I.7-94 | 20 | 100 |
| I.6-1 | 20 | 100 |
| I.6-176 | 20 | 100 |
| I.6-41 | 20 | 100 |
| I.6-276 | 20 | 100 |
| I.6-23 | 20 | 100 |
| I.6-2 | 20 | 100 |
| I.7-339 | 20 | 100 |
| I.8-23 | 20 | 100 |
| I.8-41 | 20 | 100 |
| I.8-176 | 20 | 100 |
| I.9-23 | 20 | 100 |
| I.9-41 | 20 | 100 |
| I.6-183 | 20 | 100 |
| I.8-2 | 20 | 100 |
| I.8-1 | 20 | 100 |
| I.8-276 | 20 | 100 |
| I.7-183 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 100 |
| I.19-176 | 20 | 100 |
| I.23-176 | 20 | 100 |
| I.23-1 | 20 | 80 |
| I.19-1 | 20 | 100 |
| I.19-23 | 20 | 100 |
| I.23-23 | 20 | 100 |
| I.23-276 | 20 | 100 |

**Tabelle 9a: Nachauflaufwirkung bei 1.25g/ha gegen POLCO in %**

| Beispielnummer | Dosierung [g/ha] | POLCO |
|---|---|---|
| I.1-72 | 1,25 | 80 |
| I.2-58 | 1,25 | 100 |
| I.1-26 | 1,25 | 100 |
| I.1-23 | 1,25 | 100 |
| I.1-71 | 1,25 | 100 |
| I.7-72 | 1,25 | 80 |
| I.6-23 | 1,25 | 90 |
| I.8-41 | 1,25 | 80 |
| I.8-1 | 1,25 | 90 |
| I.9-291 | 1,25 | 80 |
| I.19-176 | 1,25 | 100 |
| I.23-1 | 1,25 | 90 |
| I.19-23 | 1,25 | 100 |
| I.23-23 | 1,25 | 100 |

**Tabelle A9b: Nachauflaufwirkung bei 5g/ha gegen POLCO in %**

| Beispielnummer | Dosierung [g/ha] | POLCO |
|---|---|---|
| I.1-72 | 5 | 100 |
| I.1-1 | 5 | 100 |
| I.2-72 | 5 | 80 |
| I.2-71 | 5 | 100 |
| I.2-1 | 5 | 80 |
| I.2-23 | 5 | 100 |
| I.2-2 | 5 | 100 |
| I.2-58 | 5 | 100 |
| I.1-26 | 5 | 100 |
| I.1-23 | 5 | 100 |
| I.1-71 | 5 | 100 |
| I.7-1 | 5 | 80 |
| I.7-23 | 5 | 100 |
| I.7-176 | 5 | 100 |
| I.7-41 | 5 | 100 |
| I.7-278 | 5 | 80 |
| I.7-71 | 5 | 100 |
| I.7-115 | 5 | 100 |
| I.7-356 | 5 | 100 |
| I.7-394 | 5 | 100 |
| I.7-72 | 5 | 100 |
| I.7-179 | 5 | 100 |
| I.7-177 | 5 | 80 |
| I.6-1 | 5 | 100 |
| I.6-23 | 5 | 100 |
| I.6-2 | 5 | 100 |
| I.7-339 | 5 | 100 |
| I.8-23 | 5 | 100 |
| I.8-41 | 5 | 90 |
| I.8-176 | 5 | 80 |
| I.9-23 | 5 | 100 |
| I.9-41 | 5 | 90 |
| I.8-2 | 5 | 100 |
| I.8-1 | 5 | 100 |
| I.8-276 | 5 | 100 |
| I.9-2 | 5 | 100 |
| I.8-183 | 5 | 100 |
| I.8-291 | 5 | 100 |
| I.9-291 | 5 | 90 |
| I.19-276 | 5 | 100 |
| I.19-176 | 5 | 100 |
| I.23-176 | 5 | 100 |
| I.23-1 | 5 | 100 |
| I.19-1 | 5 | 100 |
| I.19-23 | 5 | 100 |
| I.23-23 | 5 | 100 |

**Tabelle A9c: Nachauflaufwirkung bei 20g/ha gegen POLCO in %**

| Beispielnummer | Dosierung [g/ha] | POLCO |
|---|---|---|
| I.1-72 | 20 | 100 |
| I.1-1 | 20 | 100 |
| I.2-72 | 20 | 100 |
| I.2-71 | 20 | 100 |
| I.2-1 | 20 | 100 |
| I.2-23 | 20 | 100 |
| I.2-2 | 20 | 100 |
| I.2-58 | 20 | 100 |
| I.1-26 | 20 | 100 |
| I.1-23 | 20 | 100 |
| I.1-71 | 20 | 100 |
| I.7-1 | 20 | 100 |
| I.7-176 | 20 | 100 |
| I.7-2 | 20 | 100 |
| I.9-1 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 100 |
| I.7-115 | 20 | 100 |
| I.7-356 | 20 | 100 |
| I.7-394 | 20 | 100 |
| I.7-72 | 20 | 100 |
| I.7-179 | 20 | 100 |
| I.7-177 | 20 | 100 |
| I.7-94 | 20 | 100 |
| I.6-1 | 20 | 100 |
| I.6-176 | 20 | 100 |
| I.6-41 | 20 | 100 |
| I.6-276 | 20 | 100 |
| I.6-23 | 20 | 100 |
| I.6-2 | 20 | 100 |
| I.7-339 | 20 | 100 |
| I.8-23 | 20 | 100 |
| I.8-41 | 20 | 100 |
| I.8-176 | 20 | 100 |
| I.9-23 | 20 | 100 |
| I.9-41 | 20 | 100 |
| I.6-183 | 20 | 100 |
| I.8-2 | 20 | 100 |
| I.8-1 | 20 | 100 |
| I.8-276 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 100 |
| I.19-176 | 20 | 100 |
| I.23-176 | 20 | 100 |
| I.23-1 | 20 | 100 |
| I.19-1 | 20 | 100 |
| I.19-23 | 20 | 100 |
| I.23-23 | 20 | 100 |
| I.23-276 | 20 | 100 |

**Tabelle A10a: Nachauflaufwirkung bei 1.25g/ha gegen SETVI in %**

| Beispielnummer | Dosierung [g/ha] | SETVI |
|---|---|---|
| I.1-72 | 1,25 | 100 |
| I.1-1 | 1,25 | 100 |
| I.2-72 | 1,25 | 90 |
| I.2-71 | 1,25 | 90 |
| I.2-1 | 1,25 | 100 |
| I.2-2 | 1,25 | 100 |
| I.2-58 | 1,25 | 100 |
| I.1-26 | 1,25 | 100 |
| I.1-23 | 1,25 | 100 |
| I.1-71 | 1,25 | 100 |
| I.7-1 | 1,25 | 100 |
| I.7-23 | 1,25 | 100 |
| I.7-276 | 1,25 | 100 |
| I.7-2 | 1,25 | 100 |
| I.9-1 | 1,25 | 80 |
| I.7-41 | 1,25 | 100 |
| I.7-278 | 1,25 | 100 |
| I.7-277 | 1,25 | 100 |
| I.7-291 | 1,25 | 100 |
| I.7-71 | 1,25 | 100 |
| I.7-115 | 1,25 | 100 |
| I.7-356 | 1,25 | 100 |
| I.7-394 | 1,25 | 100 |
| I.7-72 | 1,25 | 100 |
| I.7-179 | 1,25 | 100 |
| I.7-177 | 1,25 | 100 |
| I.7-94 | 1,25 | 100 |
| I.6-1 | 1,25 | 100 |
| I.6-176 | 1,25 | 100 |
| I.6-41 | 1,25 | 100 |
| I.6-276 | 1,25 | 100 |
| I.6-23 | 1,25 | 100 |
| I.6-2 | 1,25 | 100 |
| I.7-339 | 1,25 | 100 |
| I.8-23 | 1,25 | 100 |
| I.8-41 | 1,25 | 100 |
| I.8-176 | 1,25 | 100 |
| I.9-23 | 1,25 | 100 |
| I.9-41 | 1,25 | 100 |
| I.8-2 | 1,25 | 100 |
| I.8-1 | 1,25 | 100 |
| I.8-276 | 1,25 | 100 |
| I.7-183 | 1,25 | 100 |
| I.9-2 | 1,25 | 100 |
| I.9-176 | 1,25 | 100 |
| I.9-276 | 1,25 | 100 |
| I.8-183 | 1,25 | 100 |
| I.8-291 | 1,25 | 100 |
| I.9-291 | 1,25 | 100 |
| I.19-276 | 1,25 | 100 |
| I.19-176 | 1,25 | 90 |
| I.23-176 | 1,25 | 100 |
| I.23-1 | 1,25 | 100 |
| I.19-1 | 1,25 | 100 |
| I.19-23 | 1,25 | 100 |
| I.23-23 | 1,25 | 100 |
| I.23-276 | 1,25 | 100 |

**Tabelle A10b: Nachauflaufwirkung bei 5g/ha gegen SETVI in %**

| Beispielnummer | Dosierung [g/ha] | SETVI |
|---|---|---|
| I.1-72 | 5 | 100 |
| I.1-1 | 5 | 100 |
| I.2-72 | 5 | 100 |
| I.2-71 | 5 | 100 |
| I.2-1 | 5 | 100 |
| I.2-23 | 5 | 100 |
| I.2-2 | 5 | 100 |
| I.2-58 | 5 | 100 |
| I.1-26 | 5 | 100 |
| I.1-23 | 5 | 100 |
| I.1-71 | 5 | 100 |
| I.7-1 | 5 | 100 |
| I.7-23 | 5 | 100 |
| I.7-276 | 5 | 100 |
| I.7-176 | 5 | 100 |
| I.7-2 | 5 | 100 |
| I.9-1 | 5 | 100 |
| I.7-41 | 5 | 100 |
| I.7-278 | 5 | 100 |
| I.7-277 | 5 | 100 |
| I.7-291 | 5 | 100 |
| I.7-71 | 5 | 100 |
| I.7-115 | 5 | 100 |
| I.7-356 | 5 | 100 |
| I.7-394 | 5 | 100 |
| I.7-72 | 5 | 100 |
| I.7-179 | 5 | 100 |
| I.7-177 | 5 | 100 |
| I.7-94 | 5 | 100 |
| I.6-1 | 5 | 100 |
| I.6-176 | 5 | 100 |
| I.6-41 | 5 | 100 |
| I.6-276 | 5 | 100 |
| I.6-23 | 5 | 100 |
| I.6-2 | 5 | 100 |
| I.7-339 | 5 | 100 |
| I.8-23 | 5 | 100 |
| I.8-41 | 5 | 100 |
| I.8-176 | 5 | 100 |
| I.9-23 | 5 | 100 |
| I.9-41 | 5 | 100 |
| I.6-183 | 5 | 100 |
| I.8-2 | 5 | 100 |
| I.8-1 | 5 | 100 |
| I.8-276 | 5 | 100 |
| I.7-183 | 5 | 100 |
| I.9-2 | 5 | 100 |
| I.9-176 | 5 | 100 |
| I.9-276 | 5 | 100 |
| I.8-183 | 5 | 100 |
| I.8-291 | 5 | 100 |
| I.9-291 | 5 | 100 |
| I.19-276 | 5 | 100 |
| I.19-176 | 5 | 100 |
| I.23-176 | 5 | 100 |
| I.23-1 | 5 | 100 |
| I.19-1 | 5 | 100 |
| I.19-23 | 5 | 100 |
| I.23-23 | 5 | 100 |
| I.23-276 | 5 | 100 |

**Tabelle A10c: Nachauflaufwirkung bei 20g/ha gegen SETVI in %**

| Beispielnummer | Dosierung [g/ha] | SETVI |
|---|---|---|
| I.1-72 | 20 | 100 |
| I.1-1 | 20 | 100 |
| I.2-72 | 20 | 100 |
| I.2-71 | 20 | 100 |
| I.2-1 | 20 | 100 |
| I.2-23 | 20 | 100 |
| I.2-2 | 20 | 100 |
| I.2-58 | 20 | 100 |
| I.1-26 | 20 | 100 |
| I.1-23 | 20 | 100 |
| I.1-71 | 20 | 100 |
| I.7-1 | 20 | 100 |
| I.7-276 | 20 | 100 |
| I.7-176 | 20 | 100 |
| I.7-2 | 20 | 100 |
| I.9-1 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 100 |
| I.7-115 | 20 | 100 |
| I.7-356 | 20 | 100 |
| I.7-394 | 20 | 100 |
| I.7-72 | 20 | 100 |
| I.7-179 | 20 | 100 |
| I.7-177 | 20 | 100 |
| I.7-94 | 20 | 100 |
| I.6-1 | 20 | 100 |
| I.6-176 | 20 | 100 |
| I.6-41 | 20 | 100 |
| I.6-276 | 20 | 100 |
| I.6-23 | 20 | 100 |
| I.6-2 | 20 | 100 |
| I.7-339 | 20 | 100 |
| I.8-23 | 20 | 100 |
| I.8-41 | 20 | 100 |
| I.8-176 | 20 | 100 |
| I.9-23 | 20 | 100 |
| I.9-41 | 20 | 100 |
| I.6-183 | 20 | 100 |
| I.8-2 | 20 | 100 |
| I.8-1 | 20 | 100 |
| I.8-276 | 20 | 100 |
| I.7-183 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 100 |
| I.19-176 | 20 | 100 |
| I.23-176 | 20 | 100 |
| I.23-1 | 20 | 100 |
| I.19-1 | 20 | 100 |
| I.19-23 | 20 | 100 |
| I.23-23 | 20 | 100 |
| I.23-276 | 20 | 100 |

**Tabelle A11a: Nachauflaufwirkung bei 1.25g/ha gegen VERPE in %**

| Beispielnummer | Dosierung [g/ha] | VERPE |
|---|---|---|
| I.1-72 | 1,25 | 100 |
| I.1-1 | 1,25 | 80 |
| I.2-72 | 1,25 | 100 |
| I.2-71 | 1,25 | 100 |
| I.2-1 | 1,25 | 90 |
| I.2-23 | 1,25 | 80 |
| I.2-2 | 1,25 | 100 |
| I.1-26 | 1,25 | 100 |
| I.1-23 | 1,25 | 100 |
| I.1-71 | 1,25 | 100 |
| I.7-1 | 1,25 | 100 |
| I.7-23 | 1,25 | 90 |
| I.7-276 | 1,25 | 100 |
| I.7-2 | 1,25 | 80 |
| I.9-1 | 1,25 | 100 |
| I.7-41 | 1,25 | 80 |
| I.7-278 | 1,25 | 100 |
| I.7-277 | 1,25 | 100 |
| I.7-71 | 1,25 | 100 |
| I.7-115 | 1,25 | 100 |
| I.7-356 | 1,25 | 100 |
| I.7-394 | 1,25 | 100 |
| I.7-72 | 1,25 | 90 |
| I.7-179 | 1,25 | 100 |
| I.7-177 | 1,25 | 90 |
| I.7-94 | 1,25 | 100 |
| I.6-176 | 1,25 | 80 |
| I.6-23 | 1,25 | 100 |
| I.6-2 | 1,25 | 100 |
| I.7-339 | 1,25 | 80 |
| I.8-23 | 1,25 | 80 |
| I.8-176 | 1,25 | 100 |
| I.9-23 | 1,25 | 80 |
| I.8-2 | 1,25 | 100 |
| I.8-1 | 1,25 | 100 |
| I.8-276 | 1,25 | 100 |
| I.9-2 | 1,25 | 100 |
| I.9-176 | 1,25 | 100 |
| I.9-276 | 1,25 | 80 |
| I.8-291 | 1,25 | 100 |
| I.9-291 | 1,25 | 100 |
| I.19-276 | 1,25 | 100 |
| I.19-176 | 1,25 | 100 |
| I.23-176 | 1,25 | 100 |
| I.23-1 | 1,25 | 80 |
| I.19-1 | 1,25 | 100 |
| I.19-23 | 1,25 | 100 |
| I.23-276 | 1,25 | 80 |

**Tabelle A11b: Nachauflaufwirkung bei 5g/ha gegen VERPE in %**

| Beispielnummer | Dosierung [g/ha] | VERPE |
|---|---|---|
| I.1-72 | 5 | 100 |
| I.1-1 | 5 | 100 |
| I.2-72 | 5 | 100 |
| I.2-71 | 5 | 100 |
| I.2-1 | 5 | 100 |
| I.2-23 | 5 | 100 |
| I.2-2 | 5 | 100 |
| 1.2-58 | 5 | 80 |
| I.1-26 | 5 | 100 |
| I.1-23 | 5 | 100 |
| I.1-71 | 5 | 100 |
| I.7-1 | 5 | 100 |
| I.7-23 | 5 | 100 |
| I.7-276 | 5 | 100 |
| I.7-176 | 5 | 100 |
| I.7-2 | 5 | 100 |
| I.9-1 | 5 | 100 |
| I.7-41 | 5 | 100 |
| I.7-278 | 5 | 100 |
| I.7-277 | 5 | 100 |
| I.7-291 | 5 | 100 |
| I.7-71 | 5 | 100 |
| I.7-115 | 5 | 100 |
| I.7-356 | 5 | 100 |
| I.7-394 | 5 | 100 |
| I.7-72 | 5 | 100 |
| I.7-179 | 5 | 100 |
| I.7-177 | 5 | 100 |
| I.7-94 | 5 | 100 |
| I.6-1 | 5 | 100 |
| I.6-176 | 5 | 90 |
| I.6-41 | 5 | 100 |
| I.6-276 | 5 | 100 |
| I.6-23 | 5 | 100 |
| I.6-2 | 5 | 100 |
| I.7-339 | 5 | 100 |
| I.8-23 | 5 | 100 |
| I.8-41 | 5 | 100 |
| I.8-176 | 5 | 100 |
| I.9-23 | 5 | 100 |
| I.9-41 | 5 | 100 |
| I.6-183 | 5 | 100 |
| I.8-2 | 5 | 100 |
| I.8-1 | 5 | 100 |
| I.8-276 | 5 | 100 |
| I.7-183 | 5 | 100 |
| I.9-2 | 5 | 100 |
| I.9-176 | 5 | 100 |
| I.9-276 | 5 | 100 |
| I.8-183 | 5 | 100 |
| I.8-291 | 5 | 100 |
| I.9-291 | 5 | 100 |
| I.19-276 | 5 | 100 |
| I.19-176 | 5 | 100 |
| I.23-176 | 5 | 100 |
| I.23-1 | 5 | 100 |
| I.19-1 | 5 | 100 |
| I.19-23 | 5 | 100 |
| I.23-23 | 5 | 100 |
| I.23-276 | 5 | 100 |

**Tabelle A11c: Nachauflaufwirkung bei 20g/ha gegen VERPE in %**

| Beispielnummer | Dosierung [g/ha] | VERPE |
|---|---|---|
| I.1-72 | 20 | 100 |
| I.1-1 | 20 | 100 |
| I.2-72 | 20 | 100 |
| I.2-71 | 20 | 100 |
| I.2-1 | 20 | 100 |
| I.2-23 | 20 | 100 |
| I.2-2 | 20 | 100 |
| 1.2-58 | 20 | 100 |
| I.1-26 | 20 | 100 |
| I.1-23 | 20 | 100 |
| I.1-71 | 20 | 100 |
| I.7-1 | 20 | 100 |
| I.7-276 | 20 | 100 |
| I.7-176 | 20 | 100 |
| I.7-2 | 20 | 100 |
| I.9-1 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 100 |
| I.7-115 | 20 | 100 |
| I.7-356 | 20 | 100 |
| I.7-394 | 20 | 100 |
| I.7-72 | 20 | 100 |
| I.7-179 | 20 | 100 |
| I.7-177 | 20 | 100 |
| I.7-94 | 20 | 100 |
| I.6-1 | 20 | 100 |
| I.6-176 | 20 | 100 |
| I.6-41 | 20 | 100 |
| I.6-276 | 20 | 100 |
| I.6-23 | 20 | 100 |
| I.6-2 | 20 | 100 |
| I.7-339 | 20 | 100 |
| I.8-23 | 20 | 100 |
| I.8-41 | 20 | 100 |
| I.8-176 | 20 | 100 |
| I.9-23 | 20 | 100 |
| I.9-41 | 20 | 100 |
| I.6-183 | 20 | 100 |
| I.8-2 | 20 | 100 |
| I.8-1 | 20 | 100 |
| I.8-276 | 20 | 100 |
| I.7-183 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 100 |
| I.19-176 | 20 | 100 |
| I.23-176 | 20 | 100 |
| I.23-1 | 20 | 100 |
| I.19-1 | 20 | 100 |
| I.19-23 | 20 | 100 |
| I.23-23 | 20 | 100 |
| I.23-276 | 20 | 100 |

**Tabelle A12a: Nachauflaufwirkung bei 1.25g/ha gegen VIOTR in %**

| Beispielnummer | Dosierung [g/ha] | VIOTR |
|---|---|---|
| I.1-72 | 1,25 | 100 |
| I.1-1 | 1,25 | 100 |
| I.2-72 | 1,25 | 100 |
| I.2-71 | 1,25 | 100 |
| I.2-1 | 1,25 | 100 |
| I.2-2 | 1,25 | 100 |
| 1.2-58 | 1,25 | 100 |
| I.1-26 | 1,25 | 100 |
| I.1-23 | 1,25 | 100 |
| I.1-71 | 1,25 | 100 |
| I.7-1 | 1,25 | 100 |
| I.7-23 | 1,25 | 100 |
| I.7-276 | 1,25 | 100 |
| I.7-2 | 1,25 | 100 |
| I.9-1 | 1,25 | 100 |
| I.7-41 | 1,25 | 100 |
| I.7-278 | 1,25 | 90 |
| I.7-277 | 1,25 | 80 |
| I.7-291 | 1,25 | 100 |
| I.7-71 | 1,25 | 100 |
| I.7-115 | 1,25 | 100 |
| I.7-356 | 1,25 | 100 |
| I.7-394 | 1,25 | 100 |
| I.7-72 | 1,25 | 100 |
| I.7-179 | 1,25 | 100 |
| I.7-177 | 1,25 | 100 |
| I.7-94 | 1,25 | 80 |
| I.6-1 | 1,25 | 100 |
| I.6-176 | 1,25 | 100 |
| I.6-41 | 1,25 | 100 |
| I.6-276 | 1,25 | 100 |
| I.6-23 | 1,25 | 100 |
| I.6-2 | 1,25 | 100 |
| I.7-339 | 1,25 | 100 |
| I.8-23 | 1,25 | 100 |
| I.8-41 | 1,25 | 100 |
| I.8-176 | 1,25 | 90 |
| I.9-23 | 1,25 | 100 |
| I.9-41 | 1,25 | 90 |
| I.6-183 | 1,25 | 90 |
| I.8-2 | 1,25 | 100 |
| I.8-1 | 1,25 | 100 |
| I.8-276 | 1,25 | 100 |
| I.7-183 | 1,25 | 100 |
| I.9-2 | 1,25 | 100 |
| I.9-176 | 1,25 | 100 |
| I.9-276 | 1,25 | 100 |
| I.8-183 | 1,25 | 100 |
| I.8-291 | 1,25 | 100 |
| I.9-291 | 1,25 | 100 |
| I.19-276 | 1,25 | 100 |
| I.19-176 | 1,25 | 80 |
| I.23-176 | 1,25 | 90 |
| I.23-1 | 1,25 | 80 |
| I.19-1 | 1,25 | 100 |
| I.23-23 | 1,25 | 100 |
| I.23-276 | 1,25 | 80 |

**Tabelle A12b: Nachauflaufwirkung bei 5g/ha gegen VIOTR in %**

| Beispielnummer | Dosierung [g/ha] | VIOTR |
|---|---|---|
| I.1-72 | 5 | 100 |
| I.1-1 | 5 | 100 |
| I.2-72 | 5 | 100 |
| I.2-71 | 5 | 100 |
| I.2-1 | 5 | 100 |
| I.2-23 | 5 | 100 |
| I.2-2 | 5 | 100 |
| I.2-58 | 5 | 100 |
| I.1-26 | 5 | 100 |
| I.1-23 | 5 | 100 |
| I.1-71 | 5 | 100 |
| I.7-1 | 5 | 100 |
| I.7-23 | 5 | 100 |
| I.7-276 | 5 | 100 |
| I.7-176 | 5 | 100 |
| I.7-2 | 5 | 100 |
| I.9-1 | 5 | 100 |
| I.7-41 | 5 | 100 |
| I.7-278 | 5 | 100 |
| I.7-277 | 5 | 100 |
| I.7-291 | 5 | 100 |
| I.7-71 | 5 | 100 |
| I.7-115 | 5 | 100 |
| I.7-356 | 5 | 100 |
| I.7-394 | 5 | 100 |
| I.7-72 | 5 | 100 |
| I.7-179 | 5 | 100 |
| I.7-177 | 5 | 100 |
| I.7-94 | 5 | 100 |
| I.6-1 | 5 | 100 |
| I.6-176 | 5 | 100 |
| I.6-41 | 5 | 100 |
| I.6-276 | 5 | 100 |
| I.6-23 | 5 | 100 |
| I.6-2 | 5 | 100 |
| I.7-339 | 5 | 100 |
| I.8-23 | 5 | 100 |
| I.8-41 | 5 | 100 |
| I.8-176 | 5 | 100 |
| I.9-23 | 5 | 100 |
| I.9-41 | 5 | 100 |
| I.6-183 | 5 | 100 |
| I.8-2 | 5 | 100 |
| I.8-1 | 5 | 100 |
| I.8-276 | 5 | 100 |
| I.7-183 | 5 | 100 |
| I.9-2 | 5 | 100 |
| I.9-176 | 5 | 100 |
| I.9-276 | 5 | 100 |
| I.8-183 | 5 | 100 |
| I.8-291 | 5 | 100 |
| I.9-291 | 5 | 100 |
| I.19-276 | 5 | 100 |
| I.19-176 | 5 | 100 |
| I.23-176 | 5 | 100 |
| I.23-1 | 5 | 100 |
| I.19-1 | 5 | 100 |
| I.19-23 | 5 | 100 |
| I.23-23 | 5 | 100 |
| I.23-276 | 5 | 100 |

**Tabelle A12c: Nachauflaufwirkung bei 20g/ha gegen VIOTR in %**

| Beispielnummer | Dosierung [g/ha] | VIOTR |
|---|---|---|
| I.1-72 | 20 | 100 |
| I.1-1 | 20 | 100 |
| I.2-72 | 20 | 100 |
| I.2-71 | 20 | 100 |
| I.2-1 | 20 | 100 |
| I.2-23 | 20 | 100 |
| I.2-2 | 20 | 100 |
| I.2-58 | 20 | 100 |
| I.1-26 | 20 | 100 |
| I.1-23 | 20 | 100 |
| I.1-71 | 20 | 100 |
| I.7-1 | 20 | 100 |
| I.7-276 | 20 | 100 |
| I.7-176 | 20 | 100 |
| I.7-2 | 20 | 100 |
| I.9-1 | 20 | 100 |
| I.7-41 | 20 | 100 |
| I.7-278 | 20 | 100 |
| I.7-277 | 20 | 100 |
| I.7-291 | 20 | 100 |
| I.7-71 | 20 | 100 |
| I.7-115 | 20 | 100 |
| I.7-356 | 20 | 100 |
| I.7-394 | 20 | 100 |
| I.7-72 | 20 | 100 |
| I.7-179 | 20 | 100 |
| I.7-177 | 20 | 100 |
| I.7-94 | 20 | 100 |
| I.6-1 | 20 | 100 |
| I.6-176 | 20 | 100 |
| I.6-41 | 20 | 100 |
| I.6-276 | 20 | 100 |
| I.6-23 | 20 | 100 |
| I.6-2 | 20 | 100 |
| I.7-339 | 20 | 100 |
| I.8-23 | 20 | 100 |
| I.8-41 | 20 | 100 |
| I.8-176 | 20 | 100 |
| I.9-23 | 20 | 100 |
| I.9-41 | 20 | 100 |
| I.6-183 | 20 | 100 |
| I.8-2 | 20 | 100 |
| I.8-1 | 20 | 100 |
| I.8-276 | 20 | 100 |
| I.7-183 | 20 | 100 |
| I.9-2 | 20 | 100 |
| I.9-176 | 20 | 100 |
| I.9-276 | 20 | 100 |
| I.8-183 | 20 | 100 |
| I.8-291 | 20 | 100 |
| I.9-291 | 20 | 100 |
| I.19-276 | 20 | 100 |
| I.19-176 | 20 | 100 |
| I.23-176 | 20 | 100 |
| I.23-1 | 20 | 100 |
| I.19-1 | 20 | 100 |
| I.19-23 | 20 | 100 |
| I.23-23 | 20 | 100 |
| I.23-276 | 20 | 100 |

**Tabelle A13a: Nachauflaufwirkung bei 5g/ha gegen HORMU in %**

| Beispielnummer | Dosierung [g/ha] | HORMU |
|---|---|---|
| I.1-23 | 5 | 80 |
| I.1-26 | 5 | 80 |
| I.1-71 | 5 | 80 |

**Tabelle A13b: Nachauflaufwirkung bei 20g/ha gegen HORMU in %**

| Beispielnummer | Dosierung [g/ha] | HORMU |
|---|---|---|
| I.1-1 | 20 | 100 |
| I.1-23 | 20 | 100 |
| I.1-26 | 20 | 90 |
| I.1-71 | 20 | 90 |

**Tabelle A14a: Nachauflaufwirkung bei 1.25g/ha gegen STEME in %**

| Beispielnummer | Dosierung [g/ha] | STEME |
|---|---|---|
| I.1-1 | 1,25 | 80 |
| I.1-23 | 1,25 | 80 |
| I.1-26 | 1,25 | 100 |
| I.1-71 | 1,25 | 90 |
| I.2-1 | 1,25 | 80 |

**Tabelle A14b: Nachauflaufwirkung bei 5g/ha gegen STEME in %**

| Beispielnummer | Dosierung [g/ha] | STEME |
|---|---|---|
| I.1-1 | 5 | 100 |
| I.1-23 | 5 | 100 |
| I.1-26 | 5 | 100 |
| I.1-71 | 5 | 100 |
| I.1-72 | 5 | 100 |
| I.2-1 | 5 | 100 |
| I.2-2 | 5 | 100 |
| I.2-23 | 5 | 100 |
| I.2-58 | 5 | 100 |
| I.2-71 | 5 | 100 |
| I.2-72 | 5 | 100 |

**Tabelle A14c: Nachauflaufwirkung bei 20g/ha gegen STEME in %**

| Beispielnummer | Dosierung [g/ha] | STEME |
|---|---|---|
| I.1-1 | 20 | 100 |
| I.1-23 | 20 | 100 |
| I.1-26 | 20 | 100 |
| I.1-71 | 20 | 100 |
| I.1-72 | 20 | 100 |
| I.2-1 | 20 | 100 |
| I.2-2 | 20 | 100 |
| I.2-23 | 20 | 100 |
| I.2-58 | 20 | 100 |
| I.2-71 | 20 | 100 |
| I.2-72 | 20 | 100 |

In den nachstehenden Tabllen A15 bis A19 sind die Kulturpflanzenverträglichkeiten ausgewählter Verbindungen der allgemeinen Formel (I) gemäß der Tabellen 1.1 bis I.33 bei einer Aufwandmenge entsprechend 5 g/ha oder niedriger, die bei Versuchen gemäß zuvor genannter Versuchvorschrift beobachtet wurden, dargestellt. Es werden dabei die beobachteten Effekte an ausgewählten Kulturpflanzen im Vergleich zu den unbehandelten Kontrollen angegeben (Werte in %). Die Appendices "a", "b" und "c" differenzieren nach verwendeten Dosierungen bei ansonsten gleich geprüften Kulturpflanzen.

**Tabelle A15a: Nachauflaufwirkung bei 1.25g/ha gegen BRSNW in %**

| Beispielnummer | Dosierung [g/ha] | BRSNW |
|---|---|---|
| I.1-23 | 1,25 | 20 |
| I.1-26 | 1,25 | 20 |
| I.1-71 | 1,25 | 20 |
| I.2-2 | 1,25 | 10 |
| I.2-23 | 1,25 | 20 |
| I.2-58 | 1,25 | 20 |
| I.2-71 | 1,25 | 10 |
| I.2-72 | 1,25 | 10 |
| I.7-1 | 1,25 | 20 |
| I.7-23 | 1,25 | 20 |
| I.7-115 | 1,25 | 20 |
| I.7-276 | 1,25 | 20 |

**Tabelle A15b: Nachauflaufwirkung bei 5g/ha gegen BRSNW in %**

| Beispielnummer | Dosierung [g/ha] | BRSNW |
|---|---|---|
| I.2-23 | 5 | 20 |
| I.7-176 | 5 | 20 |
| I.7-276 | 5 | 20 |

**Tabelle A15c: Nachauflaufwirkung bei 20g/ha gegen BRSNW in %**

| Beispielnummer | Dosierung [g/ha] | BRSNW |
|---|---|---|
| I.7-176 | 20 | 20 |
| I.7-276 | 20 | 20 |

**Tabelle A16a: Nachauflaufwirkung bei 1.25g/ha gegen ZEAMX in %**

| Beispielnummer | Dosierung [g/ha] | ZEAMX |
|---|---|---|
| I.1-1 | 1,25 | 20 |
| I.1-23 | 1,25 | 10 |
| I.1-26 | 1,25 | 10 |
| I.1-71 | 1,25 | 20 |
| I.1-72 | 1,25 | 10 |
| I.19-276 | 1,25 | 20 |
| I.2-1 | 1,25 | 10 |
| I.23-1 | 1,25 | 20 |
| I.23-176 | 1,25 | 20 |
| I.23-23 | 1,25 | 20 |
| I.2-58 | 1,25 | 10 |
| I.6-1 | 1,25 | 20 |
| I.6-176 | 1,25 | 10 |
| I.6-183 | 1,25 | 20 |
| I.6-2 | 1,25 | 20 |
| I.6-23 | 1,25 | 10 |
| I.6-276 | 1,25 | 10 |
| I.6-41 | 1,25 | 20 |
| I.7-115 | 1,25 | 20 |
| I.7-179 | 1,25 | 0 |
| I.7-1 | 1,25 | 20 |
| I.7-2 | 1,25 | 0 |
| I.7-277 | 1,25 | 0 |
| I.7-278 | 1,25 | 0 |
| I.7-291 | 1,25 | 10 |
| I.7-339 | 1,25 | 20 |
| I.7-356 | 1,25 | 20 |
| I.7-394 | 1,25 | 10 |
| I.7-72 | 1,25 | 20 |
| I.7-94 | 1,25 | 10 |
| I.8-1 | 1,25 | 20 |
| I.8-2 | 1,25 | 20 |
| I.8-276 | 1,25 | 20 |
| I.8-41 | 1,25 | 20 |
| I.9-1 | 1,25 | 10 |
| I.9-176 | 1,25 | 0 |
| I.9-276 | 1,25 | 0 |
| I.9-41 | 1,25 | 10 |

**Tabelle A16b: Nachauflaufwirkung bei 5g/ha gegen ZEAMX in %**

| Beispielnummer | Dosierung [g/ha] | ZEAMX |
|---|---|---|
| I.1-72 | 5 | 20 |
| I.2-1 | 5 | 10 |
| I.1-26 | 5 | 20 |
| I.6-23 | 5 | 20 |
| I.6-183 | 5 | 20 |
| I.9-276 | 5 | 0 |

**Tabelle A16c: Nachauflaufwirkung bei 20g/ha gegen ZEAMX in %**

| Beispielnummer | Dosierung [g/ha] | ZEAMX |
|---|---|---|
| I.2-1 | 20 | 20 |
| I.9-276 | 20 | 20 |

**Tabelle A17a: Nachauflaufwirkung bei 1.25g/ha gegen TRZAS in %**

| Beispielnummer | Dosierung [g/ha] | TRZAS |
|---|---|---|
| I.1-1 | 1,25 | 20 |
| I.1-23 | 1,25 | 20 |
| I.1-71 | 1,25 | 10 |
| I.1-72 | 1,25 | 20 |
| I.19-1 | 1,25 | 20 |
| I.19-176 | 1,25 | 20 |
| I.19-23 | 1,25 | 10 |
| I.19-276 | 1,25 | 20 |
| I.2-1 | 1,25 | 20 |
| I.2-2 | 1,25 | 20 |
| I.2-23 | 1,25 | 20 |
| I.23-1 | 1,25 | 20 |
| I.23-176 | 1,25 | 10 |
| I.23-23 | 1,25 | 20 |
| I.23-276 | 1,25 | 20 |
| I.2-71 | 1,25 | 20 |
| I.2-72 | 1,25 | 20 |
| I.6-1 | 1,25 | 10 |
| I.6-176 | 1,25 | 20 |
| I.6-183 | 1,25 | 0 |
| I.6-2 | 1,25 | 20 |
| I.6-23 | 1,25 | 10 |
| I.6-276 | 1,25 | 10 |
| I.6-41 | 1,25 | 10 |
| I.7-1 | 1,25 | 20 |
| I.7-115 | 1,25 | 0 |
| I.7-177 | 1,25 | 0 |
| I.7-179 | 1,25 | 0 |
| I.7-183 | 1,25 | 20 |
| I.7-2 | 1,25 | 20 |
| I.7-23 | 1,25 | 10 |
| I.7-276 | 1,25 | 20 |
| I.7-277 | 1,25 | 0 |
| I.7-291 | 1,25 | 0 |
| I.7-339 | 1,25 | 0 |
| I.7-356 | 1,25 | 10 |
| I.7-394 | 1,25 | 0 |
| I.7-41 | 1,25 | 0 |
| I.7-71 | 1,25 | 0 |
| I.7-72 | 1,25 | 10 |
| I.7-94 | 1,25 | 20 |
| I.8-1 | 1,25 | 20 |
| I.8-176 | 1,25 | 0 |
| I.8-183 | 1,25 | 0 |
| I.8-23 | 1,25 | 10 |
| I.8-276 | 1,25 | 20 |
| I.8-291 | 1,25 | 10 |
| I.8-41 | 1,25 | 0 |
| I.9-1 | 1,25 | 0 |
| I.9-176 | 1,25 | 0 |
| I.9-2 | 1,25 | 20 |
| I.9-23 | 1,25 | 0 |
| I.9-276 | 1,25 | 0 |
| I.9-291 | 1,25 | 20 |
| I.9-41 | 1,25 | 0 |

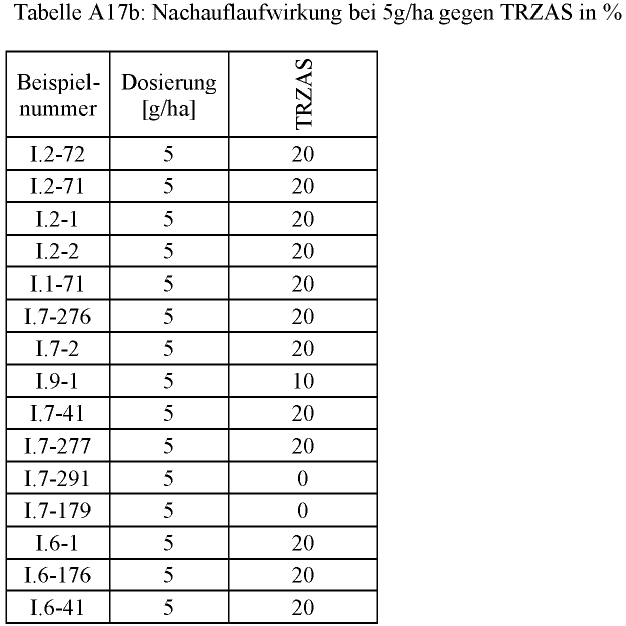

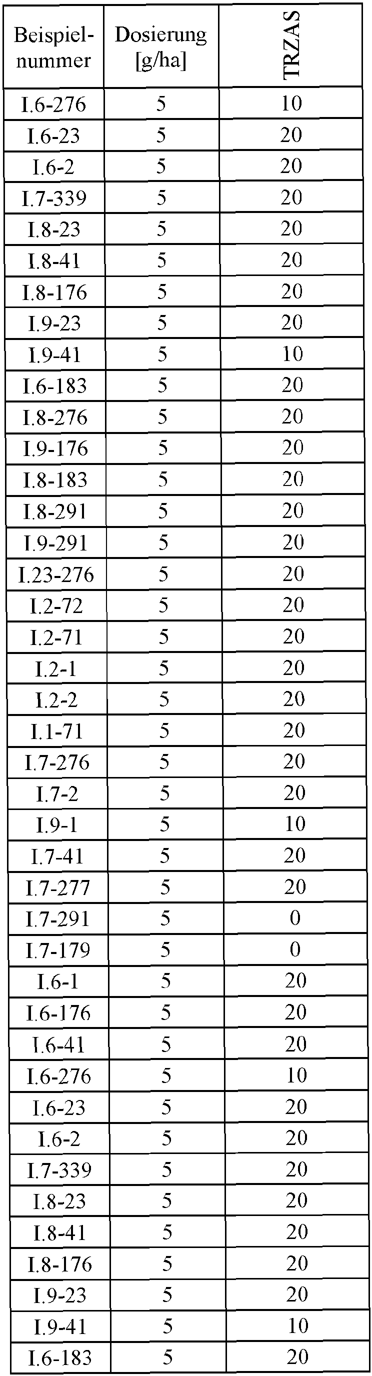

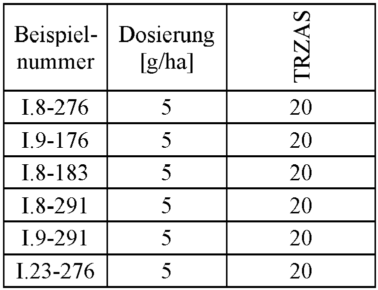

**Tabelle A17c: Nachauflaufwirkung bei 20g/ha gegen TRZAS in %**

| Beispielnummer | Dosierung [g/ha] | TRZAS |
|---|---|---|
| I.9-1 | 20 | 10 |
| I.6-176 | 20 | 20 |
| I.6-41 | 20 | 20 |
| I.6-23 | 20 | 20 |
| I.9-23 | 20 | 20 |
| I.9-41 | 20 | 20 |

**Tabelle A18a: Nachauflaufwirkung bei 1.25g/ha gegen ORYSA in %**

| Beispielnummer | Dosierung [g/ha] | ORYSA |
|---|---|---|
| I.1-72 | 1,25 | 10 |
| I.1-1 | 1,25 | 10 |
| I.2-72 | 1,25 | 0 |
| I.2-71 | 1,25 | 20 |
| I.2-1 | 1,25 | 10 |
| I.2-23 | 1,25 | 10 |
| I.2-2 | 1,25 | 10 |
| I.2-58 | 1,25 | 10 |
| I.1-26 | 1,25 | 10 |
| I.1-23 | 1,25 | 20 |
| I.7-23 | 1,25 | 10 |
| I.7-276 | 1,25 | 0 |
| I.7-2 | 1,25 | 0 |
| I.9-1 | 1,25 | 0 |
| I.7-41 | 1,25 | 0 |
| I.7-278 | 1,25 | 0 |
| I.7-277 | 1,25 | 0 |
| I.7-291 | 1,25 | 20 |
| I.7-71 | 1,25 | 20 |
| I.7-115 | 1,25 | 0 |
| I.7-356 | 1,25 | 0 |
| I.7-394 | 1,25 | 0 |
| I.7-72 | 1,25 | 0 |
| I.7-179 | 1,25 | 0 |
| I.7-177 | 1,25 | 0 |
| I.7-94 | 1,25 | 0 |
| I.6-1 | 1,25 | 0 |
| I.6-176 | 1,25 | 0 |
| I.6-41 | 1,25 | 10 |
| I.6-276 | 1,25 | 0 |
| I.6-23 | 1,25 | 0 |
| I.6-2 | 1,25 | 0 |
| I.7-339 | 1,25 | 0 |
| I.8-23 | 1,25 | 0 |
| I.8-41 | 1,25 | 0 |
| I.8-176 | 1,25 | 0 |
| I.9-23 | 1,25 | 0 |
| I.9-41 | 1,25 | 0 |
| I.6-183 | 1,25 | 0 |
| I.8-2 | 1,25 | 0 |
| I.8-1 | 1,25 | 0 |
| I.8-276 | 1,25 | 20 |
| I.7-183 | 1,25 | 0 |
| I.9-2 | 1,25 | 0 |
| I.9-176 | 1,25 | 0 |
| I.9-276 | 1,25 | 0 |
| I.8-183 | 1,25 | 0 |
| I.8-291 | 1,25 | 0 |
| I.9-291 | 1,25 | 0 |
| I.19-276 | 1,25 | 0 |
| I.19-176 | 1,25 | 0 |
| I.23-176 | 1,25 | 0 |
| I.23-1 | 1,25 | 0 |
| I.19-23 | 1,25 | 10 |
| I.23-276 | 1,25 | 0 |

**Tabelle A18b: Nachauflaufwirkung bei 5g/ha gegen ORYSA in %**

| Beispielnummer | Dosierung [g/ha] | ORYSA |
|---|---|---|
| I.1-72 | 5 | 20 |
| I.1-1 | 5 | 10 |
| I.2-72 | 5 | 20 |
| I.2-71 | 5 | 20 |
| I.2-1 | 5 | 10 |
| I.2-23 | 5 | 10 |
| I.2-2 | 5 | 10 |
| I.2-58 | 5 | 10 |
| I.7-23 | 5 | 20 |
| I.9-1 | 5 | 20 |
| I.7-41 | 5 | 20 |
| I.7-277 | 5 | 0 |
| I.7-356 | 5 | 20 |
| I.7-394 | 5 | 20 |
| I.7-94 | 5 | 20 |
| I.6-1 | 5 | 10 |
| I.6-176 | 5 | 20 |
| I.6-41 | 5 | 10 |
| I.6-276 | 5 | 10 |
| I.6-23 | 5 | 10 |
| I.6-2 | 5 | 0 |
| I.7-339 | 5 | 10 |
| I.8-23 | 5 | 0 |
| I.8-41 | 5 | 20 |
| I.8-176 | 5 | 20 |
| I.9-23 | 5 | 0 |
| I.9-41 | 5 | 0 |
| I.6-183 | 5 | 0 |
| I.8-2 | 5 | 0 |
| I.8-1 | 5 | 20 |
| I.9-176 | 5 | 20 |
| I.9-276 | 5 | 0 |
| I.8-183 | 5 | 0 |
| I.8-291 | 5 | 20 |
| I.9-291 | 5 | 0 |
| I.19-276 | 5 | 0 |
| I.23-176 | 5 | 20 |
| I.23-276 | 5 | 10 |

**Tabelle A18c: Nachauflaufwirkung bei 20g/ha gegen ORYSA in %**

| Beispielnummer | Dosierung [g/ha] | ORYSA |
|---|---|---|
| I.2-2 | 20 | 20 |
| I.2-58 | 20 | 20 |
| I.7-277 | 20 | 20 |
| I.6-1 | 20 | 20 |
| I.6-41 | 20 | 20 |
| I.6-276 | 20 | 20 |
| I.6-23 | 20 | 20 |
| I.6-2 | 20 | 20 |
| I.8-23 | 20 | 20 |
| I.9-23 | 20 | 20 |
| I.6-183 | 20 | 0 |
| I.9-276 | 20 | 0 |
| I.8-183 | 20 | 0 |
| I.9-291 | 20 | 20 |
| I.23-276 | 20 | 10 |

**Tabelle A19a: Nachauflaufwirkung bei 1.25g/ha gegen GLXMA in %**

| Beispielnummer | Dosierung [g/ha] | GLXMA |
|---|---|---|
| I.1-72 | 1,25 | 10 |
| I.1-1 | 1,25 | 0 |
| I.2-72 | 1,25 | 0 |
| I.2-71 | 1,25 | 10 |
| I.2-1 | 1,25 | 0 |
| I.2-23 | 1,25 | 0 |
| I.2-2 | 1,25 | 0 |
| I.2-58 | 1,25 | 10 |
| I.7-1 | 1,25 | 10 |
| I.7-356 | 1,25 | 0 |
| I.7-394 | 1,25 | 20 |
| I.7-177 | 1,25 | 0 |
| I.6-1 | 1,25 | 20 |
| I.6-176 | 1,25 | 20 |
| I.6-276 | 1,25 | 20 |
| I.6-23 | 1,25 | 20 |
| I.6-2 | 1,25 | 20 |
| I.9-276 | 1,25 | 0 |

**Tabelle A19b: Nachauflaufwirkung bei 5g/ha gegen GLXMA in %**

| Beispielnummer | Dosierung [g/ha] | GLXMA |
|---|---|---|
| I.1-72 | 5 | 20 |
| I.1-1 | 5 | 20 |
| I.2-72 | 5 | 10 |
| I.2-1 | 5 | 10 |
| I.2-23 | 5 | 10 |
| I.2-2 | 5 | 0 |
| I.2-58 | 5 | 10 |
| I.7-1 | 5 | 10 |
| I.7-356 | 5 | 0 |
| I.6-176 | 5 | 20 |

**Tabelle A19c: Nachauflaufwirkung bei 20g/ha gegen GLXMA in %**

| Beispielnummer | Dosierung [g/ha] | GLXMA |
|---|---|---|
| I.2-1 | 20 | 20 |
| I.2-23 | 20 | 20 |
| I.2-2 | 20 | 10 |
| I.7-1 | 20 | 10 |

Wie die Ergebnisse zeigen, weisen erfindungsgemäße Verbindungen der allgemeinen Formel (I) bei Behandlung im Nachauflauf eine gute herbizide Wirksamkeit gegen Schadpflanzen auf wie z. B. *Abutilon theophrasti* (ABUTH), *Alopecurus myosuroides* (ALOMY), *Amaranthus retroflexus* (AMARE), *Avena fatua* (AVEFA), *Echinochloa crus-galli* (ECHCG), *Hordeum murinum* (HORMU), *Lolium rigidum* (LOLRI), *Matricaria inodora* (MATIN), *Pharbitis purpurea* (PHBPU), *Polygonum convolvulus* (POLCO), *Setaria viridis* (SETVI), *Stellaria media* (STEME), *Veronica persica* (VERPE) und *Viola tricolor* (VIOTR) bei einer Aufwandmenge von 0,02 kg Aktivsubstanz oder weniger pro Hektar, sowie eine gute Kulturpflanzenverträglichkeit bei Oganismen, *wie Oryza sativa* (ORYSA), *Zea mays* (ZEAMX), *Brassica napus* (BRSNW), *Glycine max* (GLXMA) *und Triticum aestivum* (TRZAS) bei einer Aufwandmenge von 0.02 kg und weniger pro Hektar.

### B. Herbizide Wirkung und Kulturverträglichkeit im Vorauflauf

Samen von mono- bzw. dikotylen Unkraut und Kulturpflanzen wurden in Kunststoff- oder organischen Pflanztöpfen ausgelegt und mit Erde abgedeckt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 l/ha auf die Oberfläche der Abdeckerde appliziert. Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert. Beispielsweise bedeutet 100% Wirkung = Pflanzen sind abgestorben, 0% Wirkung = wie Kontrollpflanzen.

In den nachstehenden Tabllen B1 bis B10 sind die Wirkungen ausgewählter Verbindungen der allgemeinen Formel (I) gemäß der Tabellen 1.1 bis I.33 auf verschiedene Schadpflanzen und einer Aufwandmenge entsprechend 80 g/ha und niedriger, die gemäß zuvor genannter Versuchsvorschrift erhalten wurden, dargestellt. Die Appendices "a", "b" und "c" differenzieren nach verwendeten Dosierungen bei ansonsten gleich geprüften Schadpflanzen.

**Tabelle B1a: Vorauflaufwirkung bei 20g/ha gegen ABUTH in %**

| Beispielnummer | Dosierung [g/ha] | ABUTH |
|---|---|---|
| I.7-1 | 20 | 100 |
| I.7-23 | 20 | 100 |

**Tabelle B1b: Vorauflaufwirkung bei 80g/ha gegen ABUTH in %**

| Beispielnummer | Dosierung [g/ha] | ABUTH |
|---|---|---|
| I.7-1 | 80 | 100 |
| I.7-23 | 80 | 100 |

**Tabelle B2a: Vorauflaufwirkung bei 20g/ha gegen ALOMY in %**

| Beispielnummer | Dosierung [g/ha] | ALOMY |
|---|---|---|
| I.7-1 | 20 | 80 |
| I.7-23 | 20 | 90 |

**Tabelle B2b: Vorauflaufwirkung bei 80g/ha gegen ALOMY in %**

| Beispielnummer | Dosierung [g/ha] | ALOMY |
|---|---|---|
| I.7-1 | 80 | 100 |
| I.7-23 | 80 | 100 |

**Tabelle B3a: Vorauflaufwirkung bei 20g/ha gegen AMARE in %**

| Beispielnummer | Dosierung [g/ha] | AMARE |
|---|---|---|
| I.7-1 | 20 | 100 |
| I.7-23 | 20 | 100 |

**Tabelle B3b: Vorauflaufwirkung bei 80g/ha gegen AMARE in %**

| Beispielnummer | Dosierung [g/ha] | AMARE |
|---|---|---|
| I.7-1 | 80 | 100 |
| I.7-23 | 80 | 100 |

**Tabelle B4: Vorauflaufwirkung bei 80g/ha gegen AVEFA in %**

| Beispielnummer | Dosierung [g/ha] | AVEFA |
|---|---|---|
| I.7-1 | 80 | 100 |
| I.7-23 | 80 | 90 |

**Tabelle B5a: Vorauflaufwirkung bei 20g/ha gegen DIGSA in %**

| Beispielnummer | Dosierung [g/ha] | DIGSA |
|---|---|---|
| I.7-1 | 20 | 100 |
| I.7-23 | 20 | 100 |

**Tabelle B5b: Vorauflaufwirkung bei 80g/ha gegen DIGSA in %**

| Beispielnummer | Dosierung [g/ha] | DIGSA |
|---|---|---|
| I.7-1 | 80 | 100 |
| I.7-23 | 80 | 100 |

**Tabelle B6a: Vorauflaufwirkung bei 20g/ha gegen ECHCG in %**

| Beispielnummer | Dosierung [g/ha] | ECHCG |
|---|---|---|
| I.7-23 | 20 | 80 |

**Tabelle B6b: Vorauflaufwirkung bei 80g/ha gegen ECHCG in %**

| Beispielnummer | Dosierung [g/ha] | ECHCG |
|---|---|---|
| I.7-1 | 80 | 90 |
| I.7-23 | 80 | 90 |

**Tabelle B7a: Vorauflaufwirkung bei 20g/ha gegen LOLRI in %**

| Beispielnummer | Dosierung [g/ha] | LOLRI |
|---|---|---|
| I.7-23 | 20 | 90 |

**Tabelle B7b: Vorauflaufwirkung bei 80g/ha gegen LOLRI in %**

| Beispielnummer | Dosierung [g/ha] | LOLRI |
|---|---|---|
| I.7-1 | 80 | 100 |
| I.7-23 | 80 | 100 |

**Tabelle B8a: Vorauflaufwirkung bei 20g/ha gegen MATIN in %**

| Beispielnummer | Dosierung [g/ha] | MATIN |
|---|---|---|
| I.7-1 | 20 | 100 |
| I.7-23 | 20 | 100 |

**Tabelle B8b: Vorauflaufwirkung bei 80g/ha gegen MATIN in %**

| Beispielnummer | Dosierung [g/ha] | MATIN |
|---|---|---|
| I.7-1 | 80 | 100 |
| I.7-23 | 80 | 100 |

**Tabelle B9a: Vorauflaufwirkung bei 20g/ha gegen PHBPU in %**

| Beispielnummer | Dosierung [g/ha] | PHBPU |
|---|---|---|
| I.7-1 | 20 | 100 |
| I.7-23 | 20 | 100 |

**Tabelle B9b: Vorauflaufwirkung bei 80g/ha gegen PHBPU in %**

| Beispielnummer | Dosierung [g/ha] | PHBPU |
|---|---|---|
| I.7-1 | 80 | 100 |
| I.7-23 | 80 | 100 |

**Tabelle B10a: Vorauflaufwirkung bei 20g/ha gegen VERPE in %**

| Beispielnummer | Dosierung [g/ha] | VERPE |
|---|---|---|
| I.7-1 | 20 | 100 |
| I.7-23 | 20 | 100 |

**Tabelle B10b: Vorauflaufwirkung bei 80g/ha gegen VERPE in %**

| Beispielnummer | Dosierung [g/ha] | VERPE |
|---|---|---|
| I.7-1 | 80 | 100 |
| I.7-23 | 80 | 100 |

In den nachstehenden Tabellen B11 und B12 sind die Kulturpflanzenverträglichkeiten ausgewählter Verbindungen der allgemeinen Formel (I) gemäß der Tabellen 1.1 bis I.33 bei einer Aufwandmenge entsprechend 80 g/ha oder niedriger, die bei Versuchen gemäß zuvor genannter Versuchvorschrift beobachtet wurden, dargestellt. Es werden dabei die beobachteten Effekte an ausgewählten Kulturpflanzen im Vergleich zu den unbehandelten Kontrollen angegeben (Werte in %).

**Tabelle B11: Vorauflaufwirkung bei 20g/ha gegen ZEAMX in %**

| Beispielnummer | Dosierung [g/ha] | ZEAMX |
|---|---|---|
| I.7-1 | 20 | 0 |
| I.7-23 | 20 | 20 |

**Tabelle B12: Vorauflaufwirkung bei 20g/ha gegen GLXMA in %**

| Beispielnummer | Dosierung [g/ha] | GLXMA |
|---|---|---|
| I.7-23 | 20 | 10 |

Wie die Ergebnisse zeigen, weisen erfindungsgemäße Verbindungen der allgemeinen Formel (I) bei Behandlung im Vorauflauf eine gute herbizide Wirksamkeit gegen Schadpflanzen auf, z. B. gegen Schadpflanzen wie *Abutilon theophrasti* (ABUTH), *Alopecurus myosuroides* (ALOMY), *Amaranthus retroflexus* (AMARE), *Avena fatua* (AVEFA), *Digitaria sanguinalis* (DIGSA), *Echinochloa crus-galli* (ECHCG), *Lolium rigidum* (LOLRI), *Matricaria inodora* (MATIN), *Pharbitis purpurea* (PHBPU) und *Veronica persica* (VERPE) bei einer Aufwandmenge von 0.08 kg Aktivsubstanz oder weniger pro Hektar, sowie eine gute Kulturpflanzenverträglichkeit bei Oganismen, wie *Zea mays* (ZEAMX) *Glycine max* (GLXMA) bei einer Aufwandmenge von 0.02 kg, pro Hektar.

C. Vergleichende herbizide Wirkung und Kulturpflanzenverträglichkeit zweier erfindungsgemäßer Verbindungen (I.7-1 und I.7-115) mit einer literaturbekannten, strukturnahen Verbindung (WO2002/098227, a-17 und EP1106607, 3-14) im Nachauflauf.

In den nachstehenden Tabellen C1-C4 sind die Wirkungen zweier erfindungsgemäßer Verbindungen (I.7-1 und I.7-115) mit einer literaturbekannten strukturnahen Verbindung (a-17 in WO2002/098227, und 3-14 in EP 1106607) auf verschiedene Schadpflanzen bei einer Aufwandmenge entsprechend 5 g/ha und niedriger, die gemäß zuvor genannter Versuchsvorschrift erhalten wurden, dargestellt.

Hierbei unterscheiden sich die zwei erfindungsgemäßen Verbindungen (I.7-1 und I.7-115) durch Varianz eines signifikanten Strukturmerkmals von der literaturbekannten Verbindung bezüglich der Estereinheit bei gleichbleibender "Kettenlänge" der Esterfunktionalität durch die Inkorporation eines Heteroatoms bzw. durch die Inkorporation eines Heteroatoms bei gleichzeitiger Ringbildung.

**Tabelle C1**

| Verbindung | *ALOMY* (Wirkung in %) | Aufwandmenge [g/ha] |
|---|---|---|
| I.7-1 (erfindungsgemäß) | 80 | 5 |
| 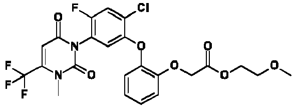 | | |
| I.7-115 (erfindungsgemäß) | 100 | 5 |
| 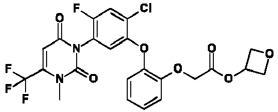 | | |
| a-17 (WO2002/098227), 3-14 (EP1106607) | 30 | 5 |
| 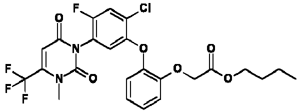 | | |

**Tabelle C2**

| Verbindung | *ECHCG* (Wirkung in %) | Aufwandmenge [g/ha] |
|---|---|---|
| I.7-1 (erfindungsgemäß) | 90 | 1.25 |
| I.7-115 (erfindungsgemäß) | 100 | 1.25 |
| a-17 (WO2002/098227), 3-14 (EP1106607) | 60 | 1.25 |

**Tabelle C3**

| Verbindung | *MATIN* (Wirkung in %) | Aufwandmenge [g/ha] |
|---|---|---|
| I.7-1 (erfindungsgemäß) | 100 | 1.25 |
| I.7-115 (erfindungsgemäß) | 100 | 1.25 |
| a-17 (WO2002/098227), 3-14 (EP1106607) | 30 | 1.25 |

**Tabelle C4**

| Verbindung | *SETVI* (Wirkung in %) | Aufwandmenge [g/ha] |
|---|---|---|
| I.7-1 (erfindungsgemäß) | 100 | 1.25 |
| I.7-115 (erfindungsgemäß) | 100 | 1.25 |
| a-17 (WO2002/098227), 3-14 (EP1106607) | 80 | 1.25 |

Wie die in den Tabellen C1 bis C4 dargestellten Ergebnisse zeigen, weisen die erfindungsgemäßen Verbindungen 1. 7-1 und 1. 7-115 im Vergleich zur literaturbekannten Verbindung a-17 (WO2002/098227) bzw. 3-14 (EP1106607) eine deutlich verbesserte herbizide Wirksamkeit gegen Schadpflanzen, wie *Alopecurus myosuroides* (ALOMY), *Echinochloa crus-galli* (ECHCG), *Matricaria inodora* (MATIN) und *Setaria viridis (SETVI)* bei einer Aufwandmenge von 5 g und weniger pro Hektar auf

In der nachstehenden Tabelle C5 sind die Wirkungen zweier erfindungsgemäßer Verbindungen (I.7-1 und I.7-115) mit einer literaturbekannten strukturnahen Verbindung (a-17 in WO2002/098227 und 3-14 in EP1106607) auf die Kulturpflanze *Oryza sativa* (ORYSA) bei einer Aufwandmenge entsprechend 1.25 g/ha, die gemäß zuvor genannter Versuchsvorschrift erhalten wurden, dargestellt.

**Tabelle C5**

| Verbindung | *ORYSA* (Wirkung in %) | Aufwandmenge [g/ha] |
|---|---|---|
| I.7-1 (erfindungsgemäß) | 0 | 1.25 |
| I.7-115 (erfindungsgemäß) | 0 | 1.25 |
| a-17 (WO2002/098227), 3-14 (EP1106607) | 20 | 1.25 |

Wie die in den Tabelle C5 dargestellten Ergebnisse zeigen, weisen die erfindungsgemäßen Verbindung I.7-1 und L7-115 im Vergleich zur literaturbekannten Verbindung a-17 (WO2002/098227) bzw. 3-14 (EP1106607) eine deutlich verbesserte Verträglichkeit hinsichtlich der Kulturpflanze *Oryza sativa* (ORYSA) bei einer Aufwandmenge von 1.25 g pro Hektar auf.

## Patentansprüche

1. Substituierte N-Phenyluracile der allgemeinen Formel (I) oder deren Salze 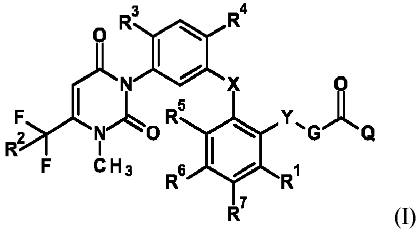 worin
R¹ für Wasserstoff, Halogen, Cyano, (C₁-C₈)-Alkyl, (C₁-C₈)-Haloalkyl, (C₁-C₈)-Alkoxy, (C₁-C₈)-Haloalkoxy steht,
R² für Wasserstoff, Fluor, Chlor, Brom, Trifluormethyl, (C₁-C₈)-Alkoxy steht,
R³ für Wasserstoff, Halogen, (C₁-C₈)-Alkoxy steht,
R⁴ für Halogen, Cyano, NO₂, C(O)NH₂, C(S)NH₂, (C₁-C₈)-Haloalkyl, (C₂-C₈)-Alkinyl steht,
R⁵, R⁶ und R⁷ unabhängig voneinander für Wasserstoff, Halogen, Cyano, (Ci-Cs)-Alkyl, (C₁-C₈)-Haloalkyl, (C₁-C₈)-Alkoxy, (C₁-C₈)-Haloalkoxy stehen,
G für unverzweigtes oder verzweigtes (C₁-C₈)-Alkylen steht,
Q für einen Rest der Formel 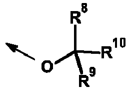 steht,
R⁸ für Wasserstoff, (C₁-C₈)-Alkyl, (C₁-C₈)-Haloalkyl, Aryl, Aryl-(C₁-C₈)-alkyl, Heteroaryl, (C₂-C₈)-Alkinyl, (C₂-C₈)-Alkenyl, C(O)R¹³, C(O)OR¹³, (C₁-C₈)-Alkoxy-(C₁-C₈)-alkyl steht,
R⁹ für Wasserstoff oder (C₁-C₈)-Alkyl steht,
R¹⁰ für Cyano, NOz, Heteroaryl, Heteroaryl-(C₁-C₈)-alkyl, Heterocyclyl, Heterocyclyl-(C₁-C₈)-alkyl, R¹¹R¹²N-(C₁-C₈)-alkyl, R¹³O-(C₁-C₈)-alkyl, Cyano-(C₁-C₈)-alkyl, (C₁-C₈)-Alkylcarbonyloxy-(C₁-C₈)-alkyl, (C₃-C₈)-Cycloalkylcarbonyloxy-(C₁-C₈)-alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, OR¹³, NR¹¹R¹², SR¹⁴, S(O)R¹⁴, SO₂R¹⁴, R¹⁴S-(C₁-C₈)-alkyl, R¹⁴(O)S-(C₁-C₈)-alkyl, R¹⁴O₂S-(C₁-C₈)-alkyl, Tris-[(C₁-C₈)-Alkyl]silyl-(C₁-C₈)-alkyl, Bis-[(C₁-C₈)-Alkyl](aryl)silyl(C₁-C₈)-alkyl, [(C₁-C₈)-Alkyl]-bis-(aryl)silyl-(C₁-C₈)-alkyl, Tris-[(C₁-C₈)-Alkyl]silyl, Bis-hydroxyboryl-(C₁-C₈)-alkyl, Bis-[(C₁-C₈)-alkoxy]boryl-(C₁-C₈)-alkyl, Tetramethyl-1,3,2-Dioxaborolan-2-yl, Tetramethyl-1,3,2-Dioxaborolan-2-yl-(C₁-C₈)-alkyl, Nitro-(C₁-C₈)-alkyl, C(O)R¹⁴, Bis-(C₁-C₈)-alkoxymethyl, Bis-(C₁-C₈)-alkoxymethyl-(C₁-C₈)-alkyl steht, oder
R⁸ und R¹⁰ mit dem Kohlenstoffatom, an das sie gebunden sind, ein vollständig gesättigtes oder teilgesättigtes, und gegebenenfalls weiter substituiertes 3 bis 10-gliedriges monocyclisches oder bicyclisches Heterocyclyl bilden,
R¹¹ und R¹² gleich oder verschieden sind und unabhängig voneinander für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, COR¹³, SO₂R¹⁴, Heterocyclyl, (C₁-C₈)-Alkoxycarbonyl, Bis-[(C₁-C₈)-alkyl]aminocarbonyl-(C₁-C₈)-alkyl, (C₁-C₈)-Alkyl-aminocarbonyl-(C₁-C₈)-alkyl, Aryl-(C₁-C₈)-alkyl-aminocarbonyl-(C₁-C₈)-alkyl, Aryl-(C₁-C₈)-alkoxycarbonyl, Heteroaryl-(C₁-C₈)-alkoxycarbonyl, (C₂-C₈)-Alkenyloxycarbonyl, (C₂-C₈)-Alkinyloxycarbonyl, Heterocyclyl-(C₁-C₈)-alkyl stehen, oder
R¹¹ und R¹² mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
R¹³ für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkoxy-(C₁-C₈)-haloalkyl, (C₁-C₈)-Alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkoxy-(C₁-C₈)-alkyl, Aryl, Aryl-(C₁-C₈)-alkyl, Aryl-(C₁-C₈)-alkoxy-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, Bis-[(C₁-C₈)-alkyl]aminocarbonyl-(C₁-C₈)-alkyl, (C₁-C₈)-Alkyl-aminocarbonyl-(C₁-C₈)-alkyl, Aryl-(C₁-C₈)-alkyl-aminocarbonyl-(C₁-C₈)-alkyl, Bis-[(C₁-C₈)-alkyl]amino-(C₂-C₆)-alkyl, (C₁-C₈)-Alkyl-amino-(C₂-C₆)-alkyl, Aryl-(C₁-C₈)-alkyl-amino-(C₂-C₆)-alkyl, R¹⁴S-(C₁-C₈)-alkyl, R¹⁴(O)S-(C₁-C₈)-alkyl, R¹⁴O₂S-(C₁-C₈)-alkyl, Hydroxycarbonyl-(C₁-C₈)-alkyl, Heterocyclyl, Heterocyclyl-(C₁-C₈)-alkyl, Tris-[(C₁-C₈)-Alkyl]silyl-(C₁-C₈)-alkyl, Bis-[(C₁-C₈)-Alkyl](aryl)silyl(C₁-C₈)-alkyl, [(C₁-C₈)-Alkyl]-bis-(aryl)silyl-(C₁-C₈)-alkyl, (C₁-C₈)-Alkylcarbonyloxy-(C₁-C₈)-alkyl, (C₃-C₈)-Cycloalkylcarbonyloxy-(C₁-C₈)-alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, Aryloxy-(C₁-C₈)-alkyl, Heteroaryloxy-(C₁-C₈)-alkyl, (C₁-C₈)-Alkoxycarbonyl steht,
R¹⁴ für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, Heterocyclyl-(C₁-C₈)-alkyl, (C₃-C₈)-Cycloalkyl-(C₁-C₈)-alkyl, (C₄-C₁₀)-Cycloalkenyl-(C₁-C₈)-alkyl, Bis-[(C₁-C₈)-alkyl]amino, (C₁-C₈)-Alkyl-amino, Aryl-(C₁-C₈)-amino, Aryl-(C₁-C₆)-alkyl-amino, Aryl-[(C₁-C₈)-alkyl]amino; (C₃-C₈)-Cycloalkyl-amino, (C₃-C₈)-Cycloalkyl-[(C₁-C₈)-alkyl]amino; N-Azetidinyl, N-Pyrrolidinyl, N-Piperidinyl. N-Morpholinyl, steht
und X und Y unabhängig voneinander für O (Sauerstoff) oder S (Schwefel) stehen.

2. Verbindungen der allgemeinen Formel (I) gemäß Anspruch 1 und/oder deren Salz, **dadurch gekennzeichnet, dass**
R¹ für Wasserstoff, Halogen, Cyano, (C₁-C₆)-Alkyl, (C₁-C₆)-Haloalkyl, (C₁-C₆)-Alkoxy, (C₁-C₆)-Haloalkoxy steht,
R² für Wasserstoff, Fluor, Chlor, Brom, Trifluormethyl, (C₁-C₆)-Alkoxy steht,
R³ für Wasserstoff, Halogen, (C₁-C₆)-Alkoxy steht,
R⁴ für Halogen, Cyano, NOz, C(O)NH₂, C(S)NH₂, (C₁-C₆)-Haloalkyl, (C₂-C₆)-Alkinyl steht,
R⁵, R⁶ und R⁷ unabhängig voneinander für Wasserstoff, Halogen, Cyano, (C₁-C₆)-Alkyl, (C₁-C₆)-Haloalkyl, (C₁-C₆)-Alkoxy, (C₁-C₆)-Haloalkoxy stehen,
G für unverzweigtes oder verzweigtes (C₁-C₆)-Alkylen steht,
Q für einen Rest der Formel 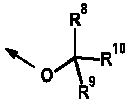 steht,
R⁸ für Wasserstoff, (C₁-C₆)-Alkyl, (C₁-C₆)-Haloalkyl, Aryl, Aryl-(C₁-C₆)-alkyl, Heteroaryl, (C₂-C₆)-Alkinyl, (C₂-C₆)-Alkenyl, C(O)R¹³, C(O)OR¹³, (C₁-C₆)-Alkoxy-(C₁-C₆)-alkyl steht,
R⁹ für Wasserstoff oder (C₁-C₄)-Alkyl steht,
R¹⁰ für Cyano, NOz, Heteroaryl, Heteroaryl-(C₁-C₆)-alkyl, Heterocyclyl, Heterocyclyl-(C₁-C₆)-alkyl, R¹¹R¹²N-(C₁-C₆)-alkyl, R¹³O-(C₁-C₆)-alkyl, Cyano-(C₁-C₆)-alkyl, (C₁-C₆)-Alkylcarbonyloxy-(C₁-C₆)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₆)-alkyl, Heterocyclylcarbonyloxy-(C₁-C₆)-alkyl, OR¹³, NR¹¹R¹², SR¹⁴, S(O)R¹⁴, SO₂R¹⁴, R¹⁴S-(C₁-C₆)-alkyl, R¹⁴(O)S-(C₁-C₆)-alkyl, R¹⁴O₂S-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, Tris-[(C₁-C₆)-Alkyl]silyl, Bis-hydroxyboryl-(C₁-C₆)-alkyl, Bis-[(C₁-C₆)-alkoxy]boryl-(C₁-C₆)-alkyl, Tetramethyl-1,3,2-Dioxaborolan-2-yl, Tetramethyl-1,3,2-Dioxaborolan-2-yl-(C₁-C₆)-alkyl, Nitro-(C₁-C₆)-alkyl, , C(O)R¹³, , Bis-(C₁-C₆)-alkoxymethyl, Bis-(C₁-C₆)-alkoxymethyl-(C₁-C₆)-alkyl steht,
R⁸ und R¹⁰ mit dem Kohlenstoffatom, an das sie gebunden sind, ein vollständig gesättigtes oder teilgesättigtes, und gegebenenfalls weiter substituiertes 3 bis 10-gliedriges monocyclisches oder bicyclisches Heterocyclyl bilden,
R¹¹ und R¹² gleich oder verschieden sind und unabhängig voneinander für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, COR¹³, SO₂R¹⁴, Heterocyclyl, (C₁-C₆)-Alkoxycarbonyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Aryl-(C₁-C₆)-alkoxycarbonyl, Heteroaryl-(C₁-C₆)-alkoxycarbonyl, (C₂-C₆)-Alkenyloxycarbonyl, (C₂-C₆)-Alkinyloxycarbonyl, Heterocyclyl-(C₁-C₆)-alkyl stehen, oder
R¹¹ und R¹² mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
R¹³ für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkoxy-(C₁-C₆)-haloalkyl, (C₁-C₆)-Alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkoxy-(C₁-C₆)-alkyl, Aryl, Aryl-(C₁-C₆)-alkyl, Aryl-(C₁-C₆)-alkoxy-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Bis-[(C₁-C₆)-alkyl]amino-(C₂-C₄)-alkyl, (C₁-C₆)-Alkyl-amino-(C₂-C₄)-alkyl, Aryl-(C₁-C₆)-alkyl-amino-(C₂-C₄)-alkyl, R¹⁴S-(C₁-C₆)-alkyl, R¹⁴(O)S-(C₁-C₆)-alkyl, R¹⁴O₂S-(C₁-C₆)-alkyl, Hydroxycarbonyl-(C₁-C₆)-alkyl, Heterocyclyl, Heterocyclyl-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, (C₁-C₆)-Alkylcarbonyloxy-(C₁-C₆)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₆)-alkyl, Heterocyclylcarbonyloxy-(C₁-C₆)-alkyl, Aryloxy-(C₁-C₆)-alkyl, Heteroaryloxy-(C₁-C₆)-alkyl, (C₁-C₆)-Alkoxycarbonyl steht,
R¹⁴ für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, Heterocyclyl-(C₁-C₆)-alkyl, (C₃-C₆)-Cycloalkyl-(C₁-C₆)-alkyl, (C₄-C₁₀)-Cycloalkenyl-(C₁-C₆)-alkyl, Bis-[(C₁-C₆)-alkyl]amino, (C₁-C₆)-Alkyl-amino, Aryl-(C₁-C₆)-amino, Aryl-(C₁-C₂)-alkyl-amino, Aryl-[(C₁-C₆)-alkyl]amino; (C₃-C₆)-Cycloalkyl-amino, (C₃-C₆)-Cycloalkyl-[(C₁-C₆)-alkyl]amino; N-Azetidinyl, N-Pyrrolidinyl, N-Piperidinyl. N-Morpholinyl, steht
und X und Y unabhängig voneinander für O (Sauerstoff) oder S (Schwefel) stehen.

3. Verbindungen der allgemeinen Formel (I) gemäß Anspruch 1 und/oder deren Salz, **dadurch gekennzeichnet, dass**
R¹ für Wasserstoff, Fluor, Chlor, Brom, Iod, Cyano, Methyl, Ethyl, Prop-1-yl, 1-Methylethyl, But-1-yl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Trifluormethyl, Difluormethyl, Pentafluorethyl, 2,2-Difluorethyl, 2,2,2-Trifluorethyl, Methoxy, Ethoxy, Prop-1-yloxy, Prop-2-yloxy, But-1-yloxy, But-2-yloxy, 2-Methylprop-1-yloxy, 1,1-Dimethyleth-1-yloxy, Difluormethoxy, Trifluormethoxy, Pentafluorethoxy, 2,2-Difluorethoxy, 2,2,2-Trifluorethoxy steht,
R² für Wasserstoff, Fluor, Chlor, Brom, Trifluormethyl, Methoxy, Ethoxy, Prop-1-yloxy, But-1-yloxy steht,
R³ für Wasserstoff, Fluor, Chlor, Brom, Methoxy, Ethoxy, Prop-1-yloxy, Prop-2-yloxy, But-1-yloxy, But-2-yloxy, 2-Methylprop-1-yloxy, 1,1-Dimethyleth-1-yloxy steht,
R⁴ für Fluor, Chlor, Brom, Cyano, NOz, C(O)NH₂, C(S)NH₂, Trifluormethyl, Difluormethyl, Pentafluorethyl, Ethinyl, Propin-1-yl, 1-Butin-1-yl, Pentin-1-yl, Hexin-1-yl steht,
R⁵, R⁶ und R⁷ unabhängig voneinander für Wasserstoff, Fluor, Chlor, Brom, Iod, Cyano, Methyl, Ethyl, Prop-1-yl, 1-Methylethyl, But-1-yl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Trifluormethyl, Difluormethyl, Pentafluorethyl, 2,2-Difluorethyl, 2,2,2-Trifluorethyl, Methoxy, Ethoxy, Prop-1-yloxy, Prop-2-yloxy, But-1-yloxy, But-2-yloxy, 2-Methylprop-1-yloxy, 1,1-Dimethyleth-1-yloxy, Difluormethoxy, Trifluormethoxy, Pentafluorethoxy, 2,2-Difluorethoxy, 2,2,2-Trifluorethoxy stehen,
G für Methylen, (Methyl)methylen, (Ethyl)methylen, (Prop-1-yl)methylen, (Prop-2-yl)methylen, (But-1-yl)methylen, (But-2-yl)methylen, (Pent-1-yl)methylen, (Pent-2-yl)methylen, (Pent-3-yl)methylen, (Dimethyl)methylen, (Diethyl)methylen, Ethylen, n-Propylen, (1-Methyl)ethyl-1-en, (2-Methyl)ethyl-1-en, n-Butylen, 1-Methylpropyl-1-en, 2-Methylpropyl-1-en, 3-Methylpropyl-1-en, 1,1-Dimethylethyl-1-en, 2,2-Dimethylethyl-1-en, 1-Ethylethyl-1-en, 2-Ethylethyl-1-en, 1-(Prop-1-yl)ethyl-1-en, 2-(Prop-1-yl)ethyl-1-en, 1-(Prop-2-yl)ethyl-1-en, 2-(Prop-2-yl)ethyl-1-en, 1,1,2-Trimethylethyl-1-en, 1,2,2-Trimethylethyl-1-en, 1,1,2,2-Tetramethylethyl-1-en, n-Pentylen, 1-Methylbutyl-1-en, 2-Methylbutyl-1-en, 3-Methylbutyl-1-en, 4-Methylbutyl-1-en, 1,1-Dimethylpropyl-1-en, 2,2-Dimethylpropyl-1-en, 3,3-Dimethylpropyl-1-en, 1,2-Dimethylpropyl-1-en, 1,3-Dimethylpropyl-1-en, 1-Ethylpropyl-1-en, n-Hexylen, 1-Methylpentyl-1-en, 2-Methylpentyl-1-en, 3-Methylpentyl-1-en, 4-Methylpentyl-1-en, 1,1-Dimethylbutyl-1-en, 1,2-Dimethylbutyl-1-en, 1,3-Di-methylbutyl-1-en, 2,2-Dimethylbutyl-1-en, 2,3-Dimethylbutyl-1-en, 3,3-Dimethylbutyl-1-en, 1-Ethylbutyl-1-en, 2-Ethylbutyl-1-en, 1,1,2-Trimethylpropyl-1-en, 1,2,2-Trimethylpropyl-1-en, 1-Ethyl-1-methylpropyl-1-en, 1-Ethyl-2-methylpropyl-1-en steht,
X und Y unabhängig voneinander für O (Sauerstoff) oder S (Schwefel) stehen
und Q für eine der nachfolgend spezifisch genannten Gruppierungen Q-1 bis Q-406 steht, wobei in den Strukturformeln der nachfolgenden Tabelle der Pfeil für eine Bindung der jeweiligen Gruppe Q zur Carbonylgruppe in der allgemeinen Formel (I) steht:
| | | | | |
|---|---|---|---|---|
| 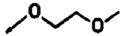 | 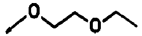 | 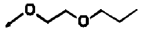 | 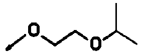 | 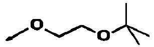 |
| Q-1 | Q-2 | Q-3 | Q-4 | Q-5 |
| | | | | |
|---|---|---|---|---|
| 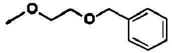 | 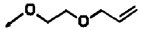 | 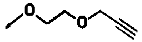 | 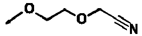 | 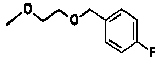 |
| Q-6 | Q-7 | Q-8 | Q-9 | Q-10 |
| | | | | |
|---|---|---|---|---|
| 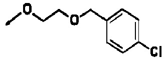 | 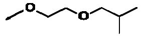 | 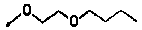 | 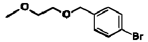 | 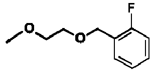 |
| Q-11 | Q-12 | Q-13 | Q-14 | Q-15 |
| | | | | |
|---|---|---|---|---|
| 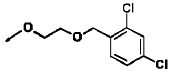 | 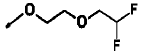 | 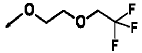 | 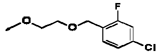 | 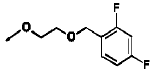 |
| Q-16 | Q-17 | Q-18 | Q-19 | Q-20 |
| | | | | |
|---|---|---|---|---|
| 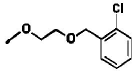 | 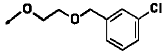 | 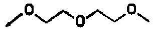 | 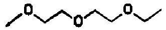 | 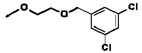 |
| Q-21 | Q-22 | Q-23 | Q-24 | Q-25 |
| | | | | |
|---|---|---|---|---|
| 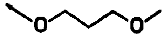 | 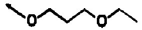 | 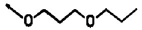 | 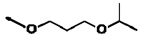 | 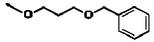 |
| Q-26 | Q-27 | Q-28 | Q-29 | Q-30 |
| | | | | |
|---|---|---|---|---|
| 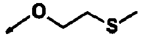 | 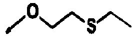 | 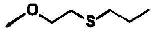 | 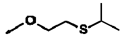 | 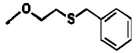 |
| Q-31 | Q-32 | Q-33 | Q-34 | Q-35 |
| | | | | |
|---|---|---|---|---|
| 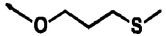 | 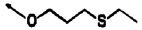 | 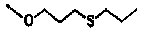 | 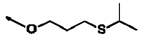 | 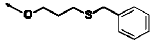 |
| Q-36 | Q-37 | Q-38 | Q-39 | Q-40 |
| | | | | |
|---|---|---|---|---|
| 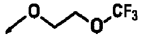 | 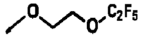 | 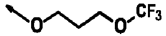 | 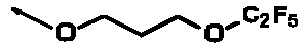 | 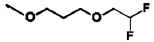 |
| Q-41 | Q-42 | Q-43 | Q-44 | Q-45 |
| | | | | |
|---|---|---|---|---|
| 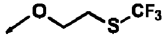 | 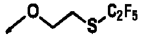 | 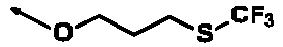 | 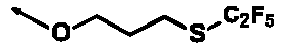 | 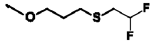 |
| Q-46 | Q-47 | Q-48 | Q-49 | Q-50 |
| | | | | |
|---|---|---|---|---|
| 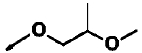 | 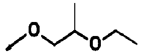 | 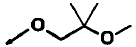 | 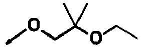 | 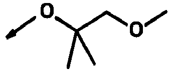 |
| Q-51 | Q-52 | Q-53 | Q-54 | Q-55 |
| | | | | |
|---|---|---|---|---|
| 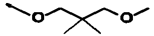 | 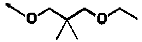 | 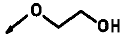 | 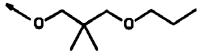 | 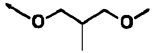 |
| Q-56 | Q-57 | Q-58 | Q-59 | Q-60 |
| | | | | |
|---|---|---|---|---|
| 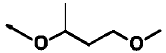 | 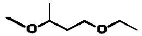 | 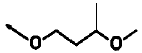 | 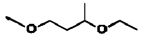 | 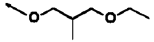 |
| Q-61 | Q-62 | Q-63 | Q-64 | Q-65 |
| | | | | |
|---|---|---|---|---|
| 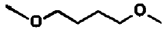 | 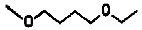 | 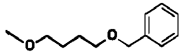 | 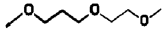 | 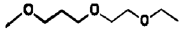 |
| Q-66 | Q-67 | Q-68 | Q-69 | Q-70 |
| | | | | |
|---|---|---|---|---|
| 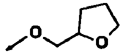 | 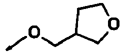 | 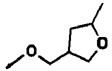 | 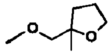 | 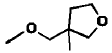 |
| Q-71 | Q-72 | Q-73 | Q-74 | Q-75 |
| | | | | |
|---|---|---|---|---|
| 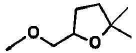 | 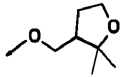 | 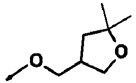 | 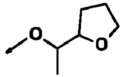 | 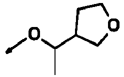 |
| Q-76 | Q-77 | Q-78 | Q-79 | Q-80 |
| | | | | |
|---|---|---|---|---|
| 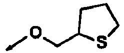 | 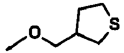 | 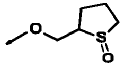 | 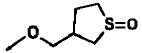 | 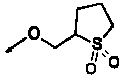 |
| Q-81 | Q-82 | Q-83 | Q-84 | Q-85 |
| | | | | |
|---|---|---|---|---|
| 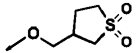 | 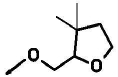 | 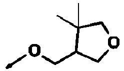 | 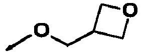 | 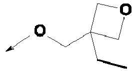 |
| Q-86 | Q-87 | Q-88 | Q-89 | Q-90 |
| | | | | |
|---|---|---|---|---|
| 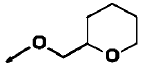 | 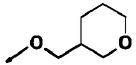 | 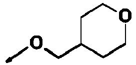 | 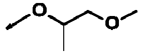 | 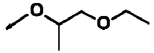 |
| Q-91 | Q-92 | Q-93 | Q-94 | Q-95 |
| | | | | |
|---|---|---|---|---|
| 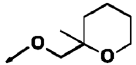 | 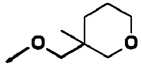 | 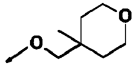 | 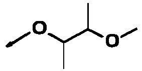 | 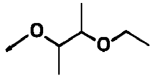 |
| Q-96 | Q-97 | Q-98 | Q-99 | Q-100 |
| | | | | |
|---|---|---|---|---|
| 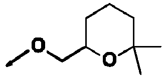 | 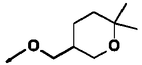 | 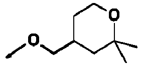 | 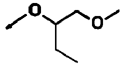 | 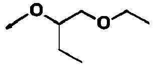 |
| Q-101 | Q-102 | Q-103 | Q-104 | Q-105 |
| | | | | |
|---|---|---|---|---|
| 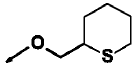 | 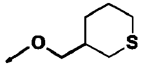 | 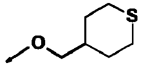 | 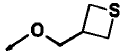 | 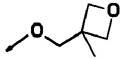 |
| Q-106 | Q-107 | Q-108 | Q-109 | Q-110 |
| | | | | |
|---|---|---|---|---|
| 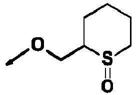 | 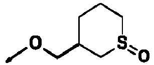 | 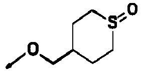 | 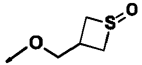 | 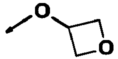 |
| Q-111 | Q-112 | Q-113 | Q-114 | Q-115 |
| | | | | |
|---|---|---|---|---|
| 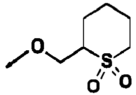 | 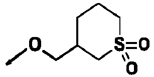 | 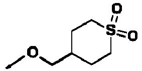 | 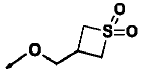 | 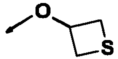 |
| Q-116 | Q-117 | Q-118 | Q-119 | Q-120 |
| | | | | |
|---|---|---|---|---|
| 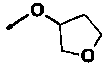 | 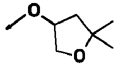 | 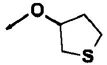 | 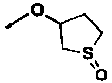 | 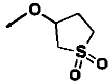 |
| Q-121 | Q-122 | Q-123 | Q-124 | Q-125 |
| | | | | |
|---|---|---|---|---|
| 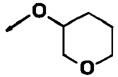 | 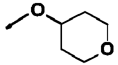 | 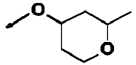 | 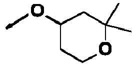 | 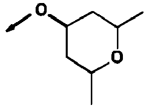 |
| Q-126 | Q-127 | Q-128 | Q-129 | Q-130 |
| | | | | |
|---|---|---|---|---|
| 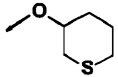 | 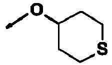 | 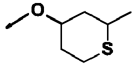 | 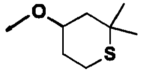 | 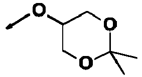 |
| Q-131 | Q-132 | Q-133 | Q-134 | Q-135 |
| | | | | |
|---|---|---|---|---|
| 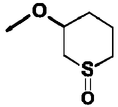 | 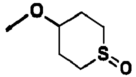 | 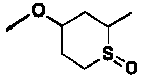 | 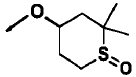 | 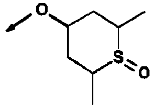 |
| Q-136 | Q-137 | Q-138 | Q-139 | Q-140 |
| | | | | |
|---|---|---|---|---|
| 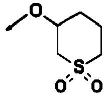 | 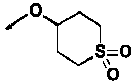 | 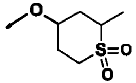 | 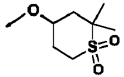 | 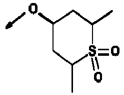 |
| Q-141 | Q-142 | Q-143 | Q-144 | Q-145 |
| | | | | |
|---|---|---|---|---|
| 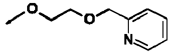 | 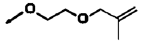 | 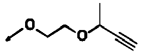 | 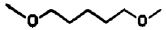 | 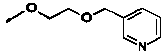 |
| Q-146 | Q-147 | Q-148 | Q-149 | Q-150 |
| | | | | |
|---|---|---|---|---|
| 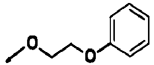 | 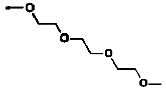 | 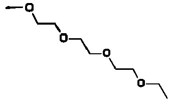 | 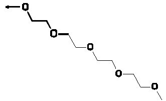 | 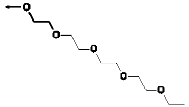 |
| Q-151 | Q-152 | Q-153 | Q-154 | Q-155 |
| | | | | |
|---|---|---|---|---|
| 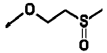 | 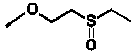 | 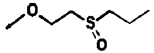 | 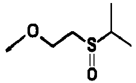 | 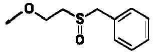 |
| Q-156 | Q-157 | Q-158 | Q-159 | Q-160 |
| | | | | |
|---|---|---|---|---|
| 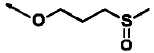 | 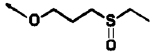 | 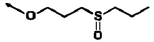 | 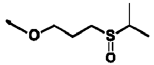 | 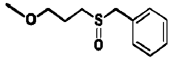 |
| Q-161 | Q-162 | Q-163 | Q-164 | Q-165 |
| | | | | |
|---|---|---|---|---|
| 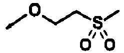 | 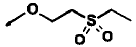 | 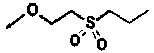 | 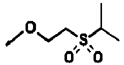 | 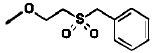 |
| Q-166 | Q-167 | Q-168 | Q-169 | Q-170 |
| | | | | |
|---|---|---|---|---|
| 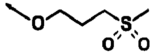 | 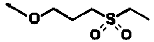 | 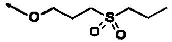 | 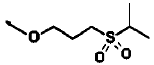 | 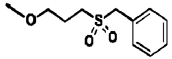 |
| Q-171 | Q-172 | Q-173 | Q-174 | Q-175 |
| | | | | |
|---|---|---|---|---|
| 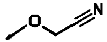 | 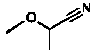 | 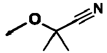 | 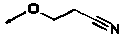 | 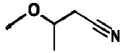 |
| Q-176 | Q-177 | Q-178 | Q-179 | Q-180 |
| | | | | |
|---|---|---|---|---|
| 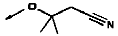 | 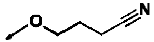 | 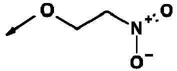 | 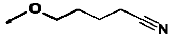 | 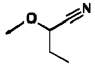 |
| Q-181 | Q-182 | Q-183 | Q-184 | Q-185 |
| | | | | |
|---|---|---|---|---|
| 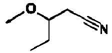 | 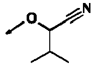 | 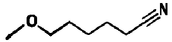 | 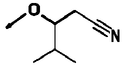 | 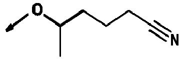 |
| 186 | 187 | 188 | 189 | 190 |
| | | | | |
|---|---|---|---|---|
| 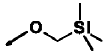 | 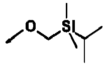 | 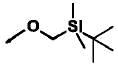 | 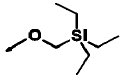 | 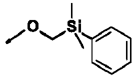 |
| Q-191 | Q-192 | Q-193 | Q-194 | Q-195 |
| | | | | |
|---|---|---|---|---|
| 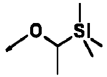 | 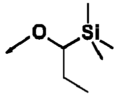 | 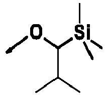 | 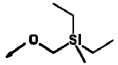 | 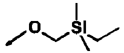 |
| Q-196 | Q-197 | Q-198 | Q-199 | Q-200 |
| | | | | |
|---|---|---|---|---|
| 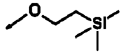 | 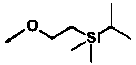 | 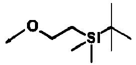 | 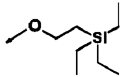 | 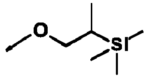 |
| Q-201 | Q-202 | Q-203 | Q-204 | Q-205 |
| | | | | |
|---|---|---|---|---|
| 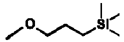 | 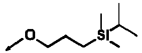 | 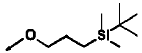 | 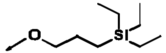 | 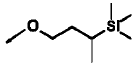 |
| Q-206 | Q-207 | Q-208 | Q-209 | Q-210 |
| | | | | |
|---|---|---|---|---|
| 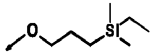 | 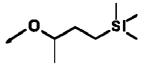 | 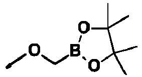 | 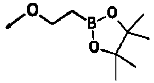 | 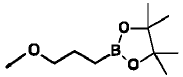 |
| Q-211 | Q-212 | Q-213 | Q-214 | Q-215 |
| | | | | |
|---|---|---|---|---|
| 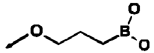 | 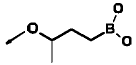 | 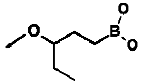 | 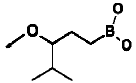 | 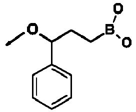 |
| Q-216 | Q-217 | Q-218 | Q-219 | Q-220 |
| | | | | |
|---|---|---|---|---|
| 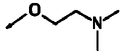 | 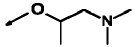 | 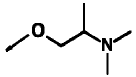 | 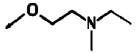 | 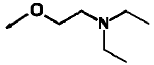 |
| Q-221 | Q-222 | Q-223 | Q-224 | Q-225 |
| | | | | |
|---|---|---|---|---|
| 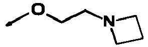 | 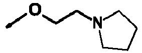 | 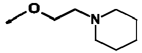 | 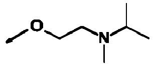 | 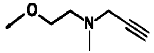 |
| Q-226 | Q-227 | Q-228 | Q-229 | Q-230 |
| | | | | |
|---|---|---|---|---|
| 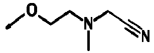 | 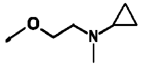 | 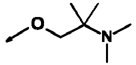 | 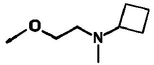 | 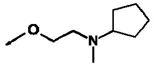 |
| Q-231 | Q-232 | Q-233 | Q-234 | Q-235 |
| | | | | |
|---|---|---|---|---|
| 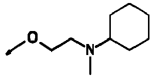 | 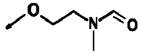 | 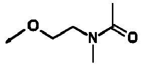 | 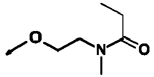 | 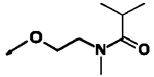 |
| Q-236 | Q-237 | Q-238 | Q-239 | Q-240 |
| | | | | |
|---|---|---|---|---|
| 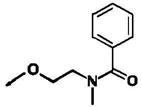 | 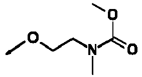 | 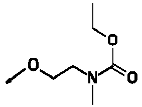 | 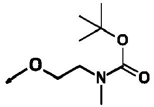 | 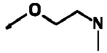 |
| Q-241 | Q-242 | Q-243 | Q-244 | Q-245 |
| | | | | |
|---|---|---|---|---|
| 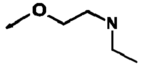 | 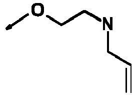 | 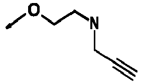 | 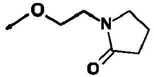 | 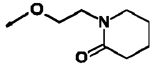 |
| Q-246 | Q-247 | Q-248 | Q-249 | Q-250 |
| | | | | |
|---|---|---|---|---|
| 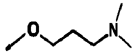 | 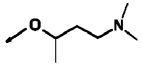 | 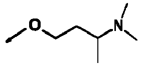 | 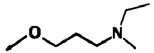 | 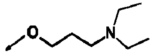 |
| Q-251 | Q-252 | Q-253 | Q-254 | Q-255 |
| | | | | |
|---|---|---|---|---|
| 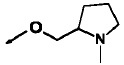 | 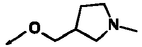 | 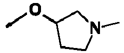 | 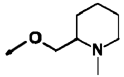 | 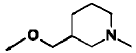 |
| Q-256 | Q-257 | Q-258 | Q-259 | Q-260 |
| | | | | |
|---|---|---|---|---|
| 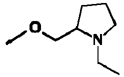 | 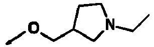 | 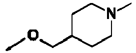 | 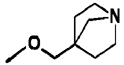 | 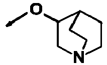 |
| Q-261 | Q-262 | Q-263 | Q-264 | Q-265 |
| | | | | |
|---|---|---|---|---|
| 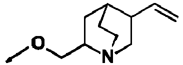 | 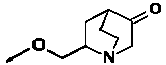 | 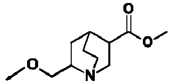 | 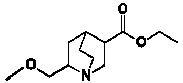 | 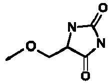 |
| Q-266 | Q-267 | Q-268 | Q-269 | Q-270 |
| | | | | |
|---|---|---|---|---|
| 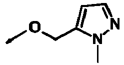 | 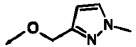 | 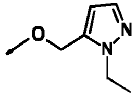 | 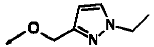 | 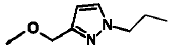 |
| Q-271 | Q-272 | Q-273 | Q-274 | Q-275 |
| | | | | |
|---|---|---|---|---|
| 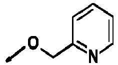 | 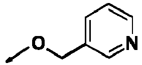 | 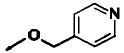 | 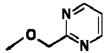 | 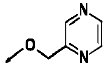 |
| Q-276 | Q-277 | Q-278 | Q-279 | Q-280 |
| | | | | |
|---|---|---|---|---|
| 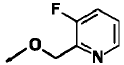 | 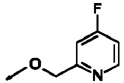 | 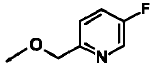 | 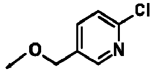 | 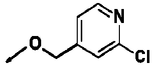 |
| Q-281 | Q-282 | Q-283 | Q-284 | Q-285 |
| | | | | |
|---|---|---|---|---|
| 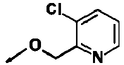 | 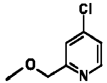 | 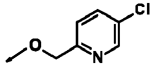 | 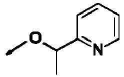 | 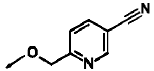 |
| Q-286 | Q-287 | Q-288 | Q-289 | Q-290 |
| | | | | |
|---|---|---|---|---|
| 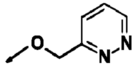 | 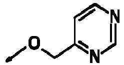 | 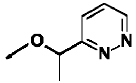 | 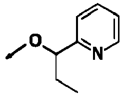 | 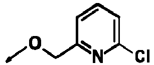 |
| Q-291 | Q-292 | Q-293 | Q-294 | Q-295 |
| | | | | |
|---|---|---|---|---|
| 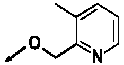 | 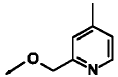 | 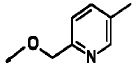 | 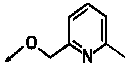 | 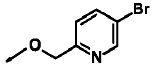 |
| Q-296 | Q-297 | Q-298 | Q-299 | Q-300 |
| | | | | |
|---|---|---|---|---|
| 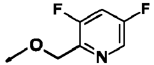 | 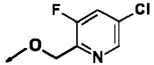 | 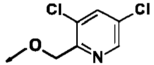 | 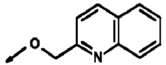 | 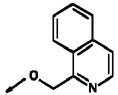 |
| Q-301 | Q-302 | Q-303 | Q-304 | Q-305 |
| | | | | |
|---|---|---|---|---|
| 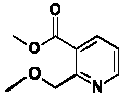 | 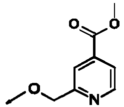 | 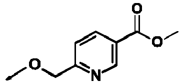 | 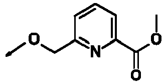 | 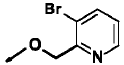 |
| Q-306 | Q-307 | Q-308 | Q-309 | Q-310 |
| | | | | |
|---|---|---|---|---|
| 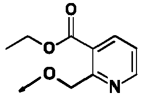 | 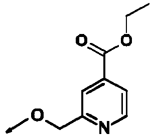 | 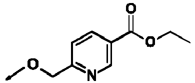 | 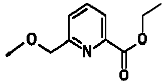 | 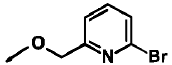 |
| Q-311 | Q-312 | Q-313 | Q-314 | Q-315 |
| | | | | |
|---|---|---|---|---|
| 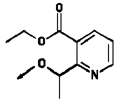 | 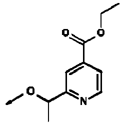 | 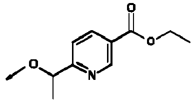 | 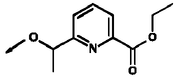 | 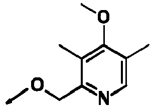 |
| Q-316 | Q-317 | Q-318 | Q-319 | Q-320 |
| | | | | |
|---|---|---|---|---|
| 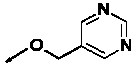 | 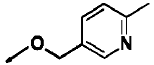 | 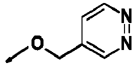 | 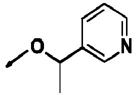 | 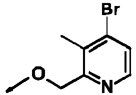 |
| Q-321 | Q-322 | Q-323 | Q-324 | Q-325 |
| | | | | |
|---|---|---|---|---|
| 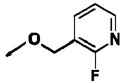 | 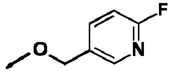 | 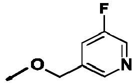 | 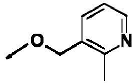 | 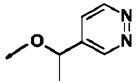 |
| Q-326 | Q-327 | Q-328 | Q-329 | Q-330 |
| | | | | |
|---|---|---|---|---|
| 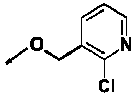 | 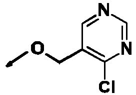 | 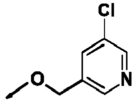 | 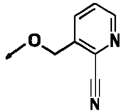 | 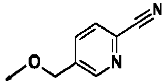 |
| Q-331 | Q-332 | Q-333 | Q-334 | Q-335 |
| | | | | |
|---|---|---|---|---|
| 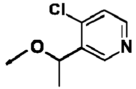 | 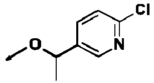 | 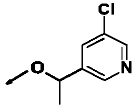 | 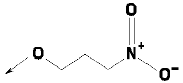 | 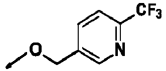 |
| Q-336 | Q-337 | Q-338 | Q-339 | Q-340 |
| | | | | |
|---|---|---|---|---|
| 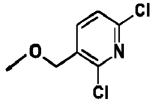 | 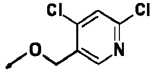 | 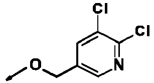 | 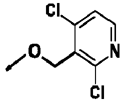 | 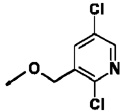 |
| Q-341 | Q-342 | Q-343 | Q-344 | Q-345 |
| | | | | |
|---|---|---|---|---|
| 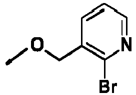 | 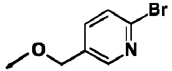 | 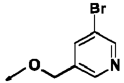 | 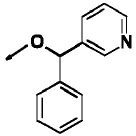 | 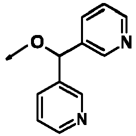 |
| Q-346 | Q-347 | Q-348 | Q-349 | Q-350 |
| | | | | |
|---|---|---|---|---|
| 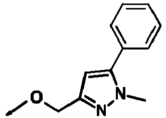 | 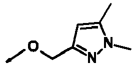 | 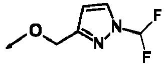 | 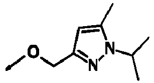 | 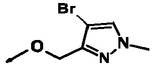 |
| Q-351 | Q-352 | Q-353 | Q-354 | Q-355 |
| | | | | |
|---|---|---|---|---|
| 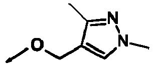 | 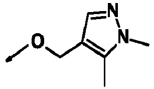 | 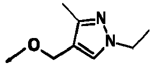 | 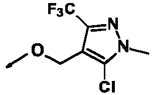 | 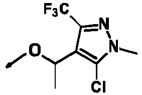 |
| Q-356 | Q-357 | Q-358 | Q-359 | Q-360 |
| | | | | |
|---|---|---|---|---|
| 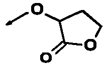 | 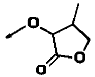 | 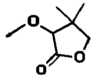 | 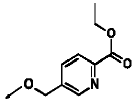 | 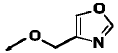 |
| Q-361 | Q-362 | Q-363 | Q-364 | Q-365 |
| | | | | |
|---|---|---|---|---|
| 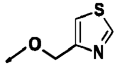 | 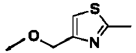 | 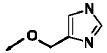 | 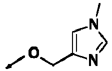 | 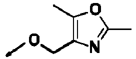 |
| Q-366 | Q-367 | Q-368 | Q-369 | Q-370 |
| | | | | |
|---|---|---|---|---|
| 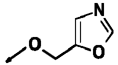 | 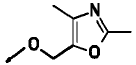 | 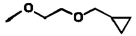 | 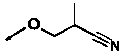 | 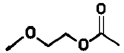 |
| Q-371 | Q-372 | Q-373 | Q-374 | Q-375 |
| | | | | |
|---|---|---|---|---|
| 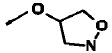 | 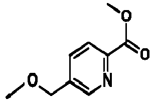 | 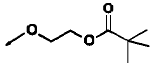 | 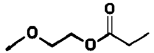 | 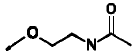 |
| Q-376 | Q-377 | Q-378 | Q-379 | Q-380 |
| | | | | |
|---|---|---|---|---|
| 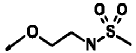 | 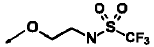 | 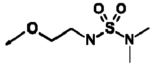 | 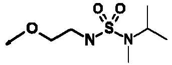 | 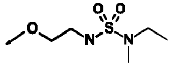 |
| Q-381 | Q-382 | Q-383 | Q-384 | Q-385 |
| | | | | |
|---|---|---|---|---|
| 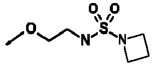 | 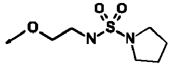 | 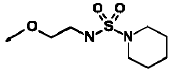 | 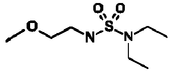 | 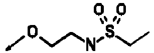 |
| Q-386 | Q-387 | Q-388 | Q-389 | Q-390 |
| | | | | |
|---|---|---|---|---|
| 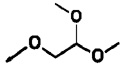 | 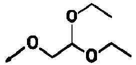 | 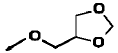 | 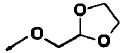 | 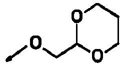 |
| Q-391 | Q-392 | Q-393 | Q-394 | Q-395 |
| | | | | |
|---|---|---|---|---|
| 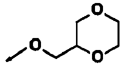 | 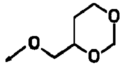 | 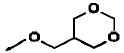 | 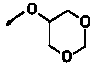 | 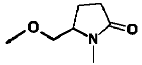 |
| Q-396 | Q-397 | Q-398 | Q-399 | Q-400 |
| | | | | |
|---|---|---|---|---|
| 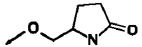 | 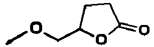 | 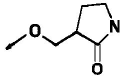 | 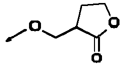 | 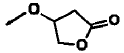 |
| Q-401 | Q-402 | Q-403 | Q-404 | Q-405 |
| |
|---|
| 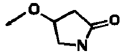 |
| Q-406 |

4. Verbindungen der allgemeinen Formel (I) gemäß Anspruch 1 und/oder deren Salz, **dadurch gekennzeichnet, dass**
R¹ für Wasserstoff, Fluor, Chlor, Brom, Iod, Cyano, Methyl, Ethyl, Trifluormethyl, Difluormethyl, Methoxy, Ethoxy, Difluormethoxy, Trifluormethoxy steht,
R² für Fluor steht,
R³ für Wasserstoff, Fluor, Chlor, Brom, Methoxy steht,
R⁴ für Fluor, Chlor, Brom, Cyano, NO₂, C(O)NHz, C(S)NH₂, Trifluormethyl, Ethinyl, Propin-1-yl steht,
R⁵, R⁶ und R⁷ unabhängig voneinander für Wasserstoff, Fluor, Chlor, Brom, Iod, Cyano, Methyl, Ethyl, Trifluormethyl, Difluormethyl, Methoxy, Ethoxy, Difluormethoxy, Trifluormethoxy stehen,
G für Methylen, (Methyl)methylen, (Ethyl)methylen, (Dimethyl)methylen, Ethylen, n-Propylen, (1-Methyl)ethyl-1-en, (2-Methyl)ethyl-1-en, n-Butylen, 1-Methylpropyl-1-en, 2-Methylpropyl-1-en, 3-Methylpropyl-1-en, 1,1-Dimethylethyl-1-en, 2,2-Dimethylethyl-1-en, 1-Ethylethyl-1-en, 2-Ethylethyl-1-en, 1-(Prop-1-yl)ethyl-1-en, 2-(Prop-1-yl)ethyl-1-en, 1-(Prop-2-yl)ethyl-1-en, 2-(Prop-2-yl)ethyl-1-en, n-Pentylen, 1-Methylbutyl-1-en, 2-Methylbutyl-1-en, 3-Methylbutyl-1-en, 4-Methylbutyl-1-en, 1,1-Dimethylpropyl-1-en, 2,2-Dimethylpropyl-1-en, 3,3-Dimethylpropyl-1-en, 1,2-Dimethylpropyl-1-en, 1,3-Dimethylpropyl-1-en, 1-Ethylpropyl-1-en, n-Hexylen steht,
X und Y unabhängig voneinander für 0 (Sauerstoff) oder S (Schwefel) stehen
und Q für eine der in Anspruch 3 spezifisch genannten Gruppierungen Q-1 bis Q-406 steht.

5. Verbindungen der allgemeinen Formel (I) gemäß Anspruch 1 und/oder deren Salz, **dadurch gekennzeichnet, dass**
R¹ für Wasserstoff, Fluor, Chlor, Brom, Cyano, Methyl, Trifluormethyl, Methoxy, Trifluormethoxy steht,
R² für Fluor steht,
R³ für Fluor steht,
R⁴ für Chlor, Brom, Cyano, NO₂, C(O)NH₂, C(S)NH₂ steht,
R⁵, R⁶ und R⁷ unabhängig voneinander für Wasserstoff, Fluor, Chlor, Brom, Cyano, Methyl, Trifluormethyl, Methoxy, Trifluormethoxy stehen,
G für Methylen, (Methyl)methylen, (Ethyl)methylen, (Dimethyl)methylen, Ethylen, n-Propylen, (1-Methyl)ethyl-1-en, (2-Methyl)ethyl-1-en, n-Butylen, 1-Methylpropyl-1-en, 2-Methylpropyl-1-en, 3-Methylpropyl-1-en, n-Pentylen, n-Hexylen steht,
X und Y unabhängig voneinander für O (Sauerstoff) oder S (Schwefel) stehen
und Q für eine der in Anspruch 3 spezifisch genannten Gruppierungen Q-1 bis Q-406 steht.

6. Verbindungen der allgemeinen Formel (I) gemäß Anspruch 1 und/oder deren Salz, **dadurch gekennzeichnet, dass**
R¹ für Wasserstoff steht,
R² für Fluor steht,
R³ für Fluor steht,
R⁴ für Chlor, Brom, Cyano, NO₂ steht,
R⁵ für Wasserstoff, Fluor, Chlor, Brom steht,
R⁶ für Wasserstoff, Fluor, Chlor, Brom, Cyano steht,
R⁷ für Wasserstoff steht,
G für Methylen, (Methyl)methylen steht,
X für O (Sauerstoff) oder S (Schwefel) steht,
Y für O (Sauerstoff) steht
und Q für eine der in Anspruch 3 spezifisch genannten Gruppierungen Q-1 bis Q-406 steht.

7. Verbindungen der allgemeinen Formel (I) gemäß Anspruch 1 und/oder deren Salz, **dadurch gekennzeichnet, dass**
R¹ für Wasserstoff steht,
R² für Fluor steht,
R³ für Fluor steht,
R⁴ für Chlor, Brom, Cyano, NO₂ steht,
R⁵ für Wasserstoff, Fluor steht,
R⁶ für Wasserstoff, Fluor, Brom, Cyano steht,
R⁷ für Wasserstoff steht,
G für Methylen, (Methyl)methylen steht,
X für O (Sauerstoff) oder S (Schwefel) steht,
Y für O (Sauerstoff) steht
und Q für eine der in Anspruch 3 spezifisch genannten Gruppierungen Q-1 bis Q-35, Q-41 bis Q-45, Q-58, Q-71 bis Q-80, Q-89, Q-94, Q-95, Q-115, Q-120 bis Q-123, Q-152 bis Q-155, Q-166 bis Q-170, Q-176 bis Q-190, Q-261 bis Q-348, Q-352 bis Q-372, Q-377, Q-391-Q-399 steht.

8. Verbindungen der allgemeinen Formel (I) gemäß Anspruch 1 und/oder deren Salz, **dadurch gekennzeichnet, dass**
R¹ für Wasserstoff steht,
R² für Fluor steht,
R³ für Fluor steht,
R⁴ für Chlor, Brom, Cyano, NO₂ steht,
R⁵ für Wasserstoff, Fluor steht,
R⁶ für Wasserstoff, Fluor, Brom, Cyano steht,
R⁷ für Wasserstoff steht,
G für Methylen, (Methyl)methylen steht,
X für O (Sauerstoff) oder S (Schwefel) steht,
Y für O (Sauerstoff) steht
und Q für eine der in Anspruch 3
spezifisch genannten Gruppierungen Q-1, Q-2, Q-3, Q-4, Q-7, Q-8, Q-9, Q-17, Q-18, Q-23, Q-24, Q-26, Q-27, Q-41, Q-42, Q-43, Q-58, Q-71, Q-72, Q-89, Q-94, Q-115, Q-121, Q-176, Q-177, Q-179, Q-183, Q-272, Q-274, Q-275, Q-276, Q-277, Q-278, Q-281, Q-282, Q-283, Q-284, Q-286, Q-288, Q-291, Q-296, Q-301, Q-302, Q-303, Q-308, Q-309, Q-321, Q-327, Q-328, Q-329, Q-331, Q-335, Q-339, Q-356, Q-365, Q-366, Q-367, Q-371, Q-394 steht.

9. Verwendung einer oder mehrere Verbindungen der allgemeinen Formel (I) wie in einem der Ansprüche 1 bis 8 definiert und/oder deren Salze, als Herbizid und/oder Pflanzenwachstumsregulator, vorzugsweise in Kulturen von Nutz- und/oder Zierpflanzen.

10. Herbizides und/oder pflanzenwachstumsregulierendes Mittel, **dadurch gekennzeichnet, dass** das Mittel eine oder mehrere Verbindungen der allgemeinen Formel (I) wie in einem der Ansprüche 1 bis 8 definiert und/oder deren Salze enthält, sowie weiter ein oder mehrere Stoffe ausgewählt aus den Gruppen (i) und/oder (ii), mit
(i) ein oder mehrere weitere agrochemisch wirksame Stoffe, vorzugsweise ausgewählt aus der Gruppe bestehend aus Insektiziden, Akariziden, Nematiziden, weiteren Herbiziden, Fungiziden, Safenern, Düngemitteln und/oder weiteren Wachstumsregulatoren,
(ii) ein oder mehrere im Pflanzenschutz übliche Formulierungshilfsmittel.

11. Verfahren zur Bekämpfung von Schadpflanzen oder zur Wachstumsregulierung von Pflanzen, **dadurch gekennzeichnet, dass** eine wirksame Menge
- einer oder mehrerer Verbindungen der allgemeinen Formel (I), wie in einem der Ansprüche 1 bis 8 definiert und/oder deren Salze, oder
- eines Mittels nach Anspruch 10
auf die Pflanzen, Pflanzensamen, den Boden, in dem oder auf dem die Pflanzen wachsen, oder die Anbaufläche appliziert wird.

## Claims

1. Substituted N-phenyluracils of the general formula (I) or salts thereof 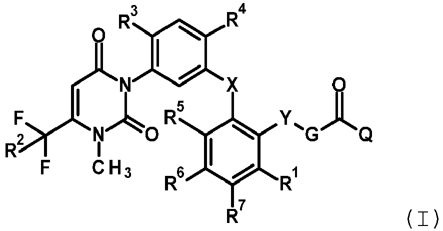 in which
R¹ is hydrogen, halogen, cyano, (C₁-C₈) -alkyl, (C₁-C₈) -haloalkyl, (C₁-C₈) -alkoxy, (C₁-C₈)-haloalkoxy,
R² is hydrogen, fluorine, chlorine, bromine, trifluoromethyl, (C1-C8) -alkoxy,
R³ is hydrogen, halogen, (C₁-C₈) -alkoxy,
R⁴ is halogen, cyano, NO₂, C(O)NH₂, C(S)NH₂, (C₁-C₈) -haloalkyl, (C₂-C₈) - alkynyl,
R⁵, R⁶ and R⁷ are independently hydrogen, halogen, cyano, (C₁-C₈) -alkyl, (C₁-C₈) -haloalkyl, (C₁-C₈) -alkoxy, (C₁-C₈) -haloalkoxy,
G is unbranched or branched (C₁-C₈) -alkylene,
Q is a radical of the formula 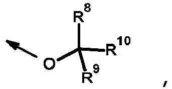
R⁸ is hydrogen, (C₁-C₈) -alkyl, (C₁-C₈) -haloalkyl, aryl, aryl- (C₁-C₈) -alkyl, heteroaryl, (C₂-C₈)-R⁹ alkynyl, (C₂-C₈)-alkenyl, C(O)R¹³, C(O)OR¹³, (C₁-C₈)-alkoxy-(C₁-C₈)-alkyl, is hydrogen or (C₁-C₈)-alkyl,
R¹⁰ is cyano, NO₂, heteroaryl, heteroaryl- (C₁-C₈)-alkyl, heterocyclyl, heterocyclyl- (C₁-C₈)-alkyl, R¹¹R¹²N- (C₁-C₈) -alkyl, R¹³O- (C₁-C₈) -alkyl, cyano-(C₁-C₈)-alkyl, (C₁-C₈)-alkylcarbonyloxy-(C₁-C₈)-alkyl, (C₃-C₈)-cycloalkylcarbonyloxy-(C₁-C₈)-alkyl, arylcarbonyloxy- (C₁-C₈) -alkyl, heteroarylcarbonyloxy-(C₁-C₈) -alkyl, heterocyclylcarbonyloxy- (C₁-C₈) -alkyl, OR¹³, NR¹¹R¹², SR¹⁴, S(O)R¹⁴, SO₂R¹⁴, R¹⁴S-(C₁-C₈)-alkyl, R¹⁴(O)S-(C₁-C₈)-alkyl, R¹⁴O₂S-(C₁-C₈)-alkyl, tris[(C₁-C₈)-alkyl]silyl-(C₁-C₈)-alkyl, bis[(C₁-C₈)-alkyl](aryl)silyl(C₁-C₈)-alkyl, [(C₁-C₈)-alkyl]bis(aryl)silyl-(C₁-C₈)-alkyl, tris[(C₁-C₈)-alkyl]silyl, bishydroxyboryl-(C₁-C₈)-alkyl, bis[(C₁-C₈)-alkoxy]boryl-(C₁-C₈)-alkyl, tetramethyl-1,3,2-dioxaborolan-2-yl, tetramethyl-1,3,2-dioxaborolan-2-yl-(C₁-C₈)-alkyl, nitro-(C₁-C₈)-alkyl, C(O)R¹⁴, bis (C₁-C₈)-alkoxymethyl, bis(C₁-C₈)-alkoxymethyl-(C₁-C₈)-alkyl, or
R⁸ and R¹⁰ together with the carbon atom to which they are bonded form a fully saturated or partly saturated 3- to 10-membered monocyclic or bicyclic heterocyclyl optionally having further substitution,
R¹¹ and R¹² are the same or different and are independently hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈) -alkyl, (C₁-C₈)-haloalkoxy-(C₁-C₈)-alkyl, (C₁-C₈)-alkylthio-(C₁-C₈)-alkyl, (C₁-C₈)-haloalkylthio-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, COR¹³, SO₂R¹⁴, heterocyclyl, (C₁-C₈)-alkoxycarbonyl, bis [(C₁-C₈)-alkyl] aminocarbonyl-(C₁-C₈)-alkyl, (C₁-C₈)-alkylaminocarbonyl-(C₁-C₈)-alkyl, aryl-(C₁-C₈)-alkylaminocarbonyl-(C₁-C₈)-alkyl, aryl-(C₁-C₈)-alkoxycarbonyl, heteroaryl-(C₁-C₈)-alkoxycarbonyl, (C₂-C₈)-alkenyloxycarbonyl, (C₂-C₈)-alkynyloxycarbonyl, heterocyclyl-(C₁-C₈)-alkyl, or
R¹¹ and R¹² together with the nitrogen atom to which they are bonded form a fully saturated or partly saturated 3- to 10-membered monocyclic or bicyclic ring optionally interrupted by heteroatoms and optionally having further substitution,
R¹³ is hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-haloalkoxy-(C₁-C₈)-alkyl, (C₁-C₈)-alkoxy-(C₁-C₈)-haloalkyl, (C₁-C₈)-alkoxy- (C₁-C₈)-alkoxy-(C₁-C₈)-alkyl, (C₁-C₈)-alkoxy- (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy- (C₁-C₈)-alkoxy-(C₁-C₈)-alkyl, aryl, aryl-(C₁-C₈)-alkyl, aryl-(C₁-C₈)-alkoxy-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, bis [(C₁-C₈)-alkyl]aminocarbonyl-(C₁-C₈)-alkyl, (C₁-C₈)-alkylaminocarbonyl-(C₁-C₈)-alkyl, aryl-(C₁-C₈)-alkylaminocarbonyl-(C₁-C₈)-alkyl, bis[(C₁-C₈)-alkyl]amino-(C₂-C₆)-alkyl, (C₁-C₈)-alkylamino-(C₂-C₆)-alkyl, aryl-(C₁-C₈)-alkylamino-(C₂-C₆)-alkyl, R¹⁴S-(C₁-C₈)-alkyl, R¹⁴(O)S-(C₁-C₈)-alkyl, R¹⁴O₂S-(C₁-C₈)-alkyl, hydroxycarbonyl-(C₁-C₈)-alkyl, heterocyclyl, heterocyclyl-(C₁-C₈)-alkyl, tris[(C₁-C₈)-alkyl]silyl-(C₁-C₈)-alkyl, bis[(C₁-C₈)-alkyl](aryl)silyl(C₁-C₈)-alkyl, [(C₁-C₈)-alkyl]bis(aryl)silyl-(C₁-C₈)-alkyl, (C₁-C₈)-alkylcarbonyloxy-(C₁-C₈)-alkyl, (C₃-C₈) cycloalkylcarbonyloxy-(C₁-C₈)-alkyl, arylcarbonyloxy-(C₁-C₈)-alkyl, heteroarylcarbonyloxy-(C₁-C₈)-alkyl, heterocyclylcarbonyloxy-(C₁-C₈)-alkyl, aryloxy-(C₁-C₈)-alkyl, heteroaryloxy-(C₁-C₈)-alkyl, (C₁-C₈)-alkoxycarbonyl,
R¹⁴ is hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, heterocyclyl-(C₁-C₈)-alkyl, (C₃-C₈)-cycloalkyl-(C₁-C₈) -alkyl, (C₄-C₁₀)-cycloalkenyl-(C₁-C₈) -alkyl, bis [(C₁-C₈)-alkyl] amino, (C₁-C₈)- alkylamino, aryl-(C₁-C₈)-amino, aryl-(C₁-C₆)-alkylamino, aryl-[(C₁-C₈)-alkyl]amino; (C₃-C₈)-cycloalkylamino, (C₃-C₈)-cycloalkyl-[(C₁-C₈)-alkyl]amino; N-azetidinyl, N-pyrrolidinyl, N-piperidinyl, N-morpholinyl,
and X and Y are independently O (oxygen) or S (sulfur).

2. Compounds of the general formula (I) according to Claim 1 and/or salt thereof, **characterized in that**
R¹ is hydrogen, halogen, cyano, (C₁-C₆)-alkyl, (C₁-C₆)-haloalkyl, (C₁-C₆)-alkoxy, (C₁-C₆)-haloalkoxy,
R² is hydrogen, fluorine, chlorine, bromine, trifluoromethyl, (C1-C6)-alkoxy,
R³ is hydrogen, halogen, (C₁-C₆)-alkoxy,
R⁴ is halogen, cyano, NO₂, C(O)NH₂, C(S)NH₂, (C₁-C₆)-haloalkyl, (C₂-C₆)-alkynyl,
R⁵, R⁶ and R⁷ are independently hydrogen, halogen, cyano, (C₁-C₆)-alkyl, (C₁-C₆)-haloalkyl, (C₁-C₆)-alkoxy, (C₁-C₆)-haloalkoxy,
G is unbranched or branched (C₁-C₆) -alkylene,
Q is a radical of the formula 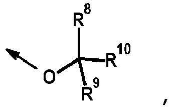
R⁸ is hydrogen, (C₁-C₆)-alkyl, (C₁-C₆)-haloalkyl, aryl, aryl-(C₁-C₆)-alkyl, heteroaryl, (C₂-C₆)-alkynyl, (C₂-C₆)-alkenyl, C(O)R¹³, C(O)OR¹³, (C₁-C₆)-alkoxy-(C₁-C₆)-alkyl,
R⁹ is hydrogen or (C₁-C₄)-alkyl,
R¹⁰ is cyano, NO₂, heteroaryl, heteroaryl-(C₁-C₆)-alkyl, heterocyclyl, heterocyclyl-(C₁-C₆)-alkyl, R¹¹R¹²N-(C₁-C₆)-alkyl, R¹³O-(C₁-C₆) -alkyl, cyano-(C₁-C₆)-alkyl, (C₁-C₆)-alkylcarbonyloxy-(C₁-C₆)-alkyl, (C₃-C₆)-cycloalkylcarbonyloxy-(C₁-C₆)-alkyl, arylcarbonyloxy-(C₁-C₆)-alkyl, heteroarylcarbonyloxy-(C₁-C₆)-alkyl, heterocyclylcarbonyloxy-(C₁-C₆)-alkyl, OR¹³, NR¹¹R¹², SR¹⁴, S(O)R¹⁴, SO₂R¹⁴, R¹⁴S-(C₁-C₆)-alkyl, R¹⁴(O)S-(C₁-C₆)-alkyl, R¹⁴O₂S-(C₁-C₆)-alkyl, tris[(C₁-C₆)-alkyl]silyl-(C₁-C₆)-alkyl, bis[(C₁-C₆)-alkyl](aryl)silyl(C₁-C₆)-alkyl, [(C₁-C₆)-alkyl]bis(aryl)silyl-(C₁-C₆)-alkyl, tris[(C₁-C₆)-alkyl]silyl, bishydroxyboryl-(C₁-C₆)-alkyl, bis[(C₁-C₆)-alkoxy]boryl-(C₁-C₆)-alkyl, tetramethyl-1,3,2-dioxaborolan-2-yl, tetramethyl-1,3,2-dioxaborolan-2-yl-(C₁-C₆)-alkyl, nitro-(C₁-C₆)-alkyl, C(O)R¹³, bis (C₁-C₆)-alkoxymethyl, bis(C₁-C₆)-alkoxymethyl-(C₁-C₆)-alkyl,
R⁸ and R¹⁰ together with the carbon atom to which they are bonded form a fully saturated or partly saturated 3- to 10-membered monocyclic or bicyclic heterocyclyl optionally having further substitution,
R¹¹ and R¹² are the same or different and are independently hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₃-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-haloalkoxy-(C₁-C₆)-alkyl, (C₁-C₆)-alkylthio-(C₁-C₆)-alkyl, (C₁-C₆)-haloalkylthio-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, (C₃-C₆)-cycloalkyl-(C₁-C₆)-alkyl, (C₄-C₁₀)-cycloalkenyl-(C₁-C₆)-alkyl, COR¹³, SO₂R¹⁴, heterocyclyl, (C₁-C₆)-alkoxycarbonyl, bis [(C₁-C₆)-alkyl] aminocarbonyl -(C₁-C₆)-alkyl, (C₁-C₆)-alkylaminocarbonyl-(C₁-C₆)-alkyl, aryl-(C₁-C₆)-alkylaminocarbonyl-(C₁-C₆)-alkyl, aryl-(C₁-C₆)-alkoxycarbonyl, heteroaryl-(C₁-C₆)-alkoxycarbonyl, (C₂-C₆)-alkenyloxycarbonyl, (C₂-C₆)-alkynyloxycarbonyl, heterocyclyl-(C₁-C₆)-alkyl, or
R¹¹ and R¹² together with the nitrogen atom to which they are bonded form a fully saturated or partly saturated 3- to 10-membered monocyclic or bicyclic ring optionally interrupted by heteroatoms and optionally having further substitution,
R¹³ is hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₃-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-haloalkoxy-(C₁-C₆)-alkyl, (C₁-C₆)-alkoxy-(C₁-C₆)-haloalkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkoxy-(C₁-C₆)-alkyl, aryl, aryl-(C₁-C₆)-alkyl, aryl-(C₁-C₆)-alkoxy-(C₁-C₆)-alkyl, heteroaryl, heteroaryl-(C₁-C₆)-alkyl, (C₃-C₆)-cycloalkyl-(C₁-C₆)-alkyl, (C₄-C₁₀)-cycloalkenyl-(C₁-C₆)-alkyl, bis[(C₁-C₆)-alkyl] aminocarbonyl-(C₁-C₆)-alkyl, (C₁-C₆)-alkylaminocarbonyl-(C₁-C₆)-alkyl, aryl-(C₁-C₆)-alkylaminocarbonyl-(C₁-C₆)-alkyl, bis[(C₁-C₆)-alkyl]amino-(C₂-C₄)-alkyl, (C₁-C₆)-alkylamino-(C₂-C₄)-alkyl, aryl-(C₁-C₆)-alkylamino-(C₂-C₄)-alkyl, R¹⁴S-(C₁-C₆)-alkyl, R¹⁴(O)S-(C₁-C₆)-alkyl, R¹⁴O₂S-(C₁-C₆)-alkyl, hydroxycarbonyl-(C₁-C₆)-alkyl, heterocyclyl, heterocyclyl-(C₁-C₆)-alkyl, tris[(C₁-C₆)-alkyl]silyl-(C₁-C₆)-alkyl, bis[(C₁-C₆)-alkyl](aryl)silyl(C₁-C₆)-alkyl, [(C₁-C₆)-alkyl]bis(aryl)silyl-(C₁-C₆)-alkyl, (C₁-C₆)-alkylcarbonyloxy-(C₁-C₆)-alkyl, (C₃-C₆)-cycloalkylcarbonyloxy-(C₁-C₆)-alkyl, arylcarbonyloxy-(C₁-C₆)-alkyl, heteroarylcarbonyloxy-(C₁-C₆)-alkyl, heterocyclylcarbonyloxy-(C₁-C₆)-alkyl, aryloxy-(C₁-C₆)-alkyl, heteroaryloxy-(C₁-C₆)-alkyl, (C₁-C₆)-alkoxycarbonyl,
R¹⁴ is hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₃-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, heterocyclyl-(C₁-C₆)-alkyl, (C₃-C₆)-cycloalkyl-(C₁-C₆)-alkyl, (C₄-C₁₀)-cycloalkenyl-(C₁-C₆)-alkyl, bis[(C₁-C₆)-alkyl]amino, (C₁-C₆)-alkylamino, aryl-(C₁-C₆)-amino, aryl-(C₁-C₂)-alkylamino, aryl-[(C₁-C₆)-alkyl]amino; (C₃-C₆)-cycloalkylamino, (C₃-C₆)-cycloalkyl-[(C₁-C₆)-alkyl]amino; N-azetidinyl, N-pyrrolidinyl, N-piperidinyl, N-morpholinyl,
and
X and Y are independently O (oxygen) or S (sulfur).

3. Compounds of the general formula (I) according to Claim 1 and/or salt thereof, **characterized in that**
R¹ is hydrogen, fluorine, chlorine, bromine, iodine, cyano, methyl, ethyl, prop-1-yl, 1-methylethyl, but-1-yl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, trifluoromethyl, difluoromethyl, pentafluoroethyl, 2,2-difluoroethyl, 2,2,2-trifluoroethyl, methoxy, ethoxy, prop-1-yloxy, prop-2-yloxy, but-1-yloxy, but-2-yloxy, 2-methylprop-1-yloxy, 1,1-dimethyleth-1-yloxy, difluoromethoxy, trifluoromethoxy, pentafluoroethoxy, 2,2-difluoroethoxy, 2,2,2-trifluoroethoxy,
R² is hydrogen, fluorine, chlorine, bromine, trifluoromethyl, methoxy, ethoxy, prop-1-yloxy, but-1-yloxy,
R³ is hydrogen, fluorine, chlorine, bromine, methoxy, ethoxy, prop-1-yloxy, prop-2-yloxy, but-1-yloxy, but-2-yloxy, 2-methylprop-1-yloxy, 1,1-dimethyleth-1-yloxy,
R⁴ is fluorine, chlorine, bromine, cyano, NO₂, C(O)NH₂, C(S)NH₂, trifluoromethyl, difluoromethyl, pentafluoroethyl, ethynyl, propyn-1-yl, 1-butyn-1-yl, pentyn-1-yl, hexyn-1-yl,
R⁵, R⁶ and R⁷ are independently hydrogen, fluorine, chlorine, bromine, iodine, cyano, methyl, ethyl, prop-1-yl, 1-methylethyl, but-1-yl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, trifluoromethyl, difluoromethyl, pentafluoroethyl, 2,2-difluoroethyl, 2,2,2-trifluoroethyl, methoxy, ethoxy, prop-1-yloxy, prop-2-yloxy, but-1-yloxy, but-2-yloxy, 2-methylprop-1-yloxy, 1,1-dimethyleth-1-yloxy, difluoromethoxy, trifluoromethoxy, pentafluoroethoxy, 2,2-difluoroethoxy, 2,2,2-trifluoroethoxy,
G is methylene, (methyl)methylene, (ethyl)methylene, (prop-1-yl)methylene, (prop-2-yl)methylene, (but-1-yl)methylene, (but-2-yl)methylene, (pent-1-yl)methylene, (pent-2-yl)methylene, (pent-3-yl)methylene, (dimethyl)methylene, (diethyl)methylene, ethylene, n-propylene, (1-methyl)ethyl-1-ene, (2-methyl)ethyl-1-ene, n-butylene, 1-methylpropyl-1-ene, 2-methylpropyl-1-ene, 3-methylpropyl-1-ene, 1,1-dimethylethyl-1-ene, 2,2-dimethylethyl-1-ene, 1-ethylethyl-1-ene, 2-ethylethyl-1-ene, 1-(prop-1-yl)ethyl-1-ene, 2-(prop-1-yl)ethyl-1-ene, 1-(prop-2-yl)ethyl-1-ene, 2-(prop-2-yl)ethyl-1-ene, 1,1,2-trimethylethyl-1-ene, 1,2,2-trimethylethyl-1-ene, 1,1,2,2-tetramethylethyl-1-ene, n-pentylene, 1-methylbutyl-1-ene, 2-methylbutyl-1-ene, 3-methylbutyl-1-ene, 4-methylbutyl-1-ene, 1,1-dimethylpropyl-1-ene, 2,2-dimethylpropyl-1-ene, 3,3-dimethylpropyl-1-ene, 1,2-dimethylpropyl-1-ene, 1,3-dimethylpropyl-1-ene, 1-ethylpropyl-1-ene, n-hexylene, 1-methylpentyl-1-ene, 2-methylpentyl-1-ene, 3-methylpentyl-1-ene, 4-methylpentyl-1-ene, 1,1-dimethylbutyl-1-ene, 1,2-dimethylbutyl-1-ene, 1,3-dimethylbutyl-1-ene, 2,2-dimethylbutyl-1-ene, 2,3-dimethylbutyl-1-ene, 3,3-dimethylbutyl-1-ene, 1-ethylbutyl-1-ene, 2-ethylbutyl-1-ene, 1,1,2-trimethylpropyl-1-ene, 1,2,2-trimethylpropyl-1-ene, 1-ethyl-1-methylpropyl-1-ene, 1-ethyl-2-methylpropyl-1-ene,
X and Y are independently O (oxygen) or S (sulfur)
and Q is one of the moieties Q-1 to Q-406 specifically mentioned below, where the arrow in the structural formulae in the table below represents a bond of the respective Q group to the carbonyl group in the general formula (I):
| | | | | |
|---|---|---|---|---|
| 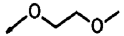 | 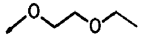 | 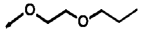 | 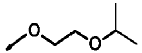 | 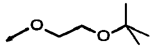 |
| Q-1 | Q-2 | Q-3 | Q-4 | Q-5 |
| | | | | |
|---|---|---|---|---|
| 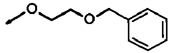 | 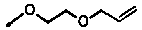 | 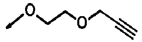 | 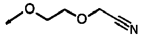 | 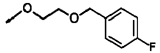 |
| Q-6 | Q-7 | Q-8 | Q-9 | Q-10 |
| | | | | |
|---|---|---|---|---|
| 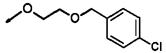 | 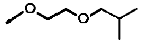 | 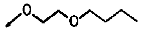 | 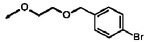 | 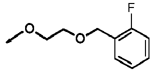 |
| Q-11 | Q-12 | Q-13 | Q-14 | Q-15 |
| | | | | |
|---|---|---|---|---|
| 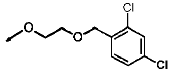 | 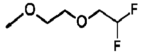 | 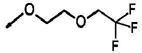 | 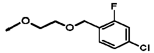 | 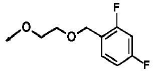 |
| Q-16 | Q-17 | Q-18 | Q-19 | Q-20 |
| | | | | |
|---|---|---|---|---|
| 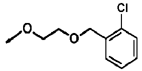 | 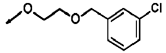 | 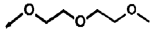 | 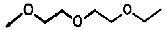 | 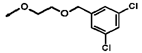 |
| Q-21 | Q-22 | Q-23 | Q-24 | Q-25 |
| | | | | |
|---|---|---|---|---|
| 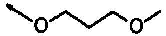 | 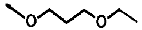 | 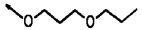 | 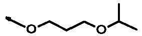 | 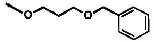 |
| Q-26 | Q-27 | Q-28 | Q-29 | Q-30 |
| | | | | |
|---|---|---|---|---|
| 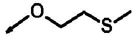 | 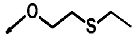 | 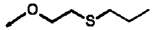 | 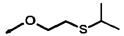 | 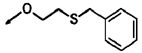 |
| Q-31 | Q-32 | Q-33 | Q-34 | Q-35 |
| | | | | |
|---|---|---|---|---|
| 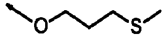 | 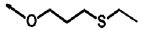 | 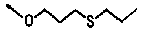 | 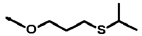 | 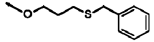 |
| Q-36 | Q-37 | Q-38 | Q-39 | Q-40 |
| | | | | |
|---|---|---|---|---|
| 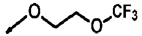 | 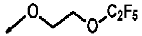 | 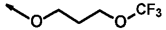 | 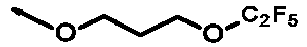 | 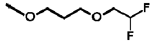 |
| Q-41 | Q-42 | Q-43 | Q-44 | Q-45 |
| | | | | |
|---|---|---|---|---|
| 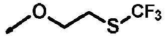 | 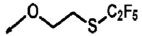 | 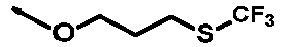 | 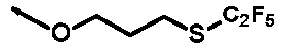 | 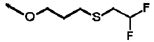 |
| Q-46 | Q-47 | Q-48 | Q-49 | Q-50 |
| | | | | |
|---|---|---|---|---|
| 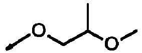 | 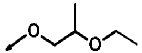 | 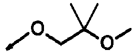 | 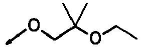 | 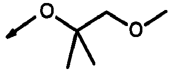 |
| Q-51 | Q-52 | Q-53 | Q-54 | Q-55 |
| | | | | |
|---|---|---|---|---|
| 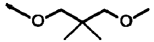 | 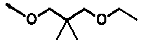 | 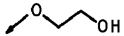 | 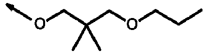 | 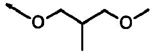 |
| Q-56 | Q-57 | Q-58 | Q-59 | Q-60 |
| | | | | |
|---|---|---|---|---|
| 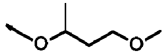 | 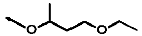 | 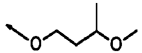 | 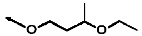 | 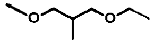 |
| Q-61 | Q-62 | Q-63 | Q-64 | Q-65 |
| | | | | |
|---|---|---|---|---|
| 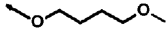 | 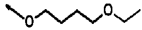 | 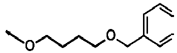 | 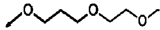 | 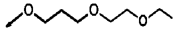 |
| Q-66 | Q-67 | Q-68 | Q-69 | Q-70 |
| | | | | |
|---|---|---|---|---|
| 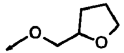 | 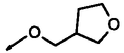 | 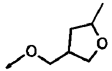 | 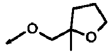 | 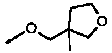 |
| Q-71 | Q-72 | Q-73 | Q-74 | Q-75 |
| | | | | |
|---|---|---|---|---|
| 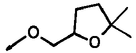 | 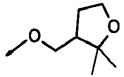 | 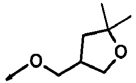 | 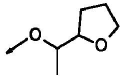 | 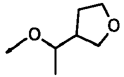 |
| Q-76 | Q-77 | Q-78 | Q-79 | Q-80 |
| | | | | |
|---|---|---|---|---|
| 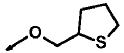 | 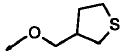 | 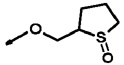 | 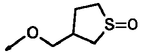 | 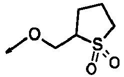 |
| Q-81 | Q-82 | Q-83 | Q-84 | Q-85 |
| | | | | |
|---|---|---|---|---|
| 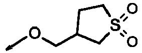 | 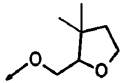 | 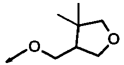 | 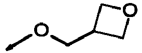 | 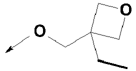 |
| Q-86 | Q-87 | Q-88 | Q-89 | Q-90 |
| | | | | |
|---|---|---|---|---|
| 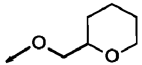 | 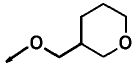 | 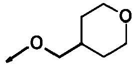 | 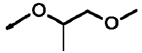 | 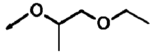 |
| Q-91 | Q-92 | Q-93 | Q-94 | Q-95 |
| | | | | |
|---|---|---|---|---|
| 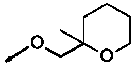 | 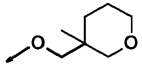 | 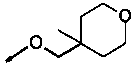 | 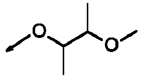 | 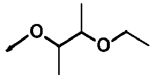 |
| Q-96 | Q-97 | Q-98 | Q-99 | Q-100 |
| | | | | |
|---|---|---|---|---|
| 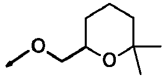 | 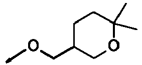 | 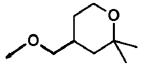 | 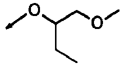 | 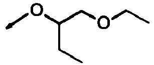 |
| Q-101 | Q-102 | Q-103 | Q-104 | Q-105 |
| | | | | |
|---|---|---|---|---|
| 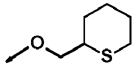 | 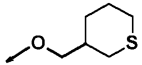 | 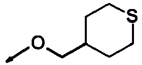 | 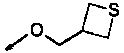 | 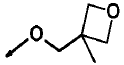 |
| Q-106 | Q-107 | Q-108 | Q-109 | Q-110 |
| | | | | |
|---|---|---|---|---|
| 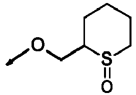 | 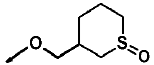 | 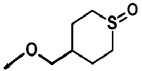 | 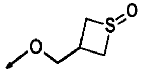 | 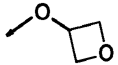 |
| Q-111 | Q-112 | Q-113 | Q-114 | Q-115 |
| | | | | |
|---|---|---|---|---|
| 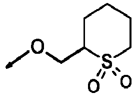 | 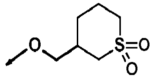 | 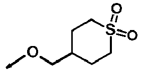 | 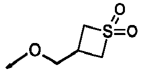 | 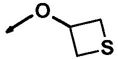 |
| Q-116 | Q-117 | Q-118 | Q-119 | Q-120 |
| | | | | |
|---|---|---|---|---|
| 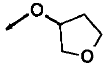 | 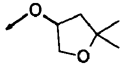 | 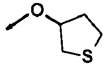 | 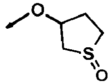 | 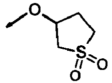 |
| Q-121 | Q-122 | Q-123 | Q-124 | Q-125 |
| | | | | |
|---|---|---|---|---|
| 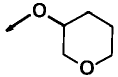 | 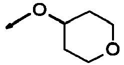 | 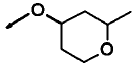 | 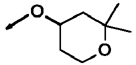 | 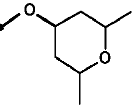 |
| Q-126 | Q-127 | Q-128 | Q-129 | Q-130 |
| | | | | |
|---|---|---|---|---|
| 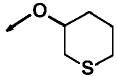 | 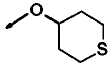 | 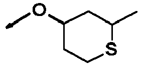 | 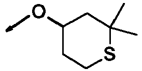 | 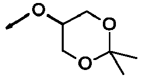 |
| Q-131 | Q-132 | Q-133 | Q-134 | Q-135 |
| | | | | |
|---|---|---|---|---|
| 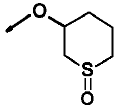 | 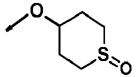 | 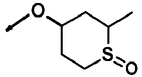 | 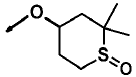 | 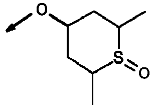 |
| Q-136 | Q-137 | Q-138 | Q-139 | Q-140 |
| | | | | |
|---|---|---|---|---|
| 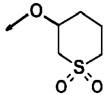 | 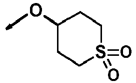 | 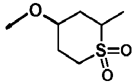 | 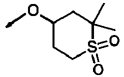 | 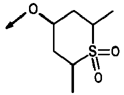 |
| Q-141 | Q-142 | Q-143 | Q-144 | Q-145 |
| | | | | |
|---|---|---|---|---|
| 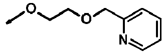 | 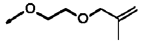 | 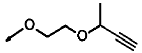 | 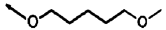 | 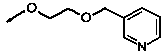 |
| Q-146 | Q-147 | Q-148 | Q-149 | Q-150 |
| | | | | |
|---|---|---|---|---|
| 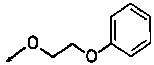 | 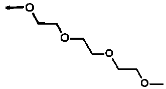 | 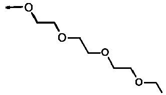 | 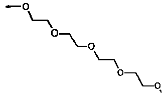 | 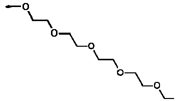 |
| Q-151 | Q-152 | Q-153 | Q-154 | Q-155 |
| | | | | |
|---|---|---|---|---|
| 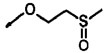 | 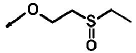 | 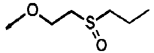 | 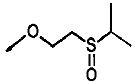 | 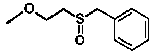 |
| Q-156 | Q-157 | Q-158 | Q-159 | Q-160 |
| | | | | |
|---|---|---|---|---|
| 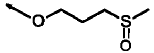 | 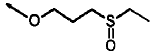 | 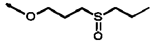 | 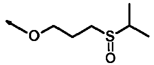 | 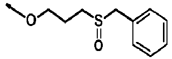 |
| Q-161 | Q-162 | Q-163 | Q-164 | Q-165 |
| | | | | |
|---|---|---|---|---|
| 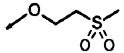 | 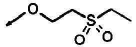 | 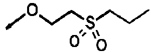 | 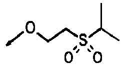 | 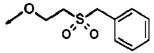 |
| Q-166 | Q-167 | Q-168 | Q-169 | Q-170 |
| | | | | |
|---|---|---|---|---|
| 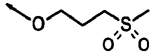 | 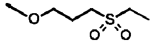 | 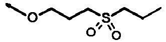 | 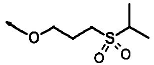 | 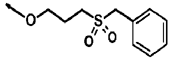 |
| Q-171 | Q-172 | Q-173 | Q-174 | Q-175 |
| | | | | |
|---|---|---|---|---|
| 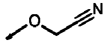 | 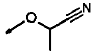 | 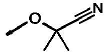 | 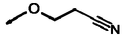 | 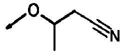 |
| Q-176 | Q-177 | Q-178 | Q-179 | Q-180 |
| | | | | |
|---|---|---|---|---|
| 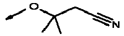 | 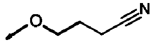 | 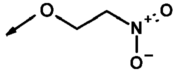 | 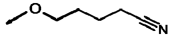 | 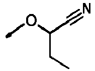 |
| Q-181 | Q-182 | Q-183 | Q-184 | Q-185 |
| | | | | |
|---|---|---|---|---|
| 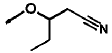 | 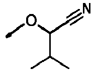 | 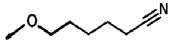 | 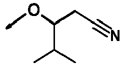 | 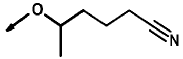 |
| 186 | 187 | 188 | 189 | 190 |
| | | | | |
|---|---|---|---|---|
| 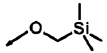 | 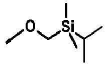 | 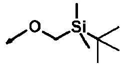 | 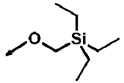 | 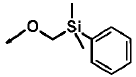 |
| Q-191 | Q-192 | Q-193 | Q-194 | Q-195 |
| | | | | |
|---|---|---|---|---|
| 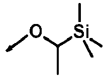 | 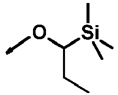 | 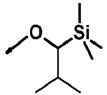 | 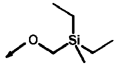 | 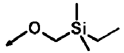 |
| Q-196 | Q-197 | Q-198 | Q-199 | Q-200 |
| | | | | |
|---|---|---|---|---|
| 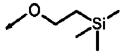 | 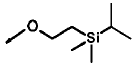 | 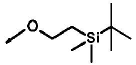 | 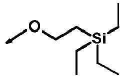 | 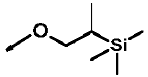 |
| Q-201 | Q-202 | Q-203 | Q-204 | Q-205 |
| | | | | |
|---|---|---|---|---|
| 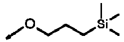 | 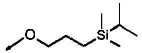 | 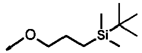 | 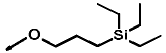 | 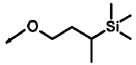 |
| Q-206 | Q-207 | Q-208 | Q-209 | Q-210 |
| | | | | |
|---|---|---|---|---|
| 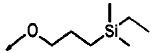 | 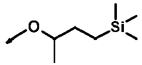 | 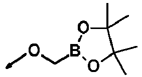 | 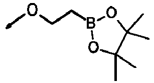 | 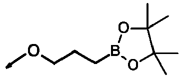 |
| Q-211 | Q-212 | Q-213 | Q-214 | Q-215 |
| | | | | |
|---|---|---|---|---|
| 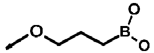 | 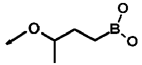 | 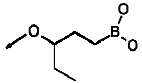 | 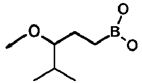 | 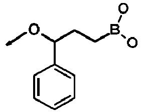 |
| Q-216 | Q-217 | Q-218 | Q-219 | Q-220 |
| | | | | |
|---|---|---|---|---|
| 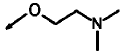 | 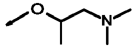 | 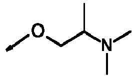 | 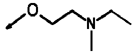 | 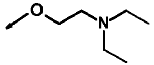 |
| Q-221 | Q-222 | Q-223 | Q-224 | Q-225 |
| | | | | |
|---|---|---|---|---|
| 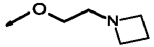 | 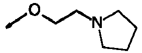 | 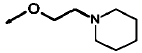 | 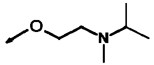 | 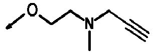 |
| Q-226 | Q-227 | Q-228 | Q-229 | Q-230 |
| | | | | |
|---|---|---|---|---|
| 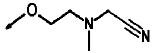 | 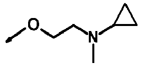 | 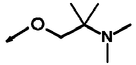 | 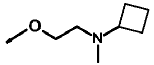 | 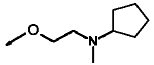 |
| Q-231 | Q-232 | Q-233 | Q-234 | Q-235 |
| | | | | |
|---|---|---|---|---|
| 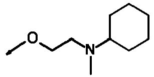 | 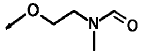 | 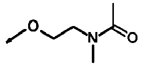 | 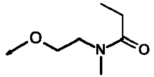 | 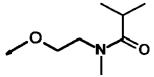 |
| Q-236 | Q-237 | Q-238 | Q-239 | Q-240 |
| | | | | |
|---|---|---|---|---|
| 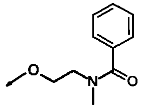 | 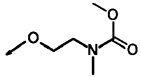 | 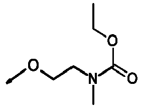 | 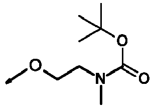 | 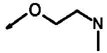 |
| Q-241 | Q-242 | Q-243 | Q-244 | Q-245 |
| | | | | |
|---|---|---|---|---|
| 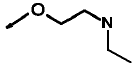 | 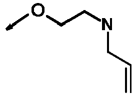 | 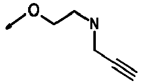 | 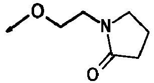 | 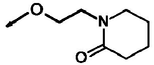 |
| Q-246 | Q-247 | Q-248 | Q-249 | Q-250 |
| | | | | |
|---|---|---|---|---|
| 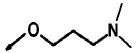 | 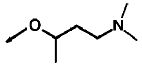 | 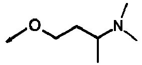 | 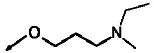 | 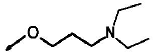 |
| Q-251 | Q-252 | Q-253 | Q-254 | Q-255 |
| | | | | |
|---|---|---|---|---|
| 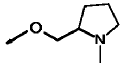 | 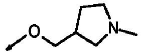 | 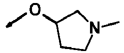 | 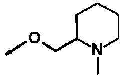 | 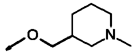 |
| Q-256 | Q-257 | Q-258 | Q-259 | Q-260 |
| | | | | |
|---|---|---|---|---|
| 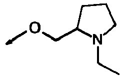 | 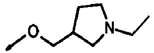 | 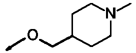 | 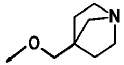 | 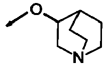 |
| Q-261 | Q-262 | Q-263 | Q-264 | Q-265 |
| | | | | |
|---|---|---|---|---|
| 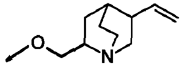 | 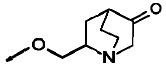 | 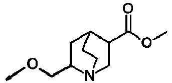 | 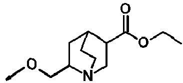 | 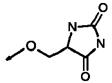 |
| Q-266 | Q-267 | Q-268 | Q-269 | Q-270 |
| | | | | |
|---|---|---|---|---|
| 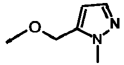 | 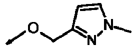 | 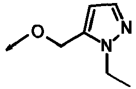 | 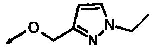 | 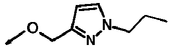 |
| Q-271 | Q-272 | Q-273 | Q-274 | Q-275 |
| | | | | |
|---|---|---|---|---|
| 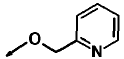 | 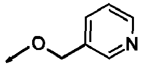 | 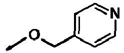 | 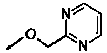 | 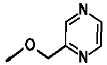 |
| Q-276 | Q-277 | Q-278 | Q-279 | Q-280 |
| | | | | |
|---|---|---|---|---|
| 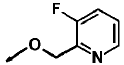 | 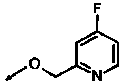 | 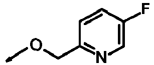 | 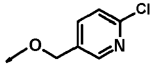 | 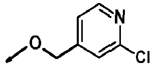 |
| Q-281 | Q-282 | Q-283 | Q-284 | Q-285 |
| | | | | |
|---|---|---|---|---|
| 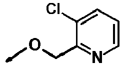 | 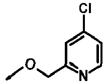 | 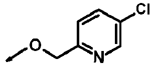 | 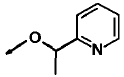 | 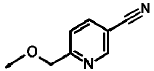 |
| Q-286 | Q-287 | Q-288 | Q-289 | Q-290 |
| | | | | |
|---|---|---|---|---|
| 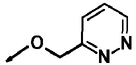 | 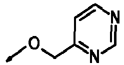 | 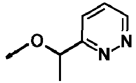 | 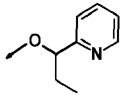 | 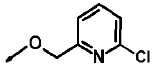 |
| Q-291 | Q-292 | Q-293 | Q-294 | Q-295 |
| | | | | |
|---|---|---|---|---|
| 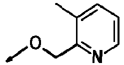 | 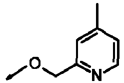 | 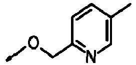 | 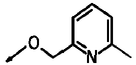 | 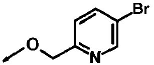 |
| Q-296 | Q-297 | Q-298 | Q-299 | Q-300 |
| | | | | |
|---|---|---|---|---|
| 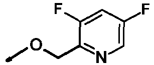 | 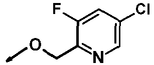 | 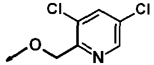 | 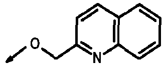 | 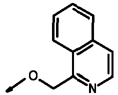 |
| Q-301 | Q-302 | Q-303 | Q-304 | Q-305 |
| | | | | |
|---|---|---|---|---|
| 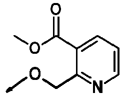 | 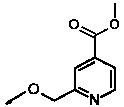 | 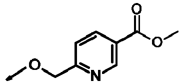 | 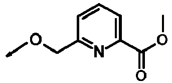 | 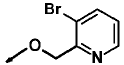 |
| Q-306 | Q-307 | Q-308 | Q-309 | Q-310 |
| | | | | |
|---|---|---|---|---|
| 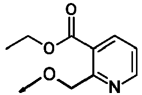 | 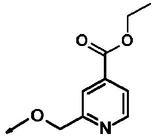 | 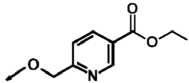 | 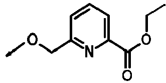 | 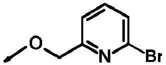 |
| Q-311 | Q-312 | Q-313 | Q-314 | Q-315 |
| | | | | |
|---|---|---|---|---|
| 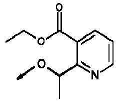 | 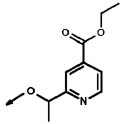 | 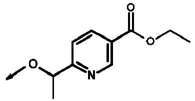 | 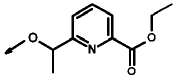 | 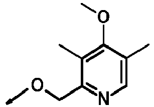 |
| Q-316 | Q-317 | Q-318 | Q-319 | Q-320 |
| | | | | |
|---|---|---|---|---|
| 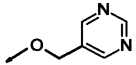 | 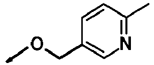 | 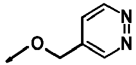 | 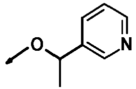 | 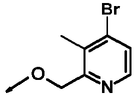 |
| Q-321 | Q-322 | Q-323 | Q-324 | Q-325 |
| | | | | |
|---|---|---|---|---|
| 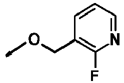 | 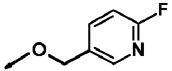 | 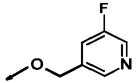 | 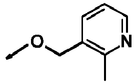 | 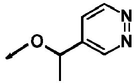 |
| Q-326 | Q-327 | Q-328 | Q-329 | Q-330 |
| | | | | |
|---|---|---|---|---|
| 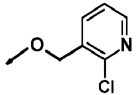 | 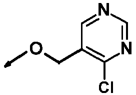 | 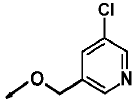 | 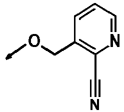 | 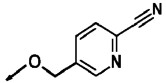 |
| Q-331 | Q-332 | Q-333 | Q-334 | Q-335 |
| | | | | |
|---|---|---|---|---|
| 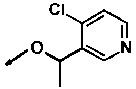 | 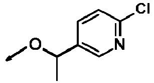 | 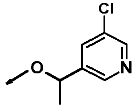 | 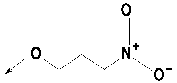 | 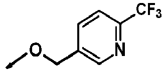 |
| Q-336 | Q-337 | Q-338 | Q-339 | Q-340 |
| | | | | |
|---|---|---|---|---|
| 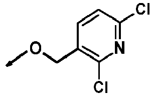 | 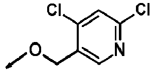 | 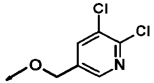 | 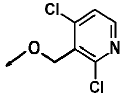 | 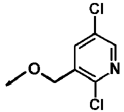 |
| Q-341 | Q-342 | Q-343 | Q-344 | Q-345 |
| | | | | |
|---|---|---|---|---|
| 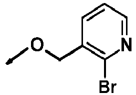 | 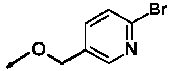 | 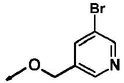 | 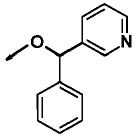 | 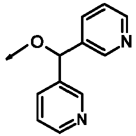 |
| Q-346 | Q-347 | Q-348 | Q-349 | Q-350 |
| | | | | |
|---|---|---|---|---|
| 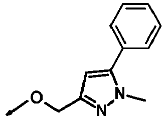 | 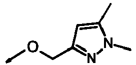 | 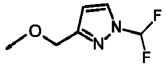 | 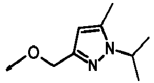 | 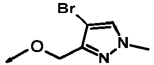 |
| Q-351 | Q-352 | Q-353 | Q-354 | Q-355 |
| | | | | |
|---|---|---|---|---|
| 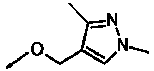 | 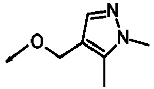 | 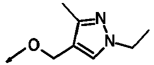 | 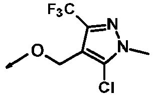 | 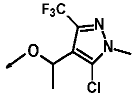 |
| Q-356 | Q-357 | Q-358 | Q-359 | Q-360 |
| | | | | |
|---|---|---|---|---|
| 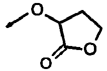 | 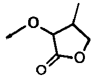 | 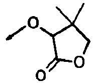 | 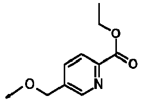 | 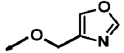 |
| Q-361 | Q-362 | Q-363 | Q-364 | Q-365 |
| | | | | |
|---|---|---|---|---|
| 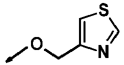 | 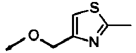 | 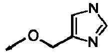 | 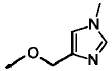 | 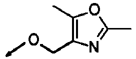 |
| Q-366 | Q-367 | Q-368 | Q-369 | Q-370 |
| | | | | |
|---|---|---|---|---|
| 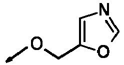 | 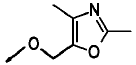 | 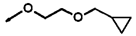 | 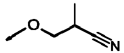 | 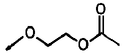 |
| Q-371 | Q-372 | Q-373 | Q-374 | Q-375 |
| | | | | |
|---|---|---|---|---|
| 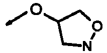 | 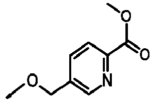 | 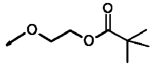 | 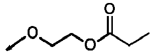 | 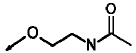 |
| Q-376 | Q-377 | Q-378 | Q-379 | Q-380 |
| | | | | |
|---|---|---|---|---|
| 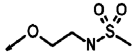 | 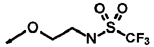 | 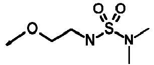 | 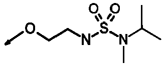 | 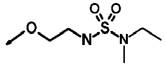 |
| Q-381 | Q-382 | Q-383 | Q-384 | Q-385 |
| | | | | |
|---|---|---|---|---|
| 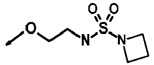 | 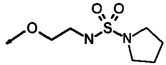 | 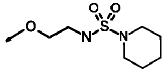 | 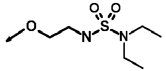 | 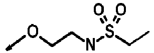 |
| Q-386 | Q-387 | Q-388 | Q-389 | Q-390 |
| | | | | |
|---|---|---|---|---|
| 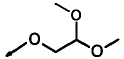 | 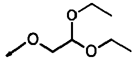 | 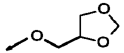 | 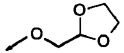 | 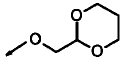 |
| Q-391 | Q-392 | Q-393 | Q-394 | Q-395 |
| | | | | |
|---|---|---|---|---|
| 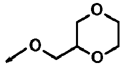 | 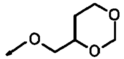 | 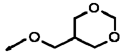 | 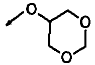 | 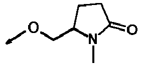 |
| Q-396 | Q-397 | Q-398 | Q-399 | Q-400 |
| | | | | |
|---|---|---|---|---|
| 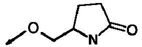 | 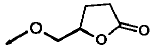 | 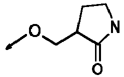 | 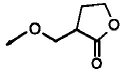 | 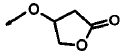 |
| Q-401 | Q-402 | Q-403 | Q-404 | Q-405 |
| |
|---|
| 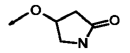 |
| Q-406 |

4. Compounds of the general formula (I) according to Claim 1 and/or salt thereof, **characterized in that**
R¹ is hydrogen, fluorine, chlorine, bromine, iodine, cyano, methyl, ethyl, trifluoromethyl, difluoromethyl, methoxy, ethoxy, difluoromethoxy, trifluoromethoxy,
R² is fluorine,
R³ is hydrogen, fluorine, chlorine, bromine, methoxy,
R⁴ is fluorine, chlorine, bromine, cyano, NO₂, C(O)NH₂, C(S)NH₂, trifluoromethyl, ethynyl, propyn-1-yl,
R⁵, R⁶, R⁷ are independently hydrogen, fluorine, chlorine, bromine, iodine, cyano, methyl, ethyl, trifluoromethyl, difluoromethyl, methoxy, ethoxy, difluoromethoxy, trifluoromethoxy,
G is methylene, (methyl)methylene, (ethyl)methylene, (dimethyl)methylene, ethylene, n-propylene, (1-methyl)ethyl-1-ene, (2-methyl)ethyl-1-ene, n-butylene, 1-methylpropyl-1-ene, 2-methylpropyl-1-ene, 3-methylpropyl-1-ene, 1,1-dimethylethyl-1-ene, 2,2-dimethylethyl-1-ene, 1-ethylethyl-1-ene, 2-ethylethyl-1-ene, 1-(prop-1-yl)ethyl-1-ene, 2-(prop-1-yl)ethyl-1-ene, 1-(prop-2-yl)ethyl-1-ene, 2-(prop-2-yl)ethyl-1-ene, n-pentylene, 1-methylbutyl-1-ene, 2-methylbutyl-1-ene, 3-methylbutyl-1-ene, 4-methylbutyl-1-ene, 1,1-dimethylpropyl-1-ene, 2,2-dimethylpropyl-1-ene, 3,3-dimethylpropyl-1-ene, 1,2-dimethylpropyl-1-ene, 1,3-dimethylpropyl-1-ene, 1-ethylpropyl-1-ene, n-hexylene,
X and Y are independently O (oxygen) or S (sulfur)
and Q is one of the moieties Q-1 to Q-406 specifically mentioned in Claim 3.

5. Compounds of the general formula (I) according to Claim 1 and/or salt thereof, **characterized in that**
R¹ is hydrogen, fluorine, chlorine, bromine, cyano, methyl, trifluoromethyl, methoxy, trifluoromethoxy,
R² is fluorine,
R³ is fluorine,
R⁴ is chlorine, bromine, cyano, NO₂, C(O)NH₂, C(S)NH₂,
R⁵, R⁶ and R⁷ are independently hydrogen, fluorine, chlorine, bromine, cyano, methyl, trifluoromethyl, methoxy, trifluoromethoxy,
G is methylene, (methyl)methylene, (ethyl)methylene, (dimethyl)methylene, ethylene, n-propylene, (1-methyl)ethyl-1-ene, (2-methyl)ethyl-1-ene, n-butylene, 1-methylpropyl-1-ene, 2-methylpropyl-1-ene, 3-methylpropyl-1-ene, n-pentylene, n-hexylene,
X and Y are independently O (oxygen) or S (sulfur)
and Q is one of the moieties Q-1 to Q-406 specifically mentioned in Claim 3.

6. Compounds of the general formula (I) according to Claim 1 and/or salt thereof, **characterized in that**
R¹ is hydrogen,
R² is fluorine,
R³ is fluorine,
R⁴ is chlorine, bromine, cyano, NO₂,
R⁵ is hydrogen, fluorine, chlorine, bromine,
R⁶ is hydrogen, fluorine, chlorine, bromine, cyano,
R⁷ is hydrogen,
G is methylene, (methyl)methylene,
X is O (oxygen) or S (sulfur),
Y is O (oxygen)
and Q is one of the moieties Q-1 to Q-406 specifically mentioned in Claim 3.

7. Compounds of the general formula (I) according to Claim 1 and/or salt thereof, **characterized in that**
R¹ is hydrogen,
R² is fluorine,
R³ is fluorine,
R⁴ is chlorine, bromine, cyano, NO₂,
R⁵ is hydrogen, fluorine,
R⁶ is hydrogen, fluorine, bromine, cyano,
R⁷ is hydrogen,
G is methylene, (methyl)methylene,
X is O (oxygen) or S (sulfur),
Y is O (oxygen)
and
Q is one of the moieties Q-1 to Q-35, Q-41 to Q-45, Q-58, Q-71 to Q-80, Q-89, Q-94, Q-95, Q-115, Q-120 to Q-123, Q-152 to Q-155, Q-166 to Q-170, Q-176 to Q-190, Q-261 to Q-348, Q-352 to Q-372, Q-377, Q-391-Q-399 specifically mentioned in Claim 3.

8. Compounds of the general formula (I) according to Claim 1 and/or salt thereof, **characterized in that**
R¹ is hydrogen,
R² is fluorine,
R³ is fluorine,
R⁴ is chlorine, bromine, cyano, NO₂,
R⁵ is hydrogen, fluorine,
R⁶ is hydrogen, fluorine, bromine, cyano,
R⁷ is hydrogen,
G is methylene, (methyl)methylene,
X is O (oxygen) or S (sulfur),
Y is O (oxygen)
and
Q is one of the moieties Q-1, Q-2, Q-3, Q-4, Q-7, Q-8, Q-9, Q-17, Q-18, Q-23, Q-24, Q-26, Q-27, Q-41, Q-42, Q-43, Q-58, Q-71, Q-72, Q-89, Q-94, Q-115, Q-121, Q-176, Q-177, Q-179, Q-183, Q-272, Q-274, Q-275, Q-276, Q-277, Q-278, Q-281, Q-282, Q-283, Q-284, Q-286, Q-288, Q-291, Q-296, Q-301, Q-302, Q-303, Q-308, Q-309, Q-321, Q-327, Q-328, Q-329, Q-331, Q-335, Q-339, Q-356, Q-365, Q-366, Q-367, Q-371, Q-394 specifically mentioned in Claim 3.

9. Use of one or more compounds of the general formula (I) as defined in any of Claims 1 to 8 and/or salts thereof, as herbicide and/or plant growth regulator, preferably in crops of useful plants and/or ornamentals.

10. Herbicidal and/or plant growth-regulating composition, **characterized in that** the composition comprises one or more compounds of the general formula (I) as defined in any of Claims 1 to 8 and/or salts thereof, and furthermore one or more substances selected from groups (i) and/or (ii), comprising
(i) one or more further agrochemically active substances, preferably selected from the group consisting of insecticides, acaricides, nematicides, further herbicides, fungicides, safeners, fertilizers and/or further growth regulators,
(ii) one or more formulation auxiliaries customary in crop protection.

11. Method of controlling harmful plants or for regulating the growth of plants, **characterized in that** an effective amount
- of one or more compounds of the general formula (I), as defined in any of Claims 1 to 8 and/or salts thereof, or
- of a composition according to Claim 10 is applied to the plants, seeds of plants, the soil in which or on which the plants grow or the area under cultivation.

## Revendications

1. N-phényluraciles substitués de formule générale (I) ou leurs sels 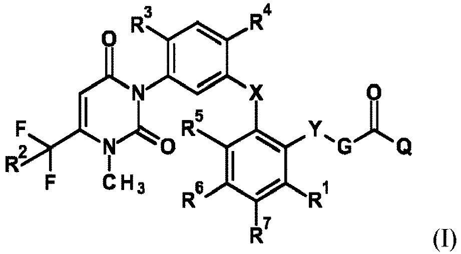 dans laquelle
R¹ représente un hydrogène, un halogène, un groupe cyano, alkyle en C₁-C₈, halogénoalkyle en C₁-C₈, alcoxy en C₁-C₈, halogénoalcoxy en C₁-C₈,
R² représente un hydrogène, un fluor, un chlore, un brome, un trifluorométhyle, un alcoxy en C₁-C₈,
R³ représente un hydrogène, un halogène, un alcoxy en C₁-C₈,
R⁴ représente un halogène, un groupe cyano, NO₂, C(O)NH₂, C(S)NH₂, halogénoalkyle en C₁-C₈, alcynyle en C₂-C₈,
R⁵, R⁶ et R⁷ représentent indépendamment les uns des autres un hydrogène, un halogène, un groupe cyano, alkyle en C₁-C₈, halogénoalkyle en C₁-C₈, alcoxy en C₁-C₈, halogénoalcoxy en C₁-C₈,
G représente un groupe alkylène en C₁-C₈ non ramifié ou ramifié,
Q représente un radical de formule 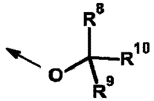
R⁸ représente un hydrogène, un groupe alkyle en C₁-C₈, halogénoalkyle en C₁-C₈, aryle, aryl-alkyle en C₁-C₈, hétéroaryle, alcynyle en C₂-C₈, alcényle en C₂-C₈, C(O)R¹³, C(O)OR¹³, alcoxy en C₁-C₈-alkyle en C₁-C₈,
R⁹ représente un hydrogène ou un alkyle en C₁-C₈,
R¹⁰ représente un groupe cyano, NO₂, hétéroaryle, hétéroaryl-alkyle en C₁-C₈, hétérocyclyle, hétérocyclyl-alkyle en C₁-C₈, R¹¹R¹²N-alkyle en C₁-C₈, R¹³O-alkyle en C₁-C₈, cyano-alkyle en C₁-C₈, alkyle en C₁-C₈-carbonyloxy-alkyle en C₁-C₈, cycloalkyle en C₃-C₈-carbonyloxy-alkyle en C₁-C₈, arylcarbonyloxy-alkyle en C₁-C₈, hétéroarylcarbonyloxy-alkyle en C₁-C₈, hétérocyclylcarbonyloxy-alkyle en C₁-C₈, OR¹³, NR¹¹R¹², SR¹⁴, S(O)R¹⁴, SO₂R¹⁴, R¹⁴S-alkyle en C₁-C₈, R¹⁴(O)S-alkyle en C₁-C₈, R¹⁴O₂S-alkyle en C₁-C₈, tris- [alkyle en C₁-C₈]silyl-alkyle en C₁-C₈, bis- [alkyle en C₁-C₈](aryl) silylalkyle en C₁-C₈, [alkyle en C₁-C₈]-bis-(aryl)silylalkyle en C₁-C₈, tris-[alkyle en C₁-C₈]silyle, bis-hydroxyboryl-alkyle en C₁-C₈, bis- [alcoxy en C₁-C₈]boryl-alkyle en C₁-C₈, tétraméthyl-1,3,2-dioxaborolan-2-yle, tétraméthyl-1,3,2-dioxaborolan-2-yl-alkyle en C₁-C₈, nitro-alkyle en C₁-C₈, C(O)R¹⁴, bis-alcoxy en C₁-C₈-méthyle, bis-alcoxy en C₁-C₈-méthyl-alkyle en C₁-C₈, ou
R⁸ et R¹⁰, avec l'atome de carbone auquel ils sont liés, forment un hétérocyclyle monocyclique ou bicyclique à 3 à 10 chaînons, entièrement saturé ou partiellement saturé ou éventuellement encore plus substitué,
R¹¹ et R¹² sont identiques ou différents et représentent indépendamment l'un de l'autre un hydrogène, un groupe alkyle en C₁-C₈, alcényle en C₂-C₈, alcynyle en C₂-C₈, cyanoalkyle en C₁-C₈, halogénoalkyle en C₁-C₁₀, halogénoalcényle en C₂-C₈, halogénoalcynyle en C₃-C₈, cycloalkyle en C₃-C₁₀, halogénocycloalkyle en C₃-C₁₀, cycloalcényle en C₄-C₁₀, halogénocycloalcényle en C₄-C₁₀, alcoxy en C₁-C₈-alkyle en C₁-C₈, halogénoalcoxy en C₁-C₈-alkyle en C₁-C₈, alkyle en C₁-C₈-thio-alkyle en C₁-C₈, halogénoalkyle en C₁-C₈-thio-alkyle en C₁-C₈, alcoxy en C₁-C₈-halogénoalkyle en C₁-C₈, aryle, aryl-alkyle en C₁-C₈, hétéroaryle, hétéroaryl-alkyle en C₁-C₈, cycloalkyle en C₃-C₈-alkyle en C₁-C₈, cycloalcényle en C₄-C₁₀-alkyle en C₁-C₈, COR¹³, SO₂R¹⁴, hétérocyclyle, alcoxy en C₁-C₈-carbonyle, bis- [alkyle en C₁-C₈] aminocarbonyl-alkyle en C₁-C₈, alkyle en C₁-C₈-aminocarbonyl-alkyle en C₁-C₈, aryl-alkyle en C₁-C₈-aminocarbonyl-alkyle en C₁-C₈, aryl-alcoxy en C₁-C₈-carbonyle, hétéroaryl-alcoxy en C₁-C₈-carbonyle, alcényloxy en C₂-C₈-carbonyle, alcynyloxy en C₂-C₈-carbonyle, hétérocyclyl-alkyle en C₁-C₈, ou
R¹¹ et R¹², avec l'atome d'azote auquel ils sont liés, forment un cycle monocyclique ou bicyclique à 3 à 10 chaînons, entièrement saturé ou partiellement saturé, éventuellement interrompu par des hétéroatomes et éventuellement encore plus substitué,
R¹³ représente un hydrogène, un groupe alkyle en C₁-C₈, alcényle en C₂-C₈, alcynyle en C₂-C₈, cyanoalkyle en C₁-C₈, halogénoalkyle en C₁-C₁₀, halogénoalcényle en C₂-C₈, halogénoalcynyle en C₃-C₈, cycloalkyle en C₃-C₁₀, halogénocycloalkyle en C₃-C₁₀, cycloalcényle en C₄-C₁₀, halogénocycloalcényle en C₄-C₁₀, alcoxy en C₁-C₈-alkyle en C₁-C₈, halogénoalcoxy en C₁-C₈-alkyle en C₁-C₈, alcoxy en C₁-C₈-halogénoalkyle en C₁-C₈, alcoxy en C₁-C₈-alcoxy en C₁-C₈-alkyle en C₁-C₈, alcoxy en C₁-C₈-alcoxy en C₁-C₈-alcoxy en C₁-C₈-alkyle en C₁-C₈, alcoxy en C₁-C₈-alcoxy en C₁-C₈-alcoxy en C₁-C₈-alcoxy en C₁-C₈-alkyle en C₁-C₈, aryle, aryl-alkyle en C₁-C₈, aryl-alcoxy en C₁-C₈-alkyle en C₁-C₈, hétéroaryle, hétéroaryl-alkyle en C₁-C₈, cycloalkyle en C₃-C₈-alkyle en C₁-C₈, cycloalcényle en C₄-C₁₀-alkyle en C₁-C₈, bis- [alkyle en C₁-C₈]aminocarbonyl-alkyle en C₁-C₈, alkyle en C₁-C₈-aminocarbonyl-alkyle en C₁-C₈, aryl-alkyle en C₁-C₈-aminocarbonyl-alkyle en C₁-C₈, bis- [alkyle en C₁-C₈]amino-alkyle en C₂-C₆, alkyle en C₁-C₈-amino-alkyle en C₂-C₆, aryl-alkyle en C₁-C₈-amino-alkyle en C₂-C₆, R¹⁴S-alkyle en C₁-C₈, R¹⁴(O)S-alkyle en C₁-C₈, R¹⁴O₂S-alkyle en C₁-C₈, hydroxycarbonyl-alkyle en C₁-C₈, hétérocyclyle, hétérocyclyl-alkyle en C₁-C₈, tris- [alkyle en C₁-C₈]silyl-alkyle en C₁-C₈, bis- [alkyle en C₁-C₈](aryl)silylalkyle en C₁-C₈, [alkyle en C₁-C₈]-bis-(aryl)silylalkyle en C₁-C₈, alkyle en C₁-C₈-carbonyloxy-alkyle en C₁-C₈, cycloalkyle en C₃-C₈-carbonyloxy-alkyle en C₁-C₈, arylcarbonyloxy-alkyle en C₁-C₈, hétéroarylcarbonyloxy-alkyle en C₁-C₈, hétérocyclylcarbonyloxy-alkyle en C₁-C₈, aryloxy-alkyle en C₁-C₈, hétéroaryloxy-alkyle en C₁-C₈, alcoxy en C₁-C₈-carbonyle,
R¹⁴ représente un hydrogène, un groupe alkyle en C₁-C₈, alcényle en C₂-C₈, alcynyle en C₂-C₈, cyanoalkyle en C₁-C₈, halogénoalkyle en C₁-C₁₀, halogénoalcényle en C₂-C₈, halogénoalcynyle en C₃-C₈, cycloalkyle en C₃-C₁₀, halogénocycloalkyle en C₃-C₁₀, cycloalcényle en C₄-C₁₀, halogénocycloalcényle en C₄-C₁₀, alcoxy en C₁-C₈-alkyle en C₁-C₈, alcoxy en C₁-C₈-halogénoalkyle en C₁-C₈, aryle, aryl-alkyle en C₁-C₈, hétéroaryle, hétéroaryl-alkyle en C₁-C₈, hétérocyclyl-alkyle en C₁-C₈, cycloalkyle en C₃-Ce-alkyle en C₁-C₈, cycloalcényle en C₄-C₁₀-alkyle en C₁-C₈, bis- [alkyle en C₁-C₈]amino, alkyle en C₁-C₈-amino, aryl- (C₁-C₈)-amino, aryl-alkyle en C₁-C₆-amino, aryl-[alkyle en C₁-C₈]amino ; cycloalkyle en C₃-C₈-amino, cycloalkyle en C₃-C₈-[alkyle en C₁-C₈]amino ; N-azétidinyle, N-pyrrolidinyle, N-pipéridinyle, N-morpholinyle,
et
X et Y représentent indépendamment l'un de l'autre O (oxygène) ou S (soufre).

2. Composés de formule générale (I) selon la revendication 1 et/ou leur sel, **caractérisés en ce que** R¹ représente un hydrogène, un halogène, un groupe cyano, alkyle en C₁-C₆, halogénoalkyle en C₁-C₆, alcoxy en C₁-C₆, halogénoalcoxy en C₁-C₆,
R² représente un hydrogène, un fluor, un chlore, un brome, un trifluorométhyle, un alcoxy en C₁-C₆,
R³ représente un hydrogène, un halogène, un alcoxy en C₁-C₆,
R⁴ représente un halogène, un groupe cyano, NO₂, C(O)NH₂, C(S)NH₂, halogénoalkyle en C₁-C₆, alcynyle en C₂-C₆,
R⁵, R⁶ et R⁷ représentent indépendamment l'un de l'autre un hydrogène, un halogène, un groupe cyano, alkyle en C₁-C₆, halogénoalkyle en C₁-C₆, alcoxy en C₁-C₆, halogénoalcoxy en C₁-C₆,
G représente un alkylène en C₁-C₆ non ramifié ou ramifié,
Q représente un radical de formule 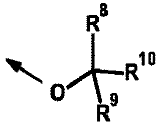
R⁸ représente un hydrogène, un groupe alkyle en C₁-C₆, halogénoalkyle en C₁-C₆, aryle, aryl-alkyle en C₁-C₆, hétéroaryle, alcynyle en C₂-C₆, alcényle en C₂-C₆, C(O)R¹³, C(O)OR¹³, alcoxy en C₁-C₆-alkyle en C₁-C₆,
R⁹ représente un hydrogène ou un alkyle en C₁-C₄,
R¹⁰ représente un groupe cyano, NO₂, hétéroaryle, hétéroaryl-alkyle en C₁-C₆, hétérocyclyle, hétérocyclyl-alkyle en Ci-Ce, R¹¹R¹²N-alkyle en C₁-C₆, R¹³O-alkyle en C₁-C₆, cyano-alkyle en C₁-C₆, alkyle en C₁-C₆-carbonyloxy-alkyle en C₁-C₆, cycloalkyle en C₃-C₆-carbonyloxy-alkyle en C₁-C₆, arylcarbonyloxy-alkyle en C₁-C₆, hétéroarylcarbonyloxy-alkyle en C₁-C₆, hétérocyclylcarbonyloxy-alkyle en C₁-C₆, OR¹³, NR¹¹R¹², SR¹⁴, S(O)R¹⁴, SO₂R¹⁴, R¹⁴S-alkyle en C₁-C₆, R¹⁴(O)S-alkyle en C₁-C₆, R¹⁴O₂S-alkyle en C₁-C₆, tris- [alkyle en C₁-C₆]silyl-alkyle en C₁-C₆, bis- [alkyle en C₁-C₆](aryl) silyl-alkyle en C₁-C₆, [alkyle en C₁-C₆]-bis- (aryl) silyl-alkyle en C₁-C₆, tris- [alkyle en C₁-C₆]silyle, bis-hydroxyboryl-alkyle en C₁-C₆, bis- [alcoxy en C₁-C₆]boryl-alkyle en C₁-C₆, tétraméthyl-1,3,2-dioxaborolan-2-yle, tétraméthyl-1,3,2-dioxaborolan-2-yl-alkyle en C₁-C₆, nitro-alkyle en C₁-C₆, C(O)R¹³, bis-alcoxy en C₁-C₆-méthyle, bis-alcoxy en C₁-C₆-méthyl-alkyle en C₁-C₆,
R⁸ et R¹⁰, avec l'atome de carbone auquel ils sont liés, forment un hétérocyclyle monocyclique ou bicyclique à 3 à 10 chaînons, entièrement saturé ou partiellement saturé et éventuellement encore plus substitué,
R¹¹ et R¹² sont identiques ou différents et représentent indépendamment l'un de l'autre un hydrogène, un groupe alkyle en C₁-C₆, alcényle en C₂-C₆, alcynyle en C₂-C₆, cyanoalkyle en C₁-C₆, halogénoalkyle en C₁-C₁₀, halogénoalcényle en C₂-C₆, halogénoalcynyle en C₃-C₆, cycloalkyle en C₃-C₁₀, halogénocycloalkyle en C₃-C₁₀, cycloalcényle en C₄-C₁₀, halogénocycloalcényle en C₄-C₁₀, alcoxy en C₁-C₆-alkyle en C₁-C₆, halogénoalcoxy en C₁-C₆-alkyle en C₁-C₆, alkyle en C₁-C₆- thio-alkyle en C₁-C₆, halogénoalkyle en C₁-C₆-thio-alkyle en C₁-C₆, alcoxy en C₁-C₆-halogénoalkyle en C₁-C₆, aryle, aryl-alkyle en C₁-C₆, hétéroaryle, hétéroaryl-alkyle en C₁-C₆, cycloalkyle en C₃-C₆-alkyle en C₁-C₆, cycloalcényle en C₄-C₁₀-alkyle en C₁-C₆, COR¹³, SO₂R¹⁴, hétérocyclyle, alcoxy en C₁-C₆-carbonyle, bis- [alkyle en C₁-C₆] aminocarbonyl -alkyle en C₁-C₆, alkyle en C₁-C₆-aminocarbonyl-alkyle en C₁-C₆, aryl-alkyle en C₁-C₆-aminocarbonyl-alkyle en C₁-C₆, aryl-alcoxy en C₁-C₆-carbonyle, hétéroaryl-alcoxy en C₁-C₆-carbonyle, alcényloxy en C₂-C₆-carbonyle, alcynyloxy en C₂-C₆-carbonyle, hétérocyclyl-alkyle en C₁-C₆, ou
R¹¹ et R¹², avec l'atome d'azote auquel ils sont liés, forment un cycle monocyclique ou bicyclique à 3 à 10 chaînons, entièrement saturé ou partiellement saturé, éventuellement interrompu par des hétéroatomes et éventuellement encore plus substitué,
R¹³ représente un hydrogène, un groupe alkyle en C₁-C₆, alcényle en C₂-C₆, alcynyle en C₂-C₆, cyanoalkyle en C₁-C₆, halogénoalkyle en C₁-C₁₀, halogénoalcényle en C₂-C₆, halogénoalcynyle en C₃-C₆, cycloalkyle en C₃-C₁₀, halogénocycloalkyle en C₃-C₁₀, cycloalcényle en C₄-C₁₀, halogénocycloalcényle en C₄-C₁₀, alcoxy en C₁-C₆-alkyle en C₁-C₆, halogénoalcoxy en C₁-C₆-alkyle en C₁-C₆, alcoxy en C₁-C₆-halogénoalkyle en C₁-C₆, alcoxy en C₁-C₆-alcoxy en C₁-C₆-alkyle en C₁-C₆, alcoxy en C₁-C₆-alcoxy en C₁-C₆-alcoxy en C₁-C₆-alkyle en C₁-C₆, alcoxy en C₁-C₆-alcoxy en C₁-C₆-alcoxy en C₁-C₆-alcoxy en C₁-C₆-alkyle en C₁-C₆, aryle, aryl-alkyle en C₁-C₆, aryl-alcoxy en C₁-C₆-alkyle en C₁-C₆, hétéroaryle, hétéroaryl-alkyle en C₁-C₆, cycloalkyle en C₃-C₆-alkyle en C₁-C₆, cycloalcényle en C₄-C₁₀-alkyle en C₁-C₆, bis- [alkyle en C₁-C₆]aminocarbonyl-alkyle en C₁-C₆, alkyle en C₁-C₆-aminocarbonyl-alkyle en C₁-C₆, aryl-alkyle en C₁-C₆-aminocarbonyl-alkyle en C₁-C₆, bis- [alkyle en C₁-C₆]amino-alkyle en C₂-C₄, alkyle en C₁-C₆-amino-alkyle en C₂-C₄, aryl-alkyle en C₁-C₆-amino-alkyle en C₂-C₄, R¹⁴S-alkyle en Ci-Ce, R¹⁴ (O) S-alkyle en C₁-C₆, R¹⁴O₂S-alkyle en C₁-C₆, hydroxycarbonyl-alkyle en C₁-C₆, hétérocyclyle, hétérocyclyl-alkyle en C₁-C₆, tris- [alkyle en C₁-C₆]silyl-alkyle en C₁-C₆, bis- [alkyle en C₁-C₆](aryl) silyl-alkyle en C₁-C₆, [alkyle en C₁-C₆]-bis-(aryl)silyl-alkyle en C₁-C₆, alkyle en C₁-C₆-carbonyloxy-alkyle en C₁-C₆, cycloalkyle en C₃-C₆-carbonyloxy-alkyle en C₁-C₆, arylcarbonyloxy-alkyle en C₁-C₆, hétéroarylcarbonyloxy-alkyle en C₁-C₆, hétérocyclylcarbonyloxy-alkyle en C₁-C₆, aryloxy-alkyle en C₁-C₆, hétéroaryloxy-alkyle en C₁-C₆, alcoxy en C₁-C₆-carbonyle,
R¹⁴ représente un hydrogène, un groupe alkyle en C₁-C₆, alcényle en C₂-C₆, alcynyle en C₂-C₆, cyanoalkyle en C₁-C₆, halogénoalkyle en C₁-C₁₀, halogénoalcényle en C₂-C₆, halogénoalcynyle en C₃-C₆, cycloalkyle en C₃-C₁₀, halogénocycloalkyle en C₃-C₁₀, cycloalcényle en C₄-C₁₀, halogénocycloalcényle en C₄-C₁₀, alcoxy en C₁-C₆-alkyle en C₁-C₆, alcoxy en C₁-C₆-halogénoalkyle en C₁-C₆, aryle, aryl-alkyle en C₁-C₆, hétéroaryle, hétéroaryl-alkyle en C₁-C₆, hétérocyclyl-alkyle en C₁-C₆, cycloalkyle en C₃-C₆-alkyle en C₁-C₆, cycloalcényle en C₄-C₁₀-alkyle en C₁-C₆, bis- [alkyle en C₁-C₆]amino, alkyle en C₁-C₆-amino, aryl- (C₁-C₆) -amino, aryl-alkyle en C₁-C₂-amino, aryl-[alkyle en C₁-C₆]amino ; cycloalkyle en C₃-C₆-amino, cycloalkyle en C₃-C₆-[alkyle en C₁-C₆]amino ; N-azétidinyle, N-pyrrolidinyle, N-pipéridinyle, N-morpholinyle,
et
X et Y représentent indépendamment l'un de l'autre O (oxygène) ou S (soufre).

3. Composés de formule générale (I) selon la revendication 1 et/ou leur sel, **caractérisés en ce que** R¹ représente un hydrogène, un fluor, un chlore, un brome, un iode, un groupe cyano, méthyle, éthyle, prop-1-yle, 1-méthyléthyle, but-1-yle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, trifluorométhyle, difluorométhyle, pentafluoroéthyle, 2,2-difluoroéthyle, 2,2,2-trifluoroéthyle, méthoxy, éthoxy, prop-1-yloxy, prop-2-yloxy, but-1-yloxy, but-2-yloxy, 2-méthylprop-1-yloxy, 1,1-diméthyléth-1-yloxy, difluorométhoxy, trifluorométhoxy, pentafluoroéthoxy, 2,2-difluoroéthoxy, 2,2,2-trifluoroéthoxy,
R² représente un hydrogène, un fluor, un chlore, un brome, un groupe trifluorométhyle, méthoxy, éthoxy, prop-1-yloxy, but-1-yloxy,
R³ représente un hydrogène, un fluor, un chlore, un brome, un groupe méthoxy, éthoxy, prop-1-yloxy, prop-2-yloxy, but-1-yloxy, but-2-yloxy, 2-méthylprop-1-yloxy, 1,1-diméthyléth-1-yloxy,
R⁴ représente un fluor, un chlore, un brome, un groupe cyano, NO₂, C(O)NH₂, C(S)NH₂, trifluorométhyle, difluorométhyle, pentafluoroéthyle, éthynyle, propyn-1-yle, 1-butin-1-yle, pentyn-1-yle, hexyn-1-yle, R⁵, R⁶ et R⁷ représentent indépendamment les uns des autres un hydrogène, un fluor, un chlore, un brome, un iode, un groupe cyano, méthyle, éthyle, prop-1-yle, 1-méthyléthyle, but-1-yle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-di-méthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, trifluorométhyle, difluorométhyle, pentafluoroéthyle, 2,2-difluoroéthyle, 2,2,2-trifluoroéthyle, méthoxy, éthoxy, prop-1-yloxy, prop-2-yloxy, but-1-yloxy, but-2-yloxy, 2-méthylprop-1-yloxy, 1,1-diméthyléth-1-yloxy, difluorométhoxy, trifluorométhoxy, Pentafluoroéthoxy, 2,2-difluoroéthoxy, 2,2,2-trifluoroéthoxy,
G représente un groupe méthylène, (méthyl)méthylène, (éthyl)méthylène, (prop-1-yl)méthylène, (prop-2-yl)méthylène, (but-1-yl)méthylène, (but-2-yl)méthylène, (pent-1-yl)méthylène, (pent-2-yl)méthylène, (pent-3-yl)méthylène, (diméthyl)méthylène, (diéthyl)méthylène, éthylène, n-propylène, (1-méthyl)éthyl-1-ène, (2-méthyl)éthyl-1-ène, n-butylène, 1-méthylpropyl-1-ène, 2-méthylpropyl-1-ène, 3-méthylpropyl-1-ène, 1,1-diméthyléthyl-1-ène, 2,2-diméthyléthyl-1-ène, 1-éthyléthyl-1-ène, 2-éthyléthyl-1-ène, 1-(prop-1-yl)éthyl-1-ène, 2-(prop-1-yl)éthyl-1-ène, 1-(prop-2-yl)éthyl-1-ène, 2-(prop-2-yl)éthyl-1-ène, 1,1,2-triméthyléthyl-1-ène, 1,2,2-triméthyléthyl-1-ène, 1,1,2,2-tétraméthyléthyl-1-ène, n-pentylène, 1-méthylbutyl-1-ène, 2-méthylbutyl-1-ène, 3-méthylbutyl-1-ène, 4-méthylbutyl-1-ène, 1,1-diméthylpropyl-1-ène, 2,2-diméthylpropyl-1-ène, 3,3-diméthylpropyl-1-ène, 1,2-diméthylpropyl-1-ène, 1,3-diméthylpropyl-1-ène, 1-éthylpropyl-1-ène, n-hexylène, 1-méthylpentyl-1-ène, 2-méthylpentyl-1-ène, 3-méthylpentyl-1-ène, 4-méthylpentyl-1-ène, 1,1-diméthylbutyl-1-ène, 1,2-diméthylbutyl-1-ène, 1,3-di-méthylbutyl-1-ène, 2,2-diméthylbutyl-1-ène, 2,3-diméthylbutyl-1-ène, 3,3-diméthylbutyl-1-ène, 1-éthylbutyl-1-ène, 2-éthylbutyl-1-ène, 1,1,2-triméthylpropyl-1-ène, 1,2,2-triméthylpropyl-1-ène, 1-éthyl-1-méthylpropyl-1-ène, 1-éthyl-2-méthylpropyl-1-ène,
X et Y représentent indépendamment l'un de l'autre O (oxygène) ou S (soufre),
et
Q représente l'un des groupements Q-1 à Q-406 spécifiquement mentionnés ci-après, la flèche, dans les formules développées du tableau ci-après, représentant une liaison du groupe correspondant Q au groupe carbonyle dans la formule générale (I) :
| | | | | |
|---|---|---|---|---|
| 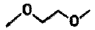 | 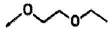 | 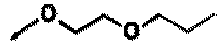 | 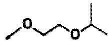 | 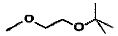 |
| Q-1 | Q-2 | Q-3 | Q-4 | Q-5 |
| | | | | |
|---|---|---|---|---|
| 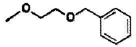 | 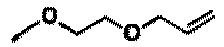 | 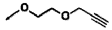 | 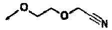 | 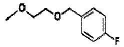 |
| Q-6 | Q-7 | Q-8 | Q-9 | Q-10 |
| | | | | |
|---|---|---|---|---|
| 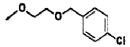 | 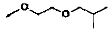 | 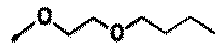 | 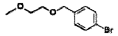 | 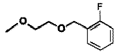 |
| Q-11 | Q-12 | Q-13 | Q-14 | Q-15 |
| | | | | |
|---|---|---|---|---|
| 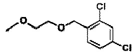 | 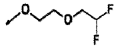 | 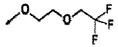 | 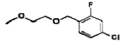 | 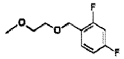 |
| Q-16 | Q-17 | Q-18 | Q-19 | Q-20 |
| | | | | |
|---|---|---|---|---|
| 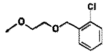 | 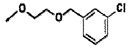 | 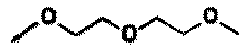 | 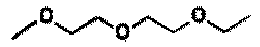 | 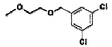 |
| Q-21 | Q-22 | Q-23 | Q-24 | Q-25 |
| | | | | |
|---|---|---|---|---|
| 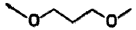 | 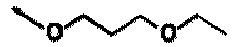 | 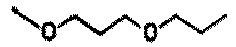 | 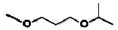 | 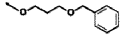 |
| Q-26 | Q-27 | Q-28 | Q-29 | Q-30 |
| | | | | |
|---|---|---|---|---|
| 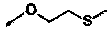 | 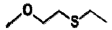 | 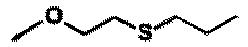 | 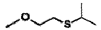 | 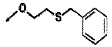 |
| Q-31 | Q-32 | Q-33 | Q-34 | Q-35 |
| | | | | |
|---|---|---|---|---|
| 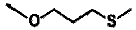 | 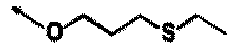 | 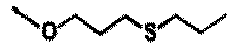 | 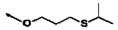 | 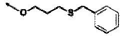 |
| Q-36 | Q-37 | Q-38 | Q-39 | Q-40 |
| | | | | |
|---|---|---|---|---|
| 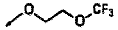 | 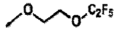 | 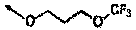 | 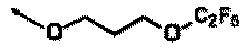 | 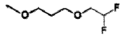 |
| Q-41 | Q-42 | Q-43 | Q-44 | Q-45 |
| | | | | |
|---|---|---|---|---|
| 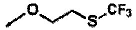 | 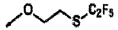 | 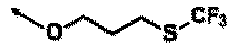 | 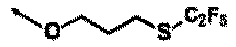 | 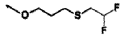 |
| Q-46 | Q-47 | Q-48 | Q-49 | Q-50 |
| | | | | |
|---|---|---|---|---|
| 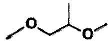 | 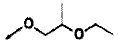 | 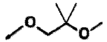 | 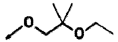 | 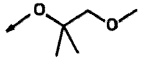 |
| Q-51 | Q-52 | Q-53 | Q-54 | Q-55 |
| | | | | |
|---|---|---|---|---|
| 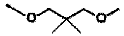 | 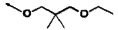 | 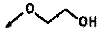 | 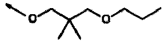 | 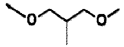 |
| Q-56 | Q-57 | Q-58 | Q-59 | Q-60 |
| | | | | |
|---|---|---|---|---|
| 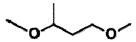 | 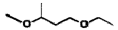 | 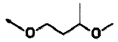 | 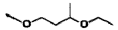 | 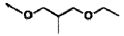 |
| Q-61 | Q-62 | Q-63 | Q-64 | Q-65 |
| | | | | |
|---|---|---|---|---|
| 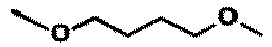 | 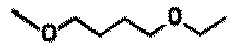 | 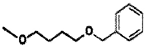 | 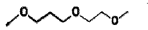 | 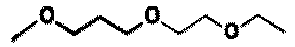 |
| Q-66 | Q-67 | Q-68 | Q-69 | Q-70 |
| | | | | |
|---|---|---|---|---|
| 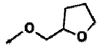 | 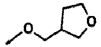 | 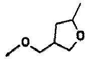 | 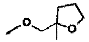 | 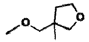 |
| Q-71 | Q-72 | Q-73 | Q-74 | Q-75 |
| | | | | |
|---|---|---|---|---|
| 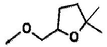 | 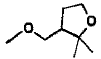 | 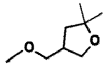 | 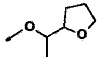 | 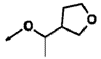 |
| Q-76 | Q-77 | Q-78 | Q-79 | Q-80 |
| | | | | |
|---|---|---|---|---|
| 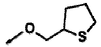 | 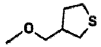 | 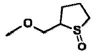 | 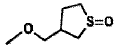 | 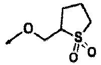 |
| Q-81 | Q-82 | Q-83 | Q-84 | Q-85 |
| | | | | |
|---|---|---|---|---|
| 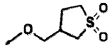 | 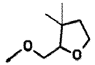 | 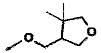 | 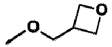 | 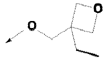 |
| Q-86 | Q-87 | Q-88 | Q-89 | Q-90 |
| | | | | |
|---|---|---|---|---|
| 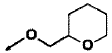 | 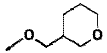 | 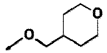 | 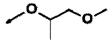 | 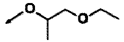 |
| Q-91 | Q-92 | Q-93 | Q-94 | Q-95 |
| | | | | |
|---|---|---|---|---|
| 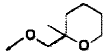 | 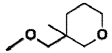 | 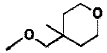 | 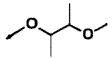 | 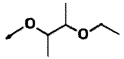 |
| Q-96 | Q-97 | Q-98 | Q-99 | Q-100 |
| | | | | |
|---|---|---|---|---|
| 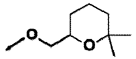 | 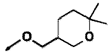 | 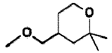 | 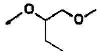 | 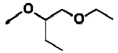 |
| Q-101 | Q-102 | Q-103 | Q-104 | Q-105 |
| | | | | |
|---|---|---|---|---|
| 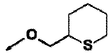 | 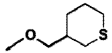 | 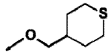 | 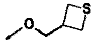 | 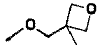 |
| Q-106 | Q-107 | Q-108 | Q-109 | Q-110 |
| | | | | |
|---|---|---|---|---|
| 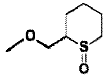 | 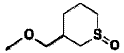 | 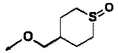 | 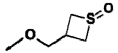 | 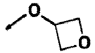 |
| Q-111 | Q-112 | Q-113 | Q-114 | Q-115 |
| | | | | |
|---|---|---|---|---|
| 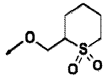 | 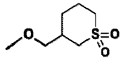 | 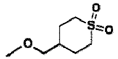 | 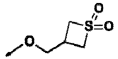 | 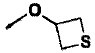 |
| Q-116 | Q-117 | Q-118 | Q-119 | Q-120 |
| | | | | |
|---|---|---|---|---|
| 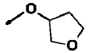 | 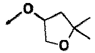 | 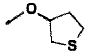 | 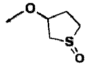 | 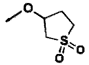 |
| Q-121 | Q-122 | Q-123 | Q-124 | Q-125 |
| | | | | |
|---|---|---|---|---|
| 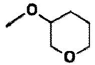 | 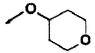 | 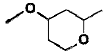 | 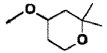 | 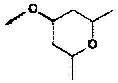 |
| Q-126 | Q-127 | Q-128 | Q-129 | Q-130 |
| | | | | |
|---|---|---|---|---|
| 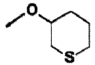 | 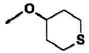 | 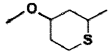 | 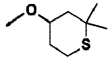 | 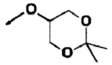 |
| Q-131 | Q-132 | Q-133 | Q-134 | Q-135 |
| | | | | |
|---|---|---|---|---|
| 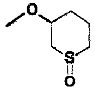 | 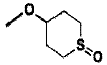 | 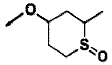 | 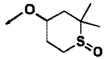 | 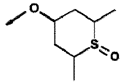 |
| Q-136 | Q-137 | Q-138 | Q-139 | Q-140 |
| | | | | |
|---|---|---|---|---|
| 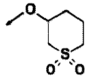 | 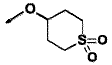 | 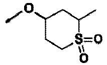 | 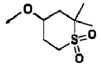 | 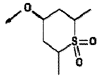 |
| Q-141 | Q-142 | Q-143 | Q-144 | Q-145 |
| | | | | |
|---|---|---|---|---|
| 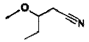 | 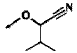 | 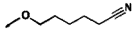 | 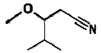 | 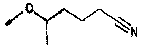 |
| 186 | 187 | 188 | 189 | 190 |
| | | | | |
|---|---|---|---|---|
| 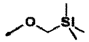 | 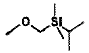 | 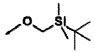 | 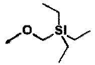 | 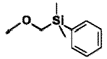 |
| Q-191 | Q-192 | Q-193 | Q-194 | Q-195 |
| | | | | |
|---|---|---|---|---|
| 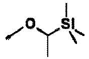 | 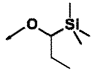 | 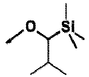 | 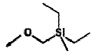 | 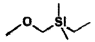 |
| Q-196 | Q-197 | Q-198 | Q-199 | Q-200 |
| | | | | |
|---|---|---|---|---|
| 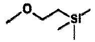 | 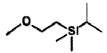 | 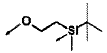 | 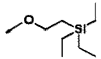 | 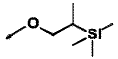 |
| Q-201 | Q-202 | Q-203 | Q-204 | Q-205 |
| | | | | |
|---|---|---|---|---|
| 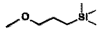 | 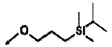 | 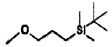 | 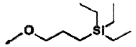 | 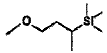 |
| Q-206 | Q-207 | Q-208 | Q-209 | Q-210 |
| | | | | |
|---|---|---|---|---|
| 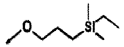 | 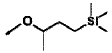 | 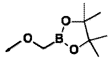 | 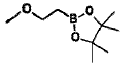 | 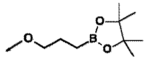 |
| Q-211 | Q-212 | Q-213 | Q-214 | Q-215 |
| | | | | |
|---|---|---|---|---|
| 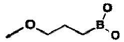 | 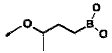 | 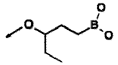 | 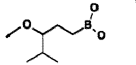 | 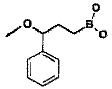 |
| Q-216 | Q-217 | Q-218 | Q-219 | Q-220 |
| | | | | |
|---|---|---|---|---|
| 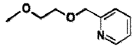 | 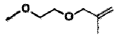 | 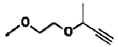 | 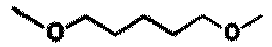 | 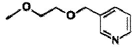 |
| Q-146 | Q-147 | Q-148 | Q-149 | Q-150 |
| | | | | |
|---|---|---|---|---|
| 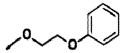 | 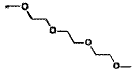 | 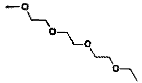 | 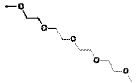 | 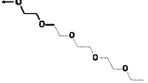 |
| Q-151 | Q-152 | Q-153 | Q-154 | Q-155 |
| | | | | |
|---|---|---|---|---|
| 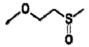 | 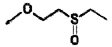 | 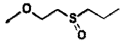 | 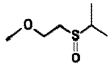 | 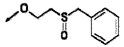 |
| Q-156 | Q-157 | Q-158 | Q-159 | Q-160 |
| | | | | |
|---|---|---|---|---|
| 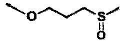 | 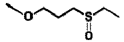 | 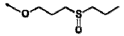 | 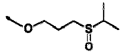 | 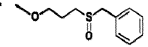 |
| Q-161 | Q-162 | Q-163 | Q-164 | Q-165 |
| | | | | |
|---|---|---|---|---|
| 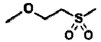 | 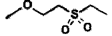 | 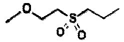 | 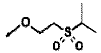 | 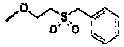 |
| Q-166 | Q-167 | Q-168 | Q-169 | Q-170 |
| | | | | |
|---|---|---|---|---|
| 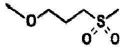 | 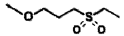 | 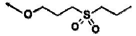 | 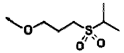 | 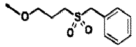 |
| Q-171 | Q-172 | Q-173 | Q-174 | Q-175 |
| | | | | |
|---|---|---|---|---|
| 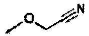 | 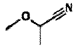 | 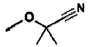 | 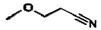 | 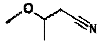 |
| Q-176 | Q-177 | Q-178 | Q-179 | Q-180 |
| | | | | |
|---|---|---|---|---|
| 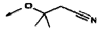 | 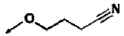 | 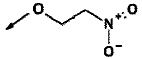 | 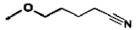 | 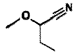 |
| Q-181 | Q-182 | Q-183 | Q-184 | Q-185 |
| | | | | |
|---|---|---|---|---|
| 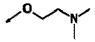 | 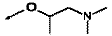 | 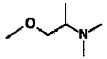 | 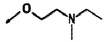 | 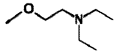 |
| Q-221 | Q-222 | Q-223 | Q-224 | Q-225 |
| | | | | |
|---|---|---|---|---|
| 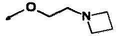 | 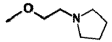 | 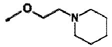 | 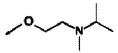 | 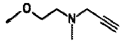 |
| Q-226 | Q-227 | Q-228 | Q-229 | Q-230 |
| | | | | |
|---|---|---|---|---|
| 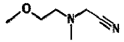 | 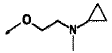 | 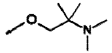 | 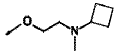 | 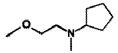 |
| Q-231 | Q-232 | Q-233 | Q-234 | Q-235 |
| | | | | |
|---|---|---|---|---|
| 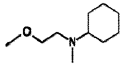 | 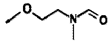 | 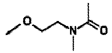 | 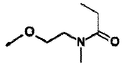 | 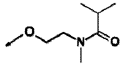 |
| Q-236 | Q-237 | Q-238 | Q-239 | Q-240 |
| | | | | |
|---|---|---|---|---|
| 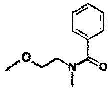 | 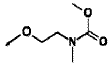 | 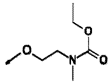 | 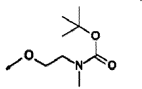 | 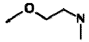 |
| Q-241 | Q-242 | Q-243 | Q-244 | Q-245 |
| | | | | |
|---|---|---|---|---|
| 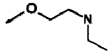 | 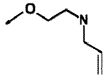 | 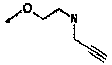 | 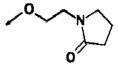 | 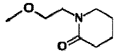 |
| Q-246 | Q-247 | Q-248 | Q-249 | Q-250 |
| | | | | |
|---|---|---|---|---|
| 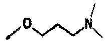 | 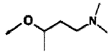 | 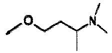 | 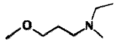 | 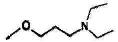 |
| Q-251 | Q-252 | Q-253 | Q-254 | Q-255 |
| | | | | |
|---|---|---|---|---|
| 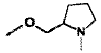 | 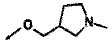 | 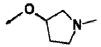 | 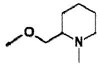 | 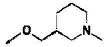 |
| Q-256 | Q-257 | Q-258 | Q-259 | Q-260 |
| | | | | |
|---|---|---|---|---|
| 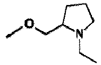 | 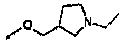 | 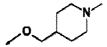 | 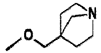 | 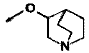 |
| Q-261 | Q-262 | Q-263 | Q-264 | Q-265 |
| | | | | |
|---|---|---|---|---|
| 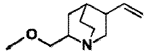 | 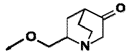 | 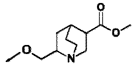 | 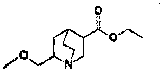 | 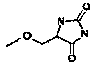 |
| Q-266 | Q-267 | Q-268 | Q-269 | Q-270 |
| | | | | |
|---|---|---|---|---|
| 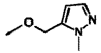 | 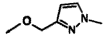 | 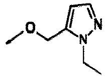 | 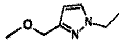 | 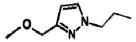 |
| Q-271 | Q-272 | Q-273 | Q-274 | Q-275 |
| | | | | |
|---|---|---|---|---|
| 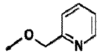 | 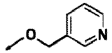 | 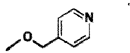 | 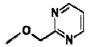 | 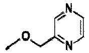 |
| Q-276 | Q-277 | Q-278 | Q-279 | Q-280 |
| | | | | |
|---|---|---|---|---|
| 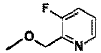 | 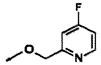 | 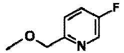 | 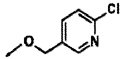 | 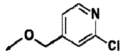 |
| Q-281 | Q-282 | Q-283 | Q-284 | Q-285 |
| | | | | |
|---|---|---|---|---|
| 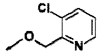 | 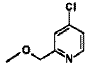 | 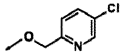 | 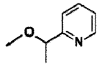 | 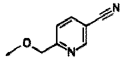 |
| Q-286 | Q-287 | Q-288 | Q-289 | Q-290 |
| | | | | |
|---|---|---|---|---|
| 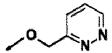 | 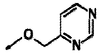 | 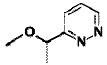 | 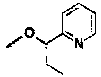 | 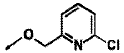 |
| Q-291 | Q-292 | Q-293 | Q-294 | Q-295 |
| | | | | |
|---|---|---|---|---|
| 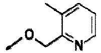 | 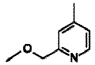 | 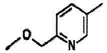 | 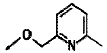 | 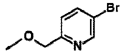 |
| Q-296 | Q-297 | Q-298 | Q-299 | Q-300 |
| | | | | |
|---|---|---|---|---|
| 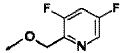 | 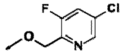 | 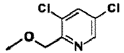 | 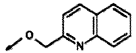 | 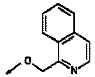 |
| Q-301 | Q-302 | Q-303 | Q-304 | Q-305 |
| | | | | |
|---|---|---|---|---|
| 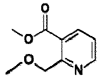 | 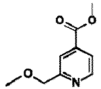 | 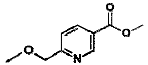 | 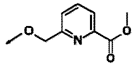 | 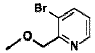 |
| Q-306 | Q-307 | Q-308 | Q-309 | Q-310 |
| | | | | |
|---|---|---|---|---|
| 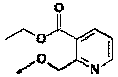 | 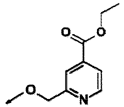 | 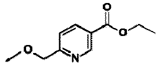 | 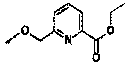 | 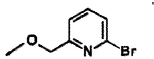 |
| Q-311 | Q-312 | Q-313 | Q-314 | Q-315 |
| | | | | |
|---|---|---|---|---|
| 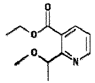 | 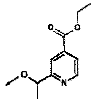 | 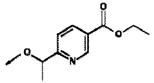 | 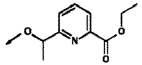 | 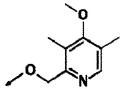 |
| Q-316 | Q-317 | Q-318 | Q-319 | Q-320 |
| | | | | |
|---|---|---|---|---|
| 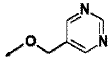 | 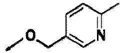 | 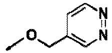 | 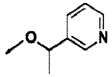 | 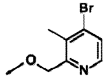 |
| Q-321 | Q-322 | Q-323 | Q-324 | Q-325 |
| | | | | |
|---|---|---|---|---|
| 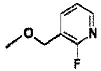 | 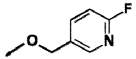 | 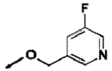 | 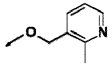 | 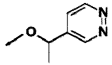 |
| Q-326 | Q-327 | Q-328 | Q-329 | Q-330 |
| | | | | |
|---|---|---|---|---|
| 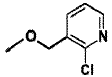 | 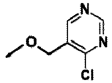 | 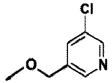 | 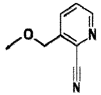 | 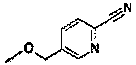 |
| Q-331 | Q-332 | Q-333 | Q-334 | Q-335 |
| | | | | |
|---|---|---|---|---|
| 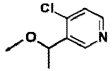 | 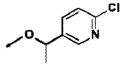 | 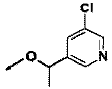 | 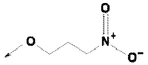 | 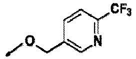 |
| Q-336 | Q-337 | Q-338 | Q-339 | Q-340 |
| | | | | |
|---|---|---|---|---|
| 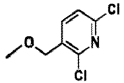 | 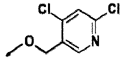 | 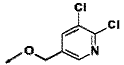 | 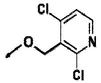 | 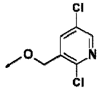 |
| Q-341 | Q-342 | Q-343 | Q-344 | Q-345 |
| | | | | |
|---|---|---|---|---|
| 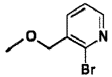 | 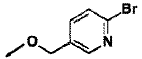 | 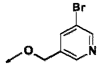 | 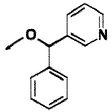 | 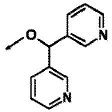 |
| Q-346 | Q-347 | Q-348 | Q-349 | Q-350 |
| | | | | |
|---|---|---|---|---|
| 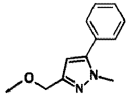 | 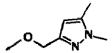 | 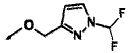 | 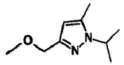 | 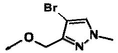 |
| Q-351 | Q-352 | Q-353 | Q-354 | Q-355 |
| | | | | |
|---|---|---|---|---|
| 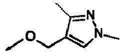 | 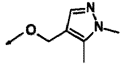 | 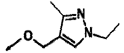 | 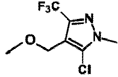 | 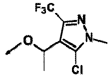 |
| Q-356 | Q-357 | Q-358 | Q-359 | Q-360 |
| | | | | |
|---|---|---|---|---|
| 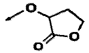 | 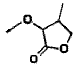 | 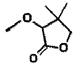 | 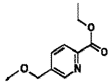 | 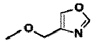 |
| Q-361 | Q-362 | Q-363 | Q-364 | Q-365 |
| | | | | |
|---|---|---|---|---|
| 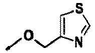 | 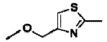 | 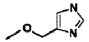 | 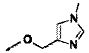 | 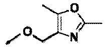 |
| Q-366 | Q-367 | Q-368 | Q-369 | Q-370 |
| | | | | |
|---|---|---|---|---|
| 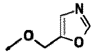 | 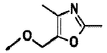 | 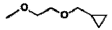 | 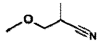 | 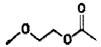 |
| Q-371 | Q-372 | Q-373 | Q-374 | Q-375 |
| | | | | |
|---|---|---|---|---|
| 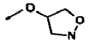 | 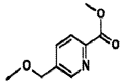 | 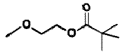 | 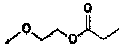 | 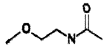 |
| Q-376 | Q-377 | Q-378 | Q-379 | Q-380 |
| | | | | |
|---|---|---|---|---|
| 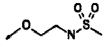 | 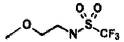 | 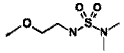 | 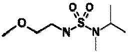 | 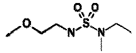 |
| Q-381 | Q-382 | Q-383 | Q-384 | Q-385 |
| | | | | |
|---|---|---|---|---|
| 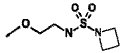 | 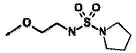 | 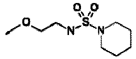 | 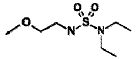 | 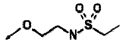 |
| Q-386 | Q-387 | Q-388 | Q-389 | Q-390 |
| | | | | |
|---|---|---|---|---|
| 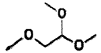 | 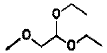 | 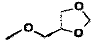 | 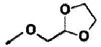 | 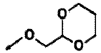 |
| Q-391 | Q-392 | Q-393 | Q-394 | Q-395 |
| | | | | |
|---|---|---|---|---|
| 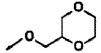 | 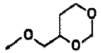 | 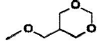 | 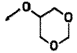 | 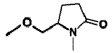 |
| Q-396 | Q-397 | Q-398 | Q-399 | Q-400 |
| | | | | |
|---|---|---|---|---|
| 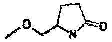 | 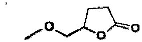 | 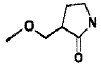 | 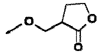 | 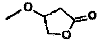 |
| Q-401 | Q-402 | Q-403 | Q-404 | Q-405 |
| |
|---|
| 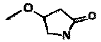 |
| Q-406 |

4. Composés de formule générale (I) selon la revendication 1 et/ou leur sel, **caractérisés en ce que** R¹ représente un hydrogène, un fluor, un chlore, un brome, un iode, un groupe cyano, méthyle, éthyle, trifluorométhyle, difluorométhyle, méthoxy, éthoxy, difluorométhoxy, trifluorométhoxy,
R² représente un fluor,y
R³ représente un hydrogène, un fluor, un chlore, un brome, un méthoxy,
R⁴ représente un fluor, un chlore, un brome, un groupe cyano, NO₂, C(O)NH₂, C(S)NH₂, trifluorométhyle, éthynyle, propyn-1-yle,
R⁵, R⁶ et R⁷ représentent indépendamment les uns des autres un hydrogène, un fluor, un chlore, un brome, un iode, un groupe cyano, méthyle, éthyle, trifluorométhyle, difluorométhyle, méthoxy, éthoxy, difluorométhoxy, trifluorométhoxy,
G représente un groupe méthylène, (méthyl)méthylène, (éthyl)méthylène, (diméthyl)méthylène, éthylène, n-propylène, (1-méthyl)éthyl-1-ène, (2-méthyl)éthyl-1-ène, n-butylène, 1-méthylpropyl-1-ène, 2-méthylpropyl-1-ène, 3-méthylpropyl-1-ène, 1,1-diméthyléthyl-1-ène, 2,2-diméthyléthyl-1-ène, 1-éthyléthyl-1-ène, 2-éthyléthyl-1-ène, 1-(prop-1-yl)éthyl-1-ène, 2-(prop-1-yl)éthyl-1-ène, 1-(prop-2-yl)éthyl-1-ène, 2-(prop-2-yl)éthyl-1-ène,
n-pentylène, 1-méthylbutyl-1-ène, 2-méthylbutyl-1-ène, 3-méthylbutyl-1-ène, 4-méthylbutyl-1-ène, 1,1-diméthylpropyl-1-ène, 2,2-diméthylpropyl-1-ène, 3,3-diméthylpropyl-1-ène, 1,2-diméthylpropyl-1-ène, 1,3-diméthylpropyl-1-ène, 1-éthylpropyl-1-ène, n-hexylène,
X et Y représentent indépendamment l'un de l'autre O (oxygène) ou S (soufre),
et
Q représente l'un des groupements Q-1 à Q-406 spécifiquement mentionnés dans la revendication 3.

5. Composés de formule générale (I) selon la revendication 1, et/ou leur sel, **caractérisés en ce que** R¹ représente un hydrogène, un fluor, un chlore, un brome, un groupe cyano, méthyle, trifluorométhyle, méthoxy, trifluorométhoxy,
R² représente un fluor,
R³ représente un fluor,
R⁴ représente un chlore, un brome, un groupe cyano, NO₂, C(O)NH₂, C(S)NH₂,
R⁵, R⁶ et R⁷ représentent indépendamment les uns des autres un hydrogène, un fluor, un chlore, un brome, un groupe cyano, méthyle, trifluorométhyle, méthoxy, trifluorométhoxy,
G représente un groupe méthylène, (méthyl)méthylène, (éthyl)méthylène, (diméthyl)méthylène, éthylène, n-propylène, (1-méthyl)éthyl-1-ène, (2-méthyl)éthyl-1-ène, n-butylène, 1-méthylpropyl-1-ène, 2-méthylpropyl-1-ène, 3-méthylpropyl-1-ène, n-pentylène, n-hexylène,
X et Y représentent indépendamment l'un de l'autre O (oxygène) ou S (soufre)
et
Q représente l'un des groupements Q-1 à Q-406 spécifiquement mentionnés dans la revendication 3.

6. Composés de formule générale (I) selon la revendication 1 et/ou leur sel, **caractérisés en ce que**
R¹ représente un hydrogène,
R² représente un fluor,
R³ représente un fluor,
R⁴ représente un chlore, un brome, un cyano, NO₂,
R⁵ représente un hydrogène, un fluor, un chlore, un brome,
R⁶ représente un hydrogène, un fluor, un chlore, un brome, un cyano,
R⁷ représente un hydrogène,
G représente un groupe méthylène, (méthyl)méthylène,
X représente O (oxygène) ou S (soufre),
Y représente O (oxygène)
et
Q représente l'un des groupements Q-1 à Q-406 spécifiquement mentionnés dans la revendication 3.

7. Composés de formule générale (I) selon la revendication 1 et/ou leur sel, **caractérisés en ce que**
R¹ représente un hydrogène,
R² représente un fluor,
R³ représente un fluor,
R⁴ représente un chlore, un brome, un cyano, NO₂,
R⁵ représente un hydrogène, un fluor,
R⁶ représente un hydrogène, un fluor, un brome, un cyano,
R⁷ représente un hydrogène,
G représente un groupe méthylène, (méthyl)méthylène,
X représente O (oxygène) ou S (soufre),
Y représente O (oxygène)
et
Q représente l'un des groupements spécifiquement mentionnés dans la revendication 3 Q-1 à Q-35, Q-41 à Q-45, Q-58, Q-71 à Q-80, Q-89, Q-94, Q-95, Q-115, Q-120 à Q-123, Q-152 à Q-155, Q-166 à Q-170, Q-176 à Q-190, Q-261 à Q-348, Q-352 à Q-372, Q-377, Q-391-Q-399.

8. Composés de formule générale (I) selon la revendication 1 et/ou leur sel, **caractérisés en ce que**
R¹ représente un hydrogène,
R² représente un fluor,
R³ représente un fluor,
R⁴ représente un chlore, un brome, un cyano, NO₂,
R⁵ représente un hydrogène, un fluor,
R⁶ représente un hydrogène, un fluor, un brome, un cyano,
R⁷ représente un hydrogène,
G représente le groupe méthylène, (méthyl)méthylène,
X représente O (oxygène) ou S (soufre),
Y représente O (oxygène)
et
Q représente l'un des groupements spécifiquement mentionnés dans la revendication 3 Q-1, Q-2, Q-3, Q-4, Q-7, Q-8, Q-9, Q-17, Q-18, Q-23, Q-24, Q-26, Q-27, Q-41, Q-42, Q-43, Q-58, Q-71, Q-72, Q-89, Q-94, Q-115, Q-121, Q-176, Q-177, Q-179, Q-183, Q-272, Q-274, Q-275, Q-276, Q-277, Q-278, Q-281, Q-282, Q-283, Q-284, Q-286, Q-288, Q-291, Q-296, Q-301, Q-302, Q-303, Q-308, Q-309, Q-321, Q-327, Q-328, Q-329, Q-331, Q-335, Q-339, Q-356, Q-365, Q-366, Q-367, Q-371, Q-394.

9. Utilisation d'un ou plusieurs composés de formule générale (I) tels que définis dans l'une des revendications 1 à 8 et/ou de leurs sels en tant qu'herbicide et/ou régulateur de croissance des plantes, de préférence dans des cultures de plantes utiles et/ou ornementales.

10. Produit herbicide et/ou régulateur de croissance des plantes, **caractérisé en ce que** le produit contient un ou plusieurs composés de formule générale (I) tels que définis dans l'une des revendications 1 à 8 et/ou leurs sels, ainsi qu'en outre une ou plusieurs substances choisies dans les groupes (i) et/ou (ii), comportant
(i) une ou plusieurs autres substances à activité agrochimique, de préférence choisies dans le groupe consistant en les insecticides, les acaricides, les nématicides, d'autres herbicides, les fongicides, les phytoprotecteurs, les engrais et/ou d'autres régulateurs de croissance,
(ii) un ou plusieurs autres adjuvants de formulation usuels en protection des plantes.

11. Procédé de lutte contre les plantes nuisibles ou de régulation de la croissance de plantes, **caractérisé en ce qu'**on applique une quantité active
- d'un ou plusieurs composés de formule générale (I), tels que définis dans l'une des revendications 1 à 8 et/ou de leurs sels, ou
- d'un produit selon la revendication 10, sur les plantes, les semences de plantes, le sol dans lequel ou sur lequel poussent les plantes, ou sur la surface cultivée.
